# EUROPEAN PATENT SPECIFICATION

(11) **EP 1 708 694 B1**
(45) Date of publication and mention of the grant of the patent: **11.07.2012**
(21) Application number: 05722794.4
(22) Date of filing: 31.01.2005
(51) Int. Cl.: A61K 31/335

(54) **COMPOSITIONS AND METHODS FOR TREATING CONTRACTURE**
ZUSAMMENSETZUNGEN UND VERFAHREN ZUR BEHANDLUNG VON KONTRAKTUR
COMPOSITIONS ET PROCEDES POUR LE TRAITEMENT DE LA CONTRACTURE

(30) Priority: 30.01.2004 US 540660 P
(43) Date of publication of application: 11.10.2006
(73) Proprietor: ANGIOTECH INTERNATIONAL AG, 6304 Zug (CH)
(72) Inventor: AVELAR, Rui, Vancouver, British Columbia V6J 2W7 (CA); LIGGINS, Richard, T., Coquitlam, British Columbia V3K 5K7 (CA); TOLEIKIS, Philip, M., Vancouver, British Columbia V6P 5N8 (CA); LOSS, Troy, A., E., North Vancouver, British Columbia V7L 3G (CA); GRAVETT, David, M., Vancouver, British Columbia V5Z 1Y8 (CA); MAITI, Arpita, Vancouver, British Columbia V5Z 4A6 (CA)
(74) Representative: Gowshall, Jonathan Vallance
(86) International application number: PCT/US2005/003800
(87) International publication number: WO 2005/074913

(56) References cited:
- EP-A- 1 508 331
- WO-A-98/24427
- WO-A-98/24427
- US-A1- 2002 183 380
- US-A1- 2002 183 380
- US-A1- 2004 001 872

## Description

### Technical Field

The present invention relates generally to pharmaceutical compositions and methods for preventing conditions associated with reduced mobility or loss of function and articulation.

### Background of the Invention

The normal function of a joint and its movement can be severely impaired by scar and abnormal tissue formation that takes place both inside and outside the joint. The result is reduced mobility of a joint or extra-articular structure such as a muscle, tendon, or ligament. Reduced mobility can involve permanently shortened distances between tissues or a reduced maximum possible lengthening or shortening of tissues. When the impaired mobility results from one of these conditions, it is generally referred to as a contracture. The term "contracture" is often used interchangeably with the terms such as "stiff joint" or arthrofibrosis.

Contractures can be associated with or caused by a variety of conditions, for example, metabolic disorders, ischemia, burns, injury (*e.g*., to joint, capsule, bone, cartilage, tendon, ligament or muscle), fractures, subluxation, dislocations, crush injuries, prolonged immobilization (*e.g*., immobilization of a joint in a cast or splint), and paralysis. Surgical procedures may also precipitate contractures, as in the case of operations involving the shoulder (*e.g*., rotator cuff repair or diagnostic inspection), elbow, and hand. Other procedures involving joint reduction after a dislocation, or repairs of tendon, ligament, capsule and bone may also induce joint contractures. Procedures to remove scar and abnormal tissue in contracted joints often fail because the surgery itself represents a controlled injury. Thus, the process of removing abnormal and scarred tissue further stimulates the formation of scarred and abnormal tissue. As a result, the procedures offered today have limited success and at times, can actually make a patient worse.

Certain joints and procedures have higher tendencies for contractures. For example, a hip or knee arthroplasty generally has a low rate of joint stiffness after a procedure, but a shoulder has a significantly higher rate. An anterior cruciate repair has an incidence of arthrofibrosis ranging from 3% to 15% depending on the surgeon and repair performed (*e.g*., semi-tendinous/gracilis or bone-patellar tendon bone repair). Joints such as the elbow have a high tendency and can form some degree of contracture in 30% to 70% of patients. Shoulders may form contractures not only in response to trauma, but can also form spontaneously, for example, a frozen shoulder with a capsule that has thickened without any obvious precipitant.

In certain cases, the contracture may have a hereditary basis and have the primary scar and abnormal tissue growth take place outside of the joint. Dupytren's contracture represents a condition whereby the connective tissue in the palmer aspect of the hand begins to scar and thicken leading to deformation of the hand at the site of the thickening and loss of range of motion of the fingers. Equivalent scenarios exist in the penis (Peyronie's disease), and on the plantar aspect of the foot (Ledderhose's disease).

Treatment for contractures today only addresses the issue after a contracture is already established. Interventions including only physiotherapy and range of motion exercises are used but have very limited success. Surgical interventions include manipulation under anesthesia (*i.e*., essentially putting the patient to sleep and then breaking down the adhesion by forcing the joint). Unfortunately, this often reignites the inflammation and proliferation in the tissue and the reformation of the scar and stiffness. Surgery may involve an open procedure, releasing and removing the restricting scar and abnormal tissue or the operation can also be done through an arthroscope, whereby the scar and restricting tissue is released and removed using special tools. Surgical interventions often fail, and may actually make the condition worse, since the surgery itself is a controlled injury or trauma, which can cause the tissue to lay down even more scar in response to the surgical injury.

Pharmacological therapy has been attempted with limited or no success. Agents most often used include non-steriodal anti-inflammatories, steroids and radiation. Pharmacological treatments for various types of contracture have included administration of hyaluronic acid (*i.e*., HEALON-R, Pharmacia Inc., Piscataway, NJ) into joints *(*Clin. Rheumatol. 20: 98-103, 2001; Acta Orthop. Scand. 62: 323-6, 1991); oral administration of antihistamines to rabbits *(*J. Hand Surg. 18: 1080-5, 1993); and intra-articular injection of dimethlysulfoxide, systemic steroids, and non-steroidal anti-inflammatories. Recombinant human superoxide dismutase (U.S. 6,312,720), calmodulin blockertrifluroperizine (U.S. 6,525,100), collagenase and calcium channel blockers have been disclosed as therapy for patients suffering from Peyronie's disease; matrix metalloproteinase inhibitors have been disclosed for inhibiting contraction (see, *e.g*., U.S. Patent No. 6,093,398); and use of dimethylsulfoxide, oxygen free radical scavengers, including colchicine, allopurinol, and methylhydrazine, interferon, collagenases, steroids, such as triamcinolone and clobetasol (Hand Clinics 15: 97-107, 1999), verapamil, nifedipine, diltiazem, amalodipine, felodipine, isradipine, nicardipine, nimodipine, nisoldipine, bepridil (see, *e.g*., U.S. Patent Nos. 6,353,028 and 6,031,005) and fluroquinolone (U.S. Patent No. 6,060,474) have been injected locally into fibrous tissue in an attempt to treat Dupuytren's contracture. To date, however, none of the pharmacological treatments described above have been approved for treating contracture in human patients.

### SUMMARY OF THE INVENTION

The present invention provides the claimed compositions, for use in devices and methods for the treatment of contracture, and in particular, for use in human and animal patients. The compositions described herein may be used after an injury in order to prevent or minimize contracture formation. In the case of established contracture, the compositions of the invention can be used to complement a release procedure (*e.g*., forced manipulation, open release, arthroscopic release, or debulking of scar) to prevent the recurrence of scarred and abnormal tissue which can lead to a contracture. The administration may be intra-articular in cases where the contracture is caused by an intra-articular scar, or may used peri-articularly where the contracture is caused by not only scarring within the joints, but also by scar tissue outside the joint. An example of the latter would include interphalangeal contractures, not only is the scar within the joint, the outside volar plate is also involved. The use or administration of the instant compositions provides for an efficacious treatment which is reasonably safe and well tolerated and may further provide other related advantages. The drug contained in the compositions of the invention may be selected from a variety of therapeutically active compounds which will provide symptomatic, disease modifying or prophylaxis effect in conditions associated with contracture. The method of use of such compositions may also vary, but includes all routes of administration, doses, and dosing frequencies which will provide such a benefit.

In one aspect, a method for treating contracture is provided that includes administering to a patient in need thereof a therapeutically effective amount of a composition comprising a therapeutic agent effective in treating contracture. The contracture may affect a joint, such as an elbow, a shoulder, a knee, an ankle, a hip, a finger joint, a wrist, a toe joint, a temporomandibular joint, a facet joint, an otic bone joint, or a combination thereof, or soft tissue, such as muscles, tendons, ligaments, fat, joint capsule, synovium, or other connective tissue (*e.g*., fascia), or a combination thereof. The contracture may be induced by a genetic predisposition such as in the case of a Dupuytren's contracture, a Peyronie's contracture, a Ledderhose's contracture, or ischemia, such as in the case of a Volkmann's contracture. In another aspect, the contracture is due to inflammation, degeneration, injury, infection, hypertrophy, a neurological condition, a metabolic condition, infection, ischemia, idiopathic, or a combination thereof.

In certain aspects, the contracture is due to injury, such as a trauma (*e.g*., burns, crushes, cuts, tears, disruptions, impacts, and tractions). In another aspect, the contracture is due to a fracture (which may occur in or around a joint, such as an elbow or hip), a subluxation, a dislocation (*e.g*., in the ankle, knee, shoulder, finger or elbow), or a joint (*e.g*., shoulder, elbow, hip, temporomandibular joint, facet, finger, knee, ankle, or toe) disruption or there may be no identifiable cause (*e.g*., frozen shoulder). The injury may be due to a surgical procedure, such as an open surgical procedure or a minimally invasive procedure, such as, *e.g*., an arthroscopic, or an endoscopic procedure.

In certain embodiments, the contracture affects soft tissue such as muscles, tendons, ligaments, fat, synovium, capsule, fascia, connective tissue, or a combination thereof.

In another aspect, the contracture is due to hypertrophy. The hypertrophy may affect a canal, such as a carpel, tarsal, or cubital tunnel.

In yet another aspect, the contracture is due to a neurological condition, such as paralysis or stroke.

In yet another aspect, the contracture is due to metabolic condition, such as diabetes, haemophilia, gout, or pseudo gout.

The composition includes at least one drug efficacious in treating contracture. Optionally, the composition may contain more than one drug from the same or a different drug class. The selected drug may be a cell cycle inhibitor, such as an anti-microtubule agent, an antimetabolite, an alkylating agent, a vinca alkaloid, a camptothecin, mitoxantrone, etoposide, doxorubicin, methotrexate, 5-fluorouracil, peloruside A, mitomycin C, or an analog thereof, or a CDK-2 inhibitor. In one aspect, the therapeutic agent is an anti-microtubule agent. In one aspect, the anti-microtubule agent is a taxane, such as paclitaxel or an analogue or derivative thereof. In certain embodiments, the taxane is paclitaxel.

In certain embodiments, the selected drug effective in treating contracture is a phosphodiesterase III inhibitor (*e.g*., milrinone, olprinone, or a derivative or analogue thereof.

In certain other embodiments, the therapeutic agent is a bisphosphonate (*e.g*., clodronate, alendronate, pamidronate, zoledronate, etidronate, and analogues and derivatives thereof).

In certain embodiments, the therapeutic agent is a macrolide antibiotic (*e.g*., rapamycin, everolimus, azathioprine, tacrolimus, azithromycin, and analogues and derivatives thereof).

In certain embodiments, the therapeutic agent is a phosphodiesterase IV inhibitor (*e.g*., rolipram, cilomilast, or an analogue or derivative thereof).

In certain embodiments, the therapeutic agent is a p38 MAP kinase inhibitor (*e.g*., BIRB-798, SB220025, RO-320-1195, RWJ-67657, RWJ-68354, SCIO-469, and analogues and derivatives thereof).

In certain embodiments, the therapeutic agent is an ICE inhibitor (*e.g*., an (aryl)acyloxymethyl ketone).

In certain embodiments, the therapeutic agent is a phenothiazine, such as chlorpromazine.

In certain embodiments, the therapeutic agent is a cytokine modulator, chemokine modulator (*e.g*., TNF alpha, IL-1, and IL-6), MCP-1 modulator, IL-8 modulator, TGF beta modulator, or an analogue or derivative thereof.

In certain embodiments, the therapeutic agent is selected from the group consisting of diacerein, doxycycline, and leflunamide.

In certain embodiments, the therapeutic agent is a NFκB inhibitor (*e.g*., Bay 11-7082 or Bay 11-7085, or an analogue or derivative thereof).

In certain embodiments, the therapeutic agent is an inosine monophosphate dehydrogenase (IMPDH) inhibitor (*e.g*., mycophenolic acid, mycophenolic mofetil, ribavarin, aminothiadiazole, thiophenfurin, viramidine, merimepodib, tiazofurin, and analogues and derivatives thereof).

In certain embodiments, the therapeutic agent is an antioxidant selected from the group consisting of Na ascorbate, alpha-tocopherol, and analogues and derivatives thereof.

In certain embodiments, the therapeutic agent is an angiogenesis inhibitor selected from the group consisting of angiostatic steroids (*e.g*., squaline), cartilage derived proteins and factors, thrombospondin, matrix metalloproteinases (*e.g*., collagenases, gelatinases A and B, stromelysins 1, 2 and 3, martilysin, metalloelastase, MT1-MMP, MT2-MMP, MT3-MMP, MT4-MMP, Bay 12-9566, AG-3340, CGS270231, D5140, D1927, and D2163), and phytochemicals (*e.g*., genistein, daidzein, leuteolin, apigenin, 3 hydroxyflavone, 2',3'-dihydroxyflavone, 3',4'-dihydroxyflavone, and fisetin) and analogues and derivatives thereof.

In certain embodiments, the therapeutic agent may be a cGMP stimulant, a vitronectin antagonist, a 5-lipoxygenase inhibitor, a chemokine receptor antagonist, a cyclin dependent protein kinase inhibitor, an epidermal growth factor (EGF) receptor kinase inhibitor, an elastase inhibitor, a factor Xa inhibitor, a farnesyltransferase inhibitor, a fibrinogen antagonist, a guanylate cyclase stimulant, a heat shock protein 90 antagonist, an HMGCoA reductase inhibitor, a hydroorotate dehydrogenase inhibitor, an IKK2 inhibitor, an IRAK antagonist, an IL-4 agonist, an immunomodulatory agent, a leukotriene inhibitor, a NO antagonist, a thromboxane A2 antagonist, a TNFα antagonist, a TACE Inhibitor, a tyrosine kinase inhibitor, a fibroblast growth factor inhibitor, a protein kinase inhibitor, a PDGF receptor kinase inhibitor, an endothelial growth factor receptor kinase inhibitor, a retinoic acid receptor antagonist, a platelet derived growth factor receptor kinase inhibitor, a fibronogin antagonist, an antimycotic agent, a phospholipase A1 inhibitor, a histamine H1/H2/H3 receptor antagonist, a GPIIb/IIIa receptor antagonist, an endothelin receptor antagonist, a peroxisome proliferator-activated receptor agonist, an estrogen receptor agent, a somatostatin analogue, a neurokinin antagonist, a neurokinin 3 antagonist, a neurokinin antagonist, a VLA-4 antagonist, an osteoclast inhibitor, a DNA topoisomerase ATP hydrolysing inhibitor, an angiotensin I converting enzyme inhibitor, an angiotensin II antagonist, an enkephalinase inhibitor, a peroxisome proliferator-activated receptor gamma agonist insulin sensitizer, a protein kinase C inhibitor, a rho-associated kinase (ROCK) inhibitor, a CXCR3 inhibitor, a Itk Inhibitor, a cytosolic phospholipase A₂-alpha Inhibitors, a PPAR agonist, an immunosuppressant, an Erb inhibitor, an apoptosis agonist, a lipocortin agonist, a VCAM-1 antagonist, a collagen antagonist, an alpha 2 integrin antagonist, a nitric oxide inhibitor, a cathepsin inhibitor, a Jun kinase inhibitors, a COX-2 inhibitor, a non-steroidal anti-inflammatory agent, a caspase inhibitor, an IGF-1 agonist, or a bFGF agonist.

In certain embodiments, the therapeutic agent may be selected from the following compounds: antimicrotubule agents including taxanes (*e.g*., paclitaxel and docetaxel), other microtubule stabilizing agents and vinca alkaloids (*e.g*., vinblastine and vincristine sulfate), haloguginone and its salt forms (halofuginone bromide), mycophenolic acid, mithramycin, puromycin, nogalamycin, 17-DMAG, nystatin, rapamycin, mitoxantrone, duanorubicin, gemcitabine, camptothecin, epothilone B, simvastatin, anisomycin, mitomycin C, epirubicin hydrochloride, topotecan, fascaplysin, podophyllotoxin, and chromomycin A3.

In certain embodiments, the composition comprises between about 0.01 mg/ml to about 100 mg/ml of a therapeutic agent. In certain embodiment, the composition comprises between about 0.1 mg/ml to about 10 mg/ml of a therapeutic agent.

The therapeutic agent may be administered by intraarticular, periarticular, peritendinal or soft tissue injection. The therapeutic agent may be injected as a single dose or in multiple doses. In one embodiment, between 2 and 6 doses are administered between once a day and once a week. In certain embodiments, the total single locally administered dose does not exceed 20 mg. In certain embodiments, the total single locally administered dose is between about 0.1 µg to about 20 mg (*e.g*., between about 1 µg to 15 mg).

In certain embodiments of the invention, compositions may be combined for use. For example, a composition having a drug effective in treating contracture may be combined in its use with a second composition having one or more drugs effective in treating contracture or one or more of the related conditions discussed herein, such as infection, swelling, pain, or inflammation. In one aspect, the second therapeutic agent is selected from the following classes of agents: anti-infectives, anaesthetics, analgesics, antibiotics, narcotics, and steroidal and non-steroidal anti-inflammatory agents. For example, the second therapeutic agent may be an opiate, such as codeine, meperidine, methadone, morphine, pentazocine, fentanyl, hydromorphone, oxycodone, or oxymorphone, including salts, derivatives, and analogues thereof. In another aspect, the second therapeutic agent is an anti-inflammatory agent, such as a non-steroidal anti-inflammatory agent (*e.g*., aspirin, ibuprofen, indomethacin, naproxen, prioxicam, diclofenac, tolmetin, fenoclofenac, meclofenamate, mefenamic acid, etodolac, sulindac, carprofen, fenbufen, fenoprofen, flurbiprofen, ketoprofen, oxaprozin, tiaprofenic acid, phenylbutazone diflunisal, salsalte, and salts and analogues and derivatives thereof), or a steroidal anti-inflammatory agent, such as hydrocortisone or an ester thereof.

When more than one agent is administered, the additional agent may be administered to the patient at the same time as the initial agent or in series. In certain embodiments, the administration of the second agent may occur within one hour or less, or may occur between about 1 hour and about 24 hours following the first therapeutic agent. The therapeutic agent can be administered at the time of a procedure, or in the case of an injury, can be administered any time before a mature contracture actually forms, which can be days to weeks after the inciting event.

Certain compositions may be useful as an injectable formulation and as such may contain one or more excipients. The excipient(s) may be polymers or non-polymers and may function to provide viscosity, sterility, isotonicity, controlled drug release, stability or other desirable characteristics to the formulation. In certain embodiments the excipient may provide a mechanical or biological benefit of its own, for example, hyaluronic acid may provide for desired viscosity or drug release characteristics although it may also have other beneficial effects when administered into a joint in the formulation.

The composition further comprises a polymeric carrier. The polymeric carrier may be biodegradable or bioresorbable. In certain aspects, the polymer includes an ester group, a thioester group, an amide group, an anhydride group, or an ether group within the polymeric backbone. The polymer may include a polyamino acid or a polysaccharide. In certain embodiments, the polymer may include a polyamino acid or a polysaccharide, with the proviso that the therapeutic agent should not be an antimicrotubule agent. The polysaccharide may be cellulose, or hyaluronic acid or a salt or derivative thereof. The polymer may include a polyalkylene oxide, such as polyethylene glycol or polypropylene oxide or a copolymer thereof. In certain embodiments, the polyalkylene oxide is a polyethylene glycol-polypropylene oxide diblock or triblock copolymer. The polymer may include a branched polymer or a linear copolymer. In one aspect, the polymer is formed from one or more monomers selected from the group consisting of L-lactide, DL-lactide, glycolide, and caprolactone. In one aspect, the polymer is poly(DL-lactide) or a copolymer thereof. In another aspect, the polymer includes poly(lactide-co-glycolide).

The polymer is a block copolymer (*e.g*., diblock or triblock copolymer).

In The invention, (a) the block copolymer comprises one or more blocks A and block B, (b) block B is more hydrophilic than block A, and (c) the block copolymer has a molecular weight of between about 500 g/mol and about 2000 g/mol. The block copolymer is be non-thermoreversible and/or a liquid at room temperature. In certain embodiments, the block copolymer is a triblock copolymer comprising a carbonate monomer. In certain embodiments, the triblock copolymer has an average molecular weight of between about 600 and about 1500 g/mol.

In certain embodiments, the triblock copolymer has a weight percent water soluble fraction of less than about 25%, about 50% or about 75%.

In certain embodiments, the triblock copolymer dissolves in a solvent having a δh Hansen solubility parameter value of no less than 22, 32, or 42.

In certain embodiments, the composition further comprise a diluent. Such a diluent may be selected from the group consisting of a polyethylene glycol (PEG), PEG derivatives, polypropylene glycol, and polypropylene glycol derivatives. In certain embodiments, the diluent has a molecular weight of between about 100 g/mol and about 500 g/mol.

In certain embodiments, the triblock copolymer is an ABA triblock copolymer, wherein the B block comprises a polyalkylene oxide (*e.g*., polyethylene glycol) having a molecular weight of between about 200 g/mol to about 600 g/mol (*e.g*., about 400 g/mol), and the A blocks comprise a polymer having about a 90:10 mole ratio of trimethylene carbonate (TMC) and glycolide (Gly) residues and have a total molecular weight of about 900 g/mol. In certain embodiments, the composition further comprises a PEG or a derivative thereof having a molecular weight of between about 100 g/mol and 500 g/mol (*e.g*., about 300 g/mol).

In certain embodiments, the therapeutic agent is paclitaxel, which may be present in the composition at a concentration of between about 0.1 mg/ml to about 1 mg/ml (*e.g*., about 0.15 mg/ml, about 0.3 mg/ml, or about 0.6 mg/ml).

The instant, compositions may include a non-polymeric carrier. Representative examples of non-polymeric carriers include phospholipids, a co-solvent, a non-ionic surfactant, such as TWEEN, or a surfactant that includes a polyethylene glycol moiety and at least one ester bond. Composition comprising phospholipids may be used to achieve a therapeutic benefit with or without the attributes of a bioactive agent.

In one aspect, the composition is in the form of a solution, suspension, or emulsion. The solution may be a colloidal dispersion and may include micelles that contain at least a portion of the therapeutic agent.

In one aspect, the carrier includes a gel (*e.g*., a hydrogel). In another aspect, the carrier includes micelles. In certain embodiments, the composition includes solid particles that contain at least a portion of the therapeutic agent. The solid particles may be microspheres having a mean diameter of between about 1 µm and about 1000 µm or nanospheres having a mean diameter of about 200 to about 1000 nm.

In another aspect, the composition is in the form of a paste, ointment, cream, powder, spray, or an implant, which may be implanted during a surgical procedure. The implant may be an orthopedic implant (*e.g*., pins, screws, plates, grafts, anchors, joint replacement devices, and bone implants) and may include one or more types of metals, metal alloys, and inorganic salts. In one aspect the orthopedic implant includes a coating in which at least a part of the therapeutic agent is contained. In one aspect, the implant is a suture, sponge, pledget, film, membrane, or fabric.

Compositions may be prepared for their ultimate clinical use by incorporation into kits, or using processes such as sterilization and addition of outer packaging. Kits may include one or more solid or liquid components to be combined with one or more liquid components such that a composition suitable for administration is prepared at some time prior to its use. In certain embodiments, at least one component of the kit is sterile. For example, microspheres may be constituted with a solution immediately prior to injection, or two liquids may be combined prior to injection.

In one aspect, the disclosure provides a kit for treating contracture. The kit includes a first composition that includes a therapeutically effective amount of a therapeutic agent, wherein the therapeutic agent is active in treating contracture. In one aspect, the therapeutic agent included in the instant kit is paclitaxel or a derivative or analogue thereof. The kit further includes a second composition that includes an excipient (*e.g*., a buffer). In one embodiment, the first composition is in the form of microspheres. In another embodiment, the second composition is in the form of a solution.

In one aspect, the disclosure provides a kit for treating contracture that includes an implant comprising a therapeutically effective amount of a therapeutic agent, wherein the therapeutic agent is active in treating contracture. In one aspect, the therapeutic agent included in the instant kit is paclitaxel or a derivative or analogue thereof. The kit further includes a device for insertion or implantation of the implant.

Other aspects of the disclosure relate to methods of use of compositions and regimes for contracture treatment. These methods include the administration of compositions, the use of kits, the methods of manufacture of compositions and kits. Treatment regimes include doses, administration schedules which may include dosing frequencies or durations, the combination therapies, and selection of the route of administration.

In one aspect, a method for treating contracture or the recurrence of contracture is described that includes: a) combining a first composition, wherein the first composition comprises a therapeutically effective amount of a therapeutic agent, wherein the therapeutic agent is active in treating (*e.g*., inhibiting) joint contracture or recurrence of joint contracture, and a second composition, wherein the second composition comprises an excipient; and b) injecting the combined first and second compositions into the joint, into the vicinity of a joint or into soft tissue during a clinical procedure. The timing of the intervention may be at the time of clinical presentation, at the time of a procedure or after a procedure.

In another aspect, a method for treating joint contracture is provided that includes administering to a joint a therapeutically effective amount of a composition including a therapeutic agent effective in treating contracture or the recurrence of the contracture.

In yet another aspect, the disclosure provides a method for treating a Dupytren's contracture or recurrence of a Dupytren's contracture, including administering to the site of the contracture before, at the time of or after a release procedure, a therapeutically effective amount of a composition comprising a therapeutic agent effective in treating contracture or its recurrence.

In yet another aspect, a method for treating a Volkmann's contracture is provided. The method includes administering to the site of the contracture during, at the time or after a release procedure, a therapeutically effective amount of a composition comprising a therapeutic agent effective in treating contracture.

In yet another aspect, the disclosure provides a method for treating a Ledderhose's contracture including administering to the site of the contracture during, at the time or after a release procedure, a therapeutically effective amount of a composition comprising a therapeutic agent effective in treating contracture.

In yet another aspect, the disclosure provides a method for treating a Peyronie's contracture. The method includes administering to the site of the contracture during, at the time or after a release procedure, a therapeutically effective amount of a composition comprising a therapeutic agent effective in treating contracture.

The methods described herein may include one or more of the therapeutic agents described herein. In one aspect, the therapeutic agent is paclitaxel or a derivative or analogue thereof.

In another aspect, the present disclosure provides a method for treating contracture, comprising: a) providing a composition that comprises an ABA triblock copolymer and about 0.1 mg/ml to about 1 mg/ml of paclitaxel, wherein (i) the triblock copolymer comprises two A blocks and a B block, (ii) the B block comprises a polyalkylene oxide having a molecular weight of between about 400 g/mol, and (iii) the A blocks comprise a polymer having about a 90:10 mole ratio of trimethylene carbonate (TMC) and glycolide (Gly) residues, and have a total molecular weight of about 900 g/mol; and b) injecting the composition into the vicinity of a joint during an operative procedure.

The present invention provides a composition comprising: a) a block copolymer comprising one or more blocks A and block B, wherein (i) block B is more hydrophilic than block A, (ii) the block copolymer has a molecular weight of between about 500 g/mol and about 2000 g/mol, (iii)
the copolymer is non-thermoreversible and is a liquid at room temperature; and a therapeutic agent effective in treating contracture (*e.g*., paclitaxel).

In another aspect, the present invention provides a composition comprising (a) an ABA triblock copolymer, wherein the B block comprises a polyalkylene oxide (*e.g*., polyethylene glycol) having a molecular weight of between about 200 g/mol to about 600 g/mol (*e.g*., about 400 g/mol), and the A blocks comprise a polymer having about a 90:10 mole ratio of trimethylene carbonate (TMC) and glycolide (Gly) residues and have a total molecular weight of about 900 g/mol, and (b) a therapeutic agent effective in treating contracture (*e.g*., paclitaxel). In certain embodiments, the composition further comprises a diluent (*e.g*., PEG having a molecular weight of about 300 g/mol).

These and other aspects of the present invention will become evident upon reference to the following detailed description and attached drawings. In addition, various references are set forth herein which describe in more detail certain procedures, devices, or compositions.

### BRIEF DESCRIPTION OF THE DRAWINGS

Figure 1 is a bar graph showing the percentage increase in knee width (swelling) as a function of paclitaxel concentration for various formulations.
Figure 2 shows a guinea pig knee joint at sacrifice 7 days after intraarticular administration of 0.1 ml of 15 mg/ml paclitaxel in PLURONIC F127 gel. A) Necrosis visible on the exterior of the lateral capsule. B) Subcutaneous swelling and fluid build up in the joint space. C) Swollen fat pad with significant vascular tissue growth.
Figure 3 shows a guinea pig knee joint at sacrifice 7 days after intraarticular administration of 0.1 ml of 7.5 mg/ml paclitaxel as micellar paclitaxel in hyaluronic acid gel. The treated joint (right) appears normal, with identical appearance to the untreated joint (left).
Figure 4 shows a guinea pig knee joint at sacrifice 7 days after intraarticular administration of 0.1 ml of (A) 1.5 mg/ml paclitaxel as microemulsion in hyaluronic acid gel and (B) 40:40:20 PEG200:water: TRANSCUTOL^{®} (ethoxydiglycol). The treated (right) joint in each animal has yellow discoloration of the infrapatellar fat pad.
Figure 5 is a bar graph showing average paclitaxel concentration in tissue 7 days after injection for various formulations. Formula 4 had an average concentration in capsule and fat pad below 0.01 µg/g.
Figure 6 is a bar graph showing average paclitaxel concentration in tissue 14 days after injection for various formulations. Formulas 3 and 4 had average concentration in all tissues that was below 0.01 µg/g.
Figure 7 is a graph showing the phase behavior and solubility of paclitaxel solutions in PEG/serum mixtures.
Figure 8 is a microscopic photograph of an excised rabbit joint showing the precipitation of paclitaxel in the joint after administration of a depot formulation.
Figure 9 is a bar graph showing percent (w/w) of water insoluble components in triblock copolymers following extraction into water at 37°C.
Figure 10 is a bar graph showing percent (w/w) of water insoluble components in triblock copolymers following extraction into water at 37°C.

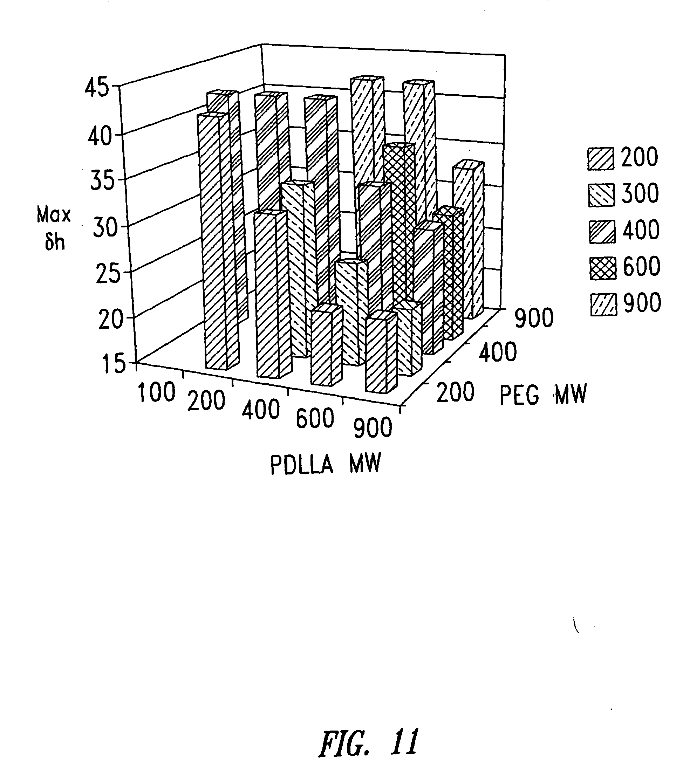

Figure 11 is a bar graph showing solubility characteristics of PEG/PDLLA triblock copolymers. Max δₕ represents the highest δₕ for all solvent systems capable of dissolving the polymer at 10 mg/ml.
Figure 12 is a bar graph showing solubility characteristics of PEG-TMC/glycolide, PEG-TMC, PPG-TMC/glycolide, and PPG-PDLLA.
Figure 13 is a graph showing the effect of concentration of PEG400-TMC/Gly(90/10)900 in PEG 300 on paclitaxel release, expressed in terms of cumulative taxane release (% of total loading).
Figure 14 is a graph showing the empirical relationship between the concentration of PEG 400 TMC/Gly(90/10) 900 triblock copolymer in PEG 300 and paclitaxel release over 3 days, expressed in terms of cumulative taxane release (% of total loading).
Figure 15 is a graph showing release profiles of PEG-PDLLA triblock co-polymers with different PEG MW and polyester MW, expressed in terms of cumulative taxane release (% of total loading).
Figure 16 is a graph showing the relationship between the molecular weight of hydrophobic blocks in triblock co-polymers and the percentage drug release in 3 days, expressed in terms of cumulative taxane release (% of total loading).
Figure 17 is a graph showing paclitaxel release profiles for triblock copolymers (structural analogues of PEG400/TMC-Gly(90/10)900) over a period of 4 days, expressed in terms of cumulative taxane release (% of total loading).
Figure 18 is a graph showing the relationship between the maximum Hansen Hydrogen Bonding Parameter (δh) and paclitaxel release, expressed in terms of cumulative taxane release (% of total loading).
Figure 19 is a ternary phase diagram showing the compositions at which phase separation was observed when water was added to PEG 400 TMC/Gly(90/10) 900 triblock copolymer/PEG 300 mixtures of various compositions.
Figure 20 is a plot showing median (N=3) concentrations of paclitaxel in tissues harvested from a rabbit knee after various time intervals following an intra-articular injection of paclitaxel in a copolymer gel formulation including 0.075 mg/ml paclitaxel in a blend of 30% PEG400-TMC/Gly(90/10)900 in PEG 300.
Figure 21 is a plot showing median (n=3) concentrations of paclitaxel in tissues harvested from a rabbit knee after various time intervals following an intra-articular injection of paclitaxel in a copolymer gel formulation including comprising 0.15 mg/ml paclitaxel in a blend of 2.5% PEG400-TMC/Gly(90/10)900 in PEG 300.

### DETAILED DESCRIPTION OF THE INVENTION

Prior to setting forth the invention, it may be helpful to an understanding thereof to set forth definitions of certain terms that will be used hereinafter.

"Contracture" as used herein refers to a permanent or longterm reduction of range of motion due to tonic spasm or fibrosis, or to loss of normal soft tissue (*e.g*., muscle, tendon, ligament, fascia, synovium, joint capsule, other connective tissue, or fat) compliance, motion or equilibrium. In general, the condition of contracture involves a fibrotic response with inflammatory components, both acute and chronic. The pathological features of contracture include the deposition of abnormal amounts and types of collagen, with the presence of fibroblasts or myofibroblasts, observed histologically in humans (J. Shoulder Elbo. Surg. 10: 353-7, 2001). The triggers for inflammation, cellular proliferation and abnormal collagen production may include; trauma, injury, drugs, irritants, metabolic disorders, neuronal problems or they may be ideopathetic. In affected joints, soft tissue both within the joint (*e.g*., capsules) and outside the joint (*e.g*., collateral ligament) have demonstrated thickening, this has been observed radiographically by MRI (J. Magn. Reson. Imagin. 5: 473-7, 1995) and on surgical exploration.

Many different types of contractures exist and may affect a joint, such as an elbow, a shoulder, a knee, an ankle, a hip, a finger joint, a wrist, a toe joint, a temporomandibular joint, an otic bone joint, a facet joint (*e.g*., a joint in the neck or back), and other extra-articular structures, such as soft tissue, muscles, tendons, ligaments, fat, synovium, joint capsule, connective tissue (*e.g*., fascia), and the volar plates. For example, after an injury to a finger joint, changes in the volar plate, a soft tissue structure that is outside the joint, can contribute to loss of finger joint motion. In certain cases, a contracture may affect a combination of one or more types of joints and/or types of soft tissue.

Contracture may be associated with a variety of conditions, including inflammation or degeneration of a joint or soft tissue; hypertrophy (including hypertrophy of soft tissue, *e.g*., muscles, tendons, ligaments, fat, joint capsule, synovium, or other connective tissue, and hypertrophic conditions that affect canals, such as carpel, tarsal, or cubital tunnel syndrome); injury; neurological conditions (*e.g*., paralysis or stroke); metabolic conditions (*e.g*., diabetes, haemophilia, gout, or pseudo gout); infection; or ischemia, or any combination of these conditions. Prolonged immobilization in a cast or splint, swelling, pain, abnormal tissue proliferation, and genetic profile are other factors that may predispose a subject to contracture. Increased compartment pressures, such as in the leg or arm, may also lead to contractures.

Risk factors that predispose patients to joint scarring and contractures include the specific joint affected (*e.g*., shoulders have a higher rate of contractures than knees), type of injury, history of contractures, inflammatory disorders, abnormal tissue proliferation disorders, hemophilia, diabetes, gender and age (*e.g*., being female over 40 years of age).

For example, in the case of a joint contracture, the thickening and fibrosis of the synovium, capsule and/or other soft tissue surrounding the joint limits the function of a joint (*e.g*., a joint in the finger). In the case of a Dupytren's contracture, the disease is a result of thickening and contraction of fibrous bands in the soft tissue (*e.g*., palmar fascia). Ledderhose Disease (plantar fibromatosis) is characterized by thickening of the plantar fascia due to local proliferation of abnormal fibrosis tissue.

Organic contractures are usually due to fibrosis within the soft tissue (*e.g*., muscle) and persist whether the subject is conscious or unconscious. Volkmann's contractures are caused by tissue degeneration produced by ischemia that leads to a late contracture involving muscles, tendons, fascia and other soft tissue.

Contracture may arise after an injury. Representative examples of traumatic injuries include burns, crushes, cuts, tears, disruptions, impacts, tractions, fracture (especially in or around a joint, such as an elbow), subluxation, dislocation (*e.g*., of a joint, such as an finger, elbow, shoulder, ankle, knee, or hip), joint disruption (*e.g*., shoulder, elbow, hip, temporomandibular joint, facet, finger, knee ankle, or toe), and other bone, cartilage, tendon or ligament injuries. Contracture related to trauma may be caused directly by the trauma, healing processes following trauma, or underlying or pre-existent conditions (*e.g*., arthritis), and may be exacerbated by immobilization during recovery or paralysis. In certain cases, trauma incurred as the result of an open surgical procedure (*e.g*., fracture reduction, rotator cuff repair, or tendon or ligament repair) or a minimally invasive procedure, such as arthroscopy, or endoscopy, may result in the formation of a contracture.

The loss of proper joint function due to joint stiffness or lack of mobility may include intra-articular and/or extra-articular contributors. Intra-articular contributors include, for example, loss of soft tissue compliance within the joint, capsular and synovial changes and thickening, and/or the formation of bands of scar tissue that can obstruct or cross within the joint limiting its function. Extra-articular contributors can include any change in the soft tissue surrounding a joint which may impact the joint function, for example, scarring, calcification, or loss compliance of a tendon or muscle which would result in an inability to fully lengthen or contract and would ultimately limit the normal range of joint movement).

"Range of Motion" abbreviated "ROM" as used herein refers to an expression derived from measurements which characterize the ability to move (*e.g*., to articulate a joint). In a joint, articulation includes rotation, flexion, extension, pronation, and supination of the joint. All of these measures of ROM are expressed in terms of degrees. In the case of motion in an elbow joint, full flexion is defined as 0°; full extension is defined as 180°. However, joints normally cannot articulate through this entire range. For example, elbows have a normal range of motion between about 20 and about 180°; however, there is variability in this range from person to person. Some joints may naturally hyperextend (motion beyond 180°), particularly under active articulation. These joints include the finger joints which have a typical range of motion between about 90 and 190°. Range of motion may be greater under active articulation (application of force) than in passive articulation. A Mayo Clinic Clinical Performance Index divides ROM in a joint into ranges of 0-50° (worst), 50-100° and >100° (best). In another similar rating a loss of <5° is considered an excellent result, and <15, <30 and >30 are considered good, fair and poor, respectively (J Bone Joint Surg Am 1988(70) 244-9).

"Carrier" as used herein refers to any of a number of matrices, solid, semi-solid or liquid which can be made to contain a therapeutic agent. The carrier may be a continuous phase, such as a suspension or a gel, or may include a plurality of phases, such as a dispersion or emulsion, or matrices, such as a coated particle (*e.g*., microparticle). The carrier may be synthetic or biologically derived and may include living tissue. The carrier may be a solid matrix having additional therapeutic utility, such as an orthopedic implant.

"Bioresorbable" as used herein refers to the property of a composition or material being able to be cleared from a body after administration to a human or animal. Bioresorption may occur by one or more of a variety of means, such as dissolution, oxidative degradation, hydrolytic degradation, enzymatic degradation, metabolism, clearance of a component or its metabolite through routes such as the kidney, intestinal tract, lung or skin. Degradative mechanisms for bioresorption are collectively termed "biodegradation" and compositions having this property are termed "biodegradable".

"Bioerodible" as used herein refers to materials which lose mass and may ultimately disappear in a physiological environment. Bioerosion results from mechanism including dissolution, degradation, fragmentation or erosion in response to mechanical force. Bioerosion may be modulated by physiological factors such as the presence of enzymes, temperature, pH or by exposure to an aqueous environment.

"Biodegradable" as used herein refers to materials for which the degradation process is at least partially mediated by, and/or performed in, a biological system. "Degradation" includes a chain scission process by which a polymer chain is cleaved into oligomers and monomers. Chain scission may occur through various mechanisms, including, for example, by chemical reaction (*e.g*., hydrolysis) or by a thermal or photolytic process. Polymer degradation may be characterized, for example, using gel permeation chromatography (GPC), which monitors the polymer molecular mass changes during erosion and drug release. "Biodegradable" also refers to materials may be degraded by an erosion process mediated by, and/or performed in, a biological system. "Erosion" refers to a process in which material is lost from the bulk. In the case of a polymeric system, the material may be a monomer, an oligomer, a part of a polymer backbone, or a part of the polymer bulk. Erosion includes (i) surface erosion, in which erosion affects only the surface and not the inner parts of a matrix; and (ii) bulk erosion, in which the entire system is rapidly hydrated and polymer chains are cleaved throughout the matrix. Depending on the type of polymer, erosion generally occurs by one of three basic mechanisms (*see*, *e.g.,* Heller, J., CRC Critical Review in Therapeutic Drug Carrier Systems (1984), 1(1), 39-90); Siepmann, J. et al., Adv. Drug Del. Rev. (2001), 48, 229-247): (1) water-soluble polymers that have been insolubilized by covalent cross-links and that solubilize as the cross-links or the backbone undergo a hydrolytic cleavage; (2) polymers that are initially water insoluble are solubilized by hydrolysis, ionization, or pronation of a pendant group; and (3) hydrophobic polymers are converted to small water-soluble molecules by backbone cleavage. Techniques for characterizing erosion include thermal analysis (*e.g*., DSC), X-ray diffraction, scanning electron microscopy (SEM), electron paramagnetic resonance spectroscopy (EPR), NMR imaging, and recording mass loss during an erosion experiment. For microspheres, photon correlation spectroscopy (PCS) and other particles size measurement techniques may be applied to monitor the size evolution of erodible devices versus time.

"Therapeutic agent" as used herein refers to those agents (*e.g*., drugs, therapeutic compounds, pharmacologically active agents and pharmacologically active compounds) which may mitigate, treat, cure or prevent (*e.g*., as a prophylactic agent) a given disease or condition. Representative examples of therapeutic agents are discussed in more detail below, and include, for example, cell cycle inhibitors, microtubule stabilizing agents, anti-angiogenic agents, cell cycle inhibitors, antithrombotic agents, and anti-inflammatory agents. Briefly, within the context of the present invention, anti-angiogenic agents should be understood to include any protein, peptide, chemical, or other molecule, which acts to inhibit vascular growth (*see*, *e.g.,* U.S. Patent Nos. 5,994,341, 5,886,026, and 5,716,981). These agents may also be referred to as bioactive agents.

"Cell cycle inhibitor" as used herein refers to any protein, peptide, chemical or other molecule which delays or impairs the ability of a cell to progress through the cell cycle and replicate.

"Anti-microtubule agent" should be understood to include any protein, peptide, chemical, or other molecule that impairs the function of microtubules, for example, through the prevention or stabilization of tubulin polymerization. A wide variety of methods may be utilized to determine the anti-microtubule activity of a particular compound including, for example, assays described by Smith et al. (Cancer Lett 79(2):213-219, 1994) and Mooberry et al., (Cancer Lett. 96(2):261-266, 1995). Representative examples of anti-microtubule agents include taxanes, cholchicine, discodermolide, vinca alkaloids (*e.g*., vinblastine and vincristine), as well as analogues and derivatives of any of these.

"Treat" or "treatment" as used herein refer to the therapeutic administration of a desired composition or compound in an amount and/or for a time sufficient to inhibit, reduce, delay, or eliminate the progression, occurrence or recurrence of, or to reduce the degree or extent of, at least one aspect or marker of contracture in a statistically or clinically significant manner. The therapeutic efficacy of a therapeutic composition according to the present invention is based on a successful clinical outcome and does not require 100% elimination of the symptoms or clinical findings associated with contracture. For example, achieving a level of a therapeutic agent at the affected site, which allows the patient to resolve, delay or prevent the onset, progression or recurrence of a contracture, or allows the patient to have a better quality of life, is sufficient. Accordingly, therapeutic agents, compositions and methods for treating contracture are provided herein. The instant methods may be used to administer the compositions described herein to a patient in need thereof who is a mammal (*e.g*., a human or any domesticated animal, such as a horse or dog).

"Fibrosis," or "scarring," or "fibrotic response" refers to the formation of fibrous (scar) tissue in response to injury or medical intervention. Therapeutic agents which inhibit fibrosis or scarring are referred to herein as "fibrosis-inhibiting agents", "fibrosis-inhibitors", "anti-scarring agents", and the like, where these agents inhibit fibrosis through one or more mechanisms including: inhibiting inflammation or the acute inflammatory response, inhibiting migration and/or proliferation of connective tissue cells (such as fibroblasts, smooth muscle cells, vascular smooth muscle cells), inhibiting angiogenesis, reducing extracellular matrix (ECM) production or promoting ECM breakdown, and/or inhibiting tissue remodeling.

"Inhibit fibrosis", "reduce fibrosis", "inhibits scarring" and the like are used synonymously to refer to the action of agents or compositions which result in a statistically significant decrease in the formation of fibrous tissue that can be expected to occur in the absence of the agent or composition.

"Inhibitor" refers to an agent which prevents a biological process from occurring or slows the rate or degree of occurrence of a biological process. The process may be a general one such as scarring or refer to a specific biological action such as a molecular process resulting in release of a cytokine.

"Antagonist" refers to an agent which prevents a biological process from occurring or slows the rate or degree of occurrence of a biological process. While the process may be a general one, typically this refers to a drug mechanism where the drug competes with a molecule for an active molecular site or prevents a molecule from interacting with the molecular site. In these situations, the effect is that the molecular process is inhibited.

"Agonist" refers to an agent which stimulates a biological process or rate or degree of occurrence of a biological process. The process may be a general one such as scarring or refer to a specific biological action such as a molecular process resulting in release of a cytokine.

"Polysaccharide" as used herein refers to a combination of at least three monosaccharides that are generally joined by glycosidic bonds. Naturally occurring polysaccharides may be purified according to accepted procedures known to those having skill in the art. Polysaccharides may be ionically or chemically cross-linked by groups such as vinyl sulfone (*see* U.S. Patent No. 4,605,691) or other polymers of low molecular weight (*see* U.S. Patent No. 4,582,865).

"Polypeptide" includes peptides, proteins, cyclic proteins, branched proteins, polyamino acids, copolymers thereof, and derivatives of each of these (including those with non-naturally occurring amino acids known in the art), which may be naturally or synthetically derived. An "isolated peptide, polypeptide, or protein" is an amino acid sequence that is essentially free from contaminating cellular components, such as carbohydrate, lipid, nucleic acid (DNA or RNA), or other proteinaceous impurities associated with the polypeptide in nature. Preferably, an isolated polypeptide is sufficiently pure for therapeutic use at a desired dose.

Any concentration ranges recited herein are to be understood to include concentrations of any integer within that range and fractions thereof, such as one tenth and one hundredth of an integer, unless otherwise indicated. Also, any number range recited herein relating to any physical feature, such as polymer subunits, size or thickness, are to be understood to include any integer within the recited range, unless otherwise indicated. It should be understood that the terms "a" and "an" as used above and elsewhere herein refer to "one or more" of the enumerated components. As used herein, the term "about" means ± 10%.

As used herein, the terms "average" or "mean" include the arithmetic mean as well as any appropriate weighted averages such as are used in the expression of polymeric molecular weight or particle size distributions.

As noted above, the present invention relates generally to compositions, for treating contracture. In one aspect, the present compositions, devices, and methods are useful in treating joint contracture, *e.g*., following surgery or injury. The disclosure provides delivering to a joint (either intra- or periarticularly) a composition that includes a therapeutic agent (with or without a polymeric carrier) that is effective at treating contracture. Administration of the therapeutic agent shortly after injury or surgery of the injured joint may markedly reduce the incidence and magnitude of joint contracture, thereby avoiding the need for additional surgical intervention (*e.g*.. to remove scar tissue) after the contracture has developed.

Within yet another aspect of the disclosure, pharmaceutical devices, products, or compositions are provided, that includes (a) a therapeutic agent in a container, and (b) a notice associated with the container in form prescribed by a governmental agency regulating the manufacture, use, or sale of devices or pharmaceuticals, which notice is reflective of approval by the agency of a device or compound that, for example, disrupts microtubule function or is anti-angiogenic or is anti-proliferative or is immunosuppressive and the like, for human or veterinary administration to treat non-tumorigenic angiogenesis-dependent diseases such as inflammatory arthritis or neovascular diseases of the eye. Briefly, Federal Law requires that the use of a pharmaceutica! agent in the therapy of humans be approved by an agency of the Federal government. Responsibility for enforcement (in the United States) is with the Food and Drug Administration, which issues appropriate regulations for securing such approval, detailed in 21 U.S.C. §§ 301-392. Regulation for biological materials that include products made from the tissues of animals, is also provided under 42 U.S.C. § 262. Similar approval is required by most countries, although, regulations may vary from country to country.

A wide variety of therapeutic agents may be delivered to a joint or soft tissue, either with or without a carrier (*e.g*., polymeric or non-polymeric), in order to treat a contracture. Discussed in more detail below arse: I) Therapeutic Agents, II) Compositions, and III) Treatment of Contracture.

### I. THERAPEUTIC AGENTS

A wide variety of agents (also referred to herein as "therapeutic agents" or "drugs") may be utilized within the context of the present invention.

Compositions of the present invention may include one or more therapeutic agents active in treating contracture. The activity of the one or more therapeutic agents may be due to inhibiting cellular processes that may be involved in the formation of the contracture state, such as inflammation including production of cytokines resulting in cell proliferation, cell migration, cell adhesion and cellular secretion and processes involved in fibrosis, such as cellular proliferation and matrix secretion. Cellular secretion may include secretion of growth factors or other factors involved in stimulation of the super-healing processes of soft tissue, such as connective tissue (*e.g*., palmar fascia or synovium) and/or hard tissue, such as tendon, fibrous bands in the hand, bone, and/or may also include secretion of a variety of matrix proteins, such as, but not limited to, collagen and proteoglycans. Processes leading to free radical production and resultant tissue damage or stimulation and release of cellular proteins also may be involved and inhibited by therapeutic agents. Formation and secretion of such proteins may result in webbed fibrous components, which reduce movement by either connecting various tissues together or by thickening some tissues, such as synovium or fibrous bands in the hand, thereby causing a reduced ability to achieve free movement of the body part. Furthermore, these protein structures may be, in the context of the fibers or tissue they are connected to, platforms for cellular accumulation and proliferation which may lead to a reduction in motion. Some cell types involved in the cellular processes described above are fibroblasts and fibroblasts with contractile activity. Fibroblasts with contractile activity would be expected to contract abnormally contributing to the contracture. This would become especially prevalent as the number of these contractile cells accumulate. Thus, drug mechanisms which lead to inhibition of proliferation of these cells may be beneficial within the context of the present invention.

When more than one therapeutic agent is present, one or more agents is/are active in treating contracture by the means described above. One or more additional therapeutic agents may be present that is/are active in treating other conditions or symptoms associated with contracture or treatments of conditions from which contracture may arise, including, without limitation, for example, drugs used in the reduction of fracture. The additional agent(s) may be administered simultaneously with a treatment for the prevention of contracture and may or may not be contained within the same composition as the pharmacologically active agent. Alternately, or in addition, the additional agent(s) may be administered before or after administration of the pharmacologically active agent. Representative examples of additional agents include, *e.g*., anti-inflammatory, antibiotic, antiinfective, analgesic or anesthetic agents, or hyaluronic acid or hyaluronic acid derivatives.

Drugs and associate classes of drugs and their derivatives and analogues effective in preventing the onset of contracture include, but are not limited to, a number of classes of compounds. Examples of agents provided are by means of description and not by means of limitation of the pharmacological class to which they belong.

### 1. Cell Cycle Inhibitors

A wide variety of cell cycle inhibitory agents can be utilized, either with or without a carrier (*e.g*., a polymer), within the context of the present invention. Within one preferred embodiment of the invention, the cell cycle inhibitor is paclitaxel, a compound which disrupts mitosis (M-phase) by binding to tubulin to form abnormal mitotic spindles or an analogue or derivative thereof. Briefly, paclitaxel is a highly derivatized diterpenoid (Wani et al., J. Am. Chem. Soc. 93:2325, 1971) which has been obtained from the harvested and dried bark of Taxus brevifolia (Pacific Yew) and Taxomyces Andreanae and Endophytic Fungus of the Pacific Yew (Stierle et al., Science 60:214-216, 1993). Paclitaxel and its formulations, prodrugs, analogues and derivatives include, for example, TAXOL (Bristol-Myers Squibb Company, New York, NY), TAXOTERE (Aventis Pharmaceuticals, France), and 3'N-desbenzoyl-3'N-t-butoxy carbonyl analogues of paclitaxel. Paclitaxel and its analogues may be readily prepared utilizing techniques known to those skilled in the art (*see*, *e.g*., Schiff et al., Nature 277:665-667, 1979; Long and Fairchild, Cancer Research 54:4355-4361, 1994; Ringel and Horwitz, J. Nat'l Cancer Inst. 83(4):288-291, 1991), or obtained from a variety of commercial sources, including for example, Sigma Chemical Co., St. Louis, Missouri (T7402 - from Taxus brevifolia).

Representative examples of paclitaxel derivatives or analogues include 7-deoxy-docetaxol, 7,8-cyclopropataxanes, N-substituted 2-azetidones, 6,7-epoxy paclitaxels, 6,7-modified paclitaxels, 10-desacetoxytaxol, 10-deacetyltaxol (from 10-deacetylbaccatin III), phosphonooxy and carbonate derivatives of taxol, taxol 2',7-di(sodium 1,2-benzenedicarboxylate, 10-desacetoxy-11,12-dihydrotaxol-10,12(18)-diene derivatives, 10-desacetoxytaxol, Protaxol (2'-and/or 7-O-ester derivatives), (2'-and/or 7-O-carbonate derivatives), asymmetric synthesis of taxol side chain, fluoro taxols, 9-deoxotaxol, 7-deoxy-9-deoxotaxol, 10-desacetoxy-7-deoxy-9-deoxotaxol, derivatives containing hydrogen or acetyl group and a hydroxy and tert-butoxycarbonylamino, sulfonated 2'-acryloyltaxol and sulfonated 2'-O-acyl acid taxol derivatives, succinyltaxol, 2'-y-aminobutyryltaxol formate, 2'-acetyl taxol, 7-acetyl taxol, 7-glycine carbamate taxol, 2'-OH-7-PEG(5000) carbamate taxol, 2'-benzoyl and 2',7-dibenzoyl taxol derivatives, other prodrugs (2'-acetyltaxol; 2',7-diacetyltaxol; 2'-succinyltaxol; 2'-(.beta.-alanyl)-taxol); 2'-.gamma.-aminobutyryltaxol formate; ethylene glycol derivatives of 2'-succinyltaxol; 2'-glutaryltaxol; 2'-(N,N-dimethylglycyl) taxol; 2'-(2-(N,N-dimethylamino)propionyl)taxol; 2'-orthocarboxybenzoyl taxol; 2'-aliphatic carboxylic acid derivatives of taxol, prodrugs {2'(N,N-diethylaminopropionyl)taxol, 2'(N,N-dimethylglycyl)taxol, 7(N,N-dimethylglycyl)taxol, 2',7-di-(N,N-dimethylglycyl)taxol, 7(N,N-diethylaminopropionyl)taxol, 2',7-di(N,N-diethylaminopropionyl)taxol, 2'-(L-glycyl)taxol, 7-(L-glycyl)taxol, 2',7-di(L-glycyl)taxol, 2'-(L-alanyl)taxol, 7-(L-alanyl)taxol, 2',7-di(L-alanyl)taxol, 2'-(L-leucyl)taxol, 7-(L-leucyl)taxol, 2',7-di(L-leucyl)taxol, 2'-(L-isoleucyl)taxol, 7-(L-isoleucyl)taxol, 2',7-di(L-isoleucyl)taxol, 2'-(L-valyl)taxol, 7-(L-valyl)taxol, 2'7-di(L-valyl)taxol, 2'-(L-phenylalanyl)taxol, 7-(L-phenylalanyl)taxol, 2',7-di(L-phenylalanyl)taxol, 2'-(L-prolyl)taxol, 7-(L-prolyl)taxol, 2',7-di(L-prolyl)taxol, 2'-(L-lysyl)taxol, 7-(L-lysyl)taxol, 2',7-di(L-lysyl)taxol, 2'-(L-glutamyl)taxol, 7-(L-glutamyl)taxol, 2',7-di(L-glutamyl)taxol, 2'-(L-arginyl)taxol, 7-(L-arginyl)taxol, 2',7-di(L-arginyl)taxol), analogues with modified phenylisoserine side chains, cephalomannine, brevifoliol, yunantaxusin and taxusin); debenzoyl-2-acyl paclitaxel derivatives, benzoate paclitaxel derivatives, phosphonooxy and carbonate paclitaxel derivatives, sulfonated 2'-acryloyltaxol; sulfonated 2'-O-acyl acid paclitaxel derivatives, 18-site-substituted paclitaxel derivatives, chlorinated paclitaxel analogues, C4 methoxy ether paclitaxel derivatives, sulfenamide taxane derivatives, brominated paclitaxel analogues, Girard taxane derivatives, nitrophenyl paclitaxel, 10-deacetyl taxol B, and 10-deacetyl taxol, benzoate derivatives of taxol, 2-aroyl-4-acyl paclitaxel analogues, orthro-ester paclitaxel analogues, 2-aroyl-4-acyl paclitaxel analogues and 1-deoxy paclitaxel and 1-deoxy paclitaxel analogues.

In one aspect, the cell cycle inhibitor is a taxane having the formula (C1): 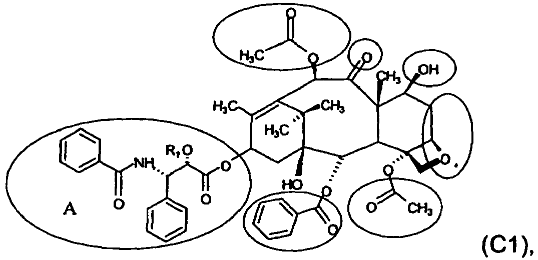 where the gray-highlighted portions may be substituted and the non-highlighted portion is the taxane core. A side-chain (labeled "A" in the diagram) is desirably present in order for the compound to have good activity as a Cell Cycle Inhibitor. Examples of compounds having this structure include paclitaxel (Merck Index entry 7117), docetaxol (TAXOTERE, Merck Index entry 3458), and 3'-desphenyl-3'-(4-ntirophenyl)-N-debenzoyl-N-(t-butoxycarbonyl)-10-deacetyltaxol.

In one aspect, suitable taxanes such as paclitaxel and its analogues and derivatives are disclosed in U.S. Patent No. 5,440,056 as having the structure (C2): 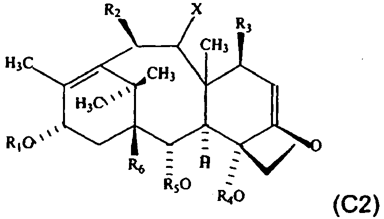 wherein X may be oxygen (paclitaxel), hydrogen (9-deoxy derivatives), thioacyl, or dihydroxyl precursors; R¹ is selected from paclitaxel or taxotere side chains or alkanoyl of the formula (C3) 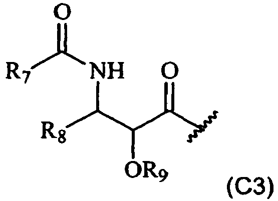 wherein R⁷ is selected from hydrogen, alkyl, phenyl, alkoxy, amino, phenoxy (substituted or unsubstituted); R₈ is selected from hydorgen, alkyl, hydroxyalkyl, alkoxyalkyl, aminoalkyl, phenyl (substituted or unsubstituted), alpha or beta-naphthyl; and R₉ is selected from hydrogen, alkanoyl, substituted alkanoyl, and aminoalkanoyl; where substitutions refer to hydroxyl, sulfhydryl, allalkoxyl, carboxyl, halogen, thioalkoxyl, N,N-dimethylamino, alkylamino, dialkylamino, nitro, and -OSO₃H, and/or may refer to groups containing such substitutions; R₂ is selected from hydrogen or oxygen-containing groups, such as hydroxyl, alkoyl, alkanoyloxy, aminoalkanoyloxy, and peptidyalkanoyloxy; R₃ is selected from hydrogen or oxygen-containing groups, such as hydroxyl, alkoyl, alkanoyloxy, aminoalkanoyloxy, and peptidyalkanoyloxy, and may further be a silyl containing group or a sulphur containing group; R₄ is selected from acyl, alkyl, alkanoyl, aminoalkanoyl, peptidylalkanoyl and aroyl; R₅ is selected from acyl, alkyl, alkanoyl, aminoalkanoyl, peptidylalkanoyl and aroyl; R₆ is selected from hydrogen or oxygen-containing groups, such as hydroxyl alkoyl, alkanoyloxy, aminoalkanoyloxy, and peptidyalkanoyloxy.

In one aspect, the paclitaxel analogues and derivatives useful as cell cycle inhibitors in the present invention are disclosed in WO 93/10076. As disclosed in this publication, the analogue or derivative should have a side chain attached to the taxane nucleus at C13, as shown in the structure below (formula C4), in order to confer antitumor activity to the taxane.

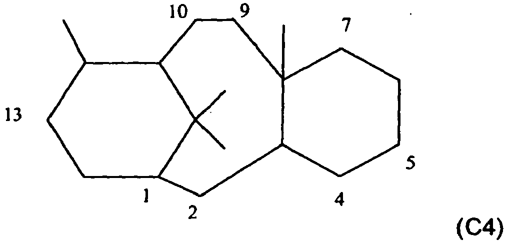

WO 93/10076 discloses that the taxane nucleus may be substituted at any position with the exception of the existing methyl groups. The substitutions may include, for example, hydrogen, alkanoyloxy, alkenoyloxy, aryloyloxy. In addition, oxo groups may be attached to carbons labeled 2, 4, 9, 10, an oxetane ring may be attached at carbons 4 and 5, and an oxirane ring may be attached to the carbon labeled 4.

In one aspect, the taxane-based cell cycle inhibitor useful in the present invention is disclosed in U.S. Patent No. 5,440,056, which discloses 9-deoxo taxanes. These are compounds lacking an oxo group at the carbon labeled 9 in the taxane structure shown above (formula C4). The taxane ring may be substituted at the carbons labeled 1, 7 and 10 (independently) with H, OH, O-R, or O-CO-R where R is an alkyl or an aminoalkyl. As well, it may be substituted at carbons labeled 2 and 4 (independently) with aryol, alkanoyl, aminoalkanoyl or alkyl groups. The side chain of formula (C3) may be substituted at R₇ and R₈ (independently) with phenyl rings, substituted phenyl rings, linear alkanes/alkenes, and groups containing H, O or N. R₉ may be substituted with H, or a substituted or unsubstituted alkanoyl group.

Taxanes in general, and paclitaxel is particular, are considered to function as a cell cycle inhibitor by acting as an anti-microtubule agent, and more specifically as a microtubule stabilizer.

In another aspect, the cell cycle inhibitor is a vinca alkaloid. Vinca alkaloids have the following general structure. They are indole-dihydroindole dimers.

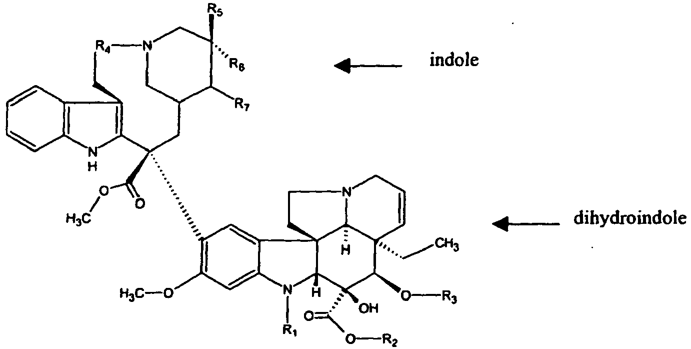

As disclosed in U.S. Patent Nos. 4,841,045 and 5,030,620, R¹ can be a formyl or methyl group or alternately H. R¹ could also be an alkyl group or an aldehyde-substituted alkyl (*e.g*., CH₂CHO). R₂ is typically a CH₃ or NH₂ group. However it can be alternately substituted with a lower alkyl ester or the ester linking to the dihydroindole core may be substituted with C(O)-R where R is NH₂, an amino acid ester or a peptide ester. R₃ is typically C(O)CH₃, CH₃ or H. Alternately a protein fragment may be linked by a bifunctional group such as maleoyl amino acid. R₃ could also be substituted to form an alkyl ester which may be further substituted. R₄ may be -CH₂- or a single bond. R₅ and R₆ may be H, OH, or a lower alkyl, typically -CH₂CH₃. Alternatively R₆ and R₇ may together form an oxetane ring. R₇ may alternately be H. Further substitutions include molecules wherein methyl groups are substituted with other alkyl groups, and whereby unsaturated rings may be derivatized by the addition of a side group such as an alkane, alkene, alkyne, halogen, ester, amide or amino group.

Exemplary vinca alkaloids are vinblastine, vincristine, vindesine, and vinorelbine, having the structures: 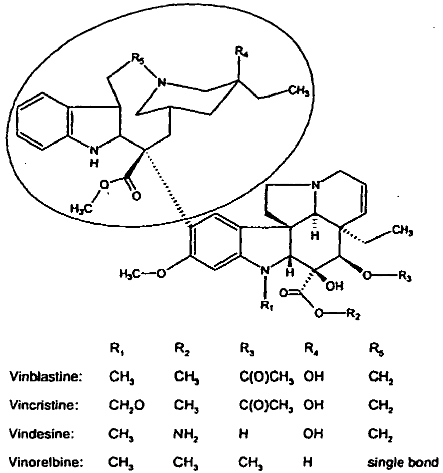 Also included is vincristine sulfate.

Analogues typically require the side group (shaded area) in order to have activity. Other suitable analogues include N-substituted vindesine sulfates (J. Med. Chem. 22(4):391-400, 1979). These compounds are thought to act as cell cycle inhibitors by functioning as anti-microtubule agents, and more specifically to inhibit polymerization. In another aspect, the cell cycle inhibitor is camptothecin, or an analogue or derivative thereof. Camptothecins have the following general structure. These compounds are thought to function as cell cycle inhibitors by being topoisomerase II Inhibitors and/or by DNA cleaving agents.

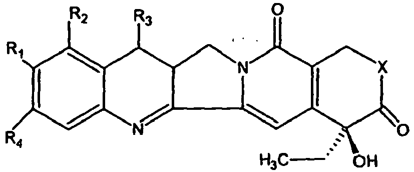

In this structure, X is typically O, but can be other groups, *e.g*., NH in the case of 21-lactam derivatives. R¹ is typically H or OH, but may be other groups, *e.g*., a terminally hydroxylated C1-3 alkane. R₂ is typically H or an amino containing group such as (CH₃)₂NHCH₂, but may be other groups *e.g*., NO₂, NH₂, halogen (as disclosed in, *e.g*., U.S. Patent No. Patent 5,552,156) or a short alkane that contains these groups. R₃ is typically H or a short alkyl such as C₂H₅. R₄ is typically H but may be other groups, *e.g*., a methylenedioxy group with R₁.

Exemplary camptothecin compounds include topotecan, irinotecan (CPT-11), 9-aminocamptothecin, 21-lactam-20(S)-camptothecin, 10,11-methylenedioxycamptothecin, SN-38, 9-nitrocamptothecin, 10-hydroxycamptothecin. Exemplary compounds have the structures:

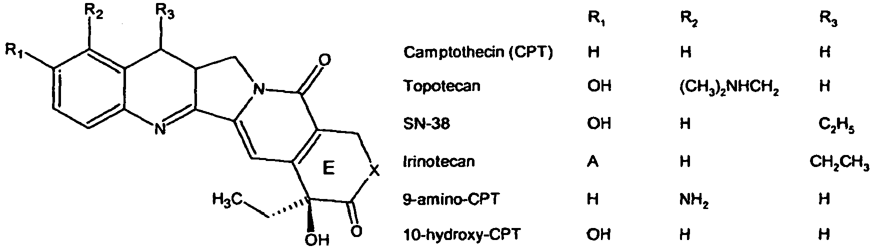

Camptothecins have the five rings shown here. The ring labeled E must be intact (the lactone rather than carboxylate form) for maximum activity and minimum toxicity.

In another aspect, the cell cycle Inhibitor is a podophyllotoxin, or a derivative or an analogue thereof. Exemplary compounds of this type are etoposide or teniposide, which have the following structures:

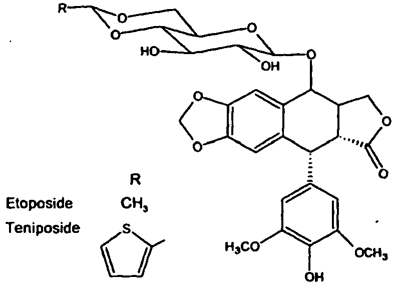

Other exemplary compounds of this type are etoposide analogues and derivatives including Cu(II)-VP-16 (etoposide) complex (Bioorg. Med. Chem. 6:1003-1008, 1998), pyrrolecarboxamidino-bearing etoposide analogues (Bioorg. Med. Chem. Lett. 7:607-612, 1997), 4β-amino etoposide analogues (Hu, University of North Carolina Dissertation, 1992), γ-lactone ring-modified arylamino etoposide analogues (J. Med. Chem. 37:287-92, 1994), N-glucosyl etoposide analogue (Tetrahedron Lett. 34:7313-16, 1993), etoposide A-ring analogues (Bioorg. Med. Chem. Lett. 2:17-22, 1992), 4'-deshydroxy-4'-methyl etoposide (Bioorg. Med. Chem. Lett. 2(10):1213-18, 1992), pendulum ring etoposide analogues (Eur. J. Cancer 26:590-3, 1990) and E-ring desoxy etoposide analogues (J. Med. Chem. 32:1418-20, 1989).

In another aspect, the cell cycle inhibitor is an anthracycline. Anthracyclines have the following general structure, where the R groups may be a variety of organic groups:

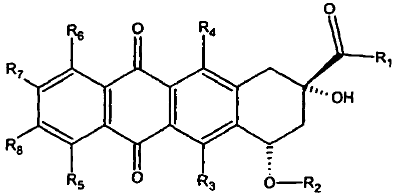

According to U.S. Patent No. 5,594,158, suitable R groups are: R₁ is CH₃ or CH₂OH; R₂ is daunosamine or H; R₃ and R₄ are independently one of OH, NO₂, NH₂, F, Cl, Br, I, CN, H or groups derived from these; R₅₋₇ are all H or R₅ and R₆ are H and R₇ and R₈ are alkyl or halogen, or vice versa: R₇ and R₈ are H and R₅ and R₆ are alkyl or halogen.

According to U.S. Patent No. 5,843,903, R₂ may be a conjugated peptide. According to U.S. Patent Nos. 4,215,062 and 4,296,105, R₅ may be OH or an ether linked alkyl group. R₁ may also be linked to the anthracycline ring by a group other than C(O), such as an alkyl or branched alkyl group having the C(O) linking moiety at its end, such as -CH₂CH(CH₂-X)C(O)-R₁, wherein X is H or an alkyl group (*e.g*., U.S. Patent No. 4,215,062). R₂ may alternately be a group linked by the functional group =N-NHC(O)-Y, where Y is a group such as a phenyl or substituted phenyl ring. Alternately, R₃ may have the following structure: 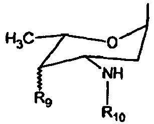 in which R₉ is OH either in or out of the plane of the ring, or is a second sugar moiety such as R₃. R₁₀ may be H or form a secondary amine with a group such as an aromatic group, saturated or partially saturated 5 or 6 membered heterocyclic having at least one ring nitrogen (U.S. Patent No. 5,843,903). When R₉ is OH and R₁₀ is H R₃ is called daunosamine. Alternately, R₁₀ may be derived from an amino acid, having the structure-C(O)CH(NHR₁₁)(R₁₂), in which R₁₁ is H, or forms a C³₋₄ membered alkylene with R₁₂. R₁₂ may be H, alkyl, aminoalkyl, amino, hydroxy, mercapto, phenyl, benzyl or methylthio (U.S. Patent No. 4,296,105).

Exemplary anthracycline are doxorubicin, daunorubicin, idarubicin, epirubicin, pirarubicin, zorubicin, and carubicin. Suitable compounds have the structures:

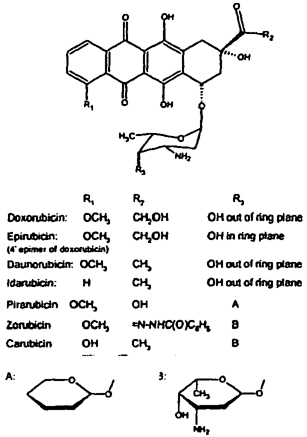

Other suitable anthracyclines are anthramycin, mitoxantrone, menogaril, nogalamycin, aclacinomycin A, olivomycin A, chromomycin A³, and plicamycin having the structures:

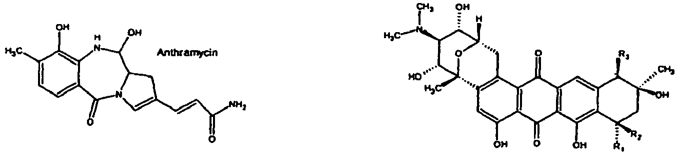

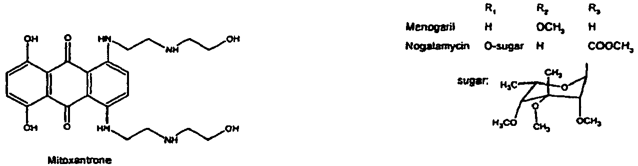

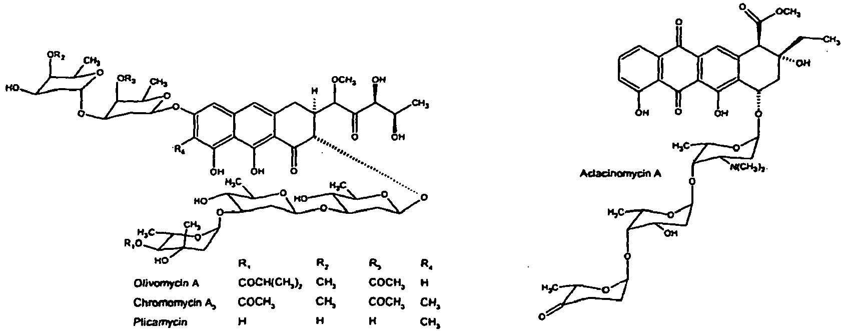

Yet other suitable anthracyclines include doxorubicin analogues and derivatives including annamycin (J. Pharm. Sci. 82:1151-1154, 1993), ruboxyl (J. Controlled Release 58:153-162, 1999), anthracycline disaccharide doxorubicin analogue (Clin. Cancer Res. 4:2833-2839, 1998), N-(trifluoroacetyl)doxorubicin and 4'-O-acetyl-N-(trifluoroacetyl)doxorubicin (Synth. Commun. 28:1109-1116, 1998), 4-demethoxy-3'-N-trifluoroacetyldoxorubicin (Drug Des. Delivery 6:123-9, 1990), 2-pyrrolinodoxorubicin (Proc. Nat'l Acad. Sci. USA. 95:1794-1799, 1998), 4-demethoxy-7-O-[2,6-dideoxy-4-O-(2,3,6-trideoxy-3-amino-α-L-lyxo-hexopyranosyl)-α-L-lyxo-hexopyranosyl]-adriamicinone doxorubicin disaccharide analogue (Carbohydr. Res. 300:11-16, 1997), piperidinyl and morpholinyl doxorubicin analogues (including FCE23762) (Cancer Chemother. Pharmacol. 38:210-216, 1996; Cancer Chemother. Pharmacol. 33:10-16, 1993; J. Nat'l Cancer Inst. 80(16):1294-8, 1988; EP 434960; Br. J. Cancer 65:703-7, 1992; 4,301,277; 4,314,054; 4,301,277; 4,585,859), enaminomalonyl-β-alanine doxorubicin derivatives (Tetrahedron Lett. 36:1413-16, 1995), cephalosporin doxorubicin derivatives (J. Med. Chem. 38:1380-5, 1995), hydroxyrubicin (Int. J. Cancer 58:85-94, 1994), (6-maleimidocaproyl)hydrazone doxorubicin derivative (Bioconjugate Chem. 4:521-7, 1993), N-(5,5-diacetoxypent-1-yl) doxorubicin (J. Med. Chem. 35:3208-14, 1992), N-hydroxysuccinimide ester doxorubicin derivatives (Biochim. Biophys. Acta 1118:83-90, 1991), polydeoxynucleotide doxorubicin derivatives (Biochim. Biophys. Acta 1129:294-302, 1991), mitoxantrone doxorubicin analogue (J. Med. Chem. 34:2373-80. 1991), AD198 doxorubicin analogue (Cancer Res. 51:3682-9, 1991), deoxydihydroiodoxorubicin (EP 275966), adriblastin (Vestn. Mosk. Univ., 16(Biol. 1):21-7, 1988), 4-demethyoxy-4'-o-methyldoxorubicin (Proc. Int. Congr. Chemother. 16:285-70-285-77, 1983), 3'-deamino-3'-hydroxydoxorubicin (Antibiot. 37:853-8, 1984), 4-demethyoxy doxorubicin analogues (Drugs Exp. Clin. Res. 10:85-90, 1984), N-L-leucyl doxorubicin derivatives (Proc. Int. Symp. Tumor Pharmacother., 179-81, 1983), 4'-deoxydoxorubicin and 4'-o-methyldoxorubicin (Int. J. Cancer 27:5-13, 1981), aglycone doxorubicin derivatives (J. Pharm. Sci. 67:1748-52, 1978), 4'-deoxy-13(S)-dihydro-4'-iododoxorubicin (EP 275966), and 4'-epidoxorubicin (Pol. J. Pharmacol. Pharm. 40:159-65, 1988; Weenen et al., Eur. J. Cancer Clin. Oncol. 20(7):919-26, 1984). These compounds are thought to function as cell cycle inhibitors by being topoisomerase inhibitors and/or by DNA cleaving agents.

In another aspect, the cell cycle inhibitor is a platinum compound. Platinum compounds are thought to function as cell cycle inhibitor by binding to DNA, *i.e*., acting as alkylating agents of DNA. In general, suitable platinum complexes may be of Pt(II) or Pt(IV) and have this basic structure: 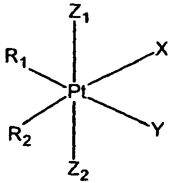 wherein X and Y are anionic leaving groups such as sulfate, phosphate, carboxylate, and halogen; R₁ and R₂ are alkyl, amine, amino alkyl any may be further substituted, and are basically inert or bridging groups. For Pt(II) complexes Z₁ and Z₂ are non-existent. For Pt(IV) Z₁ and Z₂ may be anionic groups such as halogen, hydroxy, carboxylate, ester, sulfate or phosphate (*e.g*., U.S. Patent Nos. 4,588,831 and 4,250,189).

Suitable platinum complexes may contain multiple Pt atoms (*e.g*., U.S. Patent Nos. 5,409,915 and 5,380,897). For example, bisplatinum and triplatinum complexes of the type:

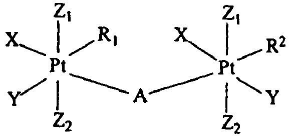

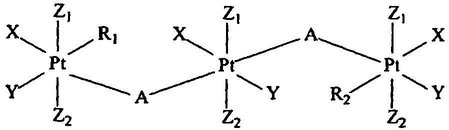

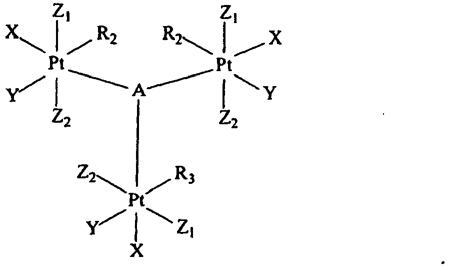

Exemplary platinum compounds are cisplatin, carboplatin, oxaliplatin, and miboplatin having the structures:

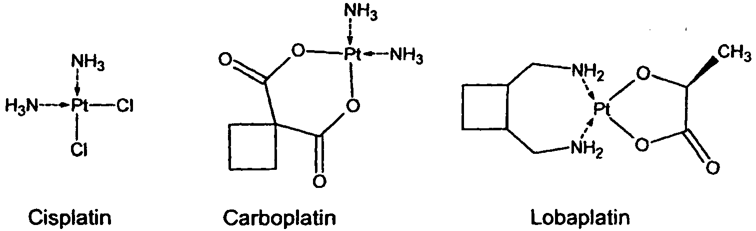

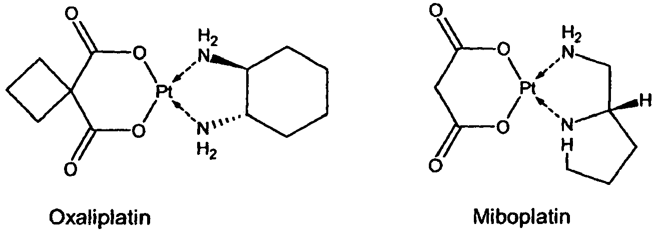

Other exemplary platinum compounds are (CPA)₂Pt[DOLYM] and (DACH)Pt[DOLYM] cisplatin (Arch. Pharmacal Res. 22:151-156, 1999), Cis-[PtCl₂(4,7-H-5-methyl-7-oxo]1,2,4[triazolo[1,5-a]pyrimidine)2] (J. Med. Chem. 41:332-338,1998), [Pt(cis-1,4-DACH)(trans-CI2)(CBDCA)] • ½MeOH cisplatin (Inorg. Chem. 36:5969-5971, 1997), 4-pyridoxate diammine hydroxy platinum (Pharm. Sci. 3:353-356, 1997), Pt(II) • • • Pt(II) (Pt₂ [NHCHN(C(CH₂)(CH₃))]₄) (Inorg. Chem. 35:7829-7835, 1996), 254-S cisplatin analogue (Neurol. Res. 18:244-247, 1996), o-phenylenediamine ligand bearing cisplatin analogues (J. Inorg. Biochem. 62:281-298, 1996), trans,cis-[Pt(OA_{C})212(en)] (J. Med. Chem. 39:2499-2507, 1996), estrogenic 1,2-diarylethylenediamine ligand (with sulfur-containing amino acids and glutathione) bearing cisplatin analogues (J. Inorg. Biochem. 62:75, 1996), cis-1,4-diaminocyclohexane cisplatin analogues (J. Inorg. Biochem. 61:291-301, 1996), 5' orientational isomer of cis-[Pt(NH³)(4-aminoTEMP-O){d(GpG)}] (J. Am. Chem. Soc. 117:10702-12, 1995), chelating diamine-bearing cisplatin analogues (J. Pharm. Sci. 84:819-23, 1995), 1,2-diarylethyleneamine ligand-bearing cisplatin analogues (J. Cancer Res. Clin. Oncol. 121:31-8, 1995), (ethylenediamine)platinum(II) complexes (J. Chem. Soc., Dalton Trans. 4:579-85, 1995), CI-973 cisplatin analogue (Int. J. Oncol. 5:597-602, 1994), cis-diamminedichloroplatinum(II) and its analogues cis-1,1-cyclobutanedicarbosylato(2R)-2-methyl-1,4-butanediam-mineplatinum(II) and cis-diammine(glycolato)platinum (J. Inorg. Biochem., 26:257-67, 1986; Cancer Res. 48:3135-9, 1988), cis-amine-cydohexylamine-dichloroplatinum(II) (Biochem. Pharmacol. 48:793-9, 1994), gem-diphosphonate cisplatin analogues (FR 2683529), (meso-1,2-bis(2,6-dichloro-4-hydroxyplenyl)ethylenediamine) dichloroplatinum(II) (J. Med. Chem. 35:4479-85, 1992), cisplatin analogues containing a tethered dansyl group (J. Am. Chem. Soc. 114:8292-3, 1992), platinum(II) polyamines (Inorg. Met.-Containing Polym. Mater., (Proc. Am. Chem. Soc. Int. Symp.), 335-61, 1990), cis-(3H)dichloro(ethylenediamine)platinum(II) (Anal. Biochem. 197:311-15, 1991), trans-diamminedichloroplatinum(II) and cis-(Pt(NH₃)2(N3-cytosine)Cl) (Biophys. Chem. 35:179-88, 1990), 3H-cis-1,2-diaminocyclohexanedichloroplatinum(II) and 3H-cis-1,2-diaminocyclohexanemalonatoplatinum (II) (Res. Commun. Chem. Pathol. Pharmacol. 64:41-58, 1989), diaminocarboxylatoplatinum (EP 296321), trans-(D,1)-1,2-diaminocyclohexane carrier ligand-bearing platinum analogues (J. Labelled Compd. Radiopharm. 25:349-57, 1988), aminoalkylaminoanthraquinone-derived cisplatin analogues (Eur. J. Med. Chem. 23:381-3, 1988), spiroplatin, iproplatin, bidentate tertiary diamine-containing cisplatinum derivatives (Inorg. Chim. Acta 152:125-34, 1988), cis-diammine(1,1-cyclobutanedicarboxylato-)platinum(II) ethylenediammine-malonatoplatinum(II) (JM40) (Radiother. Oncol. 9:157-65, 1987), JM8 and JM9 cisplatin analogues (Int. J. Androl. 10(1); 139-45, 1987), (NPr₄)₂((PtCl₄)-cis-(PtCl₂-(NH2Me)2)) (J. Chem. Soc., Chem. Commun. 6:443-5, 1987), aliphatic tricarboxylic acid platinum complexes (EP 185225), cis-dichloro(amino acid)(tert-butylamine)platinum(II) complexes (Inorg. Chim. Acta 107(4):259-67, 1985).

In another aspect, the cell cycle inhibitor is a nitrosourea. Nitrosoureas have the following general structure (C5), where typical R groups are shown below.

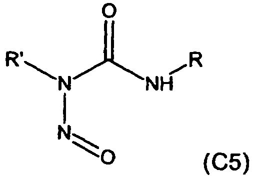

R Group:

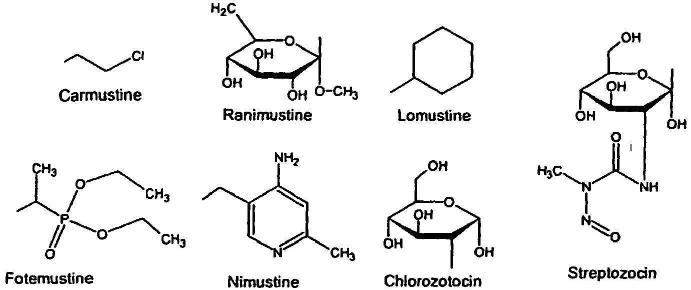

Other suitable R groups include cyclic alkanes, alkanes, halogen substituted groups, sugars, aryl and heteroaryl groups, phosphonyl and sulfonyl groups. As disclosed in U.S. Patent No. 4,367,239, R may suitably be CH₂-C(X)(Y)(Z), wherein X and Y may be the same or different members of the following groups: phenyl, cyclohexyl, or a phenyl or cyclohexyl group substituted with groups such as halogen, lower alkyl (C₁₋₄), trifluore methyl, cyano, phenyl, cyclohexyl, lower alkyloxy (C₁₋₄). Z has the following structure: -alkylene-N-R₁R₂, where R₁ and R₂ may be the same or different members of the following group: lower alkyl (C₁₋₄) and benzyl, or together R₁ and R₂ may form a saturated 5 or 6 membered heterocyclic such as pyrrolidine, piperidine, morfoline, thiomorfoline, N-lower alkyl piperazine, where the heterocyclic may be optionally substituted with lower alkyl groups.

As disclosed in U.S. Patent No. 6,096,923, R and R' of formula (C5) may be the same or different, where each may be a substituted or unsubstituted hydrocarbon having 1-10 carbons. Substitutions may include hydrocarbyl, halo, ester, amide, carboxylic acid, ether, thioether and alcohol groups. As disclosed in U.S. Patent No. 4,472,379, R of formula (C5) may be an amide bond and a pyranose structure (e.g., Methyl 2'-[N-[N-(2-chloroethyl)-N-nitroso-carbamoyl]-glycyl]amino-2'-deoxy-α-D-glucopyranoside). As disclosed in U.S. Patent No. 4,150,146, R of formula (C5) may be an alkyl group of 2 to 6 carbons and may be substituted with an ester, sulfonyl, or hydroxyl group. It may also be substituted with a carboxylic acid or CONH₂ group.

Yet other suitable nitrosoureas are exemplified by the following analogues and derivatives. 6-bromo and 6-chloro-2,3-dihydro-1,4-benzothiazines nitrosourea derivatives (Heterocycl. Commun. 2:587-592, 1996), diamino acid nitrosourea derivatives (Bioorg. Med. Chem. Lett. 4:2697-700, 1994; Bioorg. Med. Chem. 3:151-60, 1995), amino acid nitrosourea derivatives (Pharmazie 50:25-6, 1995), 3',4'-didemethoxy-3',4'-dioxo-4-deoxypodophyllotoxin nitrosourea derivatives (Heterocycles 39(1):361-9, 1994), ACNU (Immunopharmacology 23:199-204,1992), tertiary phosphine oxide nitrosourea derivatives (Pharmazie 46:603, 1991), sulfamerizine and sulfamethizole nitrosourea derivatives (Zhonghua Yaozue Zazhi 43:401-6, 1991), thymidine nitrosourea analogues (Cancer Commun. 3:119-26, 1991), 1,3-bis(2-chloroethyl)-1-nitrosourea (Cancer Res. 51:1586-90, 1991), 2,2,6,6-tetramethyl-1-oxopiperidiunium nitrosourea derivatives (USSR 1261253), 2-and 4-deoxy sugar nitrosourea derivatives (US 4,902,791), nitroxyl nitrosourea derivatives (USSR 1336489), pyrimidine (II) nitrosourea derivatives (Chung-hua Yao Hsueh Tsa Chih 41:19-26, 1989), 5-halogenocytosine nitrosourea derivatives (T'ai-wan Yao Hsueh Tsa Chih 38:37-43, 1986), 1-(2-chloroethyl)-3-isobutyl-3-(β-maltosyl)-1-nitrosourea (J. Pharmacobio-Dyn. 10:341-5, 1987), sulfur-containing nitrosoureas (Yaoxue Xuebao 21:502-9, 1986), 6-((((2-chloroethyl)nitrosoamino-)carbonyl)amino)-6-deoxysucrose (NS-1C) and 6'-((((2-chloroethyl)nitrosoamino)carbonyl)amino)-6'-deoxysucrose (NS-1 D) nitrosourea derivatives (Chemotherapy (Tokyo) 33:969-77, 1985), (JP 84219300), CNCC, RFCNU, chlorozotocin (Chemotherapy (Basel) 32:131-7, 1986), CNUA (Chemotherapy (Tokyo) 33:455-61, 1985), 1-(2-chloroethyl)-3-isobutyl-3-(β-maltosyl)-1-nitrosourea (Jpn. J. Cancer Res. (Gann) 76:651-6, 1985), choline-like nitrosoalkylureas (Izv. Akad. NAUK SSSR, Ser. Khim. 3:553-7, 1985), sulfa drug nitrosourea analogues (Proc. Nat'l Sci. Counc., Repub. China, Part A 8(1):18-22, 1984), DONU (J. Jpn. Soc. Cancer Ther. 17:2035-43, 1982), dimethylnitrosourea (Izv. Akad. NAUK SSSR, Ser. Biol. 3:439-45, 1984), GANU (Cancer Chemother. Pharmacol. 10(3):167-9, 1983), 5-aminomethyl-2'-deoxyuridine nitrosourea analogues (Shih Ta Hsueh Pao (Taipei) 27:681-9, 1982), TA-077 (Cancer Chemother. Pharmacol. 9:134-9, 1982), gentianose nitrosourea derivatives (JP 82 80396), thiocolchicine nitrosourea analogues (Shih Ta Hsueh Pao (Taipei) 25:355-62, 1980; J. Med. Chem. 23:1440-2, 1980), 2-chloroethyl-nitrosourea (Oncology 38:39-42, 1981), pyridine and piperidine nitrosourea derivatives (J. Med. Chem. 23:848-51, 1980), phensuzimide nitrosourea derivatives (J. Med. Chem. 23:324-6, 1980), ergoline nitrosourea derivatives (J. Med. Chem. 22:32-5, 1979), glucopyranose nitrosourea derivatives (JP 7895917), 1-(2-chloroethyl)-3-cyclohexyl-1-nitrosourea (J. Med. Chem. 21:514-20, 1978), 4-(3-(2-chloroethyl)-3-nitrosoureid-o)-cis-cyclohexanecarboxylic acid (Cancer Treat. Rep. 61:J1513-18, 1977), IOB-252 (Rev. Roum. Med., Virol. 28:J 55-61, 1977), 1-tetrahydroxycyclopentyl-3-nitroso-3-(2-chloroethyl)-urea (4,039,578), d-1-1-(β-chloroethyl)-3-(2-oxo-3-hexahydroazepinyl)-1-nitrosourea (3,859,277) and gentianose nitrosourea derivatives (JP 57080396). These nitrosourea compounds are thought to function as cell cycle inhibitors by binding to DNA, that is, by functioning as DNA alkylating agents.

In another aspect, the cell cycle inhibitor is a nitroimidazole, where exemplary nitroimidazoles are metronidazole, benznidazole, etanidazole, and misonidazole, having the structures:

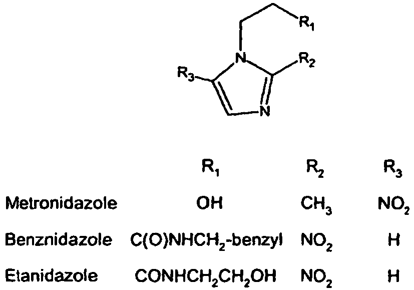

Suitable nitroimidazole compounds are disclosed in, e.g., U.S. Patent Nos. 4,371,540 and 4,462,992. Others include 5-substituted-4-nitroimidazoles (Int. J. Radiat. Biol. Relat. Stud. Phys., Chem. Med. 40:153-61, 1981), SR-2508 (Int. J. Radiat. Oncol., Biol. Phys. 7:695-703, 1981), chiral [[(2-bromoethyl)-amino]methyl]-nitro-1H-imidazole-1-ethanol (U.S. Patent Nos. 5,543,527; 4,797,397; 5,342,959), 2-nitroimidazole derivatives (U.S. Patent Nos. 4,797,397, 5,270,330, EP 0 513 351 B1), fluorine-containing nitroimidazole (U.S. Patent No. 5,304,654), fluorine containing 3-nitro-1,2,4-triazole (Publication Number 02076861 A (Japan), Mar. 31, 1988), 5-thiotretrazole derivative or its salt (Publication Number 61010511 A (Japan), Jun. 26, 1984), Publication Number 61167616 A (Japan) Jan. 22, 1985), imidazole derivatives (Publication Number 6203767 A (Japan) Aug. 1, 1985; Publication Number 62030768 A (Japan) Aug. 1, 1985; Publication Number 62030777 A (Japan) Aug. 1, 1985), 4-nitro-1,2,3-triazole (Publication Number 62039525 A (Japan), Aug. 15,1985), 3-nitro-1,2,4-triazole (Publication Number 62138427 A (Japan), Dec. 12, 1985), Publication Number 63099017 A (Japan), Nov. 21, 1986), 4,5-dinitroimidazole derivative (Publication Number 63310873 A (Japan) Jun. 9, 1987), nitrotriazole compound (Publication Number 07149737 A (Japan) Jun. 22, 1993), 4,5-dimethylmisonidazole (Biochem. Pharmacol. 43:1337-44, 1992), and azo and azoxy misonidazole derivatives (Int. J. Radiat. Biol. Relat. Stud. Phys., Chem. Med. 45:469-77, 1984).

In another aspect, the cell cycle inhibitor is a folic acid antagonist, such as methotrexate or derivatives or analogues thereof, including edatrexate, trimetrexate, raltitrexed, piritrexim, denopterin, tomudex, and pteropterin. Methotrexate analogues have the following general structure:

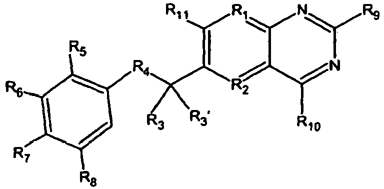

The identity of the R group may be selected from organic groups, particularly those groups set forth in U.S. Patent Nos. 5,166,149 and 5,382,582. For example, R₁ may be N, R₂ may be N or C(CH₃), R₃ and R₃' may H or alkyl, *e.g*., CH₃, R₄ may be a single bond or NR, where R is H or alkyl group. R_{5,6,8} may be H, OCH₃, or alternately they can be halogens or hydro groups. R₇ is a side chain of the general structure: 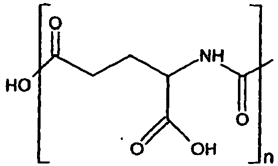 wherein n = 1 for methotrexate, n = 3 for pteropterin. The carboxyl groups in the side chain may be esterified or form a salt such as a Zn²⁺ salt. R₉ and R₁₀ can be NH₂ or may be alkyl substituted.

Exemplary folic acid antagonist compounds have the structures:

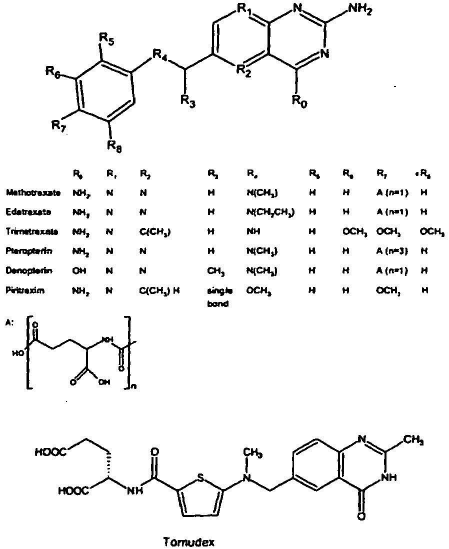

Other suitable methotrexate analogues and derivatives include indoline ring and a modified ornithine or glutamic acid-bearing methotrexate derivatives (Chem. Pharm. Bull. 45:1146-1150, 1997), alkyl-substituted benzene ring C bearing methotrexate derivatives (Chem. Pharm. Bull. 44:2287-2293, 1996), benzoxazine or benzothiazine moiety-bearing methotrexate derivatives (J. Med. Chem. 40:105-111, 1997), 10-deazaaminopterin analogues (J. Med. Chem. 40:370-376, 1997), 5-deazaaminopterin and 5,10-dideazaaminopterin methotrexate analogues (J. Med. Chem. 40:377-384, 1997), indoline moiety-bearing methotrexate derivatives (Chem. Pharm. Bull. 44:1332-1337, 1996), lipophilic amide methotrexate derivatives (World Meet. Pharm., Biopharm. Pharm. Technol., 563-4, 1995), L-threo-(2S, 4S)-4-fluoroglutamic acid and DL-3,3-difluoroglutamic acid-containing methotrexate analogues (J. Med. Chem. 39:56-65, 1996), methotrexate tetrahydroquinazoline analogue (J. Heterocycl. Chem. 32(1):243-8, 1995), N-(α-aminoacyl) methotrexate derivatives (Pteridines 3:101-2, 1992), biotin methotrexate derivatives (Pteridines 3:131-2, 1992), D-glutamic acid or D-erythro, threo-4-fluoroglutamic acid methotrexate analogues (Biochem. Pharmacol. 42:2400-3, 1991), β,γ-methano methotrexate analogues (Pteridines 2:133-9, 1991), 10-deazaaminopterin (10-EDAM) analogue (Chem. Biol. Pteridines, Proc. Int. Symp. Pteridines Folic Acid Deriv., 1027-30, 1989), γ-tetrazole methotrexate analogue (Chem. Biol. Pteridines, Proc. Int. Symp. Pteridines Folic Acid Deriv., 1154-7, 1989), N-(L-α-aminoacyl) methotrexate derivatives (Heterocycles 28:751-8, 1989), meta and ortho isomers of aminopterin (J. Med. Chem. 32:2582, 1989), hydroxymethylmethotrexate (DE 267495), γ-fluoromethotrexate (Cancer Res. 49:4517-25, 1989), gem-diphosphonate methotrexate analogues (WO 88/06158), α- and γ-substituted methotrexate analogues (Tetrahedron 44:5375-87, 1988), 5-methyl-5-deaza methotrexate analogues (4,725,687), Nδ-acyl-Nα-(4-amino-4-deoxypteroyl)-L-omithine derivatives (J. Med. Chem. 31:1332-7, 1988), 8-deaza methotrexate analogues Cancer Res. 48:1481-8, 1988), acivicin methotrexate analogue (J. Med. Chem. 30:1463-9, 1987), polymeric platinol methotrexate derivative (Polym. Sci. Technol. (Plenum), 35(Adv. Biomed. Polym.):311-24, 1987), methotrexate-γ-dimyristoylphophatidylethanolamine (Biochim. Biophys. Acta 917:211-18, 1987), deoxyuridylate methotrexate derivatives (Chem. Biol. Pteridines, Pteridines Folid Acid Deriv., Proc. Int. Symp. Pteridines Folid Acid Deriv.: Chem., Biol. Clin. Aspects: 659-62, 1986), iodoacetyl lysine methotrexate analogue (Chem. Biol. Pteridines, Pteridines Folid Acid Deriv., Proc. Int. Symp. Pteridines Folid Acid Deriv.: Chem., Biol. Clin. Aspects: 807-9, 1986), 2,.omega.-diaminoalkanoid acid-containing methotrexate analogues (Biochem. Pharmacol. 35:2607-13, 1986), quinazoline methotrexate analogue (J. Med. Chem. 29:155-8, 1986), pyrazine methotrexate analogue (J. Heterocycl. Chem. 22:5-6, 1985), cysteic acid and homocysteic acid methotrexate analogues (4,490,529), γ-tert-butyl methotrexate esters (J. Med. Chem. 28:660-7, 1985), fluorinated methotrexate analogues (Heterocycles 23:45-9, 1985), folate methotrexate analogue (J. Bacteriol. 160:849-53, 1984), poly (L-lysine) methotrexate conjugates (J. Med. Chem. 27:888-93, 1984), dilysine and trilysine methotrexate derivates (J. Org. Chem. 49:1305-9, 1984), 7-hydroxymethotrexate (Cancer Res. 43:4648-52, 1983), 3',5'-dichloromethotrexate (J. Med. Chem. 26(10):1448-52, 1983), diazoketone and chloromethylketone methotrexate analogues (J. Pharm. Sci. 71:717-19, 1982), 10-propargylaminopterin and alkyl methotrexate homologs (J. Med. Chem. 25:877-80, 1982), lectin derivatives of methotrexate (JNCI 66:523-8, 1981), methotrexate polyglutamate analogues (Proc. Int. Symp. Pteridines Folid Acid Deriv.: Chem., Biol. Clin. Aspects: 985-8, 1986; Mol. Pharmacol. 17:105-10, 1980; Adv. Exp. Med. Biol., 163(Folyl Antifolyl Polyglutamates):95-100, 1983; Methods Enzymol. 122 (Vitam. Coenzymes, Pt. G):339-46, 1986; Proc. Int. Symp. Pteridines Folid Acid Deriv.: Chem., Biol. Clin. Aspects: 989-92, 1986; Cancer Res. 46(10):5020-3, 1986), phosphonoglutamic acid analogues (Eur. J. Med. Chem.-Chim. Ther. 19:267-73, 1984), halogenated methotrexate derivatives (JNCI 58:J955-8, 1977), 8-alkyl-7,8-dihydro analogues (J. Med. Chem. 20:J1323-7, 1977), 7-methyl methotrexate derivatives and dichloromethotrexate (J. Med. Chem. 17(12):J1308-11, 1974), lipophilic methotrexate derivatives and 3',5'-dichloromethotrexate (J. Med. Chem. 16:J1190-3, 1973), deaza amethopterin analogues (Ann. N.Y. Acad. Sci. 186:J227-34, 1971), and cysteic acid and homocysteic acid methotrexate analogues (EP 0142220).

These compounds are thought to function as cell cycle inhibitors by serving as antimetabolites of folic acid.

In another aspect, the cell cycle inhibitor is a cytidine analogue, such as cytarabine or derivatives or analogues thereof, including enocitabine, FMdC ((E(-2'-deoxy-2'-(fluoromethylene)cytidine), gemcitabine, 5-azacitidine, ancitabine, and 6-azauridine. Exemplary compounds have the structures:

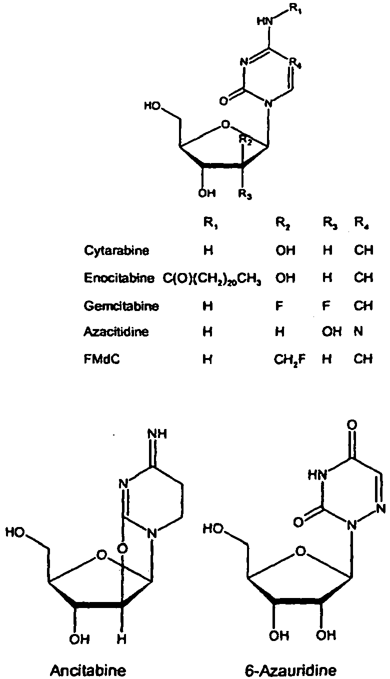

These compounds are thought to function as cell cycle inhibitors as acting as antimetabolites of pyrimidine.

In another aspect, the cell cycle inhibitor is a pyrimidine analogue. In one aspect, the pyrimidine analogues have the general structure: 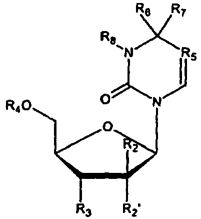 wherein positions 2', 3' and 5' on the sugar ring (R₂, R₃ and R₄, respectively) can be H, hydroxyl, phosphoryl (*e.g*., U.S. Patent No. 4,086,417) or ester (*e.g*., U.S. Patent No. 3,894,000). Esters can be of alkyl, cycloalkyl, aryl or heterocyclo/aryl types. The 2' carbon can be hydroxylated at either R₂ or R₂', the other group is H. Alternately, the 2' carbon can be substituted with halogens, *e.g*., fluoro or difluoro cytidines such as Gemcytabine. Alternately, the sugar can be substituted for another heterocyclic group such as a furyl group or for an alkane, an alkyl ether or an amide linked alkane such as C(O)NH(CH₂)₅CH₃. The 2° amine can be substituted with an aliphatic acyl (R₁) linked with an amide (*e.g*., U.S. Patent No. 3,991,045) or urethane (*e.g*., U.S. Patent No. 3,894,000) bond. It can also be further substituted to form a quaternary ammonium salt. R₅ in the pyrimidine ring may be N or CR, where R is H, halogen containing groups, or alkyl (see, *e.g*., U.S. Patent No. 4,086,417). R₆ and R⁷ can together can form an oxo group or R₆ = -NH-R₁ and R₇ = H. R₈ is H or R₇ and R₈ together can form a double bond or R₈ can be X, where X is:

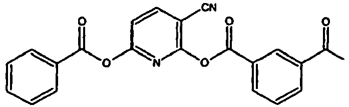

Specific pyrimidine analogues are disclosed in U.S. Patent No. 3,894,000 (*e.g*., 2'-O-palmityl-ara-cytidine, 3'-O-benzoyl-ara-cytidine, and more than 10 other examples); U.S. Patent No. 3,991,045 (*e.g*., N4-acyl-1-β-D-arabinofuranosylcytosine, and numerous acyl groups derivatives as listed therein, such as palmitoyl.

In another aspect, the cell cycle inhibitor is a fluoro-pyrimidine analogue, such as 5-fluorouracil, or an analogues or derivative thereof, including carmofur, doxifluridine, emitefur, tegafur, and floxuridine. Exemplary compounds have the structures:

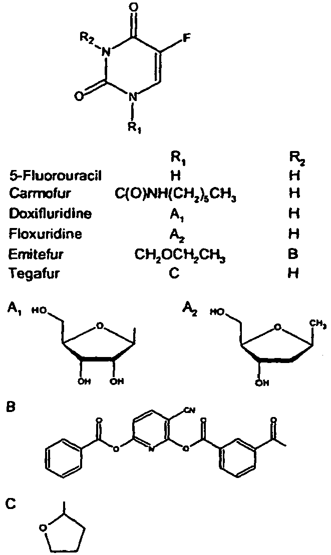

Other suitable fluoropyrimidine analogues include 5-FudR (5-fluoro-deoxyuridine), or an analogues or derivative thereof, including 5-iododeoxyuridine (5-ludR), 5-bromodeoxyuridine (5-BudR), fluorouridine triphosphate (5-FUTP), and fluorodeoxyuridine monophosphate (5-dFUMP). Exemplary compounds have the structures:

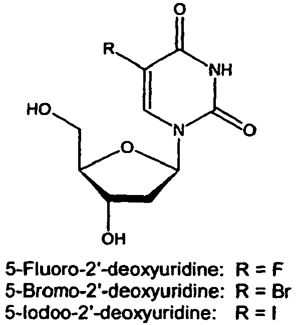

Yet other suitable fluoropyrimidine analogues include DUdR, 5-CldC, (d)H4U or 5-halo-2'-halo-2'-deoxy-cytidine or -uridine derivatives (U.S. Patent No. 4,894,364), N3-alkylated analogues of 5-fluorouracil (J. Chem. Soc., Perkin Trans. 1:3145-3146, 1998), 5-fluorouracil derivatives with 1,4-oxaheteroepane moieties (Tetrahedron 54:13295-13312, 1998), 5-fluorouracil and nucleoside analogues (Anticancer Res. 17:21-27, 1997), cis- and trans-5-fluoro-5,6-dihydro-6-alkoxyuracil (Br. J. Cancer 68:702-7, 1993), cyclopentane 5-fluorouracil analogues (Can. J. Chem. 70:1162-9, 1992), A-OT-fluorouracil (Zongguo Yiyao Gongye Zazhi 20:513-15, 1989), N4-trimethoxybenzoyl-5'-deoxy-5-fluorocytidine and 5'-deoxy-5-fluorouridine (Chem. Pharm. Bull. 38:998-1003, 1990), 1-hexylcarbamoyl-5-fluorouracil (J. Pharmacobio-Dun. 3:478-81, 1980; Maehara et al., Chemotherapy (Basel) 34:484-9, 1988), uracil-1-(2-tetrahydrofuryl)-5-fluorouracil (Oncology 45:144-7, 1988), 1-(2'-deoxy-2'-fluoro-β-D-arabinofuranosyl)-5-fluorouracil (Mol. Pharmacol. 31:301-6, 1987), doxifluridine (Oyo Yakuri 29:803-31, 1985), 5'-deoxy-5-fluorouridine (Eur. J. Cancer 16:427-32, 1980), 1-acetyl-3-O-toluyl-5-fluorouracil (J. Med. Sci. 28:49-66, 1979), 5-fluorouracil-m-formylbenzene-sulfonate (JP 55059173), N'-(2-furanidyl)-5-fluorouracil (JP 53149985) and 1-(2-tetrahydrofuryl)-5-fluorouracil (JP 52089680);

These compounds are thought to function as cell cycle inhibitors by serving as antimetabolites of pyrimidine.

In another aspect, the cell cycle inhibitor is a purine analogue. Purine analogues have the following general structure. 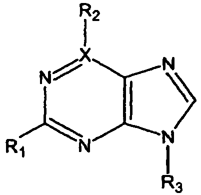 wherein X is typically carbon; R₁ is H, halogen, amine or a substituted phenyl; R₂ is H, a primary, secondary or tertiary amine, a sulfur containing group, typically -SH, an alkane, a cyclic alkane, a heterocyclic or a sugar; R₃ is H, a sugar (typically a furanose or pyranose structure), a substituted sugar or a cyclic or heterocyclic alkane or aryl group. (*e.g*., U.S. Patent No. 5,602,140) for compounds of this type.

In the case of pentostatin, X-R₂ is -CH₂CH(OH)-. In this case a second carbon atom is inserted in the ring between X and the adjacent nitrogen atom. The X-N double bond becomes a single bond.

U.S. Patent No. 5,446,139 describes suitable purine analogues of the type shown in the formula. 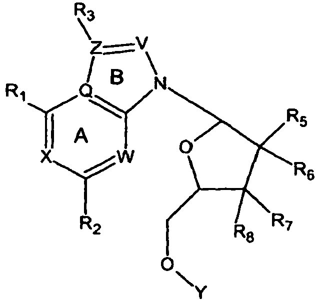 wherein N signifies nitrogen and V, W, X, Z can be either carbon or nitrogen with the following provisos. Ring A may have 0 to 3 nitrogen atoms in its structure. If two nitrogens are present in ring A, one must be in the W position. If only one is present, it must not be in the Q position. V and Q must not be simultaneously nitrogen. Z and Q must not be simultaneously nitrogen. If Z is nitrogen, R₃ is not present. Furthermore, R₁₋₃ are independently one of H, halogen, C₁₋₇ alkyl, C₁₋₇ alkenyl, hydroxyl, mercapto, C₁₋₇ alkylthio, C₁₋₇ alkoxy, C₂₋₇ alkenyloxy, aryl oxy, nitro, primary, secondary or tertiary amine containing group. R₅₋₈ are H or up to two of the positions may contain independently one of OH, halogen, cyano, azido, substituted amino, R₅ and R₇ can together form a double bond. Y is H, a C₁₋₇ alkylcarbonyl, or a mono- di or tri phosphate.

Exemplary suitable purine analogues include 6-Mercaptopurine, thiguanosine, thiamiprine, cladribine, fludaribine, tubercidin, puromycin, pentoxyfilline; where these compounds may optionally be phosphorylated. Exemplary compounds have the structures:

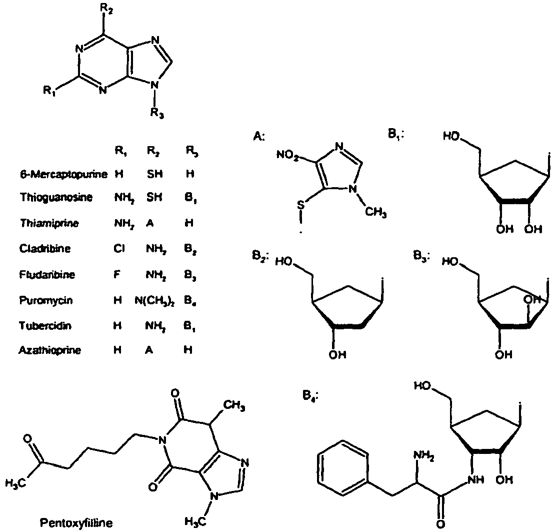

Other suitable agents of this type include mercaptopurine 6-S-aminoacyloxymethyl mercaptopurine derivatives (Chem. Pharm. Bull. 43:793-6, 1995), methyl-D-glucopyranoside mercaptopurine derivatives (Eur. J. Med. Chem. 29:149-52, 1994) and s-alkynyl mercaptopurine derivatives (Khim.-Farm. Zh. 15:65-7, 1981).

These compounds are thought to function as cell cycle inhibitors by serving as antimetabolites of purine.

In another aspect, the cell cycle inhibitor is a nitrogen mustard. Many suitable nitrogen mustards are known and are suitably used as a cell cycle inhibitor in the present invention. Suitable nitrogen mustards are also known as cyclophosphamides.

An example of a nitrogen mustard has the general structure: 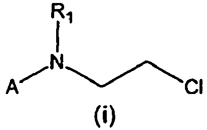 where A is: 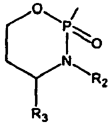 or -CH₃ or other alkane, or chlorinated alkane, typically CH₂CH(CH₃)Cl, or a polycyclic group such as B, or a substituted phenyl such as C or a heterocyclic group such as D.

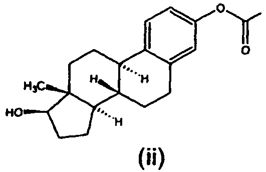

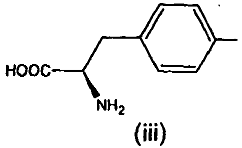

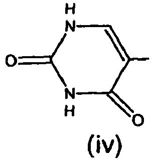

Suitable nitrogen mustards are disclosed in U.S. Patent No. 3,808,297, wherein A is:

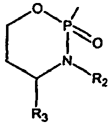

R₁₋₂ are H or CH₂CH₂Cl; R₃ is H or oxygen-containing groups such as hydroperoxy; and R₄ can be alkyl, aryl, heterocyclic.

The cyclic moiety need not be intact. U.S. Patent No. 5,472,956, 4,908,356, 4,841,085 describe the following type of structure: 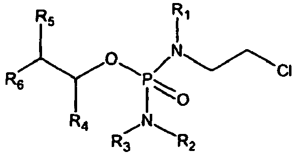 wherein R₁ is H or CH₂CH₂Cl, and R₂₋₆ are various substituent groups.

Exemplary nitrogen mustards include methylchloroethamine, and analogues or derivatives thereof, including methylchloroethamine oxide hydrochloride, novembichin, and mannomustine (a halogenated sugar). Exemplary compounds have the structures:

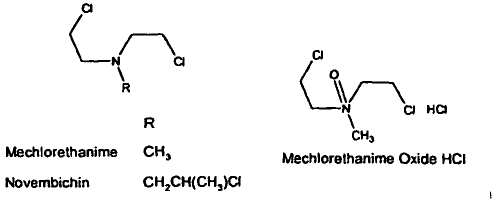

The nitrogen mustard be cyclophosphamide, Ifosfamide, perfosfamide, or torofosfamide, where these compounds have the structures:

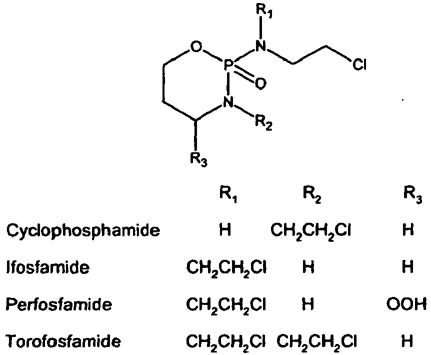

Other suitable compounds of this type are analogues or derivatives of cyclophosphamide, including 4-hydroperoxycylcophosphamide (Cancer Chemother. Pharmacol. 26:397-402, 1990), acyclouridine cyclophosphamide derivatives (Helv. Chim. Acta 73:912-15, 1990), 1,3,2-dioxa- and -oxazaphosphorinane cyclophosphamide analogues (Tetrahedron 44:6305-14, 1988), C5-substituted cyclophosphamide analogues (Spada, University of Rhode Island Dissertation, 1987), tetrahydrooxazine cyclophosphamide analogues (Valente, University of Rochester Dissertation, 1988), phenyl ketone cyclophosphamide analogues (Teratology 39:31-7, 1989), phenylketophosphamide cyclophosphamide analogues (J. Med. Chem. 29:716-27, 1986), ASTA Z-7557 cyclophosphamide analogues (Int. J. Cancer 34:883-90, 1984), 3-(1-oxy-2,2,6,6-tetramethyl-4-piperidinyl)cyclophosphamide (J. Med. Chem. 25:1106-10, 1982), 2-oxobis(2-β-chloroethylamino)-4-,6-dimethyl-1,3,2-oxazaphosphorinane cyclophosphamide (Phosphorus Sulfur 12:287-93, 1982), 5-fluoro- and 5-chlorocyclophosphamide (J. Med. Chem. 24:1399-403, 1981), cis- and trans-4-phenylcyclophosphamide (J. Med. Chem. 23:372-5, 1980), 5-bromocyclophosphamide, 3,5-dehydrocyclophosphamide (J. Med. Chem. 22:151-8, 1979), 4-ethoxycarbonyl cyclophosphamide analogues (J. Pharm. Sci. 7:709-10, 1978), arylaminotetrahydro-2H-1,3,2-oxazaphosphorine 2-oxide cyclophosphamide analogues (Arch. Pharm. (Weinheim, Ger.) 310:J,428-34, 1977), NSC-26271 cyclophosphamide analogues (Cancer Treat. Rep. 60:J381-93, 1976),benzo annulated cyclophosphamide analogues (J. Med. Chem. 18:J1251-3, 1975), 6-trifluoromethylcyclophosphamide (J. Med. Chem. 18:J1106-10, 1975), 4-methylcyclophosphamide and 6-methycyclophosphamide analogues (Biochem. Pharmacol. 24:J599-606, 1975).

The nitrogen mustard may be estramustine, or an analogue or derivative thereof, including phenesterine, prednimustine, and estramustine PO₄. Thus, suitable nitrogen mustard type cell cycle inhibitors may have the structures:

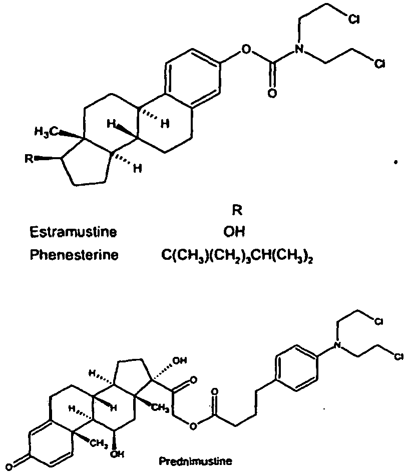

The nitrogen mustard may be chlorambucil, or an analogue or derivative thereof, including melphalan and chlormaphazine. Thus, suitable nitrogen mustard type cell cycle inhibitors may have the structures:

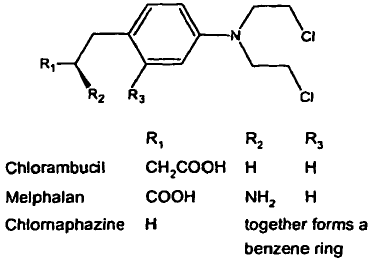

The nitrogen mustard may be uracil mustard, which has the structure:

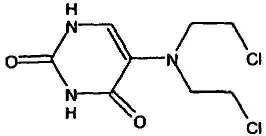

The nitrogen mustards are thought to function as cell cycle inhibitors by serving as alkylating agents for DNA.

The cell cycle inhibitor of the present invention may be a hydroxyurea. Hydroxyureas have the following general structure:

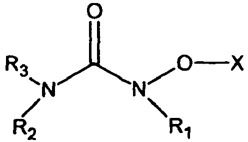

Suitable hydroxyureas are disclosed in, for example, U.S. Patent No. 6,080,874, wherein R₁ is: 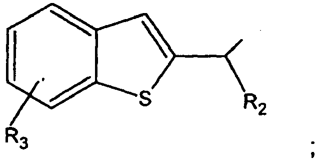 and R₁ is an alkyl group having 1-4 carbons and R₃ is one of H, acyl, methyl, ethyl, and mixtures thereof, such as a methylether.

Other suitable hydroxyureas are disclosed in, *e.g*., U.S. Patent No. 5,665,768, wherein R₁ is a cycloalkenyl group, for example, N-[3-[5-(4-fluorophenylthio)-furyl]-2-cyclopenten-1-yl]N-hydroxyurea; R₂ is H or an alkyl group having 1 to 4 carbons and R₃ is H; X is H or a cation.

Other suitable hydroxyureas are disclosed in, *e.g*., U.S. Patent No. 4,299,778, wherein R₁ is a phenyl group substituted with on or more fluorine atoms; R₂ is a cyclopropyl group; and R₃ and X is H.

Other suitable hydroxyureas are disclosed in, *e.g*., U.S. Patent No. 5,066,658, wherein R₂ and R₃ together with the adjacent nitrogen form: 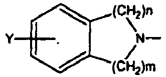 wherein m is 1 or 2, n is 0-2 and Y is an alkyl group.

In one aspect, the hydroxy urea has the structure:

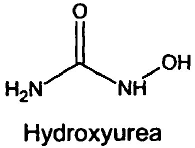

Hydroxyureas are thought to function as cell cycle inhibitors by serving to inhibit DNA synthesis.

In another aspect, the cell cycle inhibitor is a mytomicin, such as mitomycin C, or an analogue or derivative thereof, such as porphyromycin. Suitable compounds have the structures:

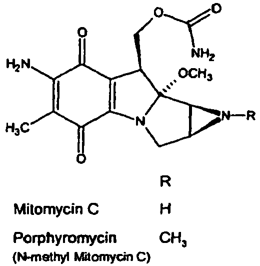

These compounds are thought to function as cell cycle inhibitors by serving as DNA alkylating agents.

In another aspect, the cell cycle inhibitor is an alkyl sulfonate, such as busulfan, or an analogue or derivative thereof, such as treosulfan, improsulfan, piposulfan, and pipobroman. Exemplary compounds have the structures:

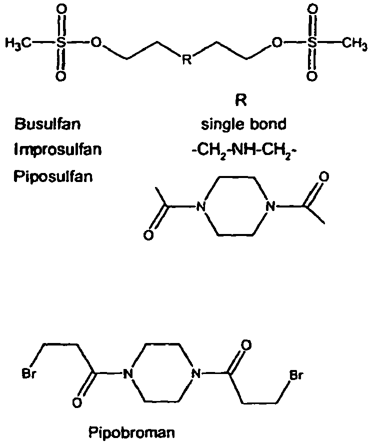

These compounds are thought to function as cell cycle inhibitors by serving as DNA alkylating agents.

In another aspect, the cell cycle inhibitor is a benzamide. In yet another aspect, the cell cycle inhibitor is a nicotinamide. These compounds have the basic structure: 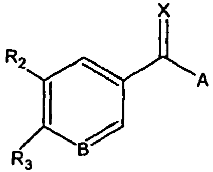 wherein X is either O or S; A is commonly NH₂ or it can be OH or an alkoxy group; B is N or C-R⁴, where R₄ is H or an ether-linked hydroxylated alkane such as OCH₂CH₂OH, the alkane may be linear or branched and may contain one or more hydroxyl groups. Alternately, B may be N-R₅ in which case the double bond in the ring involving B is a single bond. R₅ may be H, and alkyl or an aryl group (*e.g*., U.S. Patent No. 4,258,052); R₂ is H, OR₆, SR₆ or NHR₆, where R₆ is an alkyl group; and R₃ is H, a lower alkyl, an ether linked lower alkyl such as -O-Me or -O-Ethyl (*e.g*., U.S. Patent No. 5,215,738).

Suitable benzamide compounds have the structures: 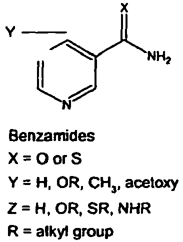 where additional compounds are disclosed in U.S. Patent No. 5,215,738, (listing some 32 compounds).

Suitable nicotinamide compounds have the structures: 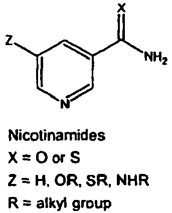 where additional compounds are disclosed in U.S. Patent No. 5,215,738 (listing some 58 compounds, *e.g*., 5-OH nicotinamide, 5-aminonicotinamide, 5-(2,3-dihydroxypropoxy) nicotinamide, and compounds having the structures: 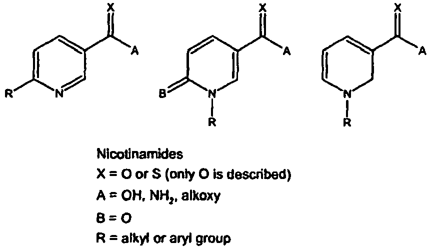 and U.S. Patent No. 4,258,052 (listing some 46 compounds, *e.g*., 1-methyl-6-keto-1,6-dihydronicotinic acid).

In one aspect, the cell cycle inhibitor is a tetrazine compound, such as temozolomide, or an analogue or derivative thereof, including dacarbazine. Suitable compounds have the structures:

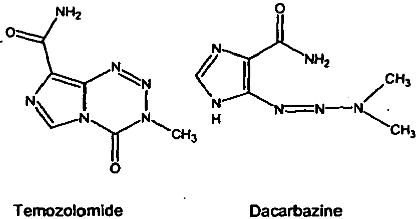

Another suitable tetrazine compound is procarbazine, including HCl and HBr salts, having the structure:

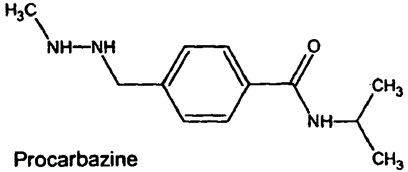

In another aspect, the cell cycle inhibitor is actinomycin D (C₁), or other members of this family, including dactinomycin, actinomycin C₂, actinomycin C₃, and actinomycin F₁. Suitable compounds have the structures:

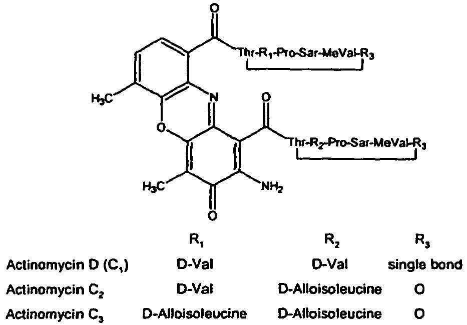

In another aspect, the cell cycle inhibitor is an aziridine compound, such as benzodepa, or an analogue or derivative thereof, including meturedepa, uredepa, and carboquone. Suitable compounds have the structures:

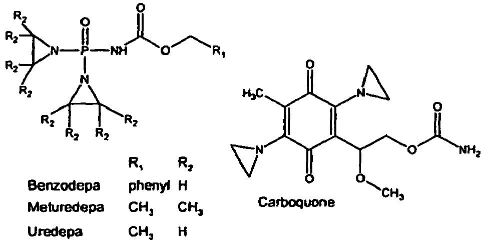

In another aspect, the cell cycle inhibitor is a halogenated sugar, such as mitolactol, or an analogue or derivative thereof, including mitobronitol and mannomustine. Examples of halogenated sugars have the structures:

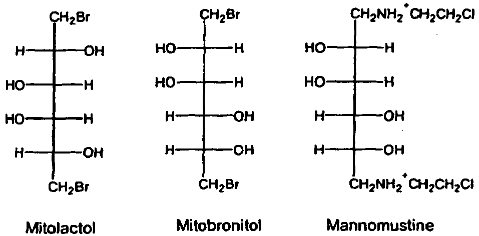

In another aspect, the cell cycle inhibitor is a diazo compound, such as azaserine, or an analogue or derivative thereof, including 6-diazo-5-oxo-L-norleucine and 5-diazouracil (also a pyrimidine analog). Suitable compounds have the structures:

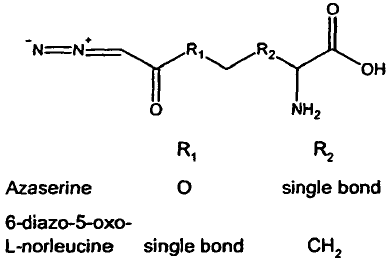

Other compounds that may serve as cell cycle inhibitor s according to the present invention are pazelliptine; wortmannin; metoclopramide; RSU; buthionine sulfoxime; tumeric; curcumin; AG337, a thymidylate synthase inhibitor; levamisole; lentinan, razoxane, indomethacin; chlorpromazine; α and interferon; MnBOPP; gadolinium texaphyrin; 4-amino-1,8-naphthalimide; staurosporine derivative of CGP; and SR-2508.

Thus, in one aspect, the cell cycle inhibitor is a DNA alkylating agent. In another aspect, the cell cycle inhibitor is an anti-microtubule agent. In another aspect, the cell cycle inhibitor is a topoisomerase inhibitor. In another aspect, the cell cycle inhibitor is a DNA cleaving agent. In another aspect, the cell cycle inhibitor is an antimetabolite. In another aspect, the cell cycle inhibitor functions by inhibiting adenosine deaminase (*e.g*., as a purine analog). In another aspect, the cell cycle inhibitor functions by inhibiting purine ring synthesis and/or as a nucleotide interconversion inhibitor (*e.g*., as a purine analogue such as mercaptopurine). In another aspect, the cell cycle inhibitor functions by inhibiting dihydrofolate reduction and/or as a thymidine monophosphate block (*e.g*., methotrexate). In another aspect, the cell cycle inhibitor functions by causing DNA damage (*e.g*., bleomycin). In another aspect, the cell cycle inhibitor functions as a DNA intercalation agent and/or RNA synthesis inhibition (*e.g*., doxorubicin). In another aspect, the cell cycle inhibitor functions by inhibiting pyrimidine synthesis (*e.g*., N-phosphonoacetyl-L-aspartate). In another aspect, the cell cycle inhibitor functions by inhibiting ribonucleotides (*e.g*., hydroxyurea). In another aspect, the cell cycle inhibitor functions by inhibiting thymidine monophosphate (*e.g*., 5-fluorouracil). In another aspect, the cell cycle inhibitor functions by inhibiting DNA synthesis (*e.g*., cytarabine). In another aspect, the cell cycle inhibitor functions by causing DNA adduct formation (*e.g*., platinum compounds). In another aspect, the cell cycle inhibitor functions by inhibiting protein synthesis (*e.g*., L-asparginase). In another aspect, the cell cycle inhibitor functions by inhibiting microtubule function (*e.g*., taxanes).

Additional cell cycle inhibitors useful in the present invention, as well as a discussion of their mechanisms of action, may be found in Hardman J.G., Limbird L.E. Molinoff R.B., Ruddon R W., Gilman A.G. editors, Chemotherapy of Neoplastic Diseases in Goodman and Gilman's The Pharmacological Basis of Therapeutics Ninth Edition, McGraw-Hill Health Professions Division, New York, 1996, pages 1225-1287. See also U.S. Patent Nos. 3,387,001; 3,808,297; 3,894,000; 3,991,045; 4,012,390; 4,057,548; 4,086,417; 4,144,237; 4,150,146; 4,210,584; 4,215,062; 4,250,189; 4,258,052; 4,259,242; 4,296,105; 4,299,778; 4,367,239; 4,374,414; 4,375,432; 4,472,379; 4,588,831; 4,639,456; 4,767,855; 4,828,831; 4,841,045; 4,841,085; 4,908,356; 4,923,876; 5,030,620; 5,034,320; 5,047,528; 5,066,658; 5,166,149; 5,190,929; 5,215,738; 5,292,731; 5,380,897; 5,382,582; 5,409,915; 5,440,056; 5,446,139; 5,472,956; 5,527,905; 5,552,156; 5,594,158; 5,602,140; 5,665,768; 5,843,903; 6,080,874; 6,096,923; and RE030561.

In another embodiment the cell-cycle inhibitor peloruside A, or a CDK-2 inhibitor, nimorazole (Cancer Chemotherapy and Biotherapy - Principles and Practice. Lippincott-Raven Publishers, New York, 1996, p.554), erythropoietin, BW12C, hydralazine, BSO, WR-2721, mono-substituted keto-aldehyde compounds (U.S. Patent No. 4,066,650), 2H-isoindolediones (U.S. Patent No. 4,494,547), nitroaniline derivatives (U.S. Patent No. 5,571,845), DNA-affnic hypoxia selective cytotoxins (U.S. Patent No. 5,602,142) halogenated DNA ligand (U.S. Patent No. 5,641,764), 1,2,4 benzotriazine oxides (U.S. Patent Nos. 5,616,584, 5,624,925, 5,175,287), nitric oxide (U.S. Patent No. 5,650,442), fluorine-containing nitroazole derivatives (U.S. Patent No. 4,927,941), copper II complexes (U.S. Patent No. 5,100,885), platinum complexes (U.S. Patent No. 4,921,963, EP 0 287 317 A3), autobiotics (U.S. Patent No. 5,147,652), acridine-intercalator (U.S. Patent No. 5,294,715), hydroxylated texaphyrins (U.S. Patent No. 5,457,183), hydroxylated compound derivative (Publication Number 011106775 A (Japan), Oct. 22,1987; Publication Number 01139596 A (Japan), Nov. 25, 1987; Publication Number 63170375 A (Japan), Jan. 7, 1987), SM 5887 (Pharma Japan 1468:20, 1995), MX-2 (Pharma Japan 1420:19, 1994), RB90740 (Br. J. Cancer, 74 Suppl. (27):S70-S74, 1996); CGP 6809 (Cancer Chemother. Pharmacol. 23(6):341-7, 1989), B-3839 (In Vivo 2(2):151-4, 1988), 7,8-polymethyleneimidazo-1,3,2-diazaphosphorines (Mendeleev Commun. 2:67, 1995), and MX068 (Pharma Japan, 658:18, 1999).

### 2. Angiogenesis Inhibitors

In one embodiment, the pharmacologically active compound is an angiogenesis inhibitor. Angiogenesis inhibitors include, without limitation, active taxanes, such as described above (*e.g*., paclitaxel and docetaxol); angiostatic steroids, such as squaline; cartilage derived proteins and factors; thrombospondin; matrix metalloproteinases (including collagenases, gelatinases A and B, stromelysins 1, 2 and 3, martilysin, metalloelastase, MT1-MMP (a progelatenase), MT2-MMP, MT3-MMP, MT4-MMP, Bay 12-9566 (Bayer), AG-3340 (Agouron), CGS270231 (Novartis), D5140, D1927, D2163 (Chiroscience)); and phytocemicals (including genistein, daidzein, leuteolin, apigenin, 3 hydroxyflavone, 2',3'-dihydroxyflavone, 3',4'-dihydroxyflavone, or fisetin). Other examples of angiogenesis inhibitors are 2-ME (NSC-659853), PI-88 (D-mannose, O-6-O-phosphono-alpha-D-mannopyranosyl-(1-3)-O-alpha-D-mannopyranosyl-(1-3)-O-alpha-D-mannopyranosyl-(1-3)-O-alpha-D-mannopyranosyl-(1-2)-hydrogen sulphate), thalidomide (1H-isoindole-1,3(2H)-dione, 2-(2,6-dioxo-3-piperidinyl)-), CDC-394, CC-5079, ENMD-0995 (S-3-amino-phthalidoglutarimide), AVE-8062A, vatalanib, SH-268, halofuginone hydrobromide, atiprimod dimaleate (2-azaspivo[4.5]decane-2-propanamine, N,N-diethyl-8,8-dipropyl, dimaleate), ATN-224, CHIR-258, combretastatin A-4 (phenol, 2-methoxy-5-[2-(3,4,5-trimethoxyphenyl)ethenyl]-, (Z)-), GCS-100LE, or an analogue or derivative thereof).

### 3. 5-Lipoxygenase Inhibitors and Antagonists

In another embodiment, the pharmacologically active compound is a 5-lipoxygenase inhibitor or antagonist (*e.g*., Wy-50295 (2-naphthaleneacetic acid, alpha-methyl-6-(2-quinolinylmethoxy), (S)-), ONO-LP-269(2,11,14-eicosatrienamide, N-(4-hydroxy-2-(1H-tetrazol-5-yl)-8-quinolinyl)-, (E,Z,Z)-), licofelone (1H-pyrrolizine-5-acetic acid, 6-(4-chlorophenyl)-2,3-dihydro-2,2-dimethyl-7-phenyl-), CMI-568 (urea, N-butyl-N-hydroxy-N'-(4-(3-(methylsulfonyl)-2-propoxy-5-(tetrahydro-5-(3,4,5-trimethoxyphenyl)-2-furanyl)phenoxy)butyl)-,trans-), IP-751 ((3R,4R)-(delta 6)-THC-DMH-11-oic acid), PF-5901 (benzenemethanol, alpha-pentyl-3-(2-quinolinylmethoxy)-), LY-293111 (benzoic acid, 2-(3-(3-((5-ethyl-4'-fluoro-2-hydroxy(1,1'-biphenyl)-4-yl)oxy)propoxy)-2-propylphenoxyf), RG-5901-A (benzenemethanol, alpha-pentyl-3-(2-quinolinylmethoxy)-, hydrochloride), rilopirox (2(1H)-pyridinone, 6-((4-(4-chlorophenoxy)phenoxy)methyl)-1-hydroxy-4-methyl-), L-674636 (acetic acid, ((4-(4-chlorophenyl)-1-(4-(2-quinolinylmethoxy)phenyl)butyl)thio)AS)), 7-((3-(4-methoxy-tetrahydro-2H-pyran-4-yl)phenyl)methoxy)-4-phenylnaphtho(2,3-c)furan-1(3H)-one, MK-886 (1H-indole-2-propanoic acid, 1-((4-chlorophenyl)methyl)-3-((1,1-dimethylethyl)thioyalpha, alpha-dimethyl-5-(1-methylethyl)-), quiflapon (1H-indole-2-propanoic acid, 1-((4-chlorophenyl)methyl)3-((1,1-dimethylethyl)thio)-alpha, alpha-dimethyl-5-(2-quinolinylmethoxy)-), quiflapon (1H-Indole-2-propanoic acid, 1-((4-chlorophenyl)methyl)-3-((1,1-dimethylethyl)thio)-alpha, alpha-dimethyl-5-(2-quinolinylmethoxy)-), docebenone (2,5-cyclohexadiene-1,4-dione, 2-(12-hydroxy-5,10-dodecadiynyl)-3,5,6-trimethyl-), zileuton (urea, N-(1-benzo(b)thien-2-ylethyl)-N-hydroxy-), or an analogue or derivative thereof).

### 4. Chemokine Receptor Antagonists CCR (1, 2, 3, & 5)

In another embodiment, the pharmacologically active compound is a chemokine receptor antagonist which inhibits one or more subtypes of CCR (1, 2, 3, and 5) (*e.g*., ONO-4128 (1,4,9-triazaspiro(5.5)undecane-2,5-dione, 1-butyl-3-(cyclohexylmethyl)-9-((2,3-dihydro-1,4-benzodioxin-6-yl)methyl-), L-381, CT-112 (L-arginine, L-threonyl-L-threonyl-L-seryl-L-glutaminyl-L-valyl-L-arginyl-L-prolyl-), AS-900004, SCH-C, ZK-811752, PD-172084, UK-427857, SB-380732, vMIP II, SB-265610, DPC-168, TAK-779 (N, N-dimethyl-N-(4-(2-(4-methylphenyl)-6,7-dihydro-5H-benzocyclohepten-8-ylcarboxamido)benyl)tetrahydro-2H-pyran-4-aminium chloride), TAK-220, KRH-1120), GSK766994, SSR-150106, or an analogue or derivative thereof). Other examples of chemokine receptor antagonists include a-Immunokine-NNS03, BX-471, CCX-282, Sch-350634; Sch-351125; Sch-417690; SCH-C, and analogues and derivatives thereof.

### 5. Cyclin Dependent Protein Kinase Inhibitors

In another embodiment, the pharmacologically active compound is a cyclin dependent protein kinase inhibitor (*e.g*., R-roscovitine, CYC-101, CYC-103, CYC-400, MX-7065, alvocidib (4H-1-Benzopyran-4-one, 2-(2-chlorophenyl)-5,7-dihydroxy-8-(3-hydroxy-1-methyl-4-piperidinyl)-, cis-(-)-), SU-9516, AG-12275, PD-0166285, CGP-79807, fascaplysin, GW-8510 (benzenesulfonamide, 4-(((Z)-(6,7-dihydro-7-oxo-8H-pyrrolo(2,3-g)benzothiazol-8-ylidene)methyl) amino)-N-(3-hydroxy-2,2-dimethylpropyl)-), GW-491619, Indirubin 3' monoxime, AZD-5438, ZK-CDK or an analogue or derivative thereof).

### 6. EGF (Epidermal Growth Factor) Receptor Kinase Inhibitors

In another embodiment, the pharmacologically active compound is an EGF (epidermal growth factor) kinase inhibitor (*e.g*., erlotinib (4-quinazolinamine, N-(3-ethynylphenyl)-6,7-bis(2-methoxyethoxy)-, monohydrochloride), erbstatin, BIBX-1382, gefitinib (4-quinazolinamine, N-(3-chloro-4-fluorophenyl)-7-methoxy-6-(3-(4-morpholinyl)propoxy)), or an analogue or derivative thereof).

### 7. Elastase Inhibitors

In another embodiment, the pharmacologically active compound is an elastase inhibitor (*e.g*., ONO-6818, sivelestat sodium hydrate (glycine, N-(2-(((4-(2,2-dimethyl-1-oxopropoxy)phenyl)sulfonyl)amino)benzoyl)-), erdosteine (acetic acid, ((2-oxo-2-((tetrahydro-2-oxo-3-thienyl)amino)ethyl)thio)-), MDL-100948A, MDL-104238 (N-(4-(4-morpholinylcarbonyl)benzoyl)-L-valyl-N'-(3,3,4,4,4-pentafluoro-1-(1-methylethyl)-2-oxobutyl)-L-2-azetamide), MDL-27324 (L-prolinamide, N-((5-(dimethylaminoyl-naphthalenyl)sulfonyl)-L-alanyl-L-alanyl-N-(3,3,3-trifluoro-1-(1-methylethyl)-2-oxopropyl)-, (S)-), SR-26831 (thieno(3,2-c)pyridinium, 5-((2-chlorophenyl)methyl)-2-(2,2-dimethyl-1-oxopropoxy)-4,5,6,7-tetrahydro-5-hydroxy-), Win-68794, Win-63110, SSR-69071 (2-(9(2-piperidinoethoxy)-4-oxo-4H-pyrido(1,2-a)pyrimidin-2-yloxymethyl)-4-(1-methylethyl)-6-methyoxy-1,2-benzisothiazol-3(2H)-one-1,1-dioxide), (N(.alpha.)-(1-adamantylsulfonyl)N(epsilon)-succinyl-L-lysyl-L-prolyl-L-valinal), Ro-31-3537 (N alpha-(1-adamantanesulphonyl)-N-(4-carboxybenzoyl)-L-lysyl-alanyl-L-valinal), R-665, FCE-28204, ((6R,7R)-2-(benzoyloxy)-7-methoxy-3-methyl-4-pivaloyl-3-cephem 1,1-dioxide), 1,2-benzisothiazol-3(2H)-one, 2-(2,4-dinitrophenyl)-, 1,1-dioxide, L-658758 (L-proline, 1-((3-((acetyloxy)methyl)-7-methoxy-8-oxo-5-thia-1-azabicyclo(4.2.0)oct-2-en-2-yl)carbonyl)-, S,S-dioxide, (6R-cis)-), L-659286 (pyrrolidine, 1-((7-methoxy-8-oxo-3-(((1,2,5,6-tetrahydro-2-methyl-5,6-dioxo-1,2,4-triazin-3-yl)thio)methyl)-5-thia-1-azabicyclo(4.2.0)oct-2-en-2-yl)carbonyl)-, S,S-dioxide, (6R-cis)-), L-680833 (benzeneacetic acid, 4-((3,3-diethyl-1-(((1-(4-methylphenyl)butyl)amino)carbonyl)-4-oxo-2-azetidinyl)oxy)-, (S-(R*,S*))-), FK-706 (L-prolinamide, N-[4-[[(carboxymethyl)amino]carbonyl]benzoyl]-L-valyl-N-[3,3,3-trifluoro-1-(1-methylethyl)-2-oxopropyl]-, monosodium salt), Roche R-665, or an analogue or derivative thereof).

### 8. Factor Xa Inhibitors

In another embodiment, the pharmacologically active compound is a factor Xa inhibitor (*e.g*., CY-222, fondaparinux sodium (alpha-D-glucopyranoside, methyl O-2-deoxy-6-O-sulfo-2-(sulfoamino)-alpha-D-glucopyranosyl-(1-4)-O-β-D-glucopyranuronosyl-(1-4)-O-2-deoxy-3,6-di-O-sulfo-2-(sulfoamino)-alpha-D-glucopyranosyl-(1-4)-O-2-O-sulfo-alpha-L-idopyranuronosyl-(1-4)-2-deoxy-2-(sulfoamino)-, 6-(hydrogen sulfate)), danaparoid sodium, or an analogue or derivative thereof).

### 9. Farnesyltransferase Inhibitors

In another embodiment, the pharmacologically active compound is a famesyltransferase inhibitor (*e.g*., dichlorobenzoprim (2,4-diamino-5-(4-(3,4-dichlorobenzylamino)-3-nitrophenyl)-6-ethylpyrimidine), B-581, B-956 (N-(8(R)-amino-2(S)-benzyl-5(S)-isopropyl-9-sulfanyl-3(Z),6(E)-nonadienoyl)-L-methionine), OSI-754, perillyl alcohol (1-cyclohexene-1-methanol, 4-(1-methylethenyl)-, RPR-114334, Ionafarnib (1-piperidinecarboxamide, 4-(2-(4-((11R)-3,10-dibromo-8-chloro-6,11-dihydro-5H-benzo(5,6)cyclohepta(1,2-b)pyridin-11-yl)-1-piperidinyl)-2-oxoethyl)-), Sch-48755, Sch-226374, (7,8-dichloro-5H-dibenzo(b,e)(1,4)diazepin-11-yl)-pyridin-3-ylmethylamine, J-104126, L-639749, L-731734 (pentanamide, 2-((2-((2-amino-3-mercaptopropyl)amino)-3-methylpentyl)amino)-3-methyl-N-(tetrahydro-2-oxo-3-furanyl)-, (3S-(3R*(2R*(2R*(S*),3S*),3R*)))-), L-744832 (butanoic acid, 2-((2-((2-((2-amino-3-mercaptopropyl)amino)-3-methylpentyl)oxy)-1-oxo-3-phenylpropyl)amino)-4-(methylsulfonyl)-, 1-methylethyl ester, (2S-(1(R*(R*)),2R*(S*),3R*))-), L-745631 (1-piperazinepropanethiol, ß-amino-2-(2-methoxyethyl)-4-(1-naphthalenylcarbonyl)-, ((3R,2S)-), N-acetyl-N-naphthylmethyl-2(S)-((1-(4-cyanobenzyl)-1H-imidazol-5-yl)acetyl)amino-3(S)-methylpentamine, (2alpha)-2-hydroxy-24,25-dihydroxylanost-8-en-3-one, BMS-316810, UCF-1-C (2,4-decadienamide, N-(5-hydroxy-5-(7-((2-hydroxy-5-oxo-1-cyclopenten-I-yl)amino-oxo-1,3,5-heptatrienyl)-2-oxo-7-oxabicyclo(4.1.0)hept-3-en-3-yl)-2,4,6-trimethyl-, (1S-(1alpha,3(2E,4E,6S*),5 alpha, 5(1E,3E,5E), 6
alpha))-), UCF-116-B, ARGLABIN (3H-oxireno[8,8a]azuleno[4,5-b]furan-8(4aH)-one, 5,6,6a,7,9a,9b-hexahydro-1,4a-dimethyl-7-methylene-, (3aR,4aS,6aS,9aS,9bRF) from ARGLABIN - Paracure, Inc. (Virginia Beach, VA), or an analogue or derivative thereof).

### 10. Fibrinogen Antagonists

In another embodiment, the pharmacologically active compound is a fibrinogen antagonist (*e.g*., 2(S)-((p-toluenesulfonyl)amino)-3-(((5,6,7,8,tetrahydro-4-oxo-5-(2-(piperidin-4-yl)ethyl)-4H-pyrazolo-(1,5-a)(1,4)diazepin-2-yl)carbonyl)-amino)propionic acid, streptokinase, urokinase, plasminogen activator, pamiteplase, monteplase, heberkinase, anistreplase, alteplase, pro-urokinase, picotamide (1,3-benzenedicarboxamide, 4-methoxy-N,N'-bis(3-pyridinylmethyl)-), or an analogue or derivative thereof).

### 11. Guanylate Cyclase Stimulants

In another embodiment, the pharmacologically active compound is a guanylate cyclase stimulant (*e.g*., isosorbide-5-mononitrate (D-glucitol, 1,4:3,6-dianhydro-, 5-nitrate), or an analogue or derivative thereof).

### 12. Heat Shock Protein 90 Antagonists

In another embodiment, the pharmacologically active compound is a heat shock protein 90 antagonist (*e.g*., geldanamycin; NSC-33050 (17-allylaminogeldanamycin), rifabutin (rifamycin XIV, 1',4-didehydro-1-deoxy-1,4-dihydro-5'-(2-methylpropyl)-1-oxo-), 17AAG, or an analogue or derivative thereof).

### 13. HMGCoA Reductase Inhibitors

In another embodiment, the pharmacologically active compound is an HMGCoA reductase inhibitor (*e.g*., BCP-671, BB-476, fluvastatin (6-heptenoic acid, 7-(3-(4-fluorophenyl)-1-(1-methylethyl)-1H-indol-2-yl)-3,5-dihydroxy-, monosodium salt, (R*,S*-(E))-(±)-), dalvastatin (2H-pyran-2-one, 6-(2-(2-(2-(4-fluoro-3-methylphenyl)-4,4,6,6-tetramethyl-1-cyclohexen-1-yl)ethenyl)tetrahydro)-4-hydroxy-, (4alpha,6ß(E))-(+/-)-), glenvastatin (2H-pyran-2-one, 6-(2-(4-(4-ftuorophenyl)-2-(1-methylethyl)-6-phenyl-3-pyridinyl)ethenyl)tetrahydro-4-hydroxy-, (4R-(4.alpha.,6ß(E)))-), S-2468, N-(1-oxododecyl)-4.alpha.,10-dimethyl-8-aza-trans-decal-3ß-ol, atorvastatin calcium (1H-Pyrrole-1-heptanoic acid, 2-(4-fluorophenyl)-ß,delta-dihydroxy-5-(1-methylethyl)-3-phenyl-4-((phenylamino)carbonyl)-, calcium salt (R-(R*,R*))-), CP-83101 (6,8-nonadienoic acid, 3,5-dihydroxy-9,9-diphenyl-, methyl ester, (R*,S*-(E))-(+/-)-), pravastatin (1-naphthaleneheptanoic acid, 1,2,6,7,8,8a-hexahydro-ß,delta,6-trihydroxy-2-methyl-8-(2-methyl-1-oxobutoxy), monosodium salt, (1S-(1.alpha.(ßS*,deltas*),2.alpha.,6.alpha.,8ß(R*),8a .alpha.))-), U-20685, pitavastatin (6-heptenoic acid, 7-(2-cyclopropyl-4-(4-fluorophenyl)-3-quinolinyl)-3,5-dihydroxy-, calcium salt (2:1), (S-(R*,S*-(E)))-), N-((1-methylpropyl)carbonyl)8-(2-(tetrahydro-4-hydroxy-6-oxo-2H-pyran-2-yl)ethyl)-perhydro-isoquinoline, dihydromevinolin (butanoic acid, 2-methyl-, 1,2,3,4,4a,7,8,8a-octahydro-3,7-dimethyl-8-(2-(tetrahydro-4-hydroxy-6-oxo-2H-pyran-2-yl)ethyl)-1-naphthalenyl ester(1.alpha.(R*),3.alpha., 4a .alpha.,7ß,8ß(2S*,4S*),8aß(3))-), HBS-107, dihydromevinolin (butanoic acid, 2-methyl-, 1,2,3,4,4a,7,8,8a-octahydro-3,7-dimethyl-8-(2-(tetrahydro-4-hydroxy-6-oxo-2H-pyran-2-yl)ethyl)-1-naphthalenyl ester(1.alpha.(R*), 3 .alpha.,4a .alpha.,7ß,8ß(2S*,4S*),8aß))-), L-669262 (butanoic acid, 2,2-dimethyl-, 1,2,6,7,8,8a-hexahydro-3,7-dimethyl-6-oxo-8-(2-(tetrahydro-4-hydroxy-6-oxo-2H-pyran-2-yl)ethyl)-1-naphthalenyl(1S-(1.alpha.,7ß,8ß(2S*,4S*),8aß))-), simvastatin (butanoic acid, 2,2-dimethyl-, 1,2,3,7,8,8a-hexahydro-3,7-dimethyl-8-(2-(tetrahydro-4-hydroxy-6-oxo-2H-pyran-2-yl)ethyl)-1-naphthalenyl ester, (1S-(1.alpha., 3.alpha.,7ß,8ß(2S*,4S*),8ß3))-), rosuvastatin calcium (6-heptenoic acid, 7-(4-(4-fluorophenyl)-6-(1-methylethyl)-2-(methyl(methylsulfonyl)amino)-5-pyrimdinyl)-3,5-dihydroxy- calcium salt (2:1) (S-(R*, S*-(E)))), meglutol (2-hydroxy-2-methyl-1,3-propandicarboxylic acid), lovastatin (butanoic acid, 2-methyl-, 1,2,3,7,8,8a-hexahydro-3,7-dimethyl-8-(2-(tetrahydro-4-hydroxy-6-oxo-2H-pyran-2-yl)ethyl)-1-naphthalenyl ester, (1S-(1 .alpha..(R*),3.alpha.,7ß,8ß(2S*,4S*),8aß))-), or an analogue or derivative thereof).

### 14. Hydroorotate Dehydrogenase Inhibitors

In another embodiment, the pharmacologically active compound is a hydroorotate dehydrogenase inhibitor (*e.g*., leflunomide (4-isoxazolecarboxamide, 5-methyl-N-(4-(trifluoromethyl)phenyl)-), laflunimus (2-propenamide, 2-cyano-3-cyclopropyl-3-hydroxy-N-(3-methyl-4(trifluoromethyl)phenyl)-, (Z)-), or atovaquone (1,4-naphthalenedione, 2-[4-(4-chlorophenyl)cyclohexyl]-3-hydroxy-, trans-, or an analogue or derivative thereof).

### 15. IKK2 Inhibitors

In another embodiment, the pharmacologically active compound is an IKK2 inhibitor (*e.g*., MLN-120B, SPC-839, or an analogue or derivative thereof).

### 16. IL-1, ICE and IRAK Antagonists

In another embodiment, the pharmacologically active compound is an IL-1, ICE or an IRAK antagonist (*e.g*., E-5090 (2-propenoic acid, 3-(5-ethyl-4-hydroxy-3-methoxy-1-naphthalenyl)-2-methyl-, (Z)-), CH-164, CH-172, CH-490, AMG-719, iguratimod (N-(3-(formylamino)-4-oxo-6-phenoxy-4H-chromen-7-yl) methanesulfonamide), AV94-88, pralnacasan (6H-pyridazino(1,2-a)(1,2)diazepine-1-carboxamide, N-((2R,3S)-2-ethoxytetrahydro-5-oxo-3-furanyl)octahydro-9-((1-isoquinolinylcarbonyl)amino)-6,10-dioxo-, (1S,9Sr), (2S-cis)-5-(benzyloxycarbonylamino-1,2,4,5,6,7-hexahydro-4-(oxoazepino(3,2,1-hi)indole-2-carbonyl)-amino)-4-oxobutanoic acid, AVE-9488, esonarimod (benzenebutanoic acid, .alpha.-((acetylthio)methyl)-4-methyl-.gamma.-oxo-), pralnacasan (6H-pyridazino(1,2-a)(1,2)diazepine-1-carboxamide, N-((2R,3S)-2-ethoxytetrahydro-5-oxo-3-furanyl)octahydro-9-((1-isoquinolinylcarbonyl)amino)-6,10-dioxo-, (1S,9S)-), tranexamic acid (cyclohexanecarboxylic acid, 4-(aminomethyl)-, trans-), Win-72052, romazarit (Ro-31-3948) (propanoic acid, 2-((2-(4-chlorophenyl)-4-methyl-5-oxazolyl)methoxy)-2-methyl-), PD-163594, SDZ-224-015 (L-alaninamide N-((phenylmethoxy)carbonyl)-L-valyl-N-(1S)-3-((2,6-dichlorobenzoyl)oxy)-1-(2-ethoxy-2-oxoethyl)-2-oxopropyl)-), L-709049 (L-alaninamide, N-acetyl-L-tyrosyl-L-valyl-N-(2-carboxy-1-formylethylr, (S)-), TA-383 (1H-imidazole, 2-(4-chlorophenyl)-4,5-dihydro-4,5-diphenyl-, monohydrochloride, cis-), EI-1507-1 (6a,12a-epoxybenz(a)anthracen-1,12(2H,7H)-dione, 3,4-dihydro-3,7-dihydroxy-8-methoxy-3-methyl-), ethyl 4-(3,4-dimethoxyphenyl)-6,7-dimethoxy-2-(1,2,4-triazol-1-yl methyl)quinoline-3-carboxylate, EI-1941-1, TJ-114, anakinra (interleukin 1 receptor antagonist (human isoform x reduced), N2-L-methionyl-), IX-207-887 (acetic acid, (10-methoxy-4H-benzo[4,5]cyclohepta[1,2-b]thien-4-ylidene)-), K-832, kineret (IL-1Ra), IL-1R Type II, NIP-1302a-3, or an analogue or derivative thereof).

### 17. IL-4 Agonists

In another embodiment, the pharmacologically active compound is an IL-4 agonist (*e.g*., glatiramir acetate (L-glutamic acid, polymer with L-alanine, L-lysine and L-tyrosine, acetate (salt)), or an analogue or derivative thereof).

### 18. Immunomodulatory Agents

In another embodiment, the pharmacologically active compound is an immunomodulatory agent (*e.g*., biolimus, ABT-578, methylsulfamic acid 3-(2-methoxyphenoxy)-2-(((methylamino)sulfonyl)oxy)propyl ester, sirolimus (also referred to as rapamycin or RAPAMUNE (American Home Products, Inc., Madison, NJ)), CCI-779 (rapamycin 42-(3-hydroxy-2-(hydroxymethyl)-2-methylpropanoate)), LF-15-0195, NPC-15669 (L-leucine, N-(((2,7-dimethyl-9H-fluoren-9-yl)methoxy)carbonyl)-), NPC-15670 (L-leucine, N-(((4,5-dimethyl-9H-fluoren-9-yl)methoxy)carbonyl)-), NPC-16570 (4-(2-(fluoren-9-yl)ethyloxy-carbonyl)aminobenzoic acid), sufosfamide (ethanol, 2-((3-(2-chloroethyl)tetrahydro-2H-1,3,2-oxazaphosphorin-2-yl)amino)-, methanesulfonate (ester), P-oxide), tresperimus (2-(N-(4-(3-aminopropylamino)butyl)carbamoyloxy)-N-(6-guanidinohexyl)acetamide), 4-(2-(fluoren-9-yl)ethoxycarbonylamino)-benzo-hydroxamic acid, iaquinimod, PBI-1411, azathioprine (6-((1-Methyl-4-nitro-1H-imidazol-5-yl)thio)1H-purine), PBI0032, beclometasone, MDL-28842 (9H-purin-6-amine, 9-(5-deoxy-5-fluoro-ß-D-threo-pent-4-enofuranosyl)-, (Z)-), FK-788, AVE-1726, ZK-90695, ZK-90695, Ro-54864, didemnin-B, Illinois (didemnin A, N-(1-(2-hydroxy-1-oxopropyl)-L-prolyl)-, (S)-), SDZ-62-826 (ethanaminium, 2-((hydroxy((1-((octadecyloxy)carbonyl)-3-piperidinyl)methoxy)phosphinyl)oxy)-N,N,N-trimethyl-, inner salt), argyrin B ((4S,7S,13R,22R)-13-Ethyl-4-(1H-indol-3-ylmethyl)-7-(4-methoxy-1H-indol-3-ylmethyl)18,22-dimethyl-16,methyl-ene-24-thia-3,6,9,12,15,18,21,26-octaazabicyclo(21.2.1)-hexacosa-1(25),23(26)-diene-2,5,8,11,14,17,20-heptaone), everolimus (rapamycin, 42-O-(2-hydroxyethyl)-), SAR-943, L-687795, 6-((4-chlorophenyl)sulfinyl)-2,3-dihydro-2-(4-methoxyphenyl)-5-methyl-3-oxo-4-pyridazinecarbonitrile, 91Y78 (1H-imidazo(4,5-c)pyridin-4-amine, 1-(3-D-ribofuranosyl-), auranofin (gold, (1-thio-f3-D-glucopyranose 2,3,4,6-tetraacetato-S)(triethylphosphine)-), 27-0-demethylrapamycin, tipredane (androsta-1,4-dien-3-one, 17-(ethylthio)-9-fluoro-11-hydroxy-17-(methylthio)-, (11ß,17.alpha.)-), Al-402, LY-178002 (4-thiazolidinone, 5-((3,5-bis(1,1-dimethylethyl)-4-hydroxyphenyl)methylene)-), SM-8849 (2-thiazolamine, 4-(1-(2-fluoro(1,1'-biphenyl)-4-yl)ethyl)-N-methyl-), piceatannol, resveratrol, triamcinolone acetonide (pregna-1,4-diene-3,20-dione, 9-fluoro-11,21-dihydroxy-16,17-((1-methylethylidene)bis(oxy))-, (11ß, 16 .alpha.)-), ciclosporin (cyclosporin A), tacrolimus (15,19-epoxy-3H-pyrido(2,1-c)(1,4)oxaazacyclotricosine-1,7,20,21(4H,23H)-tetrone, 5,6,8,11,12,13,14,15,16,17,18,19,24,25,26,26a-hexadecahydro-5,19-dihydroxy-3-(2-(4-hydroxy-3-methoxycyclohexyl)-1-methylethenyl)-14,16-dimethoxy-4,10,12,18-tetramethyl-8-(2-propenyl)-, (3S-(3R*(E(1S*,3S*,4S*)),4S*,5R*,8S*,9E,12R*,14R*,15S*,16R*,18S*,19S*,26aR*)) -), gusperimus (heptanamide, 7-((aminoiminomethyl)amino)-N-(2-((4-((3-aminopropyl)amino)butyl)amino)-1-hydroxy-2-oxoethyl)-, (+/-)-), tixocortol pivalate (pregn-4-ene-3,20-dione, 21-((2,2-dimethyl-1-oxopropyl)thio)-11,17-dihydroxy-, (11ß)-), alefacept (1-92 LFA-3 (antigen) (human) fusion protein with immunoglobulin G1 (human hinge-CH2-CH3 .gamma.1-chain), dimer), halobetasol propionate (pregna-1,4-diene-3,20-dione, 21-chloro-6,9-difluoro-11-hydroxy-16-methyl-17-(1-oxopropoxy)-, (6.alpha.,11ß,16ß)-), iloprost trometamol (pentanoic acid, 5-(hexahydro-5-hydroxy-4-(3-hydroxy-4-methyl-1-octen-6-ynyl)-2(1H)-pentalenylidene)-), beraprost (1H-cyclopenta(b)benzofuran-5-butanoic acid, 2,3,3a,8b-tetrahydro-2-hydroxy-1-(3-hydroxy-4-methyl-1-octen-6-ynyl)-), rimexolone (androsta-1,4-dien-3-one, 11-hydroxy-16,17-dimethyl-17-(1-oxopropyl)-, (11ß,16.alpha.,17ß)-), dexamethasone (pregna-1,4-diene-3,20-dione,9-fluoro-11,17,21-trihydroxy-16-methyl-, (11ß,16.alpha.)-), sulindac (cis-5-fluoro-2-methyl-1-((p-methylsulfinyl)benzylidene)indene-3-acetic acid), proglumetacin (1H-Indole-3-acetic acid, 1-(4-chlorobenzoyl)-5-methoxy-2-methyl-2-(4-(3-((4-(benzoylamino)-5-(dipropylamino)-1,5-dioxopentyl)oxy)propyl)-1-piperazinyl)ethylester, (+/-)-), alclometasone dipropionate (pregna-1,4-diene-3,20-dione, 7-chloro-11-hydroxy-16-methyl-17,21-bis(1-oxopropoxy)-, (7.alpha.,11S,16.alpha.)-), pimecrolimus (15,19-epoxy-3H-pyrido(2,1-c)(1,4)oxaazacyclotricosine-1,7,20,21(4H,23H)-tetrone, 3-(2-(4-chloro-3-methoxycydohexyl)-1-methyletheny)-8-ethyl-5,6,8,11,12,13,14,15,16,17,18,19,24,25,26,26a-hexadecahydro-5,19-dihydroxy-14,16-dimethoxy-4,10,12,18-tetramethyl-, (3S-(3R*(E(1S*,3S*,4R*)),4S*,5R*,8S*,9E,12R*,14R*,15S*,16R*,18S*,19S*,26aR*)) -), hydrocortisone-17-butyrate, mitoxantrone (9,10-anthracenedione, 1,4-dihydroxy-5,8-bis((2-((2-hydroxyethyl)amino)ethyl)amino)-), mizoribine (1H-imidazole-4-carboxamide, 5-hydroxy-1-ß-D-ribofuranosyl-), prednicarbate (pregna-1,4-diene-3,20-dione, 17-((ethoxycarbonyl)oxy)-11-hydroxy-21-(1-oxopropoxy)-, (11ß)-), iobenzarit (benzoic acid, 2-((2-carboxyphenyl)amino)-4-chloro-), glucametacin (D-glucose, 2-(((1-(4-chlorobenzoyl)-5-methoxy-2-methyl-1H-indol-3-yl)acetyl)amino)-2-deoxy-), fluocortolone monohydrate ((6 .alpha.)-fluoro-16.alpha.-methylpregna-1,4-dien-11ß,21-diol-3,20-dione), fluocortin butyl (pregna-1,4-dien-21-oic acid, 6-fluoro-11-hydroxy-16-methyl-3,20-dioxo-, butyl ester, (6.alpha.,11ß,16.alpha.)-), difluprednate (pregna-1,4-diene-3,20-dione, 21-(acetyloxy)-6,9-difluoro-11-hydroro-17-(1-oxobutoxy)-, (6 .alpha.,11ß)-), diflorasone diacetate (pregna-1,4-diene-3,20-dione, 17,21-bis(acetyloxy)-6,9-difluoro-11-hydroxy-16-methyl-, (6.alpha.,11ß,16ß), dexamethasone valerate (pregna-1,4-diene-3,20-dione, 9-fluoro-11,21-dihydroxy-16-methyl-17-((1-oxopentyl)oxyy, (11ß,16.alpha.)-), methylprednisolone, deprodone propionate (pregna-1,4-diene-3,20-dione, 11-hydroxy-17-(1-oxopropoxy)-, (11.beta.)-), bucillamine (L-cysteine, N-(2-mercapto-2-methyl-1-oxopropyl)-), amcinonide (benzeneacetic acid, 2-amino-3-benzoyl-, monosodium salt, monohydrate), acemetacin (1H-indole-3-acetic acid, 1-(4-chlorobenzoyl)-5-methoxy-2-methyl-, carboxymethyl ester), or an analogue or derivative thereof).

Further, analogues of rapamycin include tacrolimus and derivatives thereof (*e.g*., EP0184162B1 and U.S. Patent No. 6,258,823) everolimus and derivatives thereof (*e.g*., U.S. Patent No. 5,665,772). Further representative examples of sirolimus analogues and derivatives can be found in PCT Publication Nos. WO 97/10502, WO 96/41807, WO 96/35423, WO 96/03430, WO 96/00282, WO 95/16691, WO 95/15328, WO 95/07468, WO 95/04738, WO 95/04060, WO 94/25022, WO 94/21644, WO 94/18207, WO 94/10843, WO 94/09010, WO 94/04540, WO 94/02485, WO 94/02137, WO 94/02136, WO 93/25533, WO 93/18043, WO 93/13663, WO 93/11130, WO 93/10122, WO 93/04680, WO 92/14737, and WO 92/05179. Representative U.S. patents include U.S. Patent Nos. 6,342,507; 5,985,890; 5,604,234; 5,597,715; 5,583,139; 5,563,172; 5,561,228; 5,561,137; 5,541,193; 5,541,189; 5,534,632; 5,527,907; 5,484,799; 5,457,194; 5,457,182; 5,362,735; 5,324,644; 5,318,895; 5,310,903; 5,310,901; 5,258,389; 5,252,732; 5,247,076; 5,225,403; 5,221,625; 5,210,030; 5,208,241; 5,200,411; 5,198,421; 5,147,877; 5,140,018; 5,116,756; 5,109,112; 5,093,338; and 5,091,389.

The structures of sirolimus, everolimus, and tacrolimus are provided below:

| Name | Code Name | Company | Structure |
|---|---|---|---|
| Everolimus | SAR-943 | Novartis | See below |
| Sirolimus | AY-22989 | Wyeth | See below |
| RAPAMUNE | NSC-226080 | | |
| Rapamycin | | | |
| Tacrolimus | FK506 | Fujusawa | See below |

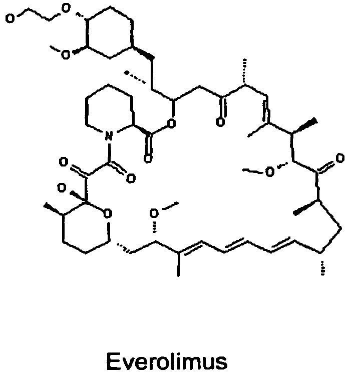

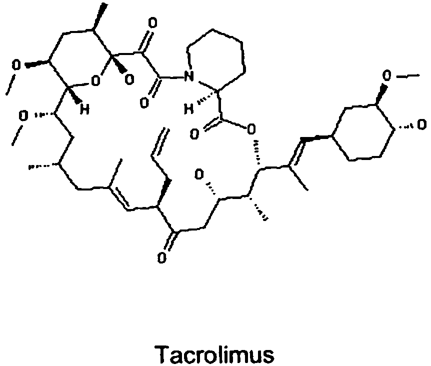

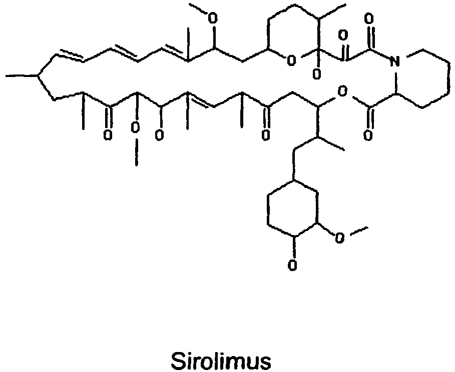

Further sirolimus analogues and derivatives include tacrolimus and derivatives thereof (*e.g*., EP0184162B1 and U.S. Patent No. 6,258,823) everolimus and derivatives thereof (*e.g*., US Patent No. 5,665,772). Further representative examples of sirolimus analogues and derivatives include ABT-578 and others may be found in PCT Publication Nos. WO 97/10502, WO 96/41807, WO 96/35423, WO 96/03430, WO 9600282, WO 95/16691, WO 9515328, WO 95/07468, WO 95/04738, WO 95/04060, WO 94/25022, WO 94/21644, WO 94/18207, WO 94/10843, WO 94/09010, WO 94/04540, WO 94/02485, WO 94/02137, WO 94/02136, WO 93/25533, WO 93/18043, WO 93/13663, WO 93/11130, WO 93/10122, WO 93/04680, WO 92/14737, and WO 92/05179. Representative U.S. patents include U.S. Patent Nos. 6,342,507; 5,985,890; 5,604,234; 5,597,715; 5,583,139; 5,563,172; 5,561,228; 5,561,137; 5,541,193; 5,541,189; 5,534,632; 5,527,907; 5,484,799; 5,457,194; 5,457,182; 5,362,735; 5,324,644; 5,318,895; 5,310,903; 5,310,901; 5,258,389; 5,252,732; 5,247,076; 5,225,403; 5,221,625; 5,210,030; 5,208,241, 5,200,411; 5,198,421; 5,147,877; 5,140,018; 5,116,756; 5,109,112; 5,093,338; and 5,091,389.

In one aspect, the fibrosis-inhibiting agent may be, *e.g*., rapamycin (sirolimus), everolimus, biolimus, tresperimus, auranofin, 27-0-demethylrapamycin, tacrolimus, gusperimus, pimecrolimus, or ABT-578.

### 19. Inosine monophosphate dehydrogenase inhibitors

In another embodiment, the pharmacologically active compound is an inosine monophosphate dehydrogenase (IMPDH) inhibitor (*e.g*., mycophenolic acid, mycophenolate mofetil (4-hexenoic acid, 6-(1,3-dihydro-4-hydroxy-6-methoxy-7-methyl-3-oxo-5-isobenzofuranyl)-4-methyl-, 2-(4-morpholinyl)ethyl ester, (E)-), ribavirin (1H-1,2,4-triazole-3-carboxamide, 1-ß-D-ribofuranosyl-), tiazofurin (4-thiazolecarboxamide, 2-ß-D-ribofuranosyl-), viramidine, aminothiadiazole, thiophenfurin, tiazofurin) or an analogue or derivative thereof. Additional representative examples are included in U.S. Patent Nos. 5,536,747, 5,807,876, 5,932,600, 6,054,472, 6,128,582, 6,344,465, 6,395,763, 6,399,773, 6,420,403, 6,479,628, 6,498,178, 6,514,979, 6,518,291, 6,541,496, 6,596,747, 6,617,323, 6,624,184, Patent Application Publication Nos. 2002/0040022A1, 2002/0052513A1, 2002/0055483A1, 2002/0068346A1, 2002/0111378A1, 2002/0111495A1, 2002/0123520A1, 2002/0143176A1, 2002/0147160A1, 2002/0161038A1, 2002/0173491A1, 2002/0183315A1, 2002/0193612A1, 2003/0027845A1, 2003/0068302A1, 2003/0105073A1, 2003/0130254A1, 2003/0143197A1, 2003/0144300A1, 2003/0166201A1, 2003/0181497A1, 2003/0186974A1, 2003/0186989A1, 2003/0195202A1, and PCT Publication Nos. WO 0024725A1, WO 00/25780A1, WO 00/26197A1, WO 00/51615A1, WO 00/56331A1, WO 00/73288A1, WO 01/00622A1, WO 01/66706A1, WO 01/79246A2, WO 01/81340A2, WO 01/85952A2, WO 02/16382A1, WO 02/18369A2, WO 2051814A1, WO 2057287A2, W02057425A2, WO 2060875A1, WO 2060896A1, WO 2060898A1, WO 2068058A2, WO 3020298A1, WO 3037349A1, WO 3039548A1, WO 3045901A2, WO 3047512A2, WO 3053958A1, WO 3055447A2, WO 3059269A2, WO 3063573A2, WO 3087071A1, WO 90/01545A1, WO 97/40028A1, WO 97/41211A1, WO 98/40381A1, and WO 99/55663A1).

### 20. Leukotriene Inhibitors

In another embodiment, the pharmacologically active compound is a leukotreine inhibitor (*e.g*., ONO-4057(benzenepropanoic acid, 2-(4-carboxybutoxy)-6-((6-(4-methoxyphenyl)-5-hexenyl)oxy)-, (E)-), ONO-LB-448, pirodomast 1,8-naphthyridin-2(1H)-one, 4-hydroxy-1-phenyl-3-(1-pyrrolidinyl), Sch-40120 (benzo(b)(1,8)naphthyridin-5(7H)-one, 10-(3-chlorophenylr6,8,9,10-tetrahydro-), L-656224 (4-benzofuranol, 7-chloro-2-((4-methoxyphenyl)methyl)-3-methyl-5-propyl-), MAFP (methyl arachidonyl fluorophosphonate), ontazolast (2-benzoxazolamine, N-(2-cyclohexyl-1-(2-pyridinyl)ethyl)-5-methyl-, (S)-), amelubant (carbamic acid, ((4-((3-((4-(1-(4-hydroxyphenyl)-1-methylethyl)phenoxy)methyl)phenyl)methoxy)phenyl)iminomethyl)- ethyl ester), SB-201993 (benzoic acid, 3-((((6-((1E)-2-carboxyethenyl)-5-((8-(4-methoxyphenyl)octyl)oxy)-2-pyridinyl)methyl)thio)methyl)-), LY-203647 (ethanone, 1-(2-hydroxy-3-propyl-4-(4-(2-(4-(1H-tetrazol-5-yl)butyl)-2H-tetrazol-5-yl)butoxy)phenyl)-), LY-210073, LY-223982 (benzenepropanoic acid, 5-(3-carboxybenzoyl)-2-((6-(4-methoxyphenyl)-5-hexenyl)oxy)-, (E)-), LY-293111 (benzoic acid, 2-(3-(3-((5-ethyl-4'-fluoro-2-hydroxy(1,1'-biphenyl)-4-yl)oxy)propoxy)-2-propylphenoxy)-), SM-9064 (pyrrolidine, 1-(4,11-dihydroxy-13-(4-methoxyphenyl)-1-oxo-5,7,9-tridecatrienyl)-, (E,E,E)-), T-0757 (2,6-octadienamide, N-(4-hydroxy-3,5-dimethylphenyl)-3,7-dimethyl-, (2E)-), or an analogue or derivative thereof).

### 21. MCP-1 Antagonists

In another embodiment, the pharmacologically active compound is a MCP-1 antagonist (*e.g*., nitronaproxen (2-napthaleneacetic acid, 6-methoxy-.alpha.-methyl 4-(nitrooxy)butyl ester (.alpha. S)-), bindarit (2-(1-benzylindazol-3-ylmethoxy)-2-methylpropanoic acid), 1-.alpha.-25 dihydroxy vitamin D₃, or an analogue or derivative thereof).

### 22. MMP Inhibitors

In another embodiment, the pharmacologically active compound is a matrix metalloproteinase (MMP) inhibitor (*e.g*., D-9120, doxycycline (2-naphthacenecarboxamide, 4-(dimethylamino)-1,4,4a,5,5a,6,11,12a-octahydro-3,5,10,12,12a-pentahydroxy-6-methyl-1,11-dioxo- (4S-(4 .alpha., 4a .alpha., 5 .alpha., 5a .alpha., 6 .alpha., 12a .alpha.))-), BB-2827, BB-1101 (2S-allyl-N1-hydroxy-3R-isobutyl-N4-(1S-methylcarbamoyl-2-phenylethyl)-succinamide), BB-2983, solimastat (N'-(2,2-dimethyl-1(S)-(N-(2-pyridyl)carbamoyl)propyl)-N4-hydroxy-2(R)-isobutyl-3(S)-methoxysuccinamide), batimastat (butanediamide, N4-hydroxy-N1-(2-(methylamino)-2-oxo-1-(phenylmethyl)ethyl)-2-(2-methylpropyl)-3-((2-thienylthio)methyl)-, (2R-(1(S*),2R*,3S*))-), CH-138, CH-5902, D-1927, D-5410, EF-13 (.gamma.-linolenic acid lithium salt), CMT-3 (2-naphthacenecarboxamide, 1,4,4a,5,5a,6,11,12a-octahydro-3,10,12,12a-tetrahydroxy-1,11-dioxo-, (4aS,5aR,12aS)-), marimastat (N-(2,2-dimethyl-1 (S)-(N-methylcarbamoyl)propyl)-N,3(S)-dihydroxy-2(R)-isobutylsuccinamide), TIMP'S,ONO-4817, rebimastat (L-Valinamide, N-((2S)-2-mercapto-1-oxo-4-(3,4,4-trimethyl-2,5-dioxo-1-imidazolidinyl)butyl)-L-leucyl-N,3-dimethyl-), PS-508, CH-715, nimesulide (methanesulfonamide, N-(4-nitro-2-phenoxyphenyl)-), hexahydro-2-(2(R)-(1 (RS)-(hydroxycarbamoyl)-4-phenylbutyl)nonanoyl)-N-(2,2,6,6-etramethyl-4-piperidinyl)-3(S)-pyridazine carboxamide, Rs-113-080, Ro-1130830, cipemastat (1-piperidinebutanamide, ß-(cyclopentylmethyl)-N-hydroxy-.gamma.-oxo-.alpha.-((3,4,4-trimethyl-2,5-dioxo-1-imidazolidinyl)methyl)-,(.alpha. R,ßR)-), 5-(4'-biphenyl)-5-(N-(4-nitrophenyl)piperazinyl)barbituric acid, 6-methoxy-1,2,3,4-tetrahydro-norharman-1-carboxylic acid, Ro-31-4724 (L-alanine, N-(2-(2-(hydroxyamino)-2-oxoethyl)-4-methyl-1-oxopentyl)-L-leucyl-, ethyl ester), prinomastat (3-thiomorpholinecarboxamide, N-hydroxy-2,2-dimethyl-4-((4-(4-pyridinyloxy) phenyl)sulfonyl)-, (3R)-), AG-3433 (1H-pyrrole-3-propanic acid, 1-(4'-cyano(1,1'-biphenyl)-4-yl)-b-((((3S)-tetrahydro-4,4-dimethyl-2-oxo-3-furanyl)amino)carbonyl)-, phenylmethyl ester, (bS)-), PNU-142769 (2H-Isoindole-2-butanamide, 1,3-dihydro-N-hydroxy-.alpha.((3S)-3-(2-methylpropyl)-2-oxo-1-(2-phenylethyl)-3-pyrrolidinyl)-1,3-dioxo-, (.alpha. R)-), (S)-1-(2-((((4,5-dihydro-5-thioxo-1,3,4-thiadiazol-2-yl)amino)-carbonyl)amino)-1-oxo-3-(pentafluorophenyl)propyl)-4-(2-pyridinyl)piperazine, SU-5402 (1H-pyrrole-3-propanoic acid, 2-((1,2-dihydro-2-oxo-3H-indol-3-ylidene)methyl)-4-methyl-), SC-77964, PNU-171829, CGS-27023A, N-hydroxy-2(R)-((4-methoxybenzene-sulfonyl)(4-picolyl)amino)-2-(2-tetrahydrofuranyl)-acetamide, L-758354 ((1,1'-biphenyl)-4-hexanoic acid, .alpha.-butyl-.gamma.-(((2,2-dimethyl-1-((methylamino)carbonyl)propyl)amino)carbonyl)-4'-fluoro-, (.alpha. S-(.alpha. R*, .gamma.S*(R*)))-, GI-155704A, CPA-926, TMI-005, XL-784, neovastat, metastat (CMT class), BB3644, BB2827 and TROCADE, or an analogue or derivative thereof). Additional representative examples are included in U.S. Patent Nos. 5,665,777; 5,985,911; 6,288,261; 5,952,320; 6,441,189; 6,235,786; 6,294,573; 6,294,539; 6,563,002; 6,071,903; 6,358,980; 5,852,213; 6,124,502; 6,160,132; 6,197,791; 6,172,057; 6,288,086; 6,342,508; 6,228,869; 5,977,408; 5,929,097; 6,498,167; 6,534,491; 6,548,524; 5,962,481; 6,197,795; 6,162,814; 6,441,023; 6,444,704; 6,462,073; 6,162,821; 6,444,639; 6,262,080; 6,486,193; 6,329,550; 6,544,980; 6,352,976; 5,968,795; 5,789,434; 5,932,763; 6,500,847; 5,925,637; 6,225,314; 5,804,581; 5,863,915; 5,859,047; 5,861,428; 5,886,043; 6,288,063; 5,939,583; 6,166,082; 5,874,473; 5,886,022; 5,932,577; 5,854,277; 5,886,024; 6,495,565; 6,642,255; 6,495,548; 6,479,502; 5,696,082; 5,700,838; 6,444,639; 6,262,080; 6,486,193; 6,329,550; 6,544,980; 6,352,976; 5,968,795; 5,789,434; 5,932,763; 6,500,847; 5,925,637; 6,225,314; 5,804,581; 5,863,915; 5,859,047; 5,861,428; 5,886,043; 6,288,063; 5,939,583; 6,166,082; 5,874,473; 5,886,022; 5,932,577; 5,854,277; 5,886,024; 6,495,565; 6,642,255; 6,495,548; 6,479,502; 5,696,082; 5,700,838; 5,861,436; 5,691,382; 5,763,621; 5,866,717; 5,902,791; 5,962,529; 6,017,889; 6,022,873; 6,022,898; 6,103,739; 6,127,427; 6,258,851; 6,310,084; 6,358,987; 5,872,152; 5,917,090; 6,124,329; 6,329,373; 6,344,457; 5,698,706; 5,872,146; 5,853,623; 6,624,144; 6,462,042; 5,981,491; 5,955,435; 6,090,840; 6,114,372; 6,566,384; 5,994,293; 6,063,786; 6,469,020; 6,118,001; 6,187,924; 6,310,088; 5,994,312; 6,180,611; 6,110,896; 6,380,253; 5,455,262; 5,470,834; 6,147,114; 6,333,324; 6,489,324; 6,362,183; 6,372,758; 6,448,250; 6,492,367; 6,380,258; 6,583,299; 5,239,078; 5,892,112; 5,773,438; 5,696,147; 6,066,662; 6,600,057; 5,990,158; 5,731,293; 6,277,876; 6,521,606; 6,168,807; 6,506,414; 6,620,813; 5,684,152; 6,451,791; 6,476,027; 6,013,649; 6,503,892; 6,420,427; 6,300,514; 6,403,644; 6,177,466; 6,569,899; 5,594,006; 6,417,229; 5,861,510; 6,156,798; 6,387,931; 6,350,907; 6,090,852; 6,458,822; 6,509,337; 6,147,061; 6,114,568; 6,118,016; 5,804,593; 5,847,153; 5,859,061; 6,194,451; 6,482,827; 6,638,952; 5,677,282; 6,365,630; 6,130,254; 6,455,569; 6,057,369; 6,576,628; 6,110,924; 6,472,396; 6,548,667; 5,618,844; 6,495,578; 6,627,411; 5,514,716; 5,256,657; 5,773,428; 6,037,472; 6,579,890; 5,932,595; 6,013,792; 6,420,415; 5,532,265; 5,691,381; 5,639,746; 5,672,598; 5,830,915; 6,630,516; 5,324,634; 6,277,061; 6,140,099; 6,455,570; 5,595,885; 6,093,398; 6,379,667; 5,641,636; 5,698,404; 6,448,058; 6,008,220; 6,265,432; 6,169,103; 6,133,304; 6,541,521; 6,624,196; 6,307,089; 6,239,288; 5,756,545; 6,020,366; 6,117,869; 6,294,674; 6,037,361; 6,399,612; 6,495,568; 6,624,177; 5,948,780; 6,620,835; 6,284,513; 5,977,141; 6,153,612; 6,297,247; 6,559,142; 6,555,535; 6,350,885; 5,627,206; 5,665,764; 5,958,972; 6,420,408; 6,492,422; 6,340,709; 6,022,948; 6,274,703; 6,294,694; 6,531,499; 6,465,508; 6,437,177; 6,376,665; 5,268,384; 5,183,900; 5,189,178; 6,511,993; 6,617,354; 6,331,563; 5,962,466; 5,861,427; 5,830,869; and 6,087,359.

### 23. NF .kappa. B Inhibitors

In another embodiment, the pharmacologically active compound is a NF .kappa. B (NFKB) inhibitor (*e.g*., AVE-0545, Oxi-104 (benzamide, 4-amino-3-chloro-N-(2-(diethylamino)ethyl)-), dexlipotam, R-flurbiprofen ((1,1'-biphenyl)-4-acetic acid, 2-fluoro-.alpha.-methyl), SP100030 (2-chloro-N-(3,5-di(trifluoromethyl)phenyl)-4-(trifluoromethyl)pyrimidine-5-carboxamide), AVE-0545, Viatris, AVE-0547, Bay 11-7082, Bay 11-7085, 15 deoxy-prostaylandin J2, bortezomib (boronic acid, ((1R)-3-methyl-1-(((2S)-1-oxo-3-phenyl-2-((pyrazinylcarbonyl)amino)propyl)amino)butyl)-, benzamide an d nicotinamide derivatives that inhibit NF-.kappa.B, such as those described in U.S. Patent Nos. 5,561,161 and 5,340,565 (OxiGene), PG490-88Na, or an analogue or derivative thereof).

### 24. NO Agonists

In another embodiment, the pharmacologically active compound is a NO antagonist (*e.g*., NCX-4016 (benzoic acid, 2-(acetyloxy)-, 3-((nitrooxy)methyl)phenyl ester, NCX-2216, L-arginine or an analogue or derivative thereof).

### 25. p38 MAP Kinase Inhibitors

In another embodiment, the pharmacologically active compound is a p38 MAP kinase inhibitor (*e.g*., GW-2286, CGP-52411, BIRB-798, SB220025, RO-320-1195, RWJ-67657, RWJ-68354, SCIO-469, SCIO-323, AMG-548, CMC-146, SD-31145, CC-8866, Ro-320-1195, PD-98059 (4H-1-benzopyran-4-one, 2-(2-amino-3-methoxyphenyl)-), CGH-2466, doramapimod, SB-203580 (pyridine, 4-(5-(4-fluorophenyl)-2-(4-(methylsulfinyl)phenyl)-1H-imidazol-4-yl)-), SB-220025 ((5-(2-amino-4-pyrimidinyl)-4-(4-fluorophenyl)-1-(4-piperidinyl)imidazole), SB-281832, PD169316, SB202190, GSK-681323, EO-1606, GSK-681323, or an analogue or derivative thereof). Additional representative examples are included in U.S. Patent Nos. 6,300,347; 6,316,464; 6,316,466; 6,376,527; 6,444,696; 6,479,507; 6,509,361; 6,579,874; 6,630,485, U.S. Patent Application Publication Nos. 2001/0044538A1; 2002/0013354A1; 2002/0049220A1; 2002/0103245A1; 2002/0151491A1; 2002/0156114A1; 2003/0018051A1; 2003/0073832A1; 2003/0130257A1; 2003/0130273A1; 2003/0130319A1; 2003/0139388A1; 20030139462A1.; 2003/0149031A1; 2003/0166647A1; 2003/0181411A1; and PCT Publication Nos. WO 00/63204A2; WO 01/21591A1; WO 01/35959A1; WO 01/74811A2; WO 02/18379A2; WO 2064594A2; WO 2083622A2; WO 2094842A2; WO 2096426A1; WO 2101015A2; WO 2103000A2; WO 3008413A1; WO 3016248A2; WO 3020715A1; WO 3024899A2; WO 3031431A1; W03040103A1; WO 3053940A1; WO 3053941A2; WO 3063799A2; WO 3079986A2; WO 3080024A2; WO 3082287A1; WO 97/44467A1; WO 99/01449A1; and WO 99/58523A1.

### 26. Phosphodiesterase Inhibitors

In another embodiment, the pharmacologically active compound is a phosphodiesterase inhibitor (*e.g*., CDP-840 (pyridine, 4-((2R)-2-(3-(cyclopentyloxy)-4-methoxyphenyl)-2-phenylethyl)-), CH-3697, CT-2820, D-22888 (imidazo(1,5-a)pyrido(3,2-e)pyrazin-6(5H)-one, 9-ethyl-2-methoxy-7-methyl-5-propyl-), D-4418 (8-methoxyquinoline-5-(N-(2,5-dichloropyridin-3-yl))carboxamide), 1-(3-cyclopentyloxy-4-methoxyphenyl)-2-(2,6-dichloro-4-pyridyl) ethanone oxime, D-4396, ONO-6126, CDC-998, CDC-801, V-11294A (3-(3-(cyclopentyloxy)-4-methoxybenzyl)-6-(ethylamino)-8-isopropyl-3H-purine hydrochloride), S,S'-methylene-bis(2-(8-cyclopropyl-3-propyl-6-(4-pyridylmethylamino)-2-thio-3H-purine)) tetrahyrochloride, rolipram (2-pyrrolidinone, 4-(3-(cyclopentyloxy)-4-methoxyphenyl)-), CP-293121, CP-353164 (5-(3-cyclopentyloxy-4-methoxyphenyl)pyridine-2-carboxamide), oxagrelate (6-phthalazinecarboxylic acid, 3,4-dihydro-1-(hydroxymethyl)-5,7-dimethyl-4-oxo-, ethyl ester), PD-168787, ibudilast (1-propanone, 2-methyl-1-(2-(1-methylethyl)pyrazolo(1,5-a)pyridin-3-yl)-), oxagrelate (6-phthalazinecarboxylic acid, 3,4-dihydro-1-(hydroxymethyl)-5,7-dimethyl-4-oxo-, ethyl ester), griseolic acid (.alpha.-L-talo-oct-4-enofuranuronic acid, 1-(6-amino-9H-purin-9-yl)-3,6-anhydro-6-C-carboxy-1,5-dideoxy-), KW-4490, KS-506, T-440, roflumilast (benzamide, 3-(cyclopropylmethoxy)-N-(3,5-dichloro-4-pyridinyl)-4-(difluoromethoxy)-), rolipram, milrinone, triflusinal (benzoic acid, 2-(acetyloxy)-4-(trifluoromethyl)-), anagrelide hydrochloride (imidazo(2,1-b)quinazolin-2(3H)-one, 6,7-dichloro-1,5-dihydro-, monohydrochloride), cilostazol (2(1H)-quinolinone, 6-(4-(1-cyclohexyl-1H-tetrazol-5-yl)butoxy)-3,4-dihydro-), propentofylline (1H-purine-2,6-dione, 3,7-dihydro-3-methyl-1-(5-oxohexyl)-7-propyl-), sildenafil citrate (piperazine, 1-((3-(4,7-dihydro-1-methyl-7-oxo-3-propyl-1H-pyrazolo(4,3-d)pyrimidin-5-yl)-4-ethoxyphenyl)sulfonyl)-4-methyl, 2-hydroxy-1,2,3-propanetricarboxylate- (1:1)), tadalafil (pyrazino(1',2':1,6)pyrido(3,4-b)indole1,4-dione, 6-(1,3-benzodioxol-5-yl)-2,3,6,7,12,12a-hexahydro-2-methyl-, (6R-trans)), vardenafil (piperazine, 1-(3-(1,4-dihydro-5-methyl(-4-oxo-7-propylimidazo(5,1-f)(1,2,4)-triazin-2-yl)-4-ethoxyphenyl)sulfonyl)-4-ethyl-), milrinone ((3,4'-bipyridine)-5-carbonitrile, 1,6-dihydro-2-methyl-6-oxo-), enoximone (2H-imidazol-2-one, 1,3-dihydro-4-methyl-5-(4-(methylthio)benzoyl)-), theophylline (1H-purine-2,6-dione, 3,7-dihydro-1,3-dimethyl-), ibudilast (1-propanone, 2-methyl-1-(2-(1-methylethyl)pyrazolo(1,5-a)pyridin-3-yl)-), aminophylline (1H-purine-2,6-dione, 3,7-dihydro-1,3-dimethyl-, compound with 1,2-ethanediamine (2:1)-), acebrophylline (7H-purine-7-acetic acid, 1,2,3,6-tetrahydro-1,3-dimethyl-2,6-dioxo-, compd. with trans-4-(((2-amino-3,5-dibromophenyl)methyl)amino)cyclohexanol (1:1)), plafibride (propanamide, 2-(4-chlorophenoxy)-2-methyl-N-(((4-morpholinylmethyl)amino)carbonyl)-), ioprinone hydrochloride (3-pyridinecarbonitrile, 1,2-dihydro-5-imidazo(1,2-a)pyridin-6-yl-6-methyl-2-oxo-, monohydrochloride-), fosfosal (benzoic acid, 2-(phosphonooxy)-), amrinone ((3,4'-bipyridin)-6(1H)-one, 5-amino-, or an analogue or derivative thereof).

Other examples of phosphodiesterase inhibitors include denbufylline (1H-purine-2,6-dione, 1,3-dibutyl-3,7-dihydro-7-(2-oxopropyl)-), propentofylline (1H-purine-2,6-dione, 3,7-dihydro-3-methyl-1-(5-oxohexyl)-7-propyl-) and pelrinone (5-pyrimidinecarbonitrile, 1,4-dihydro-2-methyl-4-oxo-6-[(3-pyridinylmethyl)amino]-).

Other examples of phosphodiesterase III inhibitors include enoximone (2H-imidazol-2-one, 1,3-dihydro-4-methyl-5-[4-(methylthio)benzoyl]-), and saterinone (3-pyridinecarbonitrile, 1,2-dihydro-5-[4-[2-hydroxy-3-[4-(2-methoxyphenyl)-1-piperazinyl]propoxy]phenyl]-6-methyl-2-oxo-).

Other examples of phosphodiesterase IV inhibitors include AWD-12-281, 3-auinolinecarboxylic acid, 1-ethyl-6-fluoro-1,4-dihydro-7-(4-methyl-1-piperazinyl)-4-oxo-), tadalafil (pyrazino(1',2':1,6)pyrido(3,4-b)indole1,4-dione, 6-(1,3-benzodioxol-5-yl)-2,3,6,7,12,12a-hexahydro-2-methyl-, (6R-trans)), and filaminast (ethanone, 1-[3-(cyclopentyloxy)-4-methoxyphenyl]-, O-(aminocarbonyl)oxime, (1E)-)

Another example of a phosphodiesterase V inhibitor is vardenafil (piperazine, 1-(3-(1,4-dihydro-5-methyl(-4-oxo-7-propylimidazo(5,1-f)(1,2,4)-triazin-2-yl)-4-ethoxyphenyl)sulfonyl)-4-ethyl-).

### 27. TGF beta Inhibitors

In another embodiment, the pharmacologically active compound is a TGF beta inhibitor (*e.g*., mannose-6-phosphate, LF-984, tamoxifen (ethanamine, 2-(4-(1,2-diphenyl-1-butenyl)phenoxy)-N,N-dimethyl-, (Z)-), tranilast, or an analogue or derivative thereof).

### 28. Thromboxane A2 Antagonists

In another embodiment, the pharmacologically active compound is a thromboxane A2 antagonist (*e.g*., CGS-22652 (3-pyridineheptanoic acid, γ-(4-(((4-chlorophenyl)sulfonyl)amino)butyl)-, (.+-.)-), ozagrel (2-propenoic acid, 3-(4-(1H-imidazol-1-ylmethyl)phenyl)-, (E)-), argatroban (2-piperidinecarboxylic acid, 1-(5-((aminoiminomethyl)amino)-1-oxo-2-(((1,2,3,4-tetrahydro-3-methyl-8-quinolinyl)sulfonyl)amino)pentyl)-4-methyl-), ramatroban (9H-carbazole-9-propanoic acid, 3-(((4-fluorophenyl)sulfonyl)amino)-1,2,3,4-tetrahydro-, (R)-), torasemide (3-pyridinesulfonamide, N-(((1-methylethyl)amino)carbonyl)-4-((3-methylphenyl)amino)-), gamma linoleic acid ((Z,Z,Z)-6,9,12-octadecatrienoic acid), seratrodast (benzeneheptanoic acid, zeta-(2,4,5-trimethyl-3,6-dioxo-1,4-cyclohexadien-1-yl)-, (+/-)-, or an analogue or derivative thereof).

### 29. TNFa Antagonists and TACE Inhibitors

In another embodiment, the pharmacologically active compound is a TNFa antagonist or TACE inhibitor (*e.g*., E-5531 (2-deoxy-6-0-(2-deoxy-3-0-(3(R)-(5(Z)-dodecenoyloxy)-decyl)-6-0-methyl-2-(3-oxotetradecanamido)-4-O-phosphono-ß-D-glucopyranosyl)-3-0-(3(R)-hydroxydecyl)-2-(3-oxotetradecanamido)-.alpha.-D-glucopyranose-1-O-phosphate), AZD-4717, glycophosphopeptical, UR-12715 (B=benzoic acid, 2-hydroxy-5-((4-(3-(4-(2-methyl-1H-imidazol(4,5-c)pyridin-1-yl)methyl)-1-piperidinyl)-3-oxo-1-phenyl-1-propenyl)phenyl)azo) (Z)), PMS-601, AM-87, xyloadenosine (9H-purin-6-amine, 9-ß-D-xylofuranosyl-), RDP-58, RDP-59, BB2275, benzydamine, E-3330 (undecanoic acid, 2-((4,5-dimethoxy-2-methyl-3,6-dioxo-1,4-cyclohexadien-1-yl)methylene)-, (E)-), N-(D,L-2-(hydroxyaminocarbonyl)methyl-4-methylpentanoyl)-L-3-(2'-naphthyl)alanyl-L-alanine, 2-aminoethyl amide, CP-564959, MLN-608, SPC-839, ENMD-0997, Sch-23863 ((2-(10,11-dihydro-5-ethoxy-5H-dibenzo (a,d) cyclohepten-S-yl)-N, N-dimethyl-ethanamine), SH-636, PKF-241-466, PKF-242-484, TNF-484A, cilomilast (cis-4-cyano-4-(3-(cyclopentyloxy)-4-methoxyphenyl)cyclohexane-1-carboxylic acid), GW-3333, GW-4459, BMS-561392, AM-87, cloricromene (acetic acid, ((8-chloro-3-(2-(diethylamino)ethyl)-4-methyl-2-oxo-2H-1-benzopyran-7-yl)oxy)-, ethyl ester), thalidomide (1H-Isoindole-1,3(2H)-dione, 2-(2,6-dioxo-3-piperidinyl)-), vesnarinone (piperazine, 1-(3,4-dimethoxybenzoyl)-4-(1,2,3,4-tetrahydro-2-oxo-6-quinolinyl)-), infliximab, lentinan, etanercept (1-235-tumor necrosis factor receptor (human) fusion protein with 236-467-immunoglobulin G1 (human .gamma.1-chain Fc fragment)), diacerein (2-anthracenecarboxylic acid, 4,5-bis(acetyloxy)-9,10-dihydro-9,10-dioxo-, CDP-870, D2E7, PEG-sTNF-R1, or an analogue or derivative thereof).

### 30. Tyrosine Kinase Inhibitors

In another embodiment, the pharmacologically active compound is a tyrosine kinase inhibitor (*e.g*., SKI-606, ER-068224, SD-208, N-(6-benzothiazolyl)-4-(2-(1-piperazinyl)pyrid-5-yl)-2-pyrimidineamine, celastrol (24,25,26-trinoroleana-1(10),3,5,7-tetraen-29-oic acid, 3-hydroxy-9,13-dimethyl-2-oxo-, (9 beta., 13.alpha., 14β,20 .alpha.)-), CP-127374 (geldanamycin, 17-demethoxy-17-(2-propenylamino)-), CP-564959, PD-171026, CGP-52411 (1H-Isoindole-1,3(2H)-dione, 4,5-bis(phenylamino)-), CGP-53716 (benzamide, N-(4-methyl-3-((4-(3-pyridinyl)-2-pyrimidinyl)amino)phenyl)-), imatinib (4-((methyl-1-piperazinyl)methyl)-N-(4-methyl-3-((4-(3-pyridinyl)-2-pyrimidinyl)amino)-phenyl)benzamide methanesulfonate), NVP-AAK980-NX, KF-250706 (13-chloro,5(R),6(S)-epoxy-14,16-dihydroxy-11-(hydroyimino)-3(R)-methyl-3,4,5,6,11,12-hexahydro-1H-2-benzoxacyclotetradecin-1-one), 5-(3-(3-methoxy-4-(2-((E)-2-phenylethenyl)-4-oxazolylmethoxy)phenyl)propyl)-3-(2-((E)-2-phenylethenyl)-4-oxazolylmethyl)-2,4-oxazolidinedione, genistein, NV-06, or an analogue or derivative thereof).

### 31. Vitronectin Inhibitors

In another embodiment, the pharmacologically active compound is a vitronectin inhibitor (*e.g*., O-(9,10-dimethoxy-1,2,3,4,5,6-hexahydro-4-((1,4,5,6-tetrahydro-2-pyrimidinyl)hydrazono)-8-benz(e)azulenyl)-N-((phenylmethoxy)carbonyl)-DL-homoserine 2,3-dihydroxypropyl ester, (2S)-benzoylcarbonylamino-3-(2-((4S)-(3-(4,5-dihydro-1H-imidazol-2-ylamino)-propyl)-2,5-dioxo-imidazolidin-1-yl)-acetylamino)-propionate, Sch-221153, S-836, SC-68448 (ß-((2-2-(((3-((aminoiminomethyl)amino)-phenyl)carbonyl)amino)acetyl)amino)-3,5-dichlorobenzenepropanoic acid), SD-7784, S-247, or an analogue or derivative thereof).

### 32. Fibroblast Growth Factor Inhibitors

In another embodiment, the pharmacologically active compound is a fibroblast growth factor inhibitor (*e.g*., CT-052923 (((2H-benzo(d)1,3-dioxalan-5-methyl)amino)(4-(6,7-dimethoxyquinazolin-4-yl)piperazinyl)methane-1-thione), or an analogue or derivative thereof).

### 33. Protein Kinase Inhibitors

In another embodiment, the pharmacologically active compound is a protein kinase inhibitor (*e.g*., KP-0201448, NPC15437 (hexanamide, 2,6-diamino-N-((1-(1-oxotridecyl)-2-piperidinyl)methyl)-), fasudil (1H-1,4-diazepine, hexahydro-1-(5-isoquinolinylsulfonyl)-), midostaurin (benzamide, N-(2,3,10,11,12,13-hexahydro-10-methoxy-9-methyl-1-oxo-9,13-epoxy-1H,9H-diindolo(1,2,3-gh:3',2',1'-Im)pyrrolo(3,4-j)(1,7)benzodiazonin-11-yl)-N-methyl-, (9.alpha.,10ß,11ß,13.alpha.)-),fasudil (1H-1,4-diazepine, hexahydro-1-(5-isoquinolinylsulfonyl)-, dexniguldipine (3,5-pyridinedicarboxylic acid, 1,4-dihydro-2,6-dimethyl-4-(3-nitrophenyl)-, 3-(4,4-diphenyl-1-piperidinyl)propyl methyl ester, monohydrochloride, (R)-), LY-317615 (1H-pyrole-2,5-dione, 3-(1-methyl-1H-indol-3-yl)-4-[1-[1-(2-pyridinylmethyl)-4-piperidinyl]-1H-indol-3-yl]-, monohydrochloride), perifosine (piperidinium, 4-[[hydroxy(octadecyloxy)phosphinyl]oxy]-1,1-dimethyl-, inner salt), LY-333531 (9H,18H-5,21:12,17-dimethenodibenzo(e,k)pyrrolo(3,4-h)(1,4,13)oxadiazacyclohexadecine-18,20(19H)-dione,9-((dimethylamino)methyl)-6,7,10,11-tetrahydro-, (S)-), Kynac; SPC-100270 (1,3-octadecanediol, 2-amino-, [S-(R*,R*)]-), Kynacyte, or an analogue or derivative thereof).

### 34. PDGF Receptor Kinase Inhibitors

In another embodiment, the pharmacologically active compound is a PDGF receptor kinase inhibitor (*e.g*., RPR-127963E, or an analogue or derivative thereof).

### 35. Endothelial Growth Factor Receptor Kinase Inhibitors

In another embodiment, the pharmacologically active compound is an endothelial growth factor receptor kinase inhibitor (*e.g*., CEP-7055, SU-0879 ((E)-3-(3,5-di-tert-butyl-4-hydroxyphenyl)-2-(aminothiocarbonyl)acrylonitrile), BIBF-1000, AG-013736 (CP-868596), AMG-706, AVE-0005, NM-3 (3-(2-methylcarboxymethyl)-6-methoxy-8-hydroxy-isocoumarin), Bay-43-9006, SU-011248, or an analogue or derivative thereof).

### 36. Retinoic Acid Receptor Antagonists

In another embodiment, the pharmacologically active compound is a retinoic acid receptor antagonist (*e.g*., etarotene (Ro-15-1570) (naphthalene, 6-(2-(4-(ethylsulfonyl)phenyl)-1-methylethenyl)-1,2,3,4-tetrahydro-1,1,4,4-tetramethyl-, (E)-), (2E,4E)-3-methyl-5-(2-((E)-2-(2,6,6-trimethyl-1-cyclohexen-1-yl)ethenyl)-1-cyclohexen-1-yl)-2,4-pentadienoic acid, tocoretinate (retinoic acid, 3,4-dihydro-2,5,7,8-tetramethyl-2-(4,8,12-trimethyltridecyl)-2H-1-benzopyran-6-yl ester, (2R*(4R*,8R*))-(±)-), aliretinoin (retinoic acid, cis-9, trans-13-), bexarotene (benzoic acid, 4-(1-(5,6,7,8-tetrahydro-3,5,5,8,8-pentamethyl-2-naphthalenyl)ethenyl)-), tocoretinate (retinoic acid, 3,4-dihydro-2,5,7,8-tetramethyl-2-(4,8,12-trimethyltridecyl)-2H-1-benzopyran-6-yl ester, [2R*(4R*,8R*)]-(±)-, or an analogue or derivative thereof).

### 37. Platelet Derived Growth Factor Receptor Kinase Inhibitors

In another embodiment, the pharmacologically active compound is a platelet derived growth factor receptor kinase inhibitor (*e.g*., leflunomide (4-isoxazolecarboxamide, 5-methyl-N-(4-(trifluoromethyl)phenyl)-, or an analogue or derivative thereof).

### 38. Fibronogin Antagonists

In another embodiment, the pharmacologically active compound is a fibrinogin antagonist (*e.g*., picotamide (1,3-benzenedicarboxamide, 4-methoxy-N,N'-bis(3-pyridinylmethyl)-, or an analogue or derivative thereof).

### 39. Antimycotic Agents

In another embodiment, the pharmacologically active compound is an antimycotic agent (*e.g*., miconazole, sulconizole, parthenolide, rosconitine, nystatin, isoconazole, fluconazole, ketoconasole, imidazole, itraconazole, terpinafine, elonazole, bifonazole, clotrimazole, conazole, terconazole (piperazine, 1-(4-((2-(2,4-dichlorophenyl)-2-(1H-1,2,4-triazol-1-ylmethyl)-1,3-dioxolan-4-yl)methoxy)phenyl)-4-(1-methylethyl)-, cis-), isoconazole (1-(2-(2-6-dichlorobenzyloxy)-2-(2-,4-dichlorophenyl)ethyl)), griseofulvin (spiro(benzofuran-2(3H),1'-(2)cyclohexane)3,4'-dione, 7-chloro-2',4,6-trimethoxy-6'methyl-, (1'S-trans)-), bifonazole (1H-imidazole, 1-((1,1'-biphenyl)-4-ylphenylmethyl)-), econazole nitrate (1-(2-((4-chlorophenyl)methoxy)-2-(2,4-dichlorophenyl)ethyl)-1H-imidazole nitrate), croconazole (1H-imidazole, 1-(1-(2-((3-chlorophenyl)methoxy)phenyl)ethenyl)-), sertaconazole (1H-Imidazole, 1-(2-((7-chlorobenzo(b)thien-3-yl)methoxy)-2-(2,4-dichlorophenyl)ethyl)-), omoconazole (1H-imidazole, 1-(2-(2-(4-chlorophenoxy)ethoxy)-2-(2,4-dichlorophenyl)-1-methylethenyl)-, (Z)-), flutrimazole (1H-imidazole, 1-((2-fluorophenyl)(4-fluorophenyl)phenylmethyl)-), fluconazole (1H-1,2,4-triazole-1-ethanol, .a)pha.-(2,4-difluorophenyl)-.alpha.-(1H-1,2,4-triazol-1-ylmethyl)-), neticonazole (1H-imidazole, 1-(2-(methylthio)-1-(2-(pentyloxy)phenyl)ethenyl)-, monohydrochloride, (E)-), butoconazole (1H-imidazole, 1-(4-(4-chlorophenyl)-2-((2,6-dichlorophenyl)thio)butyl)-, (+/-)-), clotrimazole (1-((2-chlorophenyl)diphenylmethyl)-1H-imidazole, nystatin or an analogue or derivative thereof).

### 40. Bisphosphonates

In another embodiment, the pharmacologically active compound is a bisphosphonate (*e.g*., clodronate, alendronate, pamidronate, zoledronate, or an analogue or derivative thereof).

### 41. Phospholipase A1 Inhibitors

In another embodiment, the pharmacologically active compound is a phospholipase A1 inhibitor (*e.g*., ioteprednol etabonate (androsta-1,4-diene-1 7-carboxylic acid, 17-((ethoxycarbonyl)oxy)-11-hydroxy-3-oxo-, chloromethyl ester, (11ß,17 .alpha.)-, or an analogue or derivative thereof).

### 42. Histamine H1/H2/H3 Receptor Antagonists

In another embodiment, the pharmacologically active compound is a histamine H1, H2, or H3 receptor antagonist (*e.g*., ranitidine (1,1-ethenediamine, N-(2-(((5-((dimethylamino)methyl)-2-furanyl)methyl)thio)ethyl)-N'-methyl-2-nitro-), niperotidine (N-(2-((5-((dimethylamino)methyl)furfuryl)thio)ethyl)-2-nitro-N'-piperonyl-1,1-ethenediamine), famotidine (propanimidamide, 3-(((2-((aminoiminomethyl)amino)-4-thiazolyl)methyl)thio)-N-(aminosulfonyl)-), roxitadine acetate HCl (acetamide, 2-(acetyloxy)-N-(3-(3-(1-piperidinylmethyl)phenoxy)propyl)-, monohydrochloride), lafutidine (acetamide, 2-((2-furanylmethyl)sulfinyl)-N-(4-((4-(1-piperidinylmethyl)-2-pyridinyl)oxy)-2-butenyl)-, (Z)-), nizatadine (1,1-ethenediamine, N-(2-(((2-((dimethylamino)methyl)-4-thiazolyl)methyl)thio)ethyl)-N'-methyl-2-nitro-), ebrotidine (benzenesulfonamide, N-(((2-(((2-((aminoiminomethyl)amino)-4-thiazoly)methyl)thio)ethyl)amino)methylene)-4-bromo-), rupatadine (5H-benzo(5,6)cyclohepta(1,2-b)pyridine, 8-chloro-6,11-dihydro-11-(1-((5-methyl-3-pyridinyl)methyl)-4-piperidinylidene)-, trihydrochloride-), fexofenadine HCl (benzeneacetic acid, 4-(1-hydroxy-4-(4(hydroxydiphenylmethyl)-1-piperidinyl)butyl)-.alpha., .alpha.-dimethyl-, hydrochloride, or an analogue or derivative thereof).

### 43. Macrolide Antibiotics

In another embodiment, the pharmacologically active compound is a macrolide antibiotic (*e.g*., dirithromycin (erythromycin, 9-deoxo-11-deoxy-9,11-(imino(2-(2-methoxyethoxy)ethylidene)oxy)-, (9S(R))-), flurithromycin ethylsuccinate (erythromycin, 8-fluoro-mono(ethyl butanedioate) (ester)-), erythromycin stinoprate (erythromycin, 2'-propanoate, compound with N-acetyl-L-cysteine (1:1)), clarithromycin (erythromycin, 6-O-methyl-), azithromycin (9-deoxo-9a-aza-9a-methyl-9a-homoerythromycin-A), telithromycin (3-de((2,6-dideoxy-3-C-methyl-3-O-methyl-.alpha.-L-ribo-hexopyranosyl)oxy)11,12-dideoxy-6-O-methyl-3-oxo-12,11-(oxycarbonyl((4-(4-(3-pyridinyl)-1H-imidazol-1-yl)butyl)imino))-), roxithromycin (erythromycin, 9-(O-((2-methoxyethoxy)methyl)oxime)), rokitamycin (leucomycin V, 4B-butanoate 3B-propanoate), RV-11 (erythromycin monopropionate mercaptosuccinate), midecamycin acetate (leucomycin V, 3B,9-diacetate 3,4B-dipropanoate), midecamycin (leucomycin V, 3,4B-dipropanoate), josamycin (leucomycin V, 3-acetate 4B-(3-methylbutanoate), or an analogue or derivative thereof).

### 44. GPIIb/IIIa Receptor Antagonists

In another embodiment, the pharmacologically active compound is a GPIIb or GPIIIa receptor antagonist (*e.g*., tirofiban hydrochloride (L-tyrosine, N-(butylsulfonyl)-O-(4-(4-piperidinyl)butyl)-, monohydrochloride-), eptifibatide (L-cysteinamide, N6-(aminoiminomethyl)-N2-(3-mercapto-1-oxopropyl)-L-lysylglycyl-L-.alpha.-aspartyl-L-tryptophyl-L-prolyl-, cyclic(1->6)-disulfide), xemilofiban hydrochloride, or an analogue or derivative thereof).

### 45. Endothelin Receptor Antagonists

In another embodiment, the pharmacologically active compound is an endothelin receptor antagonist (*e.g*., bosentan (benzenesulfonamide, 4-(1,1-dimethylethyl)-N-(6-(2-hydroxyethoxy)-5-(2-methoxyphenoxy)(2,2'-bipyrimidin)-4-yl)-, or an analogue or derivative thereof).

### 46. Peroxisome Proliferator-Activated Receptor Agonists

In another embodiment, the pharmacologically active compound is a peroxisome proliferator-activated receptor agonist (*e.g*., gemfibrozil (pentanoic acid, 5-(2,5-dimethylphenoxy)-2,2-dimethyl-), fenofibrate (propanoic acid, 2-(4-(4-chlorobenzoyl)phenoxy)-2-methyl-, 1-methylethyl ester), ciprofibrate (propanoic acid, 2-(4-(2,2-dichlorocyclopropyl)phenoxy)-2-methyl-), rosiglitazone maleate (2,4-thiazolidinedione, 5-((4-(2-(methyl-2-pyridinylamino)ethoxy)phenyl)methyl)-, (Z)-2-butenedioate (1:1)), pioglitazone hydrochloride (2,4-thiazolidinedione, 5-((4-(2-(5-ethyl-2-pyridinyl)ethoxy)phenyl)methyl)-, monohydrochloride (+/-)-), etofylline clofibrate (propanoic acid, 2-(4-chlorophenoxy)-2-methyl-, 2-(1,2,3,6-tetrahydro-1,3-dimethyl-2,6-dioxo-7H-purin-7-yl)ethyl ester), etofibrate (3-pyridinecarboxylic acid, 2-(2-(4-chlorophenoxy)-2-methyl-1-oxopropoxy)ethyl ester), clinofibrate (butanoic acid, 2,2'-(cyclohexylidenebis(4,1-phenyleneoxy))bis(2-methyl-)), bezafibrate (propanoic acid, 2-(4-(2-((4-chlorobenzoyl)amino)ethyl)phenoxy)-2-methyl-), binifibrate (3-pyridinecarboxylic acid, 2-(2-(4-chlorophenoxy)-2-methyl-1-oxopropoxy)-1,3-propanediyl ester), or an analogue or derivative thereof).

In one aspect, the pharmacologically active compound is a peroxisome proliferator-activated receptor .alpha. agonist, such as GW-590735, GSK-677954, GSK501516, pioglitazone hydrochloride (2,4-thiazolidinedione, 5-[[4-[2-(5-ethyl-2-pyridinyl)ethoxy]phenyl]methyl]-, monohydrochloride (+/-)-, or an analogue or derivative thereof).

### 47. Estrogen Receptor Agents

In another embodiment, the pharmacologically active compound is an estrogen receptor agent (*e.g*., estradiol, 17-β-estradiol, or an analogue or derivative thereof).

### 48. Somatostatin Analogues

In another embodiment, the pharmacologically active compound is a somatostatin analogue (*e.g*., angiopeptin, or an analogue or derivative thereof).

### 49. Neurokinin 1 Antagonists

In another embodiment, the pharmacologically active compound is a neurokinin 1 antagonist (*e.g*., GW-597599, lanepitant ((1,4'-bipiperidine)-1'-acetamide, N-(2-(acetyl((2-methoxyphenyl)methyl)amino)-1-(1H-indol-3-ylmethyl)ethyl)- (R)-), nolpitantium chloride (1-azoniabicyclo[2.2.2]octane, 1-[2-[3-(3,4-dichlorophenyl)-1-[[3-(1-methylethoxy)phenyl]acetyl]-3-piperidinyl]ethyl]-4-phenyl-, chloride, (S)-), or saredutant (benzamide, N-[4-[4-(acetylamino)-4-phenyl-1-piperidinyl]-2-(3,4-dichlorophenyl)butyl]-N-methyl-, (S)-), or vofopitant (3-piperidinamine, N-[[2-methoxy-5-[5-(trifluoromethyl)-1H-tetrazol-1-yl]phenyl]methyl]-2-phenyl-, (2S,3S)-, or an analogue or derivative thereof).

### 50. Neurokinin 3 Antagonist

In another embodiment, the pharmacologically active compound is a neurokinin 3 antagonist (*e.g*., talnetant (4-quinolinecarboxamide, 3-hydroxy-2-phenyl-N-[(1S)-1-phenylpropyl]-, or an analogue or derivative thereof).

### 51. Neurokinin Antagonis

In another embodiment, the pharmacologically active compound is a neurokinin antagonist (*e.g*., GSK-679769, GSK-823296, SR-489686 (benzamide, N-[4-[4-(acetylamino)-4-phenyl-1-piperidinyl]-2-(3,4-dichlorophenyl)butyl]-N-methyl-, (S)-), SB-223412; SB-235375 (4-quinolinecarboxamide, 3-hydroxy-2-phenyl-N-[(1S)-1-phenylpropyl]-), UK-226471, or an analogue or derivative thereof).

### 52. VLA-4 Antagonist

In another embodiment, the pharmacologically active compound is a VLA-4 antagonist (*e.g*., GSK683699, or an analogue or derivative thereof).

### 53. Osteoclast Inhibitor

In another embodiment, the pharmacologically active compound is a osteoclast inhibitor (*e.g*., ibandronic acid (phosphonic acid, [1-hydroxy-3-(methylpentylamino)propylidene] bis-), alendronate sodium, or an analogue or derivative thereof).

### 54. DNA topoisomerase ATP Hydrolysing Inhibitor

In another embodiment, the pharmacologically active compound is a DNA topoisomerase ATP hydrolysing inhibitor (*e.g*., enoxacin (1,8-naphthyridine-3-carboxylic acid, 1-ethyl-6-fluoro-1,4-dihydro-4-oxo-7-(1-piperazinyl)-), levofloxacin (7H-pyrido[1,2,3-de]-1,4-benzoxazine-6-carboxylic acid, 9-fluoro-2,3-dihydro-3-methyl-10-(4-methyl-1-piperazinyl)-7-oxo-, (S)-), ofloxacin (7H-pyrido[1,2,3-de]-1,4-benzoxazine-6-carboxylic acid, 9-fluoro-2,3-dihydro-3-methyl-10-(4-methyl-1-piperazinyl)-7-oxo-, (+/-)-), pefloxacin (3-quinolinecarboxylic acid, 1-ethyl-6-fluoro-1,4-dihydro-7-(4-methyl-1-piperazinyl)-4-oxo-), pipemidic acid (pyrido[2,3-d]pyrimidine-6-carboxylic acid, 8-ethyl-5,8-dihydro-5-oxo-2-(1-piperazinyl)-), pirarubicin (5,12-naphthacenedione, 10-[[3-amino-2,3,6-trideoxy-4-O-(tetrahydro-2H-pyran-2-yl)-.alpha.-L-lyxo-hexopyranosyl]oxy]-7,8,9,10-tetrahydro-6,8,11-trihydroxy-8-(hydroxyacetyl)-1-methoxy-, [8S-[8 .alpha.,10 .alpha.(S*)]]-), sparfloxacin (3-quinolinecarboxylic acid, 5-amino-1-cyclopropyl-7-(3,5-dimethyl-1-piperazinyl)-6,8-difluoro-1,4-dihydro-4-oxo-, cis-), AVE-6971, cinoxacin ([1,3]dioxolo[4,5-g]cinnoline-3-carboxylic acid, 1-ethyl-1,4-dihydro-4-oxo-), or an analogue or derivative thereof).

### 55. Angiotensin I Converting Enzyme Inhibitor

In another embodiment, the pharmacologically active compound is an angiotensin I converting enzyme inhibitor (*e.g*., ramipril (cyclopenta[b]pyrrole-2-carboxylic acid, 1-[2-[[1-(ethoxycarbonyl)-3-phenylpropyl]amino]-1-oxopropyl]octahydro-, [2S-[1 [R*(R*)],2 .alpha., 3aß, 6aß]]-), trandolapril (1H-indole-2-carboxylic acid, 1-[2-[(1-carboxy-3-phenylpropyl)amino]-1-oxopropyl]octahydro-, [2S-[1[R*(R*)],2 .alpha.,3a .alpha.,7aß]]-), fasidotril (L-alanine, N-[(2S)-3-(acetylthio)-2-(1,3-benzodioxol-5-ylmethyl)-1-oxopropyl]-, phenylmethyl ester), cilazapril (6H-pyridazino[1,2-a][1,2]diazepine-1-carboxylic acid, 9-[[1-(ethoxycarbonyl)-3-phenylpropyl]amino]octahydro-10-oxo-, [1S-[1 .alpha., 9.alpha.(R*)]]-), ramipril (cyclopenta[b]pyrrole-2-carboxylic acid, 1-[2-[[1-(ethoxycarbonyl)-3-phenylpropyl]amino]-1-oxopropyl]octahydro-, [2S-[1[R*(R*)], 2 .alpha.,3aß,6aß]]-, or an analogue or derivative thereof).

### 56. Angiotensin II Antagonist

In another embodiment, the pharmacologically active compound is an angiotensin II antagonist (*e.g*., HR-720 (1H-imidazole-5-carboxylic acid, 2-butyl-4-(methylthio)-1-[[2'-[[[(propylamino)carbonyl]amino]sulfonyl][1,1'-biphenyl]-4-yl]methyl]-, dipotassium salt, or an analogue or derivative thereof).

### 57. Enkephalinase Inhibitor

In another embodiment, the pharmacologically active compound is an enkephalinase inhibitor (*e.g*., Aventis 100240 (pyrido[2,1-a][2]benzazepine-4-carboxylic acid, 7-[[2-(acetylthio)-1-oxo-3-phenylpropyl]amino]-1,2,3,4,6,7,8,12b-octahydro-6-oxo-, [4S-[4 .alpha., 7 .alpha.(R*),12bß]]-), AVE-7688, or an analogue or derivative thereof).

### 58. Peroxisome Proliferator-Activated Receptor Gamma Agonist Insulin Sensitizer

In another embodiment, the pharmacologically active compound is peroxisome proliferator-activated receptor gamma agonist insulin sensitizer (*e.g*., rosiglitazone maleate (2,4-thiazolidinedione, 5-((4-(2-(methyl-2-pyridinylamino)ethoxy)phenyl)methyl)-, (Z)-2-butenedioate (1:1), farglitazar (Gl-262570, GW-2570, GW-3995, GW-5393, GW-9765), LY-929, LY-519818, LY-674, or LSN-862), or an analogue or derivative thereof).

### 59. Protein Kinase C Inhibitor

In another embodiment, the pharmacologically active compound is a protein kinase C inhibitor, such as ruboxistaurin mesylate (9H,18H-5,21:12,17-dimethenodibenzo(e,k)pyrrolo(3,4-h)(1,4,13)oxadiazacyclohexadecine-18,20(19H)-dione,9-((dimethylamino)methyl)-6,7,10,11-tetrahydro-, (S)-), safingol (1,3-octadecanediol, 2-amino-, [S-(R*,R*)]-), or enzastaurin hydrochloride (1H-pyrole-2,5-dione, 3-(1-methyl-1H-indol-3-yl)-4-[1-[1-(2-pyridinylmethyl)-4-piperidinyl]-1H-indol-3-yl]-, monohydrochloride), or an analogue or derivative thereof.

### 60. ROCK (rho-associated kinase) Inhibitors

In another embodiment, the pharmacologically active compound is a ROCK (rho-associated kinase) inhibitor, such as Y-27632, HA-1077, H-1152 and 4-1-(aminoalkyl)-N-(4-pyridyl) cyclohexanecarboxamide or an analogue or derivative thereof.

### 61. CXCR3 Inhibitors

In another embodiment, the pharmacologically active compound is a CXCR3 inhibitor such as T-487, T0906487 or analogue or derivative thereof.

### 62. Itk Inhibitors

In another embodiment, the pharmacologically active compound is an Itk inhibitor such as BMS-509744 or an analogue or derivative thereof.

### 63. Cytosolic phospholipase A₂-.alpha. Inhibitors

In another embodiment, the pharmacologically active compound is a cytosolic phospholipase A₂-.alpha. inhibitor such as efipladib (PLA-902) or analogue or derivative thereof.

### 64. PPAR Agonist

In another embodiment, the pharmacologically active compound is a PPAR Agonist (*e.g*., Metabolex ((-)-benzeneacetic acid, 4-chloro-.alpha.-[3-(trifluoromethyl)-phenoxy]-, 2-(acetylamino)ethyl ester), balaglitazone (5-(4-(3-methyl-4-oxo-3,4-dihydro-quinazolin-2-yl-methoxy)-benzyl)-thiazolidine-2,4-dione), ciglitazone (2,4-thiazolidinedione, 5-[[4-[(1-methylcyclohexyl)methoxy]phenyl]methyl]-), DRF-10945, farglitazar, GSK-677954, GW-409544, GW-501516, GW-590735, GW-590735, K-111, KRP-101, LSN-862, LY-519818, LY-674, LY-929, muraglitazar; BMS-298585 (Glycine, N-[(4-methoxyphenoxy)carbonyl]-N-[[4-[2-(5-methyl-2-phenyl-4-oxazolyl)ethoxy]phenyl]methyl]-), netoglitazone; isaglitazone (2,4-thiazolidinedione, 5-[[6-[(2-fluorophenyl)methoxy]-2-naphthalenyl]methyl]-), Actos AD-4833; U-72107A (2,4-thiazolidinedione, 5-[[4-[2-(5-ethyl-2-pyridinyl)ethoxy]phenyl]methyl]-, monohydrochloride (+/-)-), JTT-501; PNU-182716 (3,5-Isoxazolidinedione, 4-[[4-[2-(5-methyl-2-phenyl-4-oxazolyl)ethoxy]phenyl]methyl]-), AVANDIA (from SB Pharmco Puerto Rico, Inc. (Puerto Rico); BRL-48482;BRL-49653;BRL-49653c; NYRACTA and Venvia (both from (SmithKline Beecham (United Kingdom)); tesaglitazar ((2S)-2-ethoxy-3-[4-[2-[4-[(methylsulfonyl)oxy]phenyl]ethoxy]phenyl] propanoic acid), troglitazone (2,4-Thiazolidinedione, 5-[[4-[(3,4-dihydro-6-hydroxy-2,5,7,8-tetramethyl-2H-1-benzopyran-2-yl)methoxy]phenyl]methyl]-), and analogues and derivatives thereof).

### 65. Immunosuppressants

In another embodiment, the pharmacologically active compound is an immunosuppressant (*e.g*., batebulast (cyclohexanecarboxylic acid, 4-[[(aminoiminomethyl)amino]methyl]-, 4-(1,1-dimethylethyl)phenyl ester, trans-), cyclomunine, exalamide (benzamide, 2-(hexyloxy)-), LYN-001, CCI-779 (rapamycin 42-(3-hydroxy-2-(hydroxymethyl)-2-methylpropanoate)), 1726; 1726-D; AVE-1726, or an analogue or derivative thereof).

### 66. Erb Inhibitor

In another embodiment, the pharmacologically active compound is an Erb inhibitor (*e.g*., canertinib dihydrochloride (N-[4-(3-(chloro-4-fluoro-phenylamino)-7-(3-morpholin-4-yl-propoxy)-quinazolin-6-yl]-acrylamide dihydrochloride), CP-724714, or an analogue or derivative thereof).

### 67. Apoptosis Agonist

In another embodiment, the pharmacologically active compound is an apoptosis agonist (*e.g*., CEFLATONIN (CGX-635) (from Chemgenex Therapeutics, Inc., Menlo Park, CA), CHML, LBH-589, metoclopramide (benzamide, 4-amino-5-chloro-N-[2-(diethylamino)ethyl]-2-methoxy-), patupilone (4,17-dioxabicyclo(14.1.0)heptadecane-5,9-dione, 7,11-dihydroxy-8,8,10,12,16-pentamethyl-3-(1-methyl-2-(2-methyl-4-thiazolyl)ethenyl, (1R,3S,7S,10R,11S,12S,16R)), AN-9; pivanex (butanoic acid, (2,2-dimethyl-1-oxopropoxy)methyl ester), SL-100; SL-102; SL-11093; SL-11098; SL-11099; SL-93; SL-98; SL-99, or an analogue or derivative thereof).

### 68. Lipocortin Agonist

In another embodiment, the pharmacologically active compound is an lipocortin agonist (*e.g*., CGP-13774 (9.alpha.-chloro-6.alpha.-fluoro-11β,17.alpha.-dihydroxy-16.alpha.-methyl-3-oxo-1,4-androstadiene-17β-carboxylic acid-methylester-17-propionate), or analogue or derivative thereof).

### 69. VCAM-1 antagonist

In another embodiment, the pharmacologically active compound is a VCAM-1 antagonist (*e.g*., DW-908e, or an analogue or derivative thereof).

### 70. Collagen Antagonist

In another embodiment, the pharmacologically active compound is a collagen antagonist (*e.g*., E-5050 (Benzenepropanamide, 4-(2,6-dimethylheptyl)-N-(2-hydroxyethyl)-ß-methyl-), lufironil (2,4-Pyridinedicarboxamide, N,N'-bis(2-methoxyethyl)-), or an analogue or derivative thereof).

### 71. alpha. 2 Integrin Antagonist

In another embodiment, the pharmacologically active compound is an .alpha. 2 integrin antagonist (*e.g*., E-7820, or an analogue or derivative thereof).

### 72. TNF .alpha. Inhibitor

In another embodiment, the pharmacologically active compound is a TNF .alpha. inhibitor (*e.g*., ethyl pyruvate, Genz-29155, lentinan (Ajinomoto Co., Inc. (Japan)), linomide (3-quinolinecarboxamide, 1,2-dihydro-4-hydroxy-N,1-dimethyl-2-oxo-N-phenyl-), UR-1505, Enbrel, Remicade, or an analogue or derivative thereof).

### 73. Nitric Oxide Inhibitor

In another embodiment, the pharmacologically active compound is a nitric oxide inhibitor (*e.g*., guanidioethyldisulfide, or an analogue or derivative thereof).

### 74. Cathepsin Inhibitors

In another embodiment, the pharmacologically active compound is a cathepsin inhibitor (*e.g*., SB-462795 or an analogue or derivative thereof).

### 75. Antioxidants

In another embodiment, the pharmacologically active agent is an antioxidant (*e.g*., Na ascorbate, alpha-tocopherol, or an analogue or derivative thereof, or a superoxide dismutase mimetic, such as M40401 and M40403 from Metaphore and SC52608 from Monsanto or an analogue or derivative thereof, (*e.g*., S-S:-dimethyl substituted biscyclohexylpyridine Mn-based superoxide dismutase mimetics or an analogue or derivative thereof)).

### 76. Jun Kinase Inhibitors

In another embodiment, the pharmacologically active agent is a jun kinase inhibitor (*e.g*., AS601245, SP600125, or an analogue or derivative thereof).

### 77. COX-2 Inhibitors

In another embodiment, the pharmacologically active agent is a COX-2 inhibitor (*e.g*., celecoxib (sold under the trade name CELEBREX) and rofecoxib (sold under the trade name VIOXX).

### 78. Non-Steroidal Anti-inflammatory Agents

In another embodiment, the pharmacologically active agent is a non-steroidal anti-inflammatory agent (*e.g*., aspirin, ibuprofen, indomethacin, naproxen, prioxicam, diclofenac, tolmetin, fenoclofenac, meclofenamate, mefenamic acid, etodolac, sulindac, carprofen, fenbufen, fenoprofen, flurbiprofen, ketoprofen, oxaprozin, tiaprofenic acid, phenylbutazone diflunisal, salsalte, and salts and analogues thereof).

### 79. Caspase Inhibitors

In another embodiment, the pharmacologically active agent is a caspase inhibitor (*e.g*., CV 1013 or an analogue or derivative thereof).

### 80. Other Therapeutic Agents

Other agents which may be used for treating contracture include chemokines involved in pathogenesis (*e.g*., MCP-1, RANTES, and MIP-1b); NO synthase inhibitors (*e.g*., niacinamide and other ADP-ribosylation inhibitors); phenothiazine (*e.g*., chlorpromazine), cytokine modulators (*e.g*., INF .alpha., IL-1, and IL-6), chemokine modulators, cGMP stimulants, and agents that enhance the activities of growth factors IGF-1, bFGF and TGFb by decreasing proteoglycan catabolism (*e.g*., S-adenosyl methionine, rhIGF-1, rhbFGF, and rhTGFb).

In certain embodiments, the therapeutic agent effective in treating contracture is not a collagenase, a metalloproteinase inhibitor, a collagenase inhibitor, a steroid, a non-steroidal anti-inflammatory agent, a fluoroquinolone, a DNA topoisomerase ATP hydrolyzing inhibitor, enoxacin, ofloxacin, sparfloxacin, a superoxide dismutase, hyaluronic acid, antihistamine, dimethylsulfoxide, calmodulin blocker trifluroperizine, a calcium channel blocker, dimethysulfoxide, an oxygen free radical scavenger (*e.g*., colchicines, allopurinal and methylhydrazine), an interferon, a protease (*e.g*., trypsin, .alpha.-chymotrypsin, thiomcase, hyaluronidase), or insulin.

It should be apparent to one of skill in the art that potentially any agent described above (*e.g*., fibrosis-inhibiting agents) could be utilized alone, or in combination, in the practice of this embodiment. Examples of such agents for use in contracture include the following: paclitaxel, docetaxel, halofuginone bromide, mycophenolic acid, mithramycin, puromycin, nogalamycin, 17-DMAG, nystatin, rapamycin, mitoxantrone, duanorubicin, gemcitabine, camptothecin, epothilone B, simvastatin, anisomycin, mitomycin C, epirubicin hydrochloride, topotecan, fascaplysin, podophyllotoxin, and chromomycin A3 as well as analogues and derivatives of the aforementioned.

The exact dose administered will vary with the composition of the formulation, the type of joint or tissue (*e.g*., knee, shoulder, elbow, ankle, hip, finger joint, wrist, toe joint, or soft tissue, such as muscles, tendons, ligaments, fat, joint capsule, synovium or other connective tissue (*e.g*., fascia) at which the formulation is to be administered, and severity of the disease; however, certain principles can be applied in the application of this art. Drug dose can be calculated as a function of total drug dose administered or as a concentration of drug in the composition. Regardless of the method of application of the drug, the therapeutic agents, used alone or in combination, should be administered under the following dosing guidelines:

Drugs and dosage: Selected examples of therapeutic agents that may be used include but are not limited to: antimicrotubule agents including taxanes (*e.g*., paclitaxel and docetaxel), other microtubule stabilizing agents and vinca alkaloids (*e.g*., vinblastine and vincristine sulfate), halofuginone bromide, mycophenolic acid, mithramycin, puromycin, nogalamycin, 17-DMAG, nystatin, rapamycin, mitoxantrone, duanorubicin, gemcitabine, camptothecin, epothilone B, simvastatin, anisomycin, mitomycin C, epirubicin hydrochloride, topotecan, fascaplysin, podophyllotoxin, and chromomycin A3. Drugs are to be used at concentrations that range from several times more than to 10%, 5%, or even less than 1% of the concentration typically used in a single chemotherapeutic systemic dose application. Preferably, the drug is released in effective concentrations for a period ranging from 1 - 90 days. Antimicrotubule agents including taxanes such as paclitaxel and analogues and derivatives (*e.g*., docetaxel) thereof and vinca alkaloids including vinblastine and vincristine sulfate, and other agents including halofuginone bromide, mycophenolic acid, mithramycin, puromycin, nogalamycin, 17-DMAG, nystatin, rapamycin, mitoxantrone, duanorubicin, gemcitabine, camptothecin, epothilone B, simvastatin, anisomycin, mitomycin C, epirubicin hydrochloride, topotecan, fascaplysin, podophyllotoxin, and chromomycin A3 and analogues and derivatives thereof: total single locally administered dose not to exceed 20 mg (range of 0.1 µg to 20 mg); preferred 1 µg to 15 mg.

In certain embodiments, the composition comprises between about 0.01 mg/ml to about 100 mg/ml of a therapeutic agent. In certain embodiment, the composition comprises between about 0.1 mg/ml to about 10 mg/ml of a therapeutic agent.

### II. COMBINATION THERAPIES

In certain embodiments of the invention, compositions may be combined for use. For example, a composition having a drug effective in treating contracture may be combined in its use with a second composition having a drug effective in treating contracture or one or more related conditions, such as, *e.g*., pain, infection, swelling, or inflammation. Representative classes of therapeutic agents that may be used in combination therapies include, *e.g*., antibiotics, anti-infectives, anti-inflammatory agents, analgesics, narcotics, and anesthetics.

Representative examples of therapeutic agent having anti-inflammatory or analgesic activity include non-steroidal anti-inflammatory agents (such as but not limited to aspirin, ibuprofen, indomethacin, naproxen, prioxicam, diclofenac, tolmetin, fenoclofenac, meclofenamate, mefenamic acid, etodolac, sulindac, carprofen, fenbufen, fenoprofen, flurbiprofen, ketoprofen, oxaprozin, tiaprofenic acid, phenylbutazone diflunisal, salsalte, and salts and analogues thereof); opiates (such as but not limited tocodeine, meperidine, methadone, morphine, pentazocine, fentanyl, hydromorphone, oxycodone, oxymorphone, and salts and analogues thereof); and steroidal anti-inflammatories, such as but not limited to hydrocortisone, dexamethasone, triamcinolone, prednisone, cortisone, fludrocortisone and esters and analogues thereof.

Representative examples of antibiotic and anti-infective agents include, by way of example and not by way of limitation, cephalosporins (*e.g*., cefazolin, cefotaxime, cefoxitin, defuroxime, cefaclor, cefonicid, cefotetan, cefoperazone, ceftriaxone, moxalactam, and ceftazidime, and salts thereof); β-lactams (*e.g*., aztreonam and imipenem) chloramphenicol and salts thereof; erythromycins and salts thereof (*e.g*., roxithromycin, erythromycin, and its esters such as ethylsuccinate, guceptate and stearate); penicillins (*e.g*., penicillin G, amoxicillin, amdinocillin, ampicillin, carbenicillin, ticarcillin, cloxacillin, nafcillin, penicillin V, and their salts and esters); tetracyclines (such as but not limited to tetracycline, and doxycycline, and salts thereof); clindamycin, polymixin B, and sulfonamides. Also included are active analogues and derivatives of the aforementioned antibiotic and anti-infective agents.

Exemplary anaesthetics which may be included in certain compositions of the invention include, but are not limited to, methohexital sodium, thiopental sodium, etomidate, ketamine, propofol, bupivicaine, chloroprocaine, etidocaine, lidocaine, mepivicaine, prilocaine, procaine, tetracaine, benzocaine, cocaine, dibucainem dyclonnine, pramoxine, and salts (for example, hydrochlorides and sodium salts), esters, prodrugs, analogues and derivatives of the aforementioned compounds.

In certain embodiments, administration of the second agent may occur simultaneously and at the same site, being part of the same composition. In other embodiments, it may occur at the same time, but by a second administration, to the same or a different site. For example, a steroid could be given by intravenous injection while the primary therapeutic agent is administered intra-articularly. In yet other embodiments, the second agent may be given at a different time, for example, the following day or week, but as part of the same treatment regime to the same or a different site.

### III. COMPOSITIONS

The present invention provides a composition that includes one or more therapeutic agents effective in treating contracture. The composition may be in a solid, semi-solid, gel, or liquid form. Liquid compositions may be, for example, a homogenous solution or a suspension, emulsion, or dispersion of one or more phases in another. The composition may include solid components (described in further detail below), which may be defined by size, size distribution, shape, surface characteristics, water content or ability to swell, drug loading and release characteristics and bioresorbability.

Therapeutic agents may be incorporated into the compositions and devices of the invention by various methods, such as being contained (*e.g*., dispersed) in a polymeric matrix (*e.g*., a polymeric carrier), bound by covalent linkages (*e.g*., to a solid or semi-solid substrate), encapsulated in microcapsules, encapsulated in microspheres or nanospheres, or included as a component in a coating. Within certain preferred embodiments of the invention, therapeutic compositions are provided in non-capsular formulations such as microspheres (ranging from nanometers to micrometers in size), pastes, threads of various size, films and sprays.

The composition includes one or more polymeric carriers. All or some of the therapeutic agent(s) may be contained within the carrier (*e.g*., dissolved or dispersed within the carrier). The composition may include a carrier that can be formed into solid or semi-solid forms, such as a gel, a hydrogel, a suspension, a paste, a cream, an ointment, a tablet, a spray, a powder, an orthopedic implant, a fabric, a gauze or a pledget. In some embodiments, the therapeutic agent is coated onto a solid or semi-solid substrate (*e.g*., a particle or implant) with or without a carrier.

The characteristics of each type of composition are described in detail as follows:

### 1. Solutions and suspensions

In certain embodiments of the invention, a drug or drugs are contained within a carrier that is a solution or a suspension. A solution consists of molecularly dispersed or colloidally dispersed material in a liquid phase, typically an aqueous phase such as normal or buffered saline. Colloidal dispersions include micellar solutions, liposomes and microemulsions. Solutions within the scope of the invention are clear and have in them a homogeneously dispersed, therapeutically effective amount of a drug or drugs. Solutions may also contain excipients (discussed in detail below). Solutions may be made viscous by the addition of viscosity builders, such as polymers or sugar. These systems may be gels or even hydrogels, which are discussed in detail below.

Suspensions are disperse systems containing solid particles within a liquid phase, typically an aqueous phase such as normal or buffered saline. Suspensions may be characterized by the particle size of the suspended particles, the ability to maintain the suspension, the degree of flocculation and other cosmetic, or pharmaceutically relevant characteristics such as stability. The liquid phase may be a solution, having some of the drug or drugs in suspension also dissolved therein. Suspensions may contain excipients which are intended to promote the ability of the drug or drugs to remain suspended, or be easily resuspended. These may include polymers which promote flocculation and/or viscosity. As a result, some suspensions may also be considered gels or even hydrogels, which are discussed in detail below. Suspensions may be disperse systems or precursors thereto. Precursors may include solid particles and a separate liquid phase intended for later constitution of the solid particles.

Other disperse systems include emulsions, in which the first phase is a liquid dispersed within a second liquid phase. Characteristically, the two phases are largely immiscible and the dispersion is stabilized by the addition of a surfactant. Acceptable surfactants for use in the instant compositions include ionic or non-ionic surfactants and polymeric stabilizers, examples of which are well known in the art. In an emulsion, the therapeutic agent may be contained in either phase. In yet other dispersed systems within the scope of the invention, the formulation may include a liposome or a liquid crystal or precursors thereto.

### 2. Microparticles

The therapeutic composition may be a disperse system that includes a carrier formed as a microparticle. "Microparticle" as used herein refers to spheres or irregularly shaped particles having a size of less than 1 mm in diameter. Typically, the mean diameter of a microparticle may be in the range of 1-500 µm, but it may be lower, for example, in the range of 200 - 1000 nm, or lower, for example, 10 - 250 nm. Microparticles may be microspheres, which are essentially spherical and have a size in the micron range, *e.g*., a mean diameter between about 1-1000 µm. Microparticles may contain a therapeutically active amount of a drug and excipients used to form the microparticle. Microparticles may be formed with polymeric excipients, as discussed above, but may be formed with non-polymeric excipients, such as waxes, or hydrocarbon alcohols (*e.g*., cetyl alcohol and steryl alcohol). Microparticles may be formed by techniques known to those skilled in the art, including for example, spray drying, solvent evaporation or removal, hot melt microencapsulation, or ionic gelation techniques. The microspheres can be in a non-porous or a porous form.

### 3. Gels and hydrogels

In certain embodiments, the carrier may be in the form of a gel. A gel is a semi-solid characterized by relatively high yield values as described in Martin's Physical Pharmacy (Fourth Edition, Alfred Martin, Lea & Febiger, Philadelphia, 1993, pp 574-575). Gels may contain non-crosslinked materials and possess certain properties, such as elevated viscosity and elasticity, which may be reduced with increased dilution with an aqueous medium, such as water or buffer.

Certain polymers may be crosslinked to form systems that are herein defined as "hydrogels." A hydrogel will maintain an elevated level of viscosity and elasticity when diluted with an aqueous solution, such as water or buffer. Crosslinking may be accomplished by several means including covalent, hydrogen, ionic, hydrophobic bonding, chelation, complexation, and the like.

Gels and hydrogels may be fashioned into a variety of forms with specific desired properties and/or drug release characteristics. For example, polymers can be formed into gels by dispersing them into a solvent, such as water.

Hydrogels and gels within the scope of the invention may contain other semi-solid or solid materials dispersed within. These solids include, without limitation, microparticles, nanoparticles, microspheres and nanospheres, and other particles capable of being suspended within the continuous phase.

Gels with sufficiently low viscosity may be injected into the targeted site of action, for example, into the articular space. Hydrogels with sufficiently high viscosity may be inserted into a target space in or around a joint, for example, as an implant or as a component contained within a sponge or pledget.

Hydrogels may also be formed *in situ* by combining hydrogel forming components within the target site. For example, a hydrogel formulation may be injected into the target site in a precursor form. Once within the target site, the injected precursor material(s) form into a hydrogel. In certain embodiments, the hydrogel may be formed *in situ* with the aid of an external energy source, such as ultraviolet light.

A carrier gel may include a polypeptide or polysaccharide. In certain embodiments, polysaccharides and polypeptides and other polymers can be fashioned to release a therapeutic agent upon exposure to a specific triggering event such as pH (*see, e.g.,* Heller et al., "Chemically Self-Regulated Drug Delivery Systems," in Polymers in Medicine III, Elsevier Science Publishers B.V., Amsterdam, 1988, pp. 175-188; Peppas, "Fundamentals of pH- and Temperature-Sensitive Delivery Systems," in Gumy et al. (eds.), Pulsatile Drug Delivery, Wissenschaftliche Verlagsgeselischaft mbH, Stuttgart, 1993, pp. 41-55; Doelker, "Cellulose Derivatives," 1993, in Peppas and Langer (eds.), Biopolymers I, Springer-Verlag, Berlin). Representative examples of pH-sensitive polysaccharides include carboxymethyl cellulose, cellulose acetate trimellilate, hydroxypropylmethylcellulose phthalate, hydroxypropylmethylcellulose acetate succinate, chitosan and alginates. Representative examples of pH-sensitive polymers include poly(acrylic acid) and its derivatives (including, for example, homopolymers such as poly(aminocarboxylic acid); poly(acrylic acid); poly(methyl acrylic acid)), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropylmethylcellulose acetate succinate; cellulose acetate trimellilate; and chitosan. Yet other pH sensitive polymers include any mixture of a pH sensitive polymer and a water-soluble polymer.

In certain aspects, the carrier includes chitosan (poly(D-glucosamine)), chitosan derivatives (*e.g*., carboxymethyl chitosan), partially deacetylated chitin, or another polyglucosamine. Chitosan may be prepared in a gel form by dissolving a soluble form of the polymer in water. Alternatively, chitosan may be blended with a polymer matrix such as hyaluronic acid, or it may be crosslinked, with or without another polysaccharide. These or other less soluble forms of chitosan may be used to form more viscous, or solid compositions that exhibit increased dwell time upon administration, for example, in the joint space.

Likewise, polysaccharides and polypeptides and other polymers can be fashioned to be temperature sensitive (*see, e.g.,* Okano, in Proceed. Intern. Symp. Control. Rel. Bioact. Mater. 22:111-112, Controlled Release Society, Inc., 1995; Hoffman et al., "Characterizing Pore Sizes and Water 'Structure' in Stimuli-Responsive Hydrogels," Center for Bioengineering, Univ. of Washington, Seattle, WA, p. 828; Hoffman, in Migliaresi et al. (eds.), Polymers in Medicine III, Elsevier Science Publishers B.V., Amsterdam, 1988, pp. 161-167; Hoffman, in Third International Symposium on Recent Advances in Drug Delivery Systems, Salt Lake City, UT, Feb. 24-27, 1987, pp. 297-305, Sershen et al., Advanced Drug Delivery Reviews, 54:1225-1235, 2002; Chen et al., in Proceed. Intern. Symp. Control. Rel. Bioact. Mater. 22:167, Controlled Release Society, Inc., 1995; Johnston et al., Pharm. Res. 9(3):425, 1992; Tung, Int'l J. Pharm. 107:85, 1994; Harsh and Gehrke, J. Controlled Release 17:175, 1991; Bae et al., Pharm. Res. 8(4):531, 1991; Dinarvand and D'Emanuele, J. Controlled Release 36:221, 1995; Kim et al., Pharm. Res. 9(3):283-290, 1992; Bae et al., Pharm. Res. 8(5):624-628, 1991; Kono et al., J. Controlled Release 30:69, 1994; Yoshida et al., J. Controlled Release 32:97, 1994; Okano et al., J. Controlled Release 36:125, 1995; Chun and Kim, J. Controlled Release 38:39-47, 1996; D'Emanuele and Dinarvand, Int'l J. Pharm. 118:237, 1995; Katono et al., J. Controlled Release 16:215, 1991; Gutowska et al., J. Controlled Release 22:95-104, 1992; Palasis and Gehrke, J. Controlled Release 18:1-12, 1992; Paavola et al., Pharm. Res. 12(12):1997-2002, 1995).

Representative examples of thermogelling polymers include homopolymers such as poly(N-methyl-N-n-propylacrylamide), LCST=19.8°C; poly(N-n-propylacrylamide), 21.5°C; poly(N-methyl-N-isopropylacrylamide), 22.3°C; poly(N-n-propylmethacrylamide), 28.0°C; poly(N-isopropylacrylamide), 30.9°C; poly(N, n-diethylacrylamide), 32.0°C; poly(N-isopropylmethacrylamide), 44.0°C; poly(N-cyclopropylacrylamide), 45.5°C; poly(N-ethylmethyacrylamide), 50.0; poly(N-methyl-N-ethylacrylamide), 56.0°C; poly(N-cyclopropylmethacrylamide), 59.0°C; poly(N-ethylacrylamide), 72.0°C. Moreover, thermogelling polymers may be made by preparing copolymers between (among) monomers of the above, or by combining such homopolymers with other water soluble polymers (*e.g*., poly(acrylic acid), poly(methylacrylic acid), poly(acrylate), poly(butyl methacrylate), poly(acrylamide) and poly(N-n-butyl acrylamide) and derivatives thereof. Other representative examples of thermogelling polymers include cellulose ether derivatives such as hydroxypropyl cellulose, 41°C; methyl cellulose, 55°C; hydroxypropylmethyl cellulose, 66°C; and ethylhydroxyethyl cellulose, copolymers of α-hydroxy acid and poly(ethylene glycol) and PLURONICs, such as F-127 (BASF Corporation, Mount Olive, NJ). Representative examples of thermogelling polymers include PLURONIC F127, and cellulose derivatives.

An exemplary polysaccharide includes without limitation HA (also known as hyaluronan) and derivatives thereof (see, *e.g*., U.S. Patent Nos. 5,399,351, 5,266,563, 5,246,698, 5,143,724, 5,128,326, 5,099,013, 4,913,743, and 4,713,448), including esters, partial esters and salts of HA. HA as used herein includes an acidic polysaccharide of repeating subunits of D-glucuronic acid and N-acetyl-D-glucosamine, as well as salts and derivatives thereof. For example, an aqueous solution of hyaluronic acid having a non-proinflammatory molecular weight (greater than about 900 kDa) and a concentration of about 10 mg/ml would be in the form of a gel. The aqueous solution may further include one or more excipients that serve other functions, such as buffering, anti-microbial stabilization, or prevention of oxidation.

In certain aspects, a gel composition may be prepared comprising hyaluronic acid having a molecular weight between 750k and about 1 M Da or between 1 M and 5M Da, and a drug such as paclitaxel or an anti-metabolite such as 5-flurouracil. Additional excipients may be incorporated such that certain compositions of the invention further comprise a buffer, anti-microbial agent, or antioxidant. For drugs that are not sufficiently soluble in the polysaccharide gel, the composition may further comprise a co-solvent such as low molecular weight PEG (MW 200 to 400), ethoxydiglycol (*e.g*., TRANSCUTOL from Gattefosse S.A., France), pyrrolidones, for example, N-methyl-pyrrolidone, ethanol, propylene glycol, benzyl alcohol or biocompatible analogs thereof, and dimethyl sulfoxide.

Gel and gel-forming formulations may be administered to a patient by injection into a variety of intra-articular spaces and surrounding tissues, including a tendon, ligament, tendon sheath, and periarticular, periosseous, or subcutaneous space, a carpal tunnel, or the like to alleviate one or more symptoms associated with contracture, including joint stiffness, adhesion, fibrous tissue growth, loss of mobility, inflammation, pain and swelling.

### 4. Sprays

In certain embodiments of the invention, the therapeutic agent(s) is contained within a carrier that is administered as a spray. Sprays may be administered, for example, by aerosol formation, nebulization, suspension of a solution or suspension in a gas, including air, ejection of a liquid through a nozzle to form a mist or droplets, and the like. In such embodiments, a spray is meant to include the dispersed system being sprayed, as well as precursors thereto. In one embodiment, the composition may be applied as a spray, which solidifies into a film or a coating. Such sprays may include microspheres of a wide array of sizes, including for example, from 0.1 µm to 3 µm, from 10 µm to 30 µm, and from 30 µm to 100 µm. Sprays may be administered using various devices, such as syringes equipped with a sprayer or pressurized canisters equipped with atomizers. Sprays may be applied to a serosal or mucosal surface, a wound site, or a surgical site.

### 5. Sutures

In certain embodiments of the invention, the composition may include a carrier which is a suture designed to effect the closure of a wound or incision, or to fix a tissue in place. Such a suture may be fabricated of materials and by methods known to those skilled in the art. Suitable sutures may include, for example, biodegradable polymers such as polyglycolide, polylactide, polymers made from a trimethylene carbonate monomer, or co-polymers thereof. Sutures also may be formed using materials such as silk, catgut, nylon, or polypropylene. Suitable sutures may be braided or monofilamentous. An effective therapeutic agent according to the present invention may be affixed onto or within sutures by incorporation into a carrier which adheres to the suture or a portion thereof. A therapeutic agent may be introduced within the suture at the time of its manufacture or, alternatively, may be applied to the suture immediately prior to its use, for example, by dipping the suture into a medium containing the drug and allowing it to adhere to or absorb into the suture.

### 6. Sponges, Pledgets & Implantable Porous Membranes

In certain embodiments of the invention, the composition may include a carrier which is a porous material, such as a sponge, pledget or implantable porous membrane so designed as to allow for the egress of a drug contained therein. Such a device may be fabricated of materials and by methods known to those skilled in the art. Porous materials may be made of materials such as collagen, cellulose, gelatin (*e.g*., GELFOAM, available from Upjohn Company, Kalamazoo, MI), and hyaluronic acid and derivatives thereof (*e.g*., SEPRAMESH or SEPRAFILM, available from Genzyme Corporation, Cambridge, MA).

In certain embodiments, the sponge may be a pledget that includes a material, such as cotton, cellulose, gelatin, or TEFLON (E. I. du Pont de Nemours and Company, Wilmington, DE). A drug may be incorporated into a pledget by dispersing the drug in a liquid carrier and soaking the pledget in the dispersion allowing it to take up the liquid and the drug. The dispersion may be a solution or a suspension of drug and may further include other excipients. Drugs may be loaded in this manner immediately prior to use of the composition, or at an earlier time of manufacture. In certain embodiments, the liquid carrier may then be removed, for example, by drying or using pressure to expel the liquid. The pledget may be implanted or used topically or on a wound surface.

### 7. Orthopedic implants

The composition may include a carrier which is an orthopedic implant designed to provide stability or articulation to the skeletal system, including joints. Implants include pins, screws, plates, grafts (including allografts and tendon grafts), anchors, and total joint replacement devices, such as artificial knees and hips. The orthopedic implant may be fabricated of materials that include metals, such as titanium, nickel, or suitable alloys (*e.g*., steel or nickel-titanium). Suitable orthopedic implants also may include polymers, such as polyurethanes, polyethylene, polycarbonate, polyacrylates (*e.g*., polymethyl methacrylate), poly(L-lactide) or polytetrafluoroethylene. Orthopedic implants also include bone implants that contain calcium phosphate, for example, in the form of tricalcium phosphate or hydroxyapatite. Exemplary orthopedic devices also are described, for example, in The Radiology of Orthopaedic Implants: An Atlas of Techniques and Assessment Mosby Publishing (2001), Andrew A. Freiberg (Editor), William, M.D. Martel.

### 8. Films

The therapeutic compositions of the present invention may include a carrier that is formed as a film. Films generally are less than 5, 4, 3, 2 or 1 mm thick, or less than 0.75 mm or 0.5 mm thick. Such films may have other desirable features including flexibility, good tensile strength, good adhesive properties (*i.e*., readily adheres to moist or wet surfaces), and controlled permeability and biodegradation.

### 9. Meshes

The therapeutic compositions of the present invention may include a therapeutic agent and a biodegradable polymer, wherein at least some of the biodegradable polymer is in the form of a mesh. A mesh, as used herein, is a material composed of a plurality of fibers or filaments (*i.e*., a fibrous material), where the fibers or filaments are arranged in such a manner (*e.g*., interwoven, knotted, braided, overlapping, looped, knitted, interlaced, intertwined, webbed, felted, and the like) so as to form a porous structure.

A mesh may include fibers or filaments that are randomly oriented relative to each other or that are arranged in an ordered array or pattern, In one embodiment, for example, a mesh may be in the form of a fabric, such as, for example, a knitted, braided, crocheted, woven, non-woven (*e.g*., a melt-blown or wet-laid) or webbed fabric. In one embodiment, a mesh may include a natural or synthetic biodegradable polymer that may be formed into a knit mesh, a weave mesh, a sprayed mesh, a web mesh, a braided mesh, a looped mesh, and the like.

The mesh may include fibers that are of same dimension or of different dimensions, and the fibers may be formed from the same or different types of biodegradable polymers. Woven materials, for example, may include a regular or irregular array of warp and weft strands and may include one type of polymer in the weft direction and another type (having the same or a different degradation profile from the first polymer) in the warp direction. Similarly, knit materials may include one or more types (*e.g*., monofilament, multi-filament) and sizes of fibers and may include fibers made from the same or from different types of biodegradable polymers.

The structure of the mesh (*e.g*., fiber density and porosity) may impact the amount of therapeutic agent that may be loaded into the mesh. For example, a fabric having a loose weave characterized by a low fiber density and high porosity will have a lower thread count, resulting in a reduced total fiber volume and surface area. As a result, the amount of agent that may be loaded into or onto, with a fixed carrier: therapeutic agent ratio, the fibers will be lower than for a fabric having a high fiber density and lower porosity. It is preferable that the mesh also should not invoke biologically detrimental inflammatory or toxic response, should be capable of being fully metabolized in the body, have an acceptable shelf life, and be easily sterilized.

In certain embodiments, multiple mesh materials in any combination or arrangement may be used. In some embodiments, multi-layer meshes (*e.g*., device having two or more layers of material) may be used, for example, to increase the amount of drug loading.

Multi-layer constructions may also be useful, for example, to deliver more than one type of therapeutic agent. For example, a first layer of mesh material may be loaded with one type of agent and a second layer may be loaded with another type of agent. The two layers may be unconnected or connected (*e.g*., fused together, such as by heat welding or ultrasonic welding) and may be formed of the same type of fabric or from a different type of fabric having a different polymer composition and/or structure.

### 10. Pastes

Therapeutic compositions of the present invention may also be prepared in a variety of "paste" forms. For example, within one embodiment of the invention, therapeutic compositions are provided which are liquid at one temperature (*e.g*., temperature greater than 37°C, such as 40°C, 45°C, 50°C, 55°C or 60°C), and solid or semi-solid at another temperature (*e.g.*, ambient body temperature, or any temperature lower than 37°C). Such "thermopastes" may be readily made utilizing a variety of techniques (see, *e.g*., PCT Publication WO 98/24427). Other pastes may be applied as a liquid which solidify *in vivo* due to dissolution of a water-soluble component of the paste, or precipitation of encapsulated drug or excipient into the aqueous body environment. Yet other pastes may be formed by suspension of a high proportion of solid particles in a viscous carrier matrics.

### 11. Coatings

The therapeutic agent(s) may be incorporated into a carrier that forms a coating on, for example, a particle or an implantable or removable medical device, as described above. The coating typically includes a polymer that may be biodegradable or non-biodegradable. In some case, the coating may not contain a polymer. In some cases, it may be desirable that the coating be bioerodable. In certain embodiments, the coating provides controlled and sustained delivery of the agent into the target site over a particular period of time (*e.g*., minutes, hours, or days). For example, a solid or semi-solid microparticle, film, fabric, or implant (*e.g*., a screw, pin, graft, joint replacement, and the like) may be coated with a polymer, such as a hydrogel, that includes a therapeutically effective amount of a therapeutic agent, as described herein. The therapeutic agent may be admixed with the carrier, or it may be attached (*e.g*., covalently or non-covalently, for example, via electrostatic or ionic interaction) with a component of the coating material. The coating may include microparticles dispersed within the coating, where the therapeutic agent may reside either in the particles, in the carrier, or in a combination thereof. It may be desirable to include one type of therapeutic agent in the carrier composition and a second type within the particles, such that one agent may be released under one set of conditions and a second agent may be released under a second set of conditions. For example, the coating composition may include a microparticle that contains an anti-microtubule agent, such as paclitaxel, and a polymeric carrier that includes an anti-inflammatory, analgesic, or antibiotic agent. For example, a steroid such as triamcinolone may be released immediately resulting in a reduction of acute inflammation and an antimicrotubule agent may be released over 3 to 10 days in order to reduce the severity of a contracture formation. In certain embodiments, the therapeutic agent is coated directly onto the surface the substrate (*e.g*., a delivery device, such as an implant or particle). The coating may include pores that can be filled with the therapeutic agent or a combination of two or more agents.

The therapeutic agent or the therapeutic agent/carrier composition may be applied using the various coating methods that are known in the art (*e.g*., dip coating, spray coating, deposition methods such as electrospray, solvent casting, extrusion, roll coating, etc.). In some embodiments, the therapeutic agent may be attached directly to the substrate (*e.g*., by physisorption, chemisorption, ligand/receptor interaction, covalent bonds, hydrogen bonds, ionic bonds, and the like). The substrate, optionally, may be pre-treated prior to application of the therapeutic agent to enhance adhesion and/or to introduce reactive sites for attaching the drug or an intermediate (*e.g*., a linker) to the material. Surface treatment techniques are well known in the art and include, for example, applying a priming solution, plasma treatment, corona treatment, radiation treatment and surface hydrolysis, oxidation or reduction.

Coatings may be made to include more than a single polymer, and the ratio of the multiple polymeric components may be altered to control properties such as drug release rate, swelling or elasticity and other mechanical properties. Exemplary polymers suitable for use in coatings include sufficiently elastic polymers and lubricious polymers, including polyurethanes, ethylene vinyl acetate, silicones, acrylates, pyrrolidones, PARYLENE (Union Carbide) poly-para-xylylene polymers, and polyalkylene oxides.

### EXCIPIENTS

In addition to a therapeutic agent, compositions may further include one or more excipients, including but not limited to, polymeric or non-polymeric materials, phospholipids, viscosity increasing agents, pharmaceutically or veterinarilly acceptable vehicles, diluents, preservatives, stabilizers, colorants, antioxidants, binders, pore formers, density, tonicity, pH, or osmotic pressure adjusting materials, degradation accelerants, radioopaque or echogenic materials, and magnetic resonance imaging responsive materials.

Examples of polymers that may be used as excipients include natural (*e.g*., biologically derived) and synthetic materials. For example, biologically derived polymers, such as hyaluronic acid (HA) and derivatives thereof, dextran and derivatives thereof, cellulose and derivatives thereof (*e.g*., methylcellulose, hydroxypropylcellulose, hydroxypropylmethylcellulose, carboxymethylcellulose, cellulose acetate phthalate, cellulose acetate succinate, cellulose acetate butyrate, hydroxypropylmethylcellulose phthalate), chitosan and derivatives thereof, β-glucan, arabinoxylans, carrageenans, pectin, glycogen, fucoidan, chondrotin, pentosan, keratan, alginate, polypeptide (*e.g*., poly(L-glutamic acid), collagen, albumin, fibrin and gelatin), cyclodextrins, and salts and derivatives, including esters and sulphates thereof may be used as an excipient.

In some embodiments, the excipient may include a synthetic polymer, such as homopolymers, copolymers or cross-linked polymers. Polymeric excipients may be polyethers, such as polyethylene glycol, polyesters such as poly(DL-lactide), poly(glycolide), poly(glycolide-co-lactide), poly(L-lactide), poly(ε-caprolactone), or poly(δ or γ-valerolactone), polymers of acrylic acid and derivatives thereof, such as polyacrylic acid or polymethylmethacrylate, polyurethanes, polyethylene, polystyrene, ethylene vinyl acetate, poloxamers, silicones, polystyrene, polypropylene, crosslinked divinyl benzene, vinyls such as polyvinyl chloride, polyvinyl acetate, or polyvinyl alcohol, polythioesters, polyanhydrides, polyamides, and polyorthoesters. Derivatives of the aforementioned synthetic and biologically derived polymers also are suitable for use as excipients. Derivatization may be accomplished by methylation, esterification, the inclusion of unique end groups, pendant groups, or monomeric units within the backbone, spaced either randomly, regularly or with a defined density. These may include acids, bases, ionizing species, complexing species, halogens, hydrophobic groups such as phenyl containing groups, or groups with latent functionality for example, cross-linkers such as succinimides.

Compositions are provided that include a therapeutic agent (*e.g*., an anti-microtubule agent) and a carrier. The carrier may serve to provide a solid structure upon or in which the drug may be localized. Alternatively, the carrier may provide a means for the homogeneous distribution of the drug.

The carrier is a polymeric carrier. Polymeric carriers may include one or more bioresorbable or biodegradable polymer(s), one or more non-degradable polymer(s) or a combination of one or more biodegradable polymer(s) and non-degradable polymer(s). Bioerodible materials may be particularly preferred in certain embodiments.

Representative examples of bioresorbable compositions that may be used to prepare the carrier include albumin, collagen, hyaluronic acid and derivatives, sodium alginate and derivatives, chitosan and derivatives gelatin, starch, cellulose polymers (for example, methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextran and derivatives, polysaccharides, poly(caprolactone), fibrinogen, poly(hydroxyl acids), poly(L-lactide) poly(D,L lactide), poly(D,L-lactide-co-glycolide), poly(L-lactide-co-glycolide), copolymers of lactic acid and glycolic acid, copolymers of ε-caprolactone and lactide, copolymers of glycolide and e-caprolactone, copolymers of lactide and 1,4-dioxane-2-one, polymers and copolymers that include one or more of the residue units of the monomers D-lactide, L-lactide, D,L-lactide, glycolide, ε-caprolactone, trimethylene carbonate, 1,4-dioxane-2-one or 1,5-dioxepan-2-one, poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, and poly(amino acids). These compositions include copolymers of the above polymers as well as blends and combinations of the above polymers.

Representative examples of non-biodegradable polymers include ethylene-co-vinyl acetate copolymers, acrylic-based and methacrylic-based polymers [*e.g*., poly(acrylic acid), poly(methylacrylic acid), poly(methylmethacrylate), poly(hydroxyethylmethacrylate), poly(alkylcynoacrylate), poly(alkyl acrylates), poly(alkyl methacrylates)], poly(ethylene), poly(propylene), polyamides [*e.g*., nylon 6,6], poly(urethanes) [*e.g*., poly(ester urethanes), poly(ether urethanes), poly(carbonate urethanes), poly(ester-urea)], polyethers [*e.g*., poly(ethylene oxide), poly(propylene oxide), poly(ethylene oxide)-poly(propylene oxide) copolymers, diblock and triblock copolymers, poly(tetramethylene glycol)], silicone containing polymers and vinyl-based polymers [polyvinylpyrrolidone, poly(vinyl alcohol), poly(vinyl acetate phthalate), and poly(styrene-co-isobutylene-co-styrene). These compositions include copolymers as well as blends, crosslinked compositions and combinations of the above polymers. Certain non-biodegradable polymers which are water soluble may also be classed as bioresorbable, for example, water soluble, non-degradable polymers.

Preferred polymeric carriers are biodegradable, such as copolymers of lactic acid and glycolic acid, copolymers of lactide and glycolide, copolymers of lactic acid and ε-caprolactone), diblock copolymers (A-B) with block A that includes methoxypolyethylene glycol and block B that includes a polyester, for example, methoxypoly(ethylene glycol) - co - poly(D,L-lactide), and triblock copolymers (A-B-A) or (B-A-B) with block A including polyoxyalkane and block B including a polyester. Preferred polyoxyalkane blocks include polyethylene glycol, polypropylene glycol, poly(ethylene oxide-co-propylene oxide), and poly(ethylene oxide-co-propylene oxide-co-ethylene oxide). Other preferred polymeric carriers include poly(lactides), poly(glycolides), a poly(caprolactones), poly(L-lactide-co-glycolide), copolymers of lactic acid and glycolic acid, copolymers of ε-caprolactone and lactide, copolymers of glycolide and ε-caprolactone, copolymers of lactide and 1,4-dioxane-2-one, polymers and copolymers including one or more of the residue units of the monomers D-lactide, L-lactide, D,L-lactide, glycolide, ε-caprolactone, trimethylene carbonate, 1,4-dioxane-2-one, 1,5-dioxepan-2-one, or trimethylene carbonates, and combinations and blends thereof.

In certain embodiments, polymeric carriers are non-biodegradable. Exemplary non-biodegradable polymeric carries include, but are not limited to, poly(urethanes) and poly(hydroxyethylmethacrylates).

The polymer is a block copolymer. Block copolymers may be defined by the number of blocks, the order or arrangement of blocks, the total molecular weight, the ratio and type of monomers, the ratio of block lengths or weights (for block copolymers), the point of attachment of blocks (*e.g*., linear, branched or star copolymer blocks), the amount of block copolymer in the composition, and the ratio of bioactive agent to copolymer. In certain embodiments, the block copolymer is a linear, branched, star, or network polymer.

Polymeric blocks may be defined as having a distinct structure from another adjacent block. Within a single block, a copolymeric structure may also exist. For example, a diblock copolymer may comprise a block of "A" monomers and a block of alternating "A" and "B" monomers for example, as follows "AAAAAAA-BABABABABAB" or a block containing monomers "A", "B" and "C" (for example, "BBBBCCCCBBBBCCCC-AAAAAAAA"). In this case, the block copolymer contains a block of "A" monomer and a block that contains blocks of "B" and "C". This copolymer may also be characterized as a multiblock copolymer, having five blocks, one "A" block, two "B" blocks and two "C" blocks.

In certain embodiments, the polymer is a diblock polymer (AB). In certain other embodiments, the polymer is a triblock polymer (*e.g*., ABA or ABC). In yet other embodiments, the polymer is a multi-block polymer.

Copolymers may be described by a variety of nomenclatures. Herein, general polymer naming conventions are followed and abbreviations are defined. Specific diblock and triblock structures are described as follows. For diblock copolymers, the more hydrophilic block is generally named first followed by its molecular weight, *e.g*., MePEG 5000 denotes methoxypolyethylene glycol having a molecular weight of 5000 g/mol. This is followed by the more hydrophobic block with its molecular weight. For example, MePEG 5000-PDLLA 4000 denotes a diblock copolymer having a more hydrophilic block of MEPEG, MW = 5000 g/mol, and a more hydrophobic block of poly(DL-lactide), MW = 4000 g/mol, giving a polymer with total molecular weight of 9000 g/mol. For triblock copolymers of the type B-A-B the center block "A" is named first with its molecular weight followed by the external blocks "B" with their combined molecular weight. For example, "PEG 2000-PCL 2000 triblock copolymer" denotes a triblock having a center block of polyethylene glycol MW = 2000 g/mol, linked at each end with poly(e-caprolactone), both external chains having a total molecular weight of 2000 g/mol, or an average of 1000 g/mol each. When an individual block in a copolymer is itself a copolymer, its structure is defined in brackets prior to its molecular weight. For example, PEG 400-TMC/Gly (90/10) 900 is a triblock copolymer (which may be inferred by the fact that the hydrophilic block is a difunctional PEG), having a center block of PEG with MW = 400 g/mol and external blocks having a mole ratio of trimethylene carbonate (TMC) and glycolide (Gly) of 90:10 and a total molecular weight of 900 g/mol, or an average of 450 g/mol per block.

In certain embodiments, the copolymer may comprise a polymer having a bi- or multimodal molecular weight distribution, for example, a higher and lower molecular weight fraction. In certain embodiments, the copolymer may comprise a polymer with fractions having varying proportions of block length or monomer content, for example, an A-B diblock copolymer comprising 60% by weight of polymer chains with 90%mol/mol A and 10%mol/mol B and 40% by weight of polymer chains with 50%mol/mol A and 50%mol/mol-B.

Hydrophilic blocks may comprise, for example, polyethylene glycol or polypropylene glycol or a copolymer thereof (*e.g*., random, alternating or block copolymers), propylene glycol, 1,4-butanediol or poly(1-4-butanediol). These hydrophilic blocks may be reactive at more than one site (*e.g*., at two sites or more than two sites) or may be capped at one or more sites to generate less reactive sites for the preparation of diblock copolymers. Hydrophilic blocks may have molecular weights that range from between about 100 to 100,000 g/mol. Exemplary molecular weight ranges for hydrophilic blocks can be from about 200- 500 g/mol (*e.g*., about 200, 300, 340, 350, 400, 425 g/mol), or about 500-1500 g/mol (*e.g*., about 600, 725, 750, 1000 g/mol), or from about 1500-4000 g/mol (*e.g*., about 2000, 2500, 4000 g/mol), or from about 4000-10,000 g/mol (*e.g*., about 8000 g/mol), or from about 10, 000 to about 20,000 g/mol (*e.g*., about 12700 g/mol or about 20,000 g/mol). Monomers suitable for the preparation of copolymers having hydrophilic blocks include materials known to those skilled in the art, such as propylene glycol, butane diol, ethylene glycol, and the like.

In certain embodiments, a block copolymer, such as a triblock copolymer, may have structural limitations which are established to provide for a specific functional requirement. For example, the total polymer molecular weight may be sufficiently low so that the polymer is a liquid at 25°C, or have a specified maximum viscosity (*e.g*., 150 cP) at 25°C. Such a molecular weight may be, for example, about 1400 g/mol or less, or about 1000 g/mol or less, or about 900 g/mol or less. In other embodiments, the relative balance of hydrophobic (B) block(s) to hydrophilic (A) block(s) may have a specified limit, to impart properties such as drug releasing characteristics or water solubility. For example, a B-A-B type copolymer may have not more than 50%w/w of A block and not less than 50%w/w of B blocks. In other embodiments, the molecular weight of a specific block within the polymer may be specified to impart a specific characteristic, such as glass transition temperature, crystallinity, mechanical properties or drug releasing properties. For example, the molecular weight of an A block in a B-A-B polymer may be specified as being at most about 200, 400, 600, 800, 1000, 2000, 5000, 10000 or 20,000 g/mol, and/or the molecular weight of each B block may be specified as being at most about 100, 200, 300, 400, 500, 600, 700, 800, 900, 1000, 1,500, 2,000, 3,000, 4,000, 5,000, 7,500, or 10,000 g/mol,

The block copolymer comprises one or more blocks A and block B where block B is more hydrophilic than block A. The block copolymer has a molecular weight of between about 500 g/mol and about 2000 g/mol. The block copolymer is non-thermoreversible and/or a liquid at room temperature. In certain embodiments, the block copolymer is a triblock copolymer, optionally comprising a carbonate monomer. In certain embodiments, the triblock copolymer has an average molecular weight of between about 600 and about 1500 g/mol.

In certain embodiments, the block polymer is an ABA triblock copolymer wherein the B block comprises a polyalkylene oxide (*e.g*., polyethylene glycol) and the A blocks comprise a polymer having about a 90:10 mole ratio of trimethylene carbonate (TMC) and glycolide (Gly) residues. In certain embodiments, the B block has a molecular weight of between about 200 g/mol to about 600 g/mol (*e.g*., about 400 g/mol), and/or the A blocks have a total molecular weight of from about 700 g/mol to 1100 g/mol (*e.g*., about 900 g/mol).

In some embodiments, the block copolymer of the composition may be selected from those with a specific solubility characteristic. Solubility characteristics may be described in terms of the percent by mass of the polymer that is soluble in water, either before or after a purification process, such as exposing the polymer to a solvent to remove lower molecular weight or more hydrophilic or hydrophobic components. In certain embodiments, a polymer has a water soluble fraction that is less than 1, 5, 10, 20, 30, 40, 50, 60, 70, 80, or 90%w/w. In certain embodiments, complete water solubility (100%) may be desirable. Polymers with a low %w/w water soluble fraction may be used to form depot matrices for the administration of a therapeutic agent. Depot matrices that include a therapeutic agent as described herein can provide for prolonged delivery of the therapeutic agent in a patient. Polymers with a higher water soluble fraction, for example, greater than about 50 % or greater than about 80%, or that is completely water soluble, which are combined with a therapeutic agent, may be used to readily disperse the therapeutic agent upon administration to a patient. Solubility may depend on the identity of the solvents or cosolvent systems in which the polymer dissolves.

Depending on the solvent, *e.g*., a therapeutic agent effective in treating contracture may dissolve at a concentration of between about 0.001 mg/ml to about 1000 mg/ml (*e.g*., about 0.010, 0.015, 0.02, 0.1, 0.15, 0.2, 0.3, 0.6, 1, 10, 20, 50, 100, 150, 200, 400, 600, or 800 mg/ml).

Solubility may be further described in terms of the solubility parameters in which the polymer dissolves at its specified concentration level. Solubility parameters may include the interaction parameter X, Hildebrand solubility parameter 8, or partial (Hansen) solubility parameters: δp, δh and δd, describing the solvent's polarity, hydrogen bonding potential and dispersion force interaction potential, respectively. For example, a triblock or diblock polymer that will not completely dissolve at 10 or 20 mg/ml in solvents that have a characteristic δh value greater than 23 may be suitable for some applications. Yet, in other applications, a higher value may be preferred. Higher values indicate greater hydrogen bonding ability and, therefore, have a greater affinity for solvents that are capable of hydrogen bonding, such as water. A higher value of maximum observed δh for a solvent may be desirable when a more hydrophilic polymer is required. In certain embodiments, the block copolymer dissolves in a solvent having a δh value no less than 32 or 42.

In certain embodiments, the block polymer is in a solvent at a concentration of between about 1% and about 50%. In certain embodiment, the block polymer in a solvent is at a concentration of between about 2.5% to about 33%.

In certain embodiments, the composition comprises a block copolymer, and a second polymer. Suitable second polymers include copolymers and homopolymers. The second polymer may be incorporated in order to achieve or modify certain properties of the formulation such as viscosity, texture, drug release, bioadhesion or other properties described herein to be affected by polymers. For example, the polymer may be a polysaccharide, such as cellulose, chitosan, hyaluronic acid or it may be a polyacrylic acid polymer. In particular, charged polymers are particularly useful in imparting bioadhesion to the composition. In certain embodiments the polymer may be a polyether, including crosslinked polyethers or co-polymers of polyethers, including PLURONIC or TETRONIC (from BASF Corporation) polymers. In these compositions, the copolymer, for example, a triblock copolymer, may comprise a very low or very high proportion of the composition, depending on the intended use. Thus, in certain embodiments, the composition comprises no more than 10% w/w of the copolymer, while the second component is present at a concentration of at least about 50%w/w. In other embodiments, the reverse is true, and the composition comprises greater than 50%w/w of the copolymer and less than 10%w/w of the second component. In yet other embodiments, the composition may comprise greater than about 40 %, about 30%, or about 20% w/w of the copolymer.

The composition may further comprise water, in order to form a gel with a polysaccharide or other water soluble polymer. In these compositions, the copolymer may be selected to be one that is 100%w/w water soluble, micelle forming, partly water soluble (*e.g*., having a weight fraction between about 10-100% w/w that is water soluble), or may be substantially water insoluble. This selection is dependent on the intended use or desired properties of the formulation. For example, a micelle forming polymer, such as a PCL-polypropylene glycol copolymer may be selected and used to form drug loaded micelles inside a polysaccharide gel, or inside of some other polymeric aqueous gel.

In certain embodiments, the composition may comprise a diluent. Exemplary diluents include but are not limited to PEG, PEG derivatives, polypropylene glycol and polypropylene glycol derivatives. In certain embodiments the diluent has a molecular weigh of about 100, 200, 300, 400, 500, 600, 700, 800, 900, or 1000 g/mol.

In some embodiments, the composition may be used directly for a therapeutic purpose while in other, it may be used with further manipulation or processing. For example, the compositions of the invention may include precursors to final formulations or compositions. These precursors include manufacturing intermediates, materials for constitution, materials for dilution, components or a kit intended to be used together. Other components of a final composition are also possible, for example, a particulate composition may be suspended within a second composition to provide a gel or liquid suspension of particles.

In one aspect, compositions that include a block copolymer may be in the form of vesicles, micelles or reverse micelles in an aqueous environment.

In another aspect, compositions that include a block copolymer may be in the form of microspheres or microparticles, particularly those that are solid at room temperature. These microspheres may further comprise one or more therapeutic agents such as described herein.

In yet another aspect, compositions that include a block copolymer may be in a form suitable for the preparation of waxy formulations or ointments or creams or emulsions, particularly those that are semi-solid or liquid at room temperature. In these compositions, the copolymer may form a hydrophobic phase in an aqueous phase, and may be stabilized by the addition of viscosity enhancers, surfactants and other traditional pharmaceutical aids known in the art of preparation of these types of formulations.

In yet another aspect, compositions that include a block copolymer may be used for the preparation of interpenetrating networks with other polymers, particularly those which may be crosslinked or are of sufficient molecular weight.

In yet another aspect, compositions that include a block copolymer may be used for the preparation of gels, which may be aqueous or non-aqueous.

The therapeutic agent may be incorporated in a non-polymeric carrier. Non-polymeric carriers may be biodegradable or non-biodegradable and may be combined with the biodegradable or non-biodegradable compositions described above. Non-polymeric carriers may be viscous (*e.g*., having a viscosity in the range of between about 100 and about 3x10⁶ centipoise) or may be solid (having a melting point greater than ambient temperature) or a glass. Representative examples of non-polymeric carriers that may be used include sugar ester derivatives (*e.g*., sucrose acetate isobutyrate, sucrose oleate, and the like), sugar amide derivatives, fatty acids, fatty acid salts (*e.g*., calcium stearate) lipids, waxes (*e.g*., refined paraffin wax, microcrystalline wax), and vitamins (*e.g*., vitamin E).

The disclosed compositions may contain phospholipids. Phospholipids may be included in the formulation for a variety of reasons, for example, to provide lubrication at or within the target site, to enhance efficacy, to solubilize a drug, or to form a system such as an emulsion, microemulsion, liposome or liquid crystal. Phospholipids may be naturally derived and synthetic materials, which are non-toxic and biocompatible. Representative examples of phopholipids appropriate for inclusion in compositions of the invention include: lecithin, phosphatidylcholine (PC), phosphatidylethanolamine (PE), phosphatidylinositol (PI), phosphatidylserine (PS), sphingosine, cardiolipin, any derivative of sn-glycero-3-phosphoric acid that contains at least one O-acyl, or O-alkyl or O-alk-1'-enyl residue attached to the glycerol moiety; sphingosyl phosphatides referring to any lipid containing phosphorus and a long-chain base; phospholipid-like molecules, such as the alkylphosphocholines, which are known to have exhibit biological and therapeutic activities, *e.g*., phosphocholine esters of aliphatic long chain alcohols differing in chain length, unsaturation and position of the cis-double bond (Prog. Exp. Tumor Res. 34: 1, 1992).

In another aspect, the formulation may be a viscous liquid that includes a micellar or liposomal solution and a viscosity increasing agent (*e.g*., hydrogel or gel forming polymer). In one aspect, the formulation may include a continuous (aqueous) phase and a gel. The gel may include a water soluble polymer or a hydrogel, which comprises a hydrophilic polymer. The described formulation may be used to incorporate a hydrophobic drug, such as paclitaxel, into a gel or hydrogel. A liposomal or micellar matrix may be formed by, for example, reconstituting a dehydrated matrix with water, saline, or buffer. The matrix, in combination with a gel or hydrogel forming polymer, may form the desired composition. Suitable gel forming polymers include polysaccharides (*e.g.,* HA), celluloses (*e.g*., ethylcellulose), polyvinylpyrrolidone and other water soluble and biocompatible polymers (*e.g*., soluble collagen). Examples of hydrogel forming polymers include crosslinked poly(ethylene glycol)-propiondialdehyde), collagen, and other crosslinked proteins, polypeptides, and hydrophilic celluloses and other hydrophilic polymers.

In one aspect, the drug (A) effective in treating contracture (*e.g*., anti-fibrotic or an anti-proliferative agent, such as an antimetabolite or anti-microtubule agent) may be combined with a anti-inflammatory or analgesic drug (B) and at least one of (C) a phospholipid (as described herein), (D) a protein, (E) a polysaccharide, and (F) a polyether (including analogues, derivatives, cross-linked species, and copolymers of (C), (D), (E), and (F)).

The polymeric component, (D)-(F), may also provide a therapeutic benefit, such as providing a viscous medium, solubilizing or controlling release of a drug, or for altering retention of the composition or parts thereof at the site of administration.

The components (A) through (F) may be combined using standard methods known in the art, however, unique processing parameters may be required to ensure a stable, efficacious formulation. Processing parameters may include the order of mixing, maximum temperature, freeze drying, dissolution, use of high shear, or ultrasound.

In one aspect, the composition can comprise a phospholipid and at least one of (D), (E), and (F). Further, any of the components (A) through (F) may be chemically bonded to each other, or otherwise interact (*e.g*., by electrostatic, ionic, or hydrogen bonded interactions).

In addition to any of the compositions described herein, any pharmaceutically or veterinarilly acceptable vehicle, diluent, or excipient, may be included, optionally with other components. Pharmaceutically or veterinarilly acceptable excipients for therapeutic use are well known in the pharmaceutical art, and are described, for example, in Remington: The Science and Practice of Pharmacy (formerly Remington's Pharmaceutical Sciences), Lippincott Williams and Wilkins (A.R. Gennaro, ed., 20th Edition, 2000) and in CRC Handbook of Food, Drug, and Cosmetic Excipients, CRC Press (S.C. Smolinski, ed., 1992). For example, sterile saline, 5% dextrose solution, and phosphate buffered saline at physiological pH may be used.

Preservatives or stabilizers, and dyes may be provided in the composition. In one aspect, the compositions of the present invention include one or more preservatives or bacteriostatic agents present in an effective amount to preserve a composition and/or inhibit bacterial growth in a composition, for example, bismuth tribromophenate, methyl hydroxybenzoate, bacitracin, ethyl hydroxybenzoate, propyl hydroxybenzoate, erythromycin, chlorocresol, benzalkonium chlorides, and the like. Examples of the preservative include paraoxybenzoic acid esters, chlorobutanol, benzylalcohol, phenethyl alcohol, dehydroacetic acid, sorbic acid, etc. In one aspect, the compositions of the present invention include one or more bactericidal (also known as bacteriacidal) agents.

A variety of excipients may be added to impart specific properties to the formulation including, *e.g.,* colorants, antioxidants (*e.g.,* sulfites and ascorbic acid), preservatives, binders to form granules, pore formers, density, tonicity, pH or osmotic pressure adjusting materials, or degradation accelerants such as acids or bases. In certain embodiments, the compositions of this invention may further include water and/or have have a pH of about 3-9.

Examples of preservatives and bacteriostatic agents include, for example, bismuth tribromophenate, methyl hydroxybenzoate, bacitracin, ethyl hydroxybenzoate, propyl hydroxybenzoate, erythromycin, chlorocresol, benzalkonium chlorides, paraoxybenzoic acid esters, chlorobutanol, benzylalcohol, phenethyl alcohol, dehydroacetic acid, sorbic acid, and the like.

Examples of coloring agents, also referred to as dyestuffs include dyes suitable for food such as those known as F. D. and C. dyes, and natural coloring agents such as grape skin extract, beet red powder, beta carotene, carmine, turmeric, paprika, and so forth.

The composition may include radioopaque or echogenic materials and magnetic resonance imaging (MRI) responsive materials (*i.e*., MRI contrast agents) to aid in visualization of the device under ultrasound, fluoroscopy and/or MRI. For example, a delivery device may be made with or coated with a composition which is echogenic or radiopaque (*e.g*., made with echogenic or radiopaque with materials such as powdered tantalum, tungsten, barium carbonate, bismuth oxide, barium sulfate, or, by the addition of microspheres or bubbles which present an acoustic interface). For visualization under MRI, contrast agents (*e.g*., gadolinium (III) chelates or iron oxide compounds) may be incorporated into the composition or device, such as a component in a coating or within the void volume of the device (*e.g*., within a lumen, reservoir, or within the structural material used to form the device).

### FORMULATION

As noted above, therapeutic compositions of the present invention may be formulated in a variety of forms (*e.g*., microspheres, solutions, dispersions, pastes, films, sprays, coatings, gel, hydrogel, foam, sheet, mold, mesh, wrap, and the like. Further, the compositions of the present invention may be formulated to contain more than one therapeutic agent, to contain a variety of additional compounds, to have certain physical properties (e.g., elasticity, a particular melting point, or a specified release rate). Within certain embodiments of the invention, compositions may be combined in order to achieve a desired effect (*e.g*., several preparations of microspheres may be combined in order to achieve both a quick and a slow or prolonged release of one or more therapeutic agents.

Within certain aspects of the present invention, the therapeutic composition should be biocompatible, and release one or more therapeutic agents over a period of several hours, days, or, months. Within certain aspects of the present invention, the therapeutic composition releases one or more therapeutic agents over a period of several hours (*e.g*., 1 hour, 2 hours, 4 hours, 8 hours, 12 hours or 24 hours) to days (*e.g*., 1 day, 2 days, 3 days, 7 days, or 14 days) to months (*e.g*., 1 month, 2 months, 3 months, 6 months or 12 months).

Release profiles may be characterized in terms of the initial rate, time for 50%, 90% or 100% drug release, or by appropriate kinetic models such as zero-order, first order, diffusion controlled (*e.g*., square-root of time, Higuchi model) kinetics, or by the number of distinct phases of release rate (*e.g*., monophasic, biphasic, or triphasic).

The release profile may be characterized by the extent of its burst (initial) phase. For example, "quick release" or "burst" therapeutic compositions are provided that release greater than 10%, 20%, or 25% (w/v) of a therapeutic agent over a period of several hours to several days (*e.g*., 1, 6, 12 or 24 hours, or 2, 3, 7 or 10 days). Such "quick release" compositions should, within certain embodiments, be capable of releasing therapeutically effective levels (where applicable) of a desired agent. Within other embodiments, "slow release" therapeutic compositions are provided that release less than 10 to 20% (w/v) of a therapeutic agent over a period of 7 to 10 days. For microparticles, the burst phase may result in little or large amounts of drug release and consequently microparticles may be defined as "low" or "high" burst systems. For example, low burst systems may release as little as about 30, 20,10 or even 5 or 1% of the total amount loaded in the initial phase of release. High burst systems may release at least about 50, 60, 70 or even 100% of the total amount of drug in the burst phase. The duration of the burst phase is dependant on the overall intended duration of the release profile. For microparticles intended to release all of the loaded drug within hours, the burst phase may occur over several minutes (*e.g*., 1 to 30 minutes). For microparticles intended to release over several days, the burst phase may on the order of hours (*e.g*., 1 to 24 hours). For microparticles intended to release over several weeks, the burst phase may be from several hours to several days (*e.g*., 12 hours to 7 days). An exemplary release profile describing a composition's release characteristics may be a low burst, releasing less than 10% in the first 24 hours, followed by a phase of approximately zero-order release and a gradual reduction in rate after 5 days, until all of the drug is depleted.

Compositions within the scope of this invention may have a wide range of release characteristics depending on the composition. For example, a mycophenolic acid or 5-fluorouracil loaded microparticle made of a relatively hydrophilic polymer will have a high burst and release all of the drug with in several hours to a few days. Alternately, a paclitaxel loaded composition may release only a small fraction of the total dose over 5 days, with a very small burst phase.

Further, therapeutic compositions of the present invention should preferably be stable for several months and capable of being produced or maintained under sterile conditions.

In one embodiment, the drug release from these compositions can be diffusion controlled, erosion controlled or a combination of both mechanisms.

In another embodiment, the drug release can be first-order release, zero-order release or a combination of these orders of release.

Polymers and polymeric carriers of the invention may also be fashioned to have particularly desired release characteristics and/or specific properties. For example, polymers and polymeric carriers may be fashioned to release a therapeutic agent upon exposure to a specific triggering event such as pH as discussed above. Likewise, polymers and polymeric carriers may be fashioned to be temperature sensitive as discussed above.

A wide variety of forms may be fashioned by the excipients and carriers of the present invention, including for example, coatings, threads, braids, knitted or woven sheets, tubes and rod-shaped devices, (*see, e.g.,* Goodell et al., Am. J. Hosp. Pharm. 43:1454-1461, 1986; Langer et al., "Controlled release of macromolecules from polymers", in Biomedical polymers, Polymeric materials and pharmaceuticals for biomedical use, Goldberg, E.P., Nakagim, A. (eds.) Academic Press, pp. 113-137, 1980; Rhine et al., J. Pharm. Sci. 69:265-270, 1980; Brown et al., J. Pharm. Sci. 72:1181, 1983; and Bawa et al., J. Controlled Release 1:259, 1985). Therapeutic agents may be incorporated into the device by, for example, dispersion in the polymer or in the void volume of a pledget or sponge material, dissolution in the polymer matrix, coating onto, and by binding the agent(s) to the device via covalent or noncovalent linkages. The therapeutic agents may be incorporated into a secondary carrier (*e.g*., microparticles, microspheres, nanospheres, micelles, liposomes and/or emulsions) that is then incorporated into the primary carrier as described above. Within certain embodiments of the invention, therapeutic compositions are provided in formulations such as knitted or woven meshes, pastes, sheets, films, particulates, tubes, gels, foams, braids, and sprays.

Preferably, therapeutic devices or compositions of the present invention are fashioned in a variety of manners to meet a variety of intended uses. For example, a therapeutic agent is dissolved or dispersed in a biodegradable polymer carrier for intraarticular injection. The therapeutic device or composition generally should be biocompatible, and release one or more therapeutic agents over a period of several days to months with the specific release profile being appropriate for the specific indication being treated.

Therapeutic agents and compositions of the present invention may be administered either alone, or in combination with pharmaceutically or physiologically acceptable carrier, excipients or diluents. Generally, such carriers should be nontoxic to recipients at the dosages and concentrations employed. Ordinarily, the preparation of such compositions entails combining the therapeutic agent with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents.

As noted above, therapeutic agents, therapeutic compositions, or pharmaceutical compositions provided herein may be prepared for administration by a variety of different routes, including for example, periarticular injections or intraarticularly to a joint (*e.g*., direct injection with a needle or catheter, under fluoroscopy, through a portal in a arthroscope) or transdermally). Other representative routes of administration include spraying soft tissue after an open or closed procedure or administration of the therapeutic composition into the affected area through a directed route such as a needle, or leaving a therapeutic composition releasing the therapeutic agent in the area. Systemic administration of an agent may also be used.

In addition to the excipients, methods and compositions described earlier, processing methods may be required to produce compositions of the present invention.

In some aspects the compositions of the present invention are sterile. Many pharmaceuticals are manufactured to be sterile and this criterion is defined by the USP XXII <1211>. The term "USP" refers to U.S. Pharmacopeia (see www.usp.org, Rockville, MD). Sterilization in this embodiment may be accomplished by a number of means accepted in the industry and listed in the USP XXII <1211>, including without limitation autoclaving, dry heat, gas sterilization, ionizing radiation, and filtration. Sterilization may be maintained by what is termed aseptic processing, defined also in USP XXII <1211>. Acceptable gases used for gas sterilization include ethylene oxide. Acceptable radiation types used for ionizing radiation methods include gamma, for instance, from a cobalt 60 source and electron beam. A typical dose of gamma radiation is 2.5 MRad. Filtration may be accomplished using a filter with suitable pore size, such as 0.22 µm, and of a suitable material, such as TEFLON. In one aspect, when the polysaccharide is hyaluronic acid (HA) or a derivative thereof, the sterilization should be by a method other than irradiation as the HA tends to lose stability after exposure to gamma radiation. Furthermore, a sterile composition may be achieved by using a combination of these sterilization methods and optionally aseptic techniques. In certain aspects of the invention including microparticles greater than 200 nm in diameter, a method of sterilization other than filtration should be used since the particles would not pass easily through the filter. Since not all components of the composition may be conveniently sterilized by a single method, sterilization may be accomplished by sterilizing components in separate steps. The sterilized components then may be combined into the embodied composition.

In some aspects, the compositions of the present invention are contained in a container that allows them to be used for their intended purpose, *i.e*., as a pharmaceutical composition. Properties of the container that are important are a volume of empty space to allow for the addition of a constitution medium, such as water or other aqueous medium (*e.g*., saline), an acceptable light transmission characteristic in order to prevent light energy from damaging the composition in the container (refer to USP XXII <661>), an acceptable limit of extractables within the container material (refer to USP XXII), and an acceptable barrier capacity for moisture (refer to USP XXII <671>) or oxygen. In the case of oxygen penetration, this may be controlled by including in the container a positive pressure of an inert gas such as high purity nitrogen, or a noble gas such as argon.

Typical materials used to make containers for pharmaceuticals include USP Type through III and Type NP glass (refer to USP XXII <661>), polyethylene, polyvinyl chloride, TEFLON, silicone, and gray-butyl rubber. For parenterals, USP Types I to III glass and polyethylene are preferred. In addition, a container may contain more than one chamber (*e.g*., a dual chamber syringe) to allow extrusion and mixing of separate solutions to generate a single bioactive composition. In one embodiment, microparticles dispersed in a carrier component (*e.g*., a polymer) may be in a first delivery chamber and a second carrier component (*e.g*., a buffer) may be in a second delivery chamber.

Compositions may be administered to a patient as a single dosage unit or form (*e.g*., a hydrogel implant or an orthopedic device), and the compositions may be administered as a plurality of dosage units (*e.g*., in aerosol form as a spray, or a solution dispensed from a multidose tube). For example, the anti-microtubule agent formulations may be sterilized and packaged in single-use, plastic laminated pouches or glass vials of dimensions selected to provide for routine, measured dispensing.

In certain embodiments, the compositions of the present invention are subjected to a process of lyophilization, including lyophilization of any of the compositions described above to create a lyophilized powder. Alternatively, compositions of the invention may be spray dried as described above. It may be desirable to further reconstitute the lyophilized powder with water or other aqueous media, such as benzyl alcohol-containing bacteriostatic water for injection, to create a reconstituted suspension of microparticles (Bacteriostatic Water for injection, Abbott Laboratories, Abbott Park, IL).

The present disclosure also provides kits that include a therapeutic agent useful in the treatment or prevention of one or more conditions associated with reduced mobility or loss of articulation. The kit may include a first composition that includes a therapeutically effective amount of a therapeutic agent, wherein the therapeutic agent is active in treating symptoms associated with joint contracture. In one embodiment, for example, the first composition may be in the form of microspheres. The kit may include a second composition, *e.g*., a polymeric carrier, in the form of a solution. The kit may provide a set of instructions for delivering the compositions to the target site. Optionally, the kit may include a device or devices for administering the compositions. Other kits may include multiple therapeutic agents in one or more compositions. For example, a kit may be provided having a first composition which is an injectable formulation and a second which is an implant, oral or topical medication.

### IV. TREATMENT OF CONTRACTURE

In order to further the understanding of the compositions and methods for their use, representative clinical applications are discussed in more detail below.

In one aspect, the invention provides a composition for use in a method for treating a contracture. The contracture may affect a joint, such as an elbow, a shoulder, a knee, an ankle, a hip, a finger joint, a wrist, a toe joint, a temporomandibular joint, a facet joint, an otic bone joint, or a combination thereof. Alternatively, or in addition, the contracture may affect one or more types of soft tissue, such as, *e.g*., muscles, tendons, ligaments, fat, synovium, joint capsule, connective tissue, such as fascia, or a combination thereof. The contractures may arise after an injury or may be related to an underlying genetic or medical condition (such as arthritis or a hyperproliferative disease). In one aspect, the contracture may involve a thickening and fibrosis of the capsule and/or other soft tissue in (*e.g*., capsule) and around (*e.g*., volar plate) the joint which limits the function of a joint. In other cases, the contracture may be due to fibrosis within the soft tissue that may be more remote from the joint (*e.g*., muscle or palmar facia). In one aspect, the contracture may be induced by a bum or crush injury. In another aspect, the contracture may have a genetic predisposition, such as in Dupuytren's contracture, Peyronie's contracture, Ledderhose contracture, or be induced by ischemia such as in a Volkmann's contracture.

Any joint with the potential for contracture may benefit from the administration of therapeutic agents as described herein. The therapeutic agent or composition comprising the therapeutic agent may be administered, for example, after joint trauma, arthroplasty, closed or open manipulation or any other injury or procedure that may lead to a contracture.

The compositions, therapeutic agents, and methods may be used, for example, to prevent a contracture prophylactically, to prevent the recurrence of a contracture, and as an adjunct to surgical methods for treating contractures. Further, the present compositions may inhibit thickening of scar tissue at the site of intervention, which can negatively impact range of motion and appearance.

In one aspect, the patient is administered a therapeutically effective amount of a therapeutic agent (*e.g*., an anti-microtubule agent) composition, as described herein. In one aspect, the agent may be delivered directly to a target site. In another aspect, the method includes forming a therapeutic agent composition, and then introducing the composition into an aqueous environment, wherein a target site is in contact with the aqueous environment. The contracture may be treated with the above methods using, *e.g*., suspensions, solutions, gels, hydrogels, sprays, sutures, sponges, pledgets, implantable membranes, orthopedic implants, films, or microparticles that include a therapeutic agent, as described above. The above methods may be used to administer the compositions described herein by intraarticular, periarticular, or peritendinal administration, or administration into an operative site, such as an opened joint or during arthroscopy. The present compositions may be injected into the joint or surrounding tissues depending on the clinical application. Other formulations may be implanted, either temporarily or permanently. For example, a pledget containing drug may be implanted into a repair site (*e.g*., a tendon) for a period of time as short as 30 seconds during the procedure. Other implants, such as hydrogels, may be implanted in a similar procedure and remain for a period of hours, days or months, being removed by bioresorptive processes.

In one aspect, a method for treating joint contracture is provided, in which a patient in need of treatment is administered a therapeutic agent effective in treating contracture. A therapeutic agent may be administered to a joint, for example, directly after the treatment of an injury, such as a fracture or dislocation, in order to prevent the onset of the contracture. For example, a patient who has just suffered an elbow fracture, *e.g*., to the radial head, may also be administered a therapeutic agent effective in treating contracture. The agent may be administered directly to the joint, *e.g*., by intrarticular injection of a composition in accordance with the invention. In another aspect, the patient may already have a contracture that affects movement of a joint, which requires surgical intervention in order to excise the fibrotic tissue. A therapeutic agent in accordance with the present invention may be administered to the patient at the time of or after surgical removal of the tissue in order to prevent reoccurrence of the contracture.

In one embodiment, a therapeutic agent, such as an anti-microtubule agent (*e.g*., paclitaxel or a paclitaxel derivative or analogue), is injected intraarticularly into a joint or the area of a joint to treat the contracture. The same principle could be used in the case of an established contracture. The contracture can be broken down by manipulation (under anesthesia) or surgically with an open procedure or through an arthroscope, releasing, reducing or eliminating the scar. An anti-microtubule agent (*e.g*., paclitaxel or a paclitaxel derivative or analogue) can be injected intraarticularly or into the periarticular area to prevent the recurrence of the contracture.

Intra-articular injection may be performed after completion of the surgery by delivering into the joint an appropriate volume a therapeutic agent or composition that comprises a therapeutic agent through a needle that has been directly connected into one of the established portals of the surgical instrumentation. For example, in the case of a shoulder or elbow, the contracture causing tissue would be removed, broken down or dissected, and then about 3 ml to 5 ml of the intra-articular agent would be introduced through an 18G to 25G 1.5 inch needle. In the case of an open procedure, the contracture causing tissue would be dissected, pathological tissue removed and capsulotomy or synovectomy may be performed if required. After the procedure and proper irrigation of the tissue to remove any debrided or pathological tissue, the intra-articular agent may be introduced to prevent the reformation of a contracture. The therapeutic agent may be introduced at anytime during the procedure, but for reasons of retention, an optimal time may be via an intra-articular injection into the affected joint after the closure of the joint to prevent the reformation of the contracture.

In one aspect, methods are provided to prophylactically prevent the formation of a contracture either completely or partially in an elbow, knee, or shoulder, however, the method may be utilized in to treat any joint with potential to form contractures. After the traumatic event, a needle (using sterile technique) may be used to introduce the therapeutic compound intra-articularly. In the case of an elbow, elbow fractures all have the potential for late onset contracture that may become disabling because of its impact on range of motion. After the injury, a 25G needle is introduced between the radial head and olecranon process laterally injecting 1 ml to 3 ml volume containing the active compound. Range of motion is commenced immediately if permissible or the fracture is treated as per standard protocol. In the case of a shoulder, a posterior or posterior-lateral approach can be used with a 20G to 25G 1.5 inch needle. In the case of a knee, the knee can be approached antero-medially, antro-laterally or via supro-lateral approach with the same size needle to introduce the intra-articular agent. The active compound may stop, retard or limit the prolific, inflammatory and other pathological responses that lead to a contraction and the formation of contracture inducing tissue.

In another aspect, the contracture may be caused by extra-articular formation of pathological tissue, for example, a Dupuytren's contracture or the tissue surrounding a PIP joint. In the case of Dupuytren's Disease, there is thickening of the palmar tissue and often contracture of the fingers. The deformity leads to disfigurement, pain and difficulty with function. Due to less than optimal results and high incidence of recurrence, surgery is not offered until the pain or deformity (typically greater than 30 degrees contracture) is substantial Methods for treating such contractures may involve an open or closed procedure. The most common five surgical procedures employed are 1) subcutaneous fascitomy, (2) parial (selective) fasciectomy, (3) complete fasciectomy, (4) fasciectomy with skin grafting, and (5) amputation. The first 4 of these procedures are associated with a high rate of recurrence, at least 30%, which often require repeat surgery. The therapeutic agent or a composition comprising the therapeutic agent would be applied most logically after the contracture forming tissue has been resected or released and just before closure of the site. The agent or composition comprising the agent may be sprayed on, poured on, or delivered locally by any other means. Local delivery of a therapeutic compound to the affected site may prevent the reformation of the contracture forming tissue either completely or partially. Additionally, administration of a therapeutic agent in accordance with the invention may prevent, either completely or partially, the formation of a thickened scar in the area of the surgery which has the affect of limiting the flexion of the joints of the hand. In the case of a minimally invasive technique, after removal and release of the contracture tissue, the therapeutic agent would be delivered through an appropriate portal on the scope that is utilized in the procedure.

In one aspect, the present invention provides a composition for use in a method for treating the recurrence of a Dupuytren's contracture. A patient who exhibits the symptoms of Dupuytren's contracture, (*e.g*., loss of mobility of a finger and thickening of scar tissue in the palms), the scar would be surgically removed by standard or acceptable plastic surgery techniques. A therapeutic composition could then be sprayed or injected into the affected and resected area to prevent the recurrence of the contractures. The same procedures could be used in the area of the penis for conditions such as, but not limited to, crush injuries or Peyronie's contracture and to the plantar fascia for conditions such as, but not limited to, post operative scarring and Ledderhose Disease.

In the case of an established contracture, the invention is used as part of the treatment to prevent a recurrence of the contracture either completely or partially. The majority of patients who sustain elbow trauma are left with a residual contracture, and most surgeons prefer not to surgically intervene unless the contracture is greater than 45 degrees, patient has less than 100 degrees of motion or the patient is greatly limited by function or pain. The reluctance to operate is multifold but some of the major considerations include a high rate of reformation of the contracture deformity, risk of injuring the nerve structures around the elbow and infection. In fact many surgeons can only increase the range by another 45 degrees once a contracture is established in the elbow. The knee has less of a tendency for contracture formation, but as an example, can occur in 5% to 20% of patients who undergo anterior cruciate reconstruction. This arthrofibrosis may not only be disabling functionally and cause pain, but may further mature into fibrocartilage and cause joint destruction.

The treatment of established contractures involves the surgical removal or destruction of the contracture tissue, and removal of abnormal synovium or capsule in an open or closed fashion. In the case of a knee for example, the standard three ports (antro-medial, antro-lateral and cerebro-medial) are established with the inflow port cerebro-medial. A cannula may be used if desired and a 4 mm or 5 mm shaver or shaver like device may be used to remove pathological fibrotic tissue, perform a synovectomy or capsulotomy to restore normal range of motion. Blunt dissection with a probe may be sufficient to adequately break down adhesion. After the procedure, the traumatized sites typically respond by reforming the tissue responsible for the contracture.

In one aspect, the contracture may be due to idiopathic causes such as "frozen shoulder" or adhesive capsulitis. This painful and restrictive condition has no satisfactory treatment presently; steroids, anti-inflammatories, physical therapy and surgery have all been met with limited success. Introducing the therapeutic agent intra-articularly early in the disease may prevent, retard or limit the formation or progression of a "frozen shoulder", decreasing or eliminating the formation of pathological tissue, decreasing or eliminating the pain associated with the condition and increasing or preserving the range of motion. In the case of an established frozen shoulder, there is very little that can be done other than symptomatic treatment and physiotherapy, which is of limited use. If the condition is severe enough a release procedure maybe offered. The surgery is usually performed through the standard arthroscopic shoulder portals. The adhesions are bluntly dissected or removed with a shaver and a synovectomy or capsulotomy can be done to take down the tissue to stable tissue. In certain patients, the affected tissue can be up to 1 cm in thickness. After the procedure, the typical patient may begin to experience stiffness and almost immediate reformation of pathological contracture inducing tissue. Invention can be introduced into the joint at any point, but is best introduced after the procedure is complete through and established cannula before closing or through a 18G to 20G 1.5 inch needle through one of the established portals then closed with a suture or steri-strip. The release of a "frozen shoulder" may also be accomplished as an open procedure, and this is causes more trauma, is associated with a higher incidence of recurrence. The active compound can be introduced at the end of the procedure after closure of the capsule or at the very end of the operation through a 1.5 inch needle into the gleno-humeral joint using a standard, such as the structures of the anterior shoulder.

### EXAMPLES

### EXAMPLE 1

### PRODUCTION OF A MICELLAR CARRIER FOR PACLITAXEL FORMED AS A PACLITAXEL-POLYMER MATRIX

Polymer synthesis: A diblock copolymer used as a micellar carrier for paclitaxel was prepared as follows. A 60:40 methoxy polyethylene glycol (MePEG):poly(DL-lactide) diblock copolymer was prepared by combining 60 g of DL-lactide and 40 g of MePEG (MW = 2,000 g/mol) in a round bottom glass flask containing a TEFLON-coated stir bar. The mixture was heated to 140°C with stirring in a temperature controlled mineral oil bath until the components melted to form a homogeneous liquid. Then 0.1 g (or 0.5 g in some batches) of stannous 2-ethyl hexanoate was added to the molten mixture and the reaction was continued for 6 hours at 140°C with continuous stirring. The reaction was terminated by cooling the product to ambient temperature. The product, 60:40 MePEG:poly(DL-lactide) diblock copolymer, was stored in sealed containers at 2-8°C until use.

Preparation of Paclitaxel Polymer Matrix: A micellar paclitaxel composition was prepared from the diblock copolymer as follows. A solid composition capable of forming micelles upon constitution with an aqueous medium was prepared as follows. Then 41.29 g of MePEG (MW = 2,000 g/mol) was combined with 412.84 g of 60:40 MePEG:poly(DL-lactide) diblock copolymer in a stainless steel beaker, heated to 75°C in a mineral oil bath and stirred by an overhead stirring blade. Once a clear liquid was obtained, the mixture was cooled to 55°C. To the mixture was added a 200 ml solution of 45.87 g paclitaxel in tetrahydrofuran. The solvent was added at approximately 40 ml/min and the mixture stirred for 4 hours at 55°C. After mixing for this time, the liquid composition was transferred to a stainless steel pan and placed in a forced air oven at 50°C for about 48 hours to remove residual solvent. The composition was then cooled to ambient temperature and was allowed to solidify to form a micellar form of paclitaxel.

Micellar formulations for paclitaxel and other hydrophobic drugs may also be formed from other water soluble block copolymers including several synthesized according to Example 18 and determined to have a very high or complete water solubility according to Example 19 and PLURONIC polymers, such as those in Example 10.

### Reference EXAMPLE 2

### MICELLAR PACLITAXEL DISPERSED IN A HYALURONIC ACID GEL

A 2 g aliquot of paclitaxel-polymer matrix from Example 1 was dissolved in 100 ml water and the pH adjusted to between 6 and 8 by the addition of 1 M sodium hydroxide solution. Into a separate container, 1 mg of 1 MDa hyaluronic acid (Genzyme, Cambridge, MA) was added and then 1 ml of the pH adjusted paclitaxel solution was added with stirring to dissolve the hyaluronic acid. The result was a hyaluronic acid gel containing 10 mg/ml hyaluronic acid and 2 mg/ml paclitaxel. A second formulation was prepared in a similar manner to a concentration of 15 mg/ml paclitaxel by dissolving 15 g of micellar paclitaxel in 100 ml prior to pH adjustment. Using this method, by varying the paclitaxel content, formulations were prepared having paclitaxel concentrations between 1.5 and 30 mg/ml. Specifically, 1.5, 4.5, 7.5, 15 and 30 mg/ml were prepared.

### Reference EXAMPLE 3

### PACLITAXEL DISPERSED IN A MICELLAR CARRIER IN A CARRIER COMPOSED OF A FABRIC

A 2 g aliquot of paclitaxel-polymer matrix from Example 1 is dissolved in 100 ml water and the pH adjusted to between 6 and 8 by the addition of 1 M sodium hydroxide solution. The solution is used to dip carrier matrices, soaking the paclitaxel in micellar form into the carrier. A SEPRAFILM patch is dipped into the solution and allowed to soak in the liquid for 30 seconds. The patch is removed and gently rolled up and unrolled again and any liquid dripping from the fabric was allowed to come off, removing any excess liquid. Alternately, a pledget made of cotton is dipped in the same manner. The SEPRAFILM formulation is intended to be inserted into a patient without needing to withdrawn at a later time. The pledget formulation is intended to be inserted into the patient for instance adjacent to a tendon repair, and removed after a short period of time, for example 2 minutes. Using this method, by varying the paclitaxel content, formulations may be prepared having paclitaxel concentrations between 0.15 and 30 mg/ml.

### Reference EXAMPLE 4

### PACLITAXEL DISPERSED IN A MICROEMULSION IN A HYALURONIC ACID GEL

Paclitaxel in a microemulsion carrier was incorporated into a hyaluronic acid gel as follows. Forty grams of water was added to a beaker that contained 1 g hyaluronic acid (180 kDa, Bioiberica, Spain). The mixture was allowed to dissolve with stirring (400 rpm for at least 30 minutes) to form a homogeneous gel. To 38.5 g of LABRASOL was added 100 mg of paclitaxel and the mixture stirred (400 rpm for at least 20 minutes) until a clear solution formed. To the paclitaxel solution was added 5 g of LABRAFAC and 16.5 g PLUROL OLEIQUE with continued stirring for at least 10 minutes to form a visibly homogeneous mixture. The paclitaxel phase was added to the hyaluronic acid phase with further stirring for at least one hour. After stirring, the composition was allowed to stand for at least one hour to allow most of the bubbles to migrate from the gel. The product contains about 0.99 mg paclitaxel/g gel and 9.9 mg hyaluronic acid/g gel.

This composition is alternately prepared with hyaluronic acid having a molecular weight of 1 MDa (Genzyme, Cambridge, MA). In these compositions, the exact process is duplicated with the exception that longer stirring times and standing times are used for phases containing higher molecular weight hyaluronic acid. Typically, these are increased by a factor of 5 to 10. Following stirring, if a homogeneous phase is not formed, the mixture is transferred to a 100 ml syringe, attached to a second 100 ml syringe, and then transferred back and forth 30 times between the two syringes through a 1/16" ID tube to effect mixing. Following that, the mixture is allowed to stand for about 16 hours.

### Reference EXAMPLE 5

### PREPARATION OF A CO-SOLVENT/PACLITAXEL/HYALURONIC ACID FORMULATION

A hyaluronic acid gel containing paclitaxel with a co-solvent carrier is prepared as follows. 9 ml of PEG 200 is used to dissolve 30 mg of paclitaxel. Once a clear, particulate free solution results, water is added to adjust the volume to 10 ml. This "active" phase is transferred to a 10 ml syringe. In a second 10 ml syringe, 200 mg of hyaluronic acid (e.g., 1.6M Da molecular weight) is combined with 10 ml of a mixture of PEG 200 and water having a PEG:water ratio of 3:7. The powder is allowed to dissolve in the co-solvent mixture over a 16 hour period. If needed to produce a homogeneous solution, the mixture is mixed by transferring it back and forth 30 times between two syringes joined by a short piece of 1/16" ID tubing. After both syringes are prepared they are connected to a Y-connector, which is connected by its third opening to an empty 20 ml syringe. The two 10 ml syringes are placed in a syringe pump and the contents of both are pumped at the same rate into the 20 ml syringe. Once the transfer is complete, the contents of the 20 ml syringe are transferred back and forth 30 times to a second, empty 20 ml syringe attached by a short piece of 1/16" ID tubing. The result is a 20 ml solution that is a gel of hyaluronic acid (10 mg/ml) containing paclitaxel (1.5 mg/ml) in a co-solvent carrier. Using this method, by varying the paclitaxel content, formulations were prepared having paclitaxel concentrations between 0.45 and 15 mg/ml. Specifically, 0.45, 0.75, 1.5, 4.5, 7.5 and 15 mg/ml were prepared.

### Reference EXAMPLE 6

### NANOPARTICLES OF PACLITAXEL CONTAINED IN A GEL

An aliquot of nanoparticulate paclitaxel is obtained from its supplier (either commercial or non-commercial) in either an aqueous form or as a lyophilized material for constitution according to the following table.

| Nanoparticle Name | Solution Concentration | Supplier |
|---|---|---|
| HYDROPLEX Paclitaxel | 10 mg pactitaxel/ml | ImaRx |
| DISSOCUBE Paclitaxel | 10 mg paclitaxel/ml | SkyePharma PLC |
| NANOCRYSTAL Paclitaxel | 50 mg/ml paclitaxel/ml | Elan Pharmaceuticals |

Alternately, NANOCRYSTAL paclitaxel is produced using a pearl mill. The milling balls used in such mills range in size from about 0.4 mm to 3.0 mm. Current pearl materials are glass and zirconium oxide. Alternatively, the pearl mills can be made from a hard polymer, e.g., especially cross-linked polystyrene. Depending on the hardness of the drug powder and the required fineness of the particle material, the milling times range from hours to days (Liversidge, in "Drug Nanocrystals for Improved Drug Delivery" at CRS Workshop Particulate Drug Delivery Systems 11-12, July 1996, Kyoto, Japan). The preferred size range for NANOCRYSTAL is below 400 nm, and about 100 nm for paclitaxel (Liversidge & Cundy Int J Pharm 1995(125) 91). After the milling process the drug nanoparticles need to be separated from the milling balls.

The aliquot of nanoparticulate paclitaxel is diluted with a 20 mM phosphate buffered 0.9% saline solution to a final concentration of 3 mg paclitaxel/ml. A gel phase is prepared by dissolving 20 mg/ml 1 MDa hyaluronic acid (Genzyme, Cambridge, MA) in water. Alternate gel phases may be prepared utilizing other polysaccharides such as dextran, polyethylene glycols, such as PEG 20k, or polypeptides such as water soluble collagen.

A 10 ml aliquot of the gel phase is transferred to a depyrogenated serum bottle and capped with a flat bottomed stopper and sealed. A venting needle is placed in the stopper and the bottle is autoclaved at 135°C for 15 minutes. After sterilization a 10 ml aliquot of the paclitaxel phase is sterile filtered by passing it through a 0.22 µm filter into the bottle containing the gel. The contents of the bottle are mixed first by inversion of the bottle and finally by repeatedly withdrawing the contents of the bottle through a 25-gauge needle into a syringe and re-injecting the contents into the bottle until a visibly homogeneous liquid is observed. The result is a formulation containing 1.5 mg/ml paclitaxel and 10 mg/ml hyaluronic acid in a sterile buffered aqueous dispersion. The formulation is stored for a maximum of 24 hours at 2-8°C and may be used by intra-articular injection provided the vial contents are visually clear, with no signs of precipitation.

### EXAMPLE 7

### MANUFACTURE OF PACLITAXEL-LOADED PLA- AND PLGA-PEG COPOLYMER MICROSPHERES

Microspheres containing 5, 10 or 20% paclitaxel in low molecular weight star-shaped PLA and PLGA (M.W. ≈ 10,000 by gel permeation chromatography) were prepared by an oil-in-water emulsification technique. Briefly, the appropriate weights of the paclitaxel and 0.5 polymer were dissolved in 10 ml of dichloromethane and emulsified with a overhead propeller stirrer at the level of 3 (Fisher Scientific) into 100 ml 1% polyvinyl alcohol solution for about 3 hours. The formed microspheres were sieved and dried under vacuum at a temperature below 10°C. Yield of microspheres in the desired size range (53 - 90 pm) was about 50% and the encapsulation efficiency of paclitaxel in microspheres was about 98%.

Release studies were done by placing 2.5 mg of the microspheres in a 15 ml TEFLON capped tube (with 10 ml phosphate buffer saline with albumin). The microsphere/buffer solution was tested daily (three sampling at the first day) to maintain the sink condition. Release study data showed that paclitaxel was released from the star-shaped microspheres 3 to 10 times faster than the conventional linear PLA and PLGA microspheres.

### Reference EXAMPLE 8

### MANUFACTURE OF PACLITAXEL-LOADED GELATIN MICROSPHERES

For a 5% paclitaxel loaded gelatin formulation, 50 mg of paclitaxel was mixed with 950 mg of gelatin. The mixture was gradually heated up to and maintained at 70°C until the paclitaxel was completely dissolved in the molten gelatin. Mixed the solution for 30 minutes with a stirrer bar at 600 rpm. The resulted solution was cooled down to room temperature and became solidified. The solid gelatin-paclitaxel solution was ground into the microparticles until the anticipated size ranges were achieved.

### Reference EXAMPLE 9

### MANUFACTURE OF PACLITAXEL-LOADED CROSS-LINKED HYALURONIC ACID MICROSPHERES

Two hundred milligrams of hyaluronic acid (sodium salt) was dissolved in 10 ml of distilled water overnight. 3.3 mg of paclitaxel (Hauser Chemical Company, Boulder, CO) was placed in a 2 ml homogenizer and 1 ml of water was added. The paclitaxel was hand homogenized for 2 minutes to reduce the particle size. Immediately before the experiment, the homogenized paclitaxel was added into 3.3 ml of hyaluronic acid solution and mixed together using a spatula. 50 ml of light paraffin oil (Fisher Scientific) containing 250 µl of Span 80 (Fisher Scientific) was stirred at 600 rpm at 50°C using a propeller type overhead stirrer (Fisher Scientific) in a 100 ml beaker on a heating block. The hyaluronic acid-paclitaxel solution was added to the paraffin and allowed to stir for one hour at 50°C. Then, 200 µl of a 0.02% EDA carbodiimide (Aldrich) was added to the oil to initiate cross-linking of the hyaluronic acid. The hyaluronic acid microspheres were allowed to form over the next four hours. The microspheres (10 to 100 µm) were then allowed to settle under gravity and then washed three times with hexane.

### EXAMPLE 10

### PREPARATION OF PACLITAXEL-PLURONIC F127 FORMULATION

The PLURONIC F127 formulation was prepared in three stages. In the first stage, three PLURONIC-paclitaxel polymer matrices containing 0.75, 3.75, and 7.50% paclitaxel were prepared. Paclitaxel was dissolved in tetrahydrofuran and mixed with molten PLURONIC F127 at 55°C. The polymer matrix was stirred for 1 hour at 55°C, then poured onto a stainless steel tray and dried under forced air at 55°C for 16 hours. The molten polymer matrix was cooled to room temperature, covered with aluminum foil and placed in the 2-8°C cold room for 30 minutes. The solid polymer matrix was transferred to an amber glass jar and stored at 2-8°C until use.

In the second stage, three 20% w/v PLURONIC F127 micellar gels were prepared with final paclitaxel concentrations of 1.5, 4.5, 7.5, and 15 mg/ml using the paclitaxel-polymer matrices made in the first stage. A fourth gel was prepared having no paclitaxel, using PLURONIC F127. A 10 g aliquot of polymer matrix was dissolved in 42.05 g of 0.9%w/v aqueous sodium chloride and left without agitation at 2-8°C (in the walk in cold room) for at least 16 hours. A stir bar was then added and the solution stirred for an additional 4 hours at 2-8°C. From each gel, a 3 ml aliquot was dispensed into 5 ml serum vials. The solutions were lyophilized for at least 48 hours at -20°C and the lyophilized formulations sterilized by gamma radiation.

In the third stage, the lyophilized gels were constituted with 2.3 ml of sterile water. The vials were held at 2-8°C without agitation for at least 16 hours. An autoclaved stir bar was added and the gel was stirred for an additional 30 minutes. After constitution, 0.3 ml aliquots were transferred to syringes for injection. Samples preparation was scheduled so that the final stirring a dispensing steps were completed the morning that the formulation was used in biocompatibility studies.

### Reference EXAMPLE 11

### EFFICACY OF A PACLITAXEL-HYALURONIC ACID GEL IN RABBIT MODEL OF JOINT CONTRACTURE IN THE KNEE

The evaluation of paclitaxel in a hyaluronic acid gel is completed following the protocol of Trudel et al (J Rhemumatol 2000(27) 351-7; Arch Phys Med Rehabil 2000(81) 6-13; J Rheumatol 1998(25) 945-50; Arch Phys Med Rehabil 1999(80) 1542-7) as follows. Rabbits are randomized into four groups (Low Dose Treatment (n=40), High Dose Treatment (n=40), High Dose Treatment (n=40) and Control (n=20)). Within each group, half have their left knee immobilized using plate and screws, without entering the joint. The other half has their right knees immobilized in the same manner. Rabbits are anaesthetized with halothane and a 1 cm incision is made over the later aspect of the proximal femur and one over the distal tibia, to expose the bones. A Delrin plate (E.I. duPont de Nemours and Co, Wilmington DE) joins the two bones in a submuscular course such that 135° of flexion is maintained in the joint. After implantation, the skin is closed with staples. Immediately after closure of the site, each treated knee receives an intraarticular injection. Control animals receive 100 µl of a 10 mg/ml HA gel. Low, Medium and High dose treatment animals receive 100 µl of a 0.1, 0.5, or 1.5 mg/ml paclitaxel in 10 mg/ml HA gel, respectively. After 2, 4, 8, 16, or 32 weeks eight of the animals from each group are anaesthetized again, maintained at 22°C and the effect of immobilization on joint contracture are evaluated. Range of motion and the extent of flexion and contraction are measured with a goniometer and standardized torque applied to the joint. Torques of 667, 1060 and 1649 g are used. Treatment group animals are compared with Control group analysis using ANOVA and trend analyses in order to discriminate a therapeutic effect in increase range of motion, as well as a dose response. Additional doses and formulations (e.g., those in Examples 2 through 10, 15, 17, 22, and 23) may be evaluated by this method.

### Reference EXAMPLE 12

### CLINICAL STUDY TO ASSESS SAFETY AND TOLERABILITY OF PACLITAXEL FORMULATION FOR THE TREATMENT OF JOINT CONTRACTURE

Study Design: Male patients with a diagnosis of radial head fracture having a Mason score of 1 or 2 are eligible for participation in the study. Seventy-five patients are randomized into the following groups:

| Treatment | Paclitaxel Dose | Hyaluronic Acid Dose |
|---|---|---|
| Placebo | 0 | 0.2 mg in 2 ml |
| Low Dose x3 | 25% MTD | 20 mg in 2 ml |
| High Dose x3 | 75% MTD | 20 mg in 2 ml |
| Low Dose x5 | 25% MTD | 20 mg in 2 ml |
| High Dose x5 | 75% MTD | 20 mg in 2 ml |

The MTD (maximum tolerated dose) of paclitaxel given by intraarticular injection is to be determined in a dose escalation phase 1 clinical study involving 20 patients divided into four groups of 5 each receiving hyaluronic acid 20 mg in 2 ml containing paclitaxel in amounts of 0, 1, 5 and 10 mg). In the phase 1 trial, a MTD will be determined as the maximum dose in which the evaluation criteria are met, having minimally acceptable levels of:
(i) pain/discomfort at and after injection
(ii) increased swelling in the joint
(iii) decreased range of motion in the joint
(iv) neutropenia
(v) alopecia
(vi) nausea
(vii) hypersensitivity reaction
(viii) inflammation at the site of injection

After determining the MTD by these means, the clinical test to determine effectiveness of a safe dose may be initiated as follows. After receiving weekly injections according to the table in this example, the patients will be followed by visits at 6, 12 and 24 weeks after treatment. At treatment and at each follow-up visit, blood will be collected for CBC analysis, liver function tests (AST and bilirubin levels).

Enrollment: Patients enrolled in this study must be males between the age of 16-65 and be old enough to provide informed consent. Patients must be diagnosed with a Type 1 or 2 radial head fracture. The diagnosis is to be made using clinical and radiographic indices. Patients are eligible for this study if they have no major concurrent illness or laboratory abnormalities and their CBC; Neutrophils >2,500/mm³; Platelet count ≥125,000/mm³; hemoglobin ≥10 mg/dL; creatinine ≤1.4; <2x elevated liver function tests; normal clotting time.

If the patient has had prior/current treatment with TAXOL, the patient must not be treated with a paclitaxel/hyaluronic acid preparation. Patients must not have a history of joint contracture and be free of other joint disorders or systemic diseases such as rheumatoid arthritis. Prior malignancy, major organ allograft, or uncontrolled cardiac, hepatic, pulmonary, renal or central nervous system disease, known clotting deficiency or any illness that increases undue risk to patient will exclude them from this study.

Evaluation and Testing: At the time of treatment and at follow-up visits the patient will undergo blood collection as described above. Patients will also receive an X-ray at full supination and extension. X-ray data will be reviewed and scored by a blinded radiologist. Using a goniometer, the patient's range of motion will be measured in the affected and contra lateral elbows. The angles of full flexion and contraction will be measured and the range of motion therebetween calculated. The primary clinical endpoint will be a statistically significant reduction in the loss of range of motion after 24 weeks.

### Reference EXAMPLE 13

### MAXIMUM TOLERATED DOSE (MTD) DETERMINATION OF PACLITAXEL ADMINISTERED BY INTRA-ARTICULAR INJECTION IN A HYALURONIC ACID GEL

Surgical Procedures: Male Hartley guinea pigs, at least 6 weeks old, were anaesthetized using 5% isoflurane in an enclosed chamber. The animals were weighed and then transferred to the surgical table where anesthesia was maintained by nose cone with 2% isoflurane. The knee area on both legs was shaved and knee width at the head of the femur was measured on both knees. The skin on the right knee was sterilized. A 25G needle was introduced into the synovial cavity using a medial approach and 0.1ml of the test formulation was injected. Seven days after the injection, the animals were sacrificed by cardiac injection of 0.7ml Euthanyl under deep anesthesia (5% isoflurane). Sample size was N=3 for each formulation.

Assessment of tolerability: Knee function was assessed before sacrifice by recording changes in walking behavior and signs of tenderness. The animal was weighed immediately after sacrifice. The width of both knees at the head of the femur was then measured with calipers. The knee joint was dissected open by transecting the quadriceps tendon, cutting through the lateral and medial articular capsule and flipping the patella over the tibia. Knee inflammation was assessed by recording signs of swelling, vascularization, fluid accumulation and change in color in subcutaneous tissue as well as inner joint structures. All data was recorded by observers blinded to the treatment groups.

### Results:

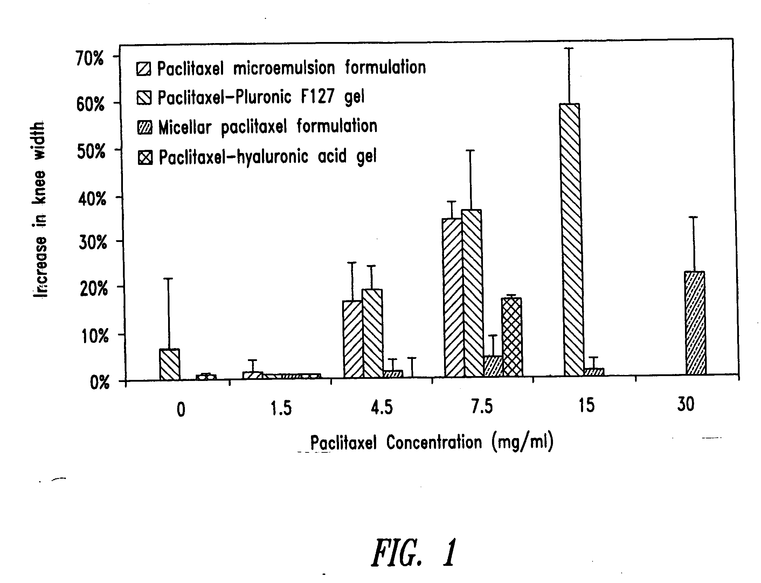

Swelling Measured by Knee Width: Knee width for the various groups is presented in FIG. 1. Knee width reflects swelling of the underlying joint structures and thus is a marker of inflammation. A clear dose-response effect was observed for the PLURONIC F127 (Example 10) and microemulsion (Example 4) formulations with doses as low as 4.5 mg/ml inducing swelling and higher doses causing more severe swelling. Paclitaxel doses of 7.5 mg/ml were inflammatory for the paclitaxel-hyaluronic acid gel formulation with lower doses (4.5 mg/ml and 1.5 mg/ml) showing no significant swelling (p > 0.05, ANOVA).

Body Weights of Guinea Pigs: All animals had normal walking behavior at the time of sacrifice and no sign of knee tenderness was observed. On average, all groups of animals gained or had stable weight.

Observations in Joint Tissues: The 7.5 mg/ml paclitaxel-hyaluronic acid gel group (formulation from Example 5) showed mild inflammation of the treated knee joint characterized by a slightly swollen knees and darken inner knee infrapatellar fat pad and knee capsule. The animals treated with 4.5 mg/ml paclitaxel HA gel had normal knees,

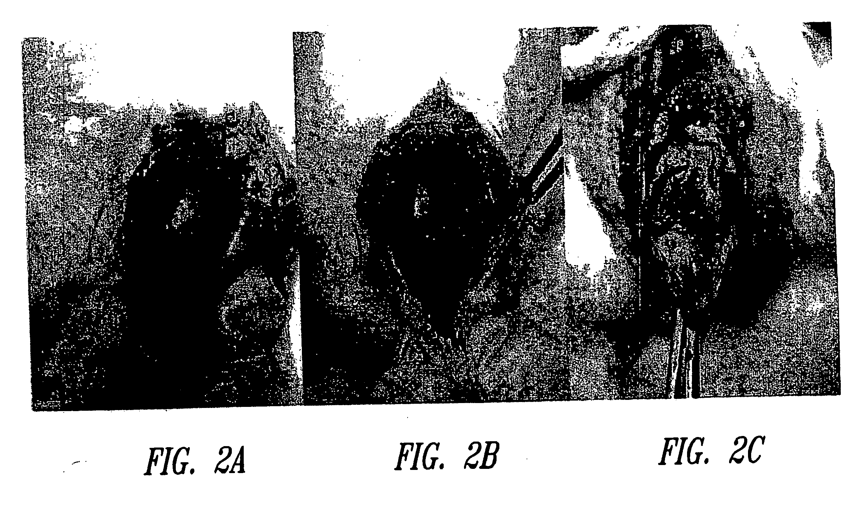

The 15 mg/ml paclitaxel in PLURONIC F127 group (formulation from Example 10) exhibited inflamed knees characterized by subcutaneous tissue swelling and fluid accumulation with highly vascularized knee capsule and swollen infrapatellar fat pad (FIG. 2). The groups treated with 7.5mg/ml and 4.5 mg/ml paclitaxel in PLURONIC F127 showed similar but less severe findings as the 15 mg/ml group. The animals treated with 1.5 mg/ml paclitaxel in PLURONIC F127 and with control PLURONIC F127 devoid of paclitaxel had normal knees.

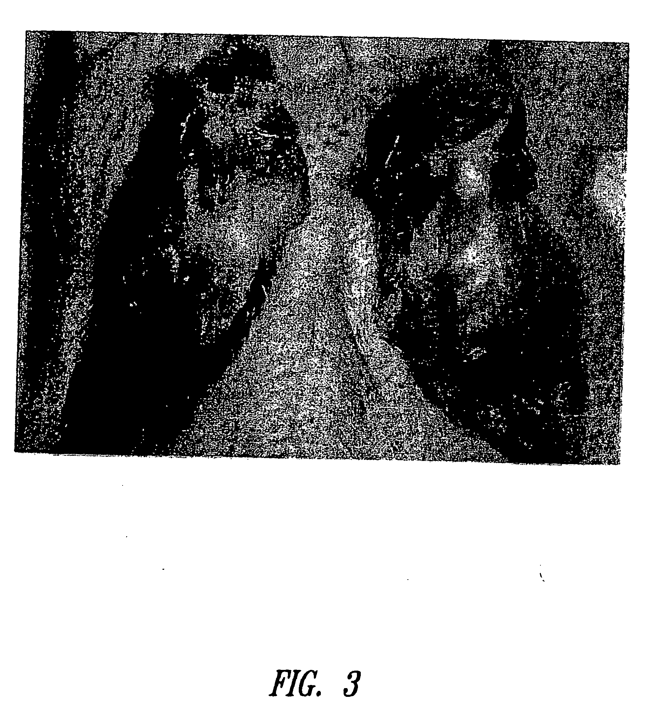

Knees treated with 30 mg/ml paclitaxel in micelles paclitaxel/hyaluronic acid gel (formulation from Example 2) exhibited mild to severe inflammation of the fibrous capsule and subcutaneous tissue with only slight inflammation of the inner joint. Knees treated with 15 mg/ml, 7.5 mg/ml (the MTD), 4.5 mg/ml and 1.5 mg/ml paclitaxel in micelles were all normal (FIG. 3).

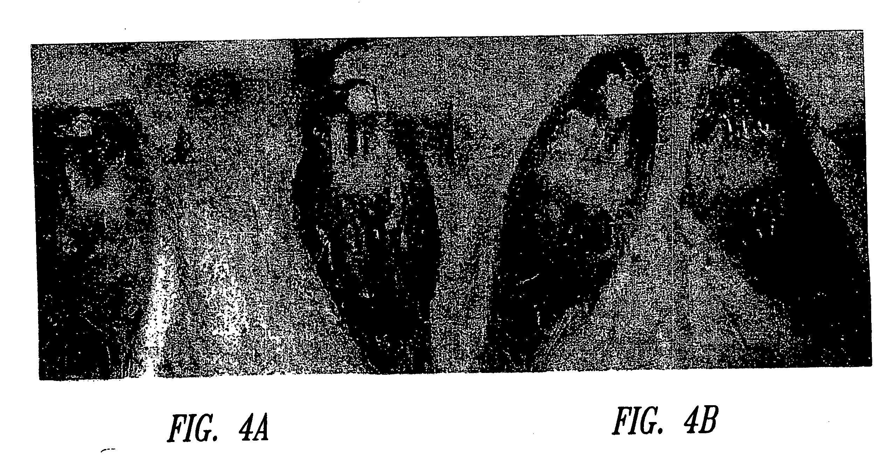

Knees treated with 7.5 mg/ml paclitaxel microemulsion gel (formulation from Example 4) exhibited severe inflammation of the fibrous capsule (swelling, vascularization) and infrapatellar fat pad. Knees treated with 4.5 mg/ml paclitaxel microemulsion gel showed less severe but noticeable signs of inflammation of the fibrous capsule and infrapatellar fat pad. Knees treated with 1.5 mg/ml paclitaxel microemulsion gel showed very mild signs of inflammation characterized by yellowish subcutaneous tissue and infrapatellar fat pad (FIG. 4A). The cause of the inflammation is not fully characterized for this formulation since no control group (without paclitaxel) was evaluated. Referring to FIG. 4B, a guinea pig knee joint at sacrifice 7 days is shown after intraarticular administration of 40:40:20 PEG200: water: TRANSCUTOL (ethoxydiglycol). The treated (right) joint has yellow discoloration of the infrapatellar fat pad.

Conclusions: This study demonstrates that the MTD for paclitaxel in the synovial cavity of guinea pig knees depends on the formulation used. Paclitaxel MTD determined 7 days after a 0.1 ml injection was 1.5 mg/ml with the PLURONIC F127 and microemulsion formulations, 4.5 mg/ml with the co-solvent formulations and 15 mg/ml with the micellar paclitaxel formulation. The difference in MTD between the various formulations most likely reflects differences in paclitaxel bioavailability due to different drug release rate and/or different formulation clearance from the knee joint.

### Reference EXAMPLE 14

### PREPARATION OF A PACLITAXEL IN CO-SOLVENT WITHOUT HYALURONIC ACID FORMULATION

In a method similar to Example 5, paclitaxel was prepared in a 60:40 PEG 300-water co-solvent, but hyaluronic acid was not included in the formulation. Paclitaxel was dissolved in PEG 300 at 7.5 mg/ml. The solution was stirred to dissolve the drug then diluted with water to a PEG:water ratio of 60:40. If necessary, the solution was pH adjusted with 0.1 M NaOH or glacial acetic acid, to a pH range of 6-8. The final paclitaxel concentration was 4.5 mg/ml Lower concentrations of paclitaxel were also used, by simply dissolving less drug in the PEG 300 at the start. Final concentration of paclitaxel in the formulation between 0.15 and 4.5 mg/ml were achieved in this manner.

Additional formulations were prepared by this means except that they were not diluted with water. Final compositions were between 0.15 and 4.5 mg/ml in PEG 300. The formulation may also be prepared with other drugs, for example 5-FU. For more hydrophilic drugs such as 5-FU, less PEG may be used, and more water substituted.

### Reference EXAMPLE 15

### PREPARATION OF 5-FLUOROURACIL (5-FU)-HYALURONIC ACID FORMULATION.

A hyaluronic acid formulation that includes 5-FU can be prepared as described. 5-FU is combined with 10 mg hyaluronic acid (1 MDa), 1 ml sterile water. The product is stirred until a uniform gel solution, free of particular polymer or drug is achieved. Alternatively, the HA and water may be combined, stirred and autoclaved to homogenize the solution. After dissolving the polymer, the 5-FU may be added with stirring. NaCl is added (as required for isotonicity), and the pH is adjusted to between 6-8 with NaOH and HCl as required. Formulations can be made with up to 12.9 mg/ml 5-FU, its measured water solubility. The formulation may be injected to the site of treatment (e.g., into a joint) in a volume appropriate to that site. For example, a knee joint might receive a 2 ml injection, whereas a finger joint or tendon sheath may receive substantially less.

### Reference EXAMPLE 16

### DISTRIBUTION OF PACLITAXEL TO JOINT TISSUES OVER A TWO WEEK PERIOD

Male rabbits were anaesthetized using 5% isoflurane in an enclosed chamber. The animals were weighed and then transferred to the surgical table where anesthesia was maintained by nose cone with 2% isoflurane. The knee area on both legs was shaved and knee width at the head of the femur was measured on both knees. The skin on the right knee was sterilized. A 25G needle was introduced into the synovial cavity using a medial approach and 0.5 mL of the test formulation was injected. At various time intervals after the injection, the animals were sacrificed by cardiac injection of 0.7 mL Euthanyl under deep anesthesia (5% isoflurane). Sample size was N=3 for each formulation. The knee joint was dissected open and the synovial membrane, the anterior cruciate ligament, the fat pad, the menisci and the cartilage were harvested. Each tissue was briefly rinsed in saline solution, blotted dry and stored individually in a scintillation vial at -20°C until paclitaxel analysis. Tissue samples were weighed and ground using a Certiprep Spex Cryomill cooled with liquid nitrogen. Milling was accomplished using three two minute agitation cycles, with 2 minute pauses between each. Paclitaxel was extracted from the frozen ground tissues with 12 ml of a 50/50 or 90/10 acidified acetonitrile/water mixture, with mixing for 30 minutes using a Labquake tube rotator. The extract was syringe filtered into an HPLC vial and analyzed by LC/MS/MS. The samples were spiked with lithium chloride to improve detection. The LC column was an ACE 3 C18 with an Upchurch guard column. The mobile phase was 1:1 acetonitrile:water with lithium chloride and acidified with acetic acid. The flow rate was 0.3 ml/min and the injection volume was 10 µl. The molecular ion was quantified. The data were used to calculate the concentration of paclitaxel in tissue, expressed in terms of µg paclitaxel per g tissue.

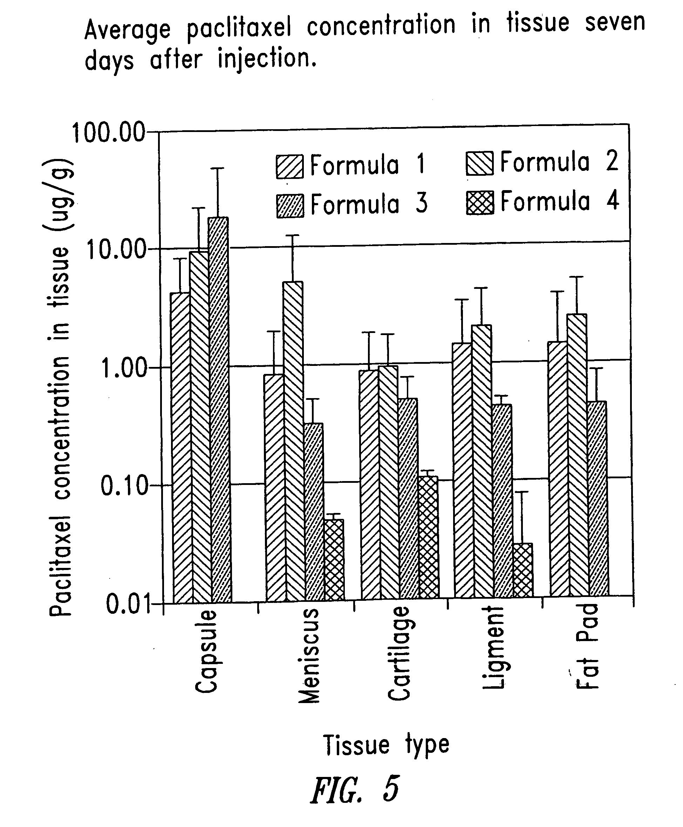

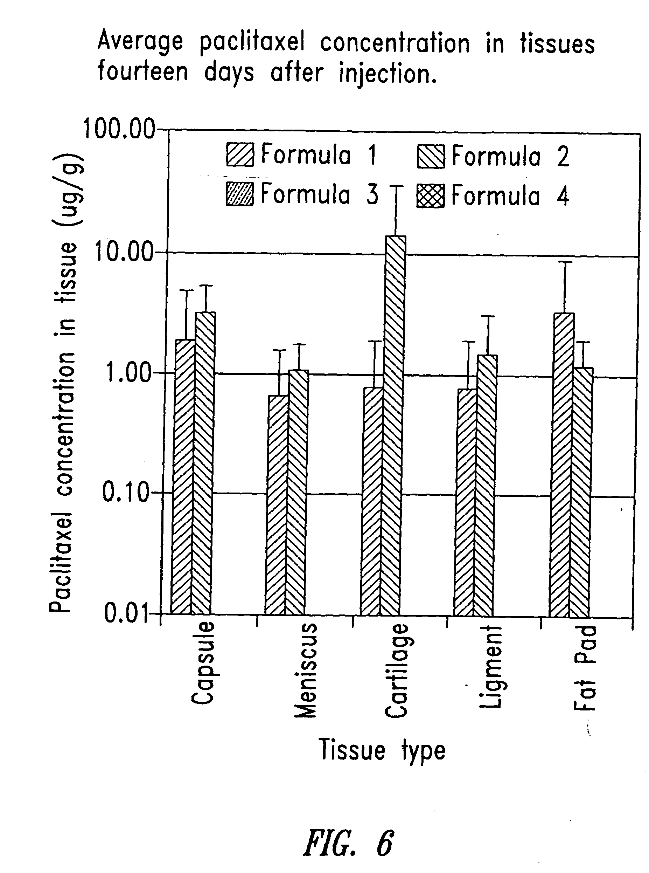

Results: Of four formulae evaluated, two demonstrated paclitaxel retention in various joint tissues for over fourteen days, while two demonstrated paclitaxel retention for at least seven days, but no quantifiable paclitaxel after fourteen days (less than 0.01 µg/g). These data are summarized in the FIG. 5 and FIG. 6 (Formula 1: 70% PEG 300, 30% water, 4.5 mg/ml paclitaxel made according to Example 5). Formula 2: co-solvent formulation with 10 mg/ml hyaluronic acid and 4.5 mg/ml paclitaxel, made according to Example 14). Formula 3: 4.5 mg/ml paclitaxel in PEG 300. Formula 4: 2.25 mg/ml paclitaxel in PEG 300, similar to those in Example 17).

### Reference EXAMPLE 17

### FORMULATIONS PROVIDE SUSTAINED PACLITAXEL CONCENTRATIONS IN TISSUES BY A DRUG DEPOT MECHANISM

The deposition of paclitaxel in the joint space after intra-articular injection was characterized by in vitro solubility studies and confirmed by visualization in rabbit joints after intra-articular injection of paclitaxel in PEG 300.

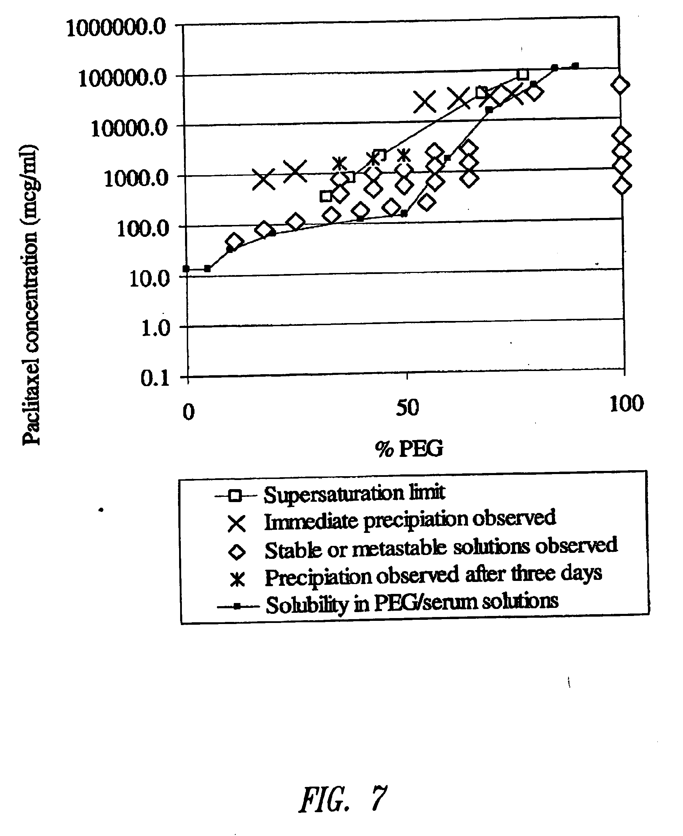

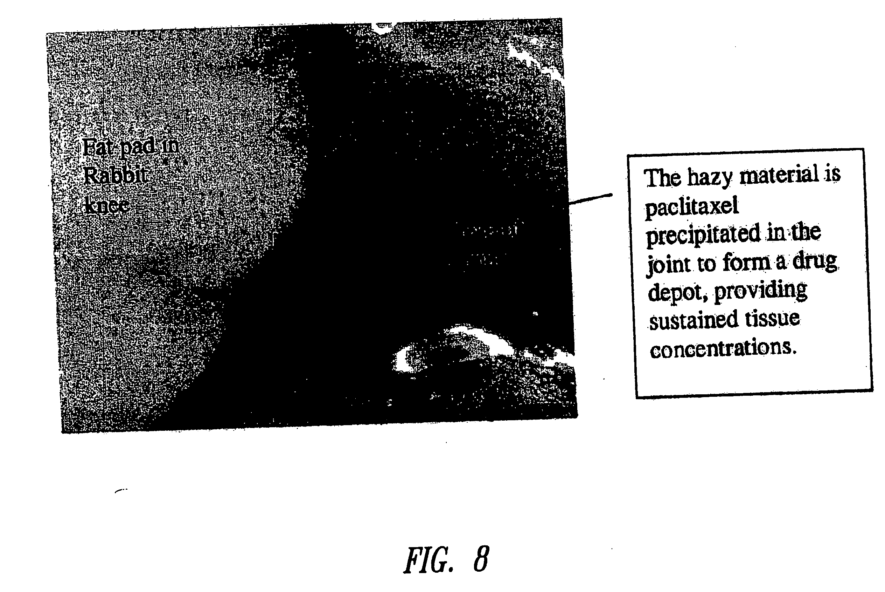

The in vitro characterization involved diluting paclitaxel solution in PEG 300 with various volumes of human serum and observing for precipitation of paclitaxel. Dilution of 45 mg/ml paclitaxel in PEG resulted in drug precipitation when the mixture was 75%v/v PEG and 25%v/v serum. When 1/10^{th} of the drug concentration (4.5 mg/ml) was tested, immediate precipitation was not observed until dilution to 25%v/v PEG. Precipitation was observed after three days in samples diluted to 50%vlv PEG with serum. At lower paclitaxel concentrations, precipitation was not observed at any level of dilution evaluated. These data are summarized in FIG. 7 below. Thus, by varying the starting paclitaxel concentration in a formulation, the degree to which the paclitaxel will precipitate upon dilution with a physiological aqueous medium can be controlled. The precipitated drug will form a depot *in vivo*, providing sustained drug levels in tissue. This was confirmed by kinetic studies (reference the new kinetics example) and by visual observation of joints injected with 4.5 mg/ml paclitaxel in PEG 300, which showed the presence of solid paclitaxel crystals in the joint space, which formed as a result of dilution of the formulation *in vivo*. (FIG. 8).

### EXAMPLE 18

### SYNTHESIS OF BLOCK COPOLYMERS.

Numerous block co-polymers were synthesized using a method similar to Polymer Synthesis in Example 1.

PEG and monomer(s) were weighed into 20x150 mm glass test tubes on a top-loading balance and sealed with screw caps. The weights used were weight ratios of their molecular weights. For example, 3.08 g of PEG 400 and 6.92 g of D,L-lactide were used to make 10 g of PEG 400-poly D,L-lactic acid (900). About 400 ml of heavy mineral oil was added into a 2 L beaker and placed on top of a hot plate. The hot plate was connected to a temperature probe which was set at 302°F (150°C), with the hot plate set to heat at setting 4 and stir at setting 3. The test tubes were put into the oil bath carefully once the temperature had equilibrated. The test tubes were vortexed after a homogeneous solution was formed and 5 µl/g polymer of stannous 2-ethylhexanoate was added to each tube as a catalyst. The tubes were vortexed and put into the oil bath for 5 hours, during which the tubes were vortexed briefly at 0.5 hours and 1.5 hours. The polymers were poured into glass dishes and were allowed to cool overnight in a fume hood.

Polyester residues of DL-lactide, glycolide, and ε-caprolactone as well as trimethylene carbonate were reacted to form copolymers with various PEG and methoxy-PEG blocks. This process was used to produce many block copolymers. In some batches the tin catalyst content was varied between 0.05 and 0.5% catalyst, most often 0.5% was used and 0.1 % was used commonly for diblock copolymer comprising MePEG. In some batches, the scale of synthesis was altered. Accordingly, reaction vessels of different sizes were used, however the same process was followed. By this means various copolymers were synthesized, as shown in Table 1, where component A was polymerized independently with each of components B, C, D, E, F, or G.

**TABLE 1**

| IDENTITY AND MOLECULAR WEIGHT OF POLYESTERS AND POLYCARBONATES IN SYNTHESIZED COPOLYMERS | | | | | | |
|---|---|---|---|---|---|---|
| A | B | C | D | E | F | G |
| PEG/ MePEG MW (g/mol) | PDLLA MW (g/mol) | PGA MW (g/mol) | PCL MW (g/mol) | PLLA MW (g/mol) | TMC MW (g/mol) | 90% TMC/10% GA MW (g/mol) |
| Triblock copolymers | | | | | | |
| PEG 200 | 200, 400, 600, 900, 2000, 5000, 10000, 15000, 17500, 20000, 22500, 25000, 30,000 | 200, 2000, 20000 | 200, 2000, . 20000 | | | |
| PEG 300 | 300, 600, 900 | | | | 300, 600, 900 | 300, 600, 900 |
| PEG 400 | 200, 400, 600, 900, 1600, 2000 | | | | 300, 600, 900 | 300, 600, 900 |
| PEG 600 | 600, 8000 | | 600, 8000 | | | |
| PEG 900 | 400, 600, 900, 2000 | | | | | |
| PEG 2000 | 200, 2000, 20000 | 200, 2000, 20000 | 200, 2000, 20000 | | | |
| PEG 5000 | 4000, 6000, 9000 | | | | | |
| PEG 8000 | 600, 8000 | | 600, 8000 | | | |
| PEG 20000 | 200, 2000, 4000, 6000, 9000, 20000 | 200, 2000, 20000 | 200, 2000, 20000 | | | |
| PPG 425 | 300, 400, 600, 900 | | | | | 300, 400, 600, 900 |
| PG | 300, 400, 600, 900 | | | | | 300, 400, 600,900 |

| Diblock Copolymers | | | | | | |
|---|---|---|---|---|---|---|
| MePEG 350 | 200, 2000, 20000 | 200 | 200, 2000, 20000 | | | |
| MePEG 750 | 200, 2000, 3000, 20000 | 200, 2000, 20000 | 200, 500, 2000, 20000 | | | |
| MePEG 2000 | 200, 857, 1333, 1636, 2000, 2444, 4000, 6000, 9000, 20000 | 200, 1333, 2000, 20000 | 200, 500, 1333, 2000, 3000, 8000, 20000 | 4667, 8000, 18000, 38000 | | |
| MePEG 5000 | 200, 2000, 2700, 3333, 4000, 6000, 7500, 9000, 20000 | 200, 2000, 20000 | 200, 2000, 20000 | 20000, 45000, 95000 | | |
| Other PEG Triblocks with mixed polyester chains: | | | | | | |
| PEG 400- Poly(D,L Lactic Acid-co-ε-Caprolactone) (900) (80%LA, 20%CL) | | | | | | |
| PEG 400- PLGA 70 (65% LA, 35% GA) | | | | | | |
| PEG 400- PLGA 170 (65% LA, 35% GA) | | | | | | |
| PEG 400- PLGA 200 (65% LA, 35% GA) | | | | | | |
| PEG 400- PLGA 400 (65% LA, 35% GA) | | | | | | |
| PEG 400- PLGA 600 (65% LA, 35% GA) | | | | | | |
| PEG 400- PLGA 900 (65% LA, 35% GA) | | | | | | |
| PEG 400- PLGA 1600 (65% LA, 35% GA) | | | | | | |
| PEG 400- PLGA 2000 (65% LA, 35% GA) | | | | | | |
| MePEG 2000-Poly valerolactone 1333; MePEG 750-Poly valerolactone 500 | | | | | | |
| MePEG 2000-Poly decanolactone 1333 | | | | | | |

| | | | | | | |
|---|---|---|---|---|---|---|
| Abbreviations in the table: PEG = polyethylene glycol; MePEG= methoxy polyethylene glycol; PDLLA = Poly D,L-lactic Acid; PLLA = poly L-lactic acid; PGA= poly glycolic acid; PCL= poly-c-caprolactone; PLGA = poly(D,L-lactic-co-glycolic acid); PPG = polypropylene glycol; PG = propylene glycol; TMC = trimethylene carbonate; GA = glycolide; LA = D,L-lactide. | | | | | | |

### EXAMPLE 19

### DETERMINATION OF THE WEIGHT PERCENT OF WATER SOLUBLE MATERIAL IN A POLYMER

Empty 50 ml plastic centrifuge tubes was tared and 1 g of polymer was weighed accurately into each tube. 10 ml of deionized water was added to each. The tubes were vortexed, transferred to a 37°C oven overnight and centrifuged at 2500 rpm for 10 minutes the next morning. The supernatant was removed and discarded to eliminate the water soluble component from the polymer. Another 10 ml of water was added and the above process was repeated. The sample was then frozen in the -20°C freezer and freeze-dried to completely remove the water. The tube was weighed and the percent mass recovery of the sample and the percent water soluble were calculated.

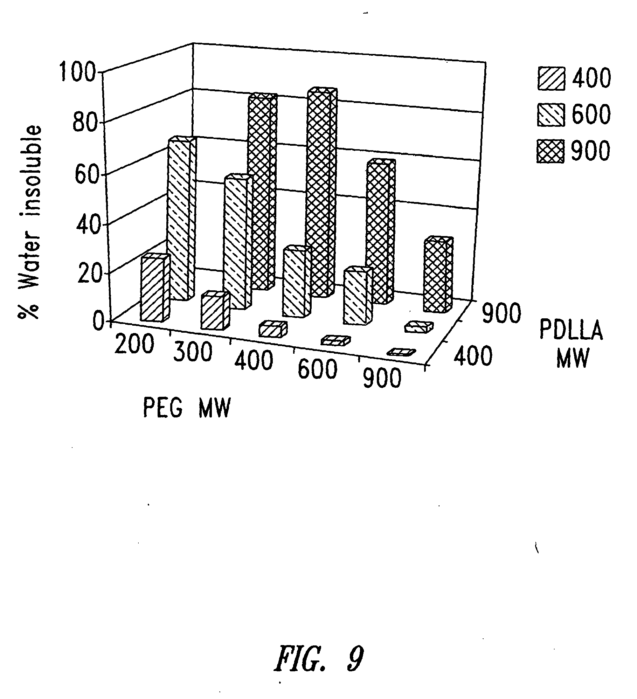

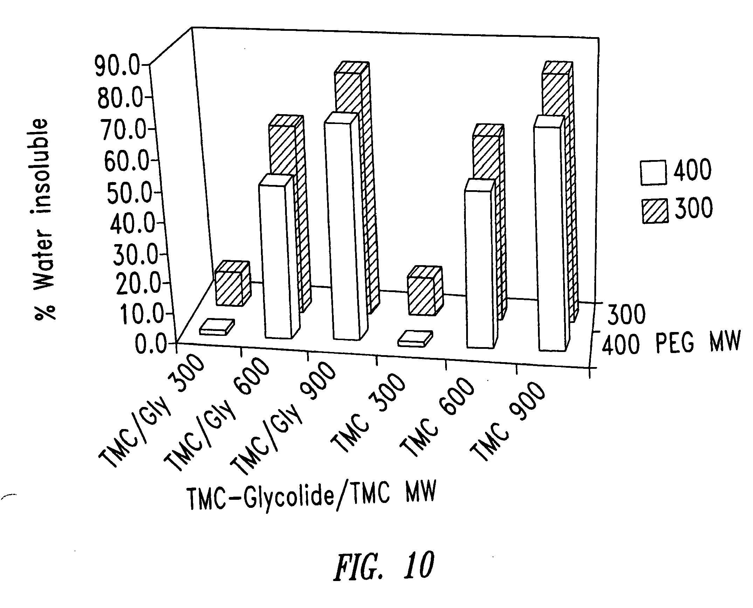

In one experiment, of four polymers tested, all were only partially soluble (25 to 40% dissolved) in water (Table 2). The increased proportion of water soluble component coincided with increasing maximum δh values measured in the solubility screening studies (FIGS. 9 and 10). However, the results were unexpected for PEG400-PLGA900 which was predicted to have a water soluble fraction greater than PEG400-PDLLA900, as the greater density of methyl groups on PDLLA give the polymer more hydrophobic properties than PLGA. The repeatability of this technique was evaluated by testing duplicate samples of PEG400-PDLLA900. The values were nearly identical (Table 2).

GPC of the polymers were obtained before and after the gravimetric study. As seen in Table 2, the number average molecular weight (Mn) increased over 10% (absolute increase of 150-222 g/mol) in all four polymers tested, indicating that the water soluble fractions were the shorter polymer chains in the material. This was expected since shorter chains had proportionally more PEG in the polymer structure, and are thus more hydrophilic.

**TABLE 2**

| WEIGHT RECOVERY OF POLYMERS IN WATER | | | | | |
|---|---|---|---|---|---|
| Polymer | % Water Soluble | Mn (before) | Mn (after) | % Increase | Absolute Mn Change |
| PEG400-PLACL (900) (20%CL, 80%LA) | 27.81 | 1172 | 1322 | 12.8 | 150 |
| PEG400-(90%TMC, 10%GA)900 | 24.87 | 1666 | 1837 | 10.3 | 171 |
| | 24.48 | | | | |
| PEG400-PDLLA (900) | 39.73 | 1069 | 1232 | 15.2 | 163 |
| PEG400-PLGA (900) (65%LA, 35%GA) | 37.29 | 1143 | 1365 | 19.4 | 222 |

A broader range of PEG-PDLLA triblocks were evaluated for percent water soluble fraction in this manner. As the molecular weight of the PEG block in the triblock copolymer increased, the weight percent of polymer recovered after incubation decreased, thus the water soluble fraction increased (FIG. 9). Conversely, as the PDLLA proportion of the triblock copolymer increased, the amount of polymer recovered also increased. PEG 400-PDLLA 900 had greater than 85% water insoluble material in the matrix, while PEG 900-PDLLA 400 was completely water soluble. Thus by altering the polymer constituents over a relatively narrow range, a wide range of water solubility properties may be achieved. The relationship of a polymer's structure to its mass percent water insoluble fraction when evaluated graphically, as in FIG. 9 indicates a regular trend which allows prediction of percent water solubility for polymers not tested, but with intermediate polymer molecular weights. Polymers made with 90%mol/mol / 10%mol/mol glycolide and 100% TMC [TMC/Gly(90/10)] ranged from nearly completely water soluble (hydrophobic block = 300 g/mol) to nearly completely insoluble (hydrophobic block = 900 g/mol) (FIG. 10).

### EXAMPLE 20

### CHARACTERIZATION OF THE "MAX δH" PARAMETER FOR A POLYMER

The Hansen solubility parameters system was developed by Charles M. Hansen in 1966 for the study of polymer solubility. According to this system, solvents are characterized by three parameters, consisting of a hydrogen bonding component, δh, a polarity component, δp, and a dispersion force component, δd, and all three parameters were related to the total Hildebrand parameter, δt, according to the equation: δt² = δh² + δp² + δd². This system is described in several texts, for example, Hansen Solubility Parameters: A User's Handbook, Charles M Hansen, CRC Press, 2000. For this characterization solubility parameters were calculated or obtained from data in this text as well as in Handbook of Solubility Parameters and Other Cohesion Parameters, 2nd edition. Allan FM Barton, CRC Press, 1991.

Around 20 mg of polymer was accurately weighed into 20 ml scintillation vials and various solvents or co-solvent mixtures were added in a ratio of 10 mg polymer/ml solvent. The vials were put into a forced air oven at 50°C overnight, and were allowed to cool to ambient temperature the next morning before making observations. The polymer was considered soluble if there were no visible solids and the solution was clear and transparent. It was very important to check the bottom of the vials as sometimes tiny solid particles were stuck at the bottom of the vial despite having a transparent appearance when viewed from the side. It was also important to note that on some occasions the solids took as long as a few days to come out of solution, especially in xylene and ethoxydiglycol. Polymer solubility was also tested in various solvent blends to assess a wide range of solubility characteristics. The maximum δh value was the highest hydrogen bonding solubility parameter (δh) for any solvent or co-solvent system in which the polymer was soluble at 10 mg/ml. The highest value possible by this method is 42, the δh of water (see, Table 3).

**TABLE 3.**

| MAXIMUM ΔH VALUES OF ALL PEG-PDLLA TESTED FOR SOLUBILITY | | | | | | | | |
|---|---|---|---|---|---|---|---|---|
| | PEG MW | | | | | | | |
| PDLLA MW | | 200 | 400 | 600 | 900 | 2000 | 5000 | 20000 |
| | 100 | * | 42 | * | * | * | * | * |
| | 200 | 42 | 42 | * | * | 42 | - | 42 |
| | 400 | 32.3 | 42 | * | 42 | * | * | * |
| | 600 | 22.9 | 33 | 36 | * | * | * | * |
| | 900 | 22.9 | 29 | * | 33 | * | * | * |
| | 1600 | * | 15 | * | * | * | * | * |
| | 2000 | 22 | * | * | 23 | 42 | - | 42 |
| | 4000 | * | * | * | * | 15 | 22.3 | 32 |
| | 6000 | * | * | * | * | 15 | 15.2 | 17.3 |
| | 9000 | * | * | * | * | 15.2 | 15.2 | 17.3 |
| | 20000 | 15 | * | * | * | 15 | * | 15 |

| | | | | | | | | |
|---|---|---|---|---|---|---|---|---|
| *These triblock copolymers were not synthesized | | | | | | | | |

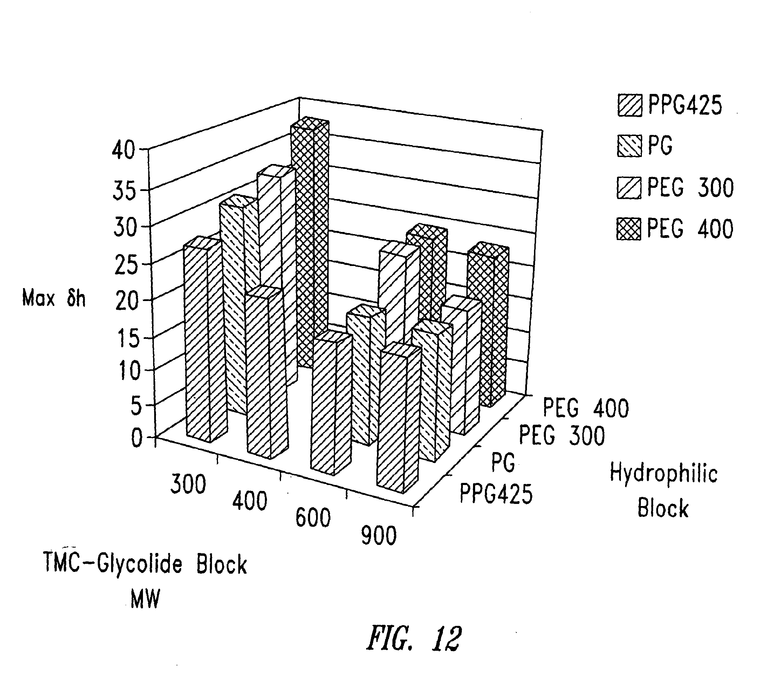

A similar solubility screen for triblock copolymers having polypropylene glycol (PPG) 425 and propylene glycol (PG) as the center hydrophilic block and various hydrophobic block structures: trimethylene carbonate (TMC), trimethylene carbonate-co-glycolide (90/10 mol ratio) (TMC/Gly) and PDLLA. For a given hydrophobic block structure and length PG and PPG 425 resulted in the same max δh for the polymers and PEGs 300 and 400 resulted in similar values as well, although for some polymers (e.g., PEG-TMC/Gly (90/10)), the PEG 400 based polymer had a slightly higher max δh (FIG. 12). Altering the hydrophobic block from 100% TMC to a 90/10 copolymer of TMC and glycolide did not alter the max δh values, yielding a data set similar to that shown in FIG. 12.

### EXAMPLE 21

### CHARACTERIZATION OF DRUG RELEASE FROM A TRIBLOCK COPOLYMER CONTAINING COMPOSITION.

### Preparation of Samples for Drug Release Study:

Around 20 mg of paclitaxel was accurately weighed and dissolved in THF to make a 1 mg/ml solution. Around 4 g of polymer was accurately weighed and 0.5 ml of the paclitaxel solution was added per gram of polymer (0.5 mg paclitaxel/gram polymer). The mixture was stirred at 450 rpm inside a 50°C forced air oven until a homogeneous solution was formed. It was then uncovered and stirred inside the oven for 1 hour. The mixture was transferred into a vacuum oven set at 50°C and vacuum was applied overnight to remove all the solvent from the polymer.

### Drug Release Assay for Paclitaxel loaded Triblock Copolymers:

Approximately 3.5 g of the 0.5 mg/g drug loaded polymer was weighed into a 16x100 mm culture tube (approximately 175 µg of total drug). 11 ml of phosphate buffered saline was dispensed into each tube through a pipette or dispenser and capped. The tubes were placed on a rotating wheel which was set at a 10° incline and rotated at 30 rpm. The apparatus was placed in a 37°C oven. The sampling time points were at 2, 4 and 7 hours on the first day, daily for the first week and every 48 hours in subsequent weeks. At each sampling time point, the sample was first centrifuged at 2600 rpm for 5 minutes. A 10 ml aliquot was then transferred by glass pipette to a clean 16x100 mm culture tube for solid phase extraction (Table 4). 10 ml of fresh phosphate buffered saline was added to the remaining 1 ml before replacing it on the rotating wheel in the incubation oven. After extraction, the elution solvent (ACN) was dried on a TurboVap with N₂ at 35°C and the solid was reconstituted in 85/15 ACN/water for HPLC analysis.

**TABLE 4**

| SPE METHOD | | | | | |
|---|---|---|---|---|---|
| Step | Action | Source | Output | Volume | (ml/min) |
| 1 | Condition | MeOH | Aq. Waste | 2 | 5 |
| 2 | Condition | H₂O | Aq. Waste | 1.5 | 5 |
| 3 | Condition | Buffer | Aq. Waste | 1 | 5 |
| 4 | Load | Sample | Aq. Waste | 2 | 3 |
| 5 | Load | Sample | Aq. Waste | 2 | 3 |
| 6 | Load | Sample | Aq. Waste | 2 | 3 |
| 7 | Load | Sample | Aq. Waste | 2 | 3 |
| 8 | Load | Sample | Aq. Waste | 2.2 | 3 |
| 9 | Purge-Cannula | ACN | Cannula | 3 | 15 |
| 10 | Rinse | Buffer | Aq. Waste | 3 | 5 |
| 11 | Rinse | H₂O | Aq. Waste | 3 | 5 |
| 12 | Rinse | Vent | Aq. Waste | 6 | 30 |
| 13 | Rinse | Vent | Aq. Waste | 6 | 30 |
| 14 | Collect | ACN | Frac. 1 | 2 | 3 |
| 15 | Purge-Cannula | DCM | Cannula | 6 | 15 |
| 16 | Rinse | DCM | Aq. Waste | 6 | 15 |
| 17 | Purge-Cannula | ACN | Cannula | 6 | 15 |
| 18 | Rinse | ACN | Aq. Waste | 6 | 15 |
| 19 | Purge-Cannula | H₂O | Cannula | 6 | 15 |
| 20 | Rinse | H₂O | Aq. Waste | 6 | 15 |

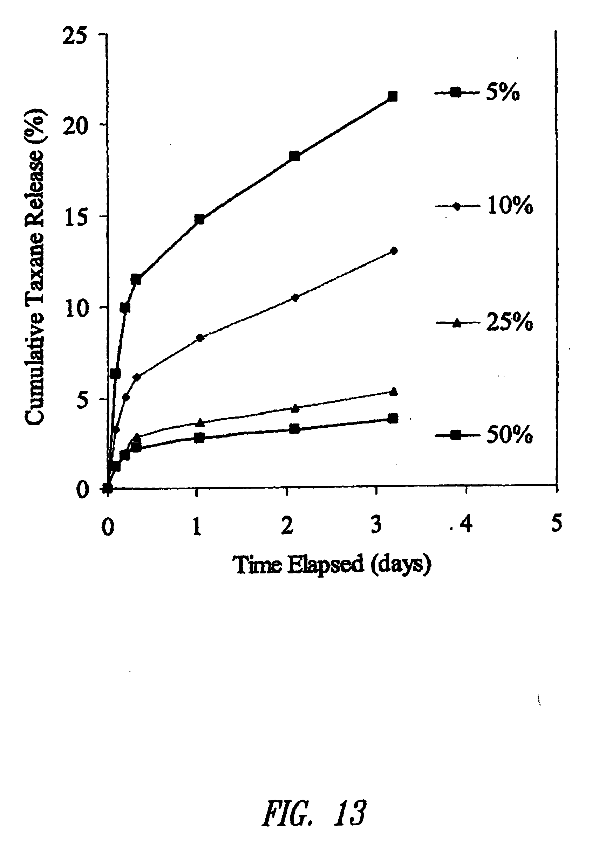

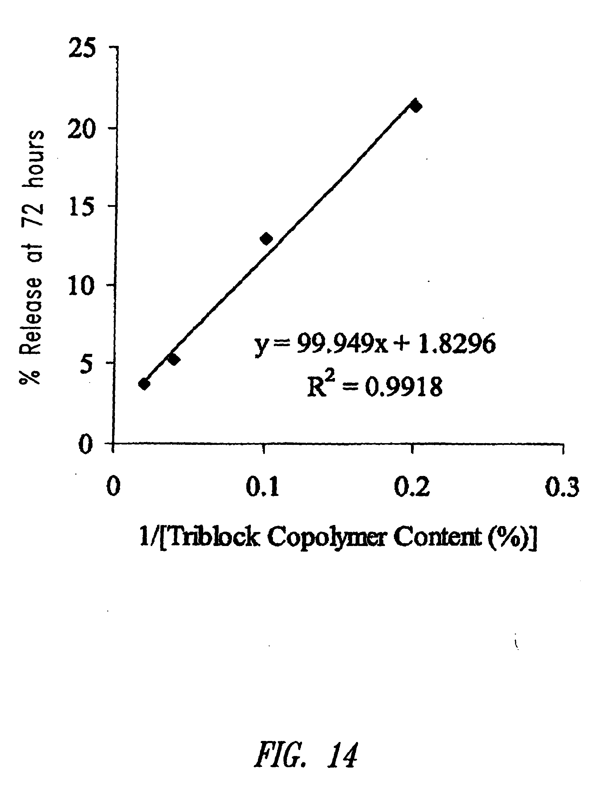

A triblock copolymer (PEG400/TMC-Gly(90/10)900) having a center hydrophilic block of PEG 400 and two hydrophobic blocks on each end having a combined molecular weight of 900 g/mol and a monomer structure of 90%mol/mol trimethylene carbonate and 10%mol/mol glycolide was dissolved in PEG 300 in various ratios and paclitaxel was added at 0.5 mg/g. Release study data demonstrate that the compositions provide for highly controlled drug release, having a limited burst phase followed by a linear phase of release. The data are shown in FIG. 13 and FIG. 14 demonstrates the high level of control over release rate by varying the proportion of this triblock copolymer in a paclitaxel formulation.

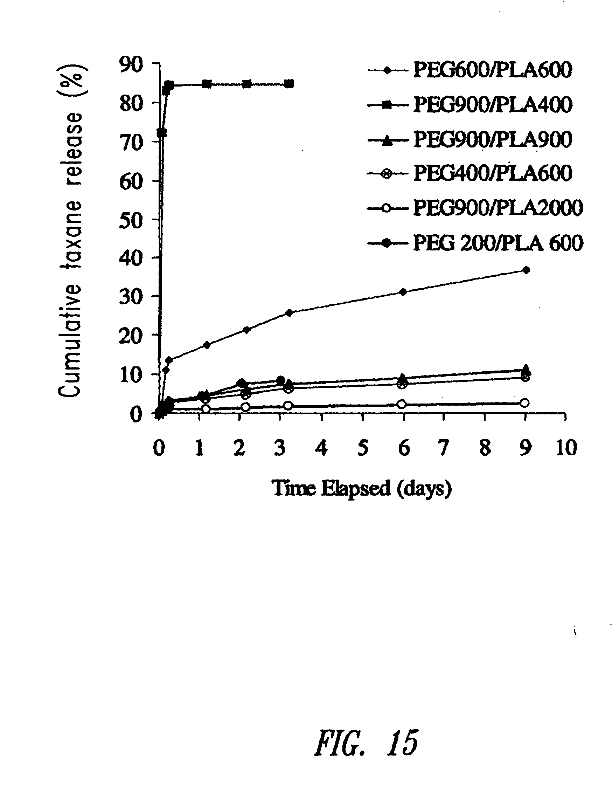

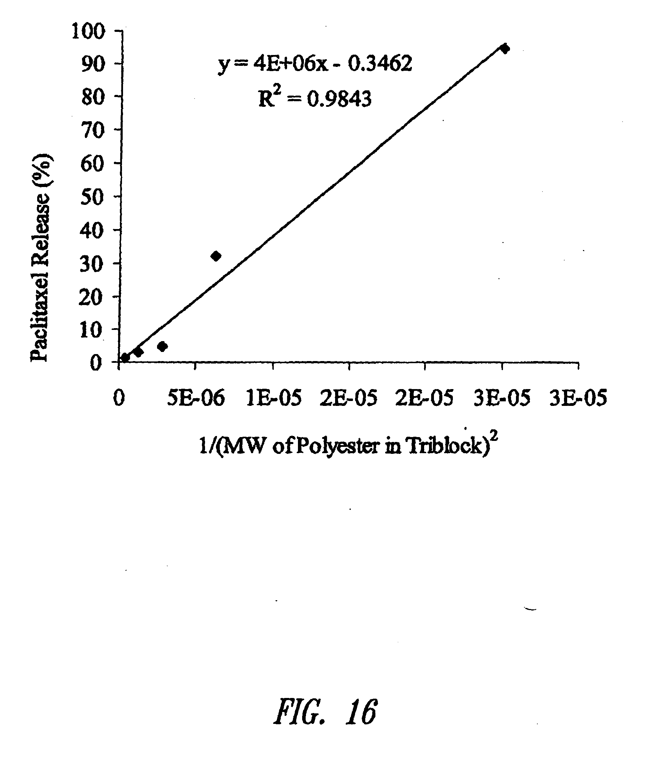

Paclitaxel release characteristics for triblocks having a range of PEG block molecular weights (200 to 900) and PDLLA block total molecular weights (400 to 2000) were evaluated (FIG. 15). In general, as the PDLLA block lengths increased or the PEG block length decreased, the extent of paclitaxel release decreased (FIG. 16). Release ranged from about 85% release in 7 hours from a water soluble copolymer (PEG900/PDLLA400) to only 2% over nine days (PEG900/PDLLA2000). An empirical relationship between extent of release and PDLLA block molecular weight was established. Release after three days was inversely proportional to the square of PDLLA block molecular weight (FIG. 16), indicating that paclitaxel release is very sensitive to the block length of PDLLA.

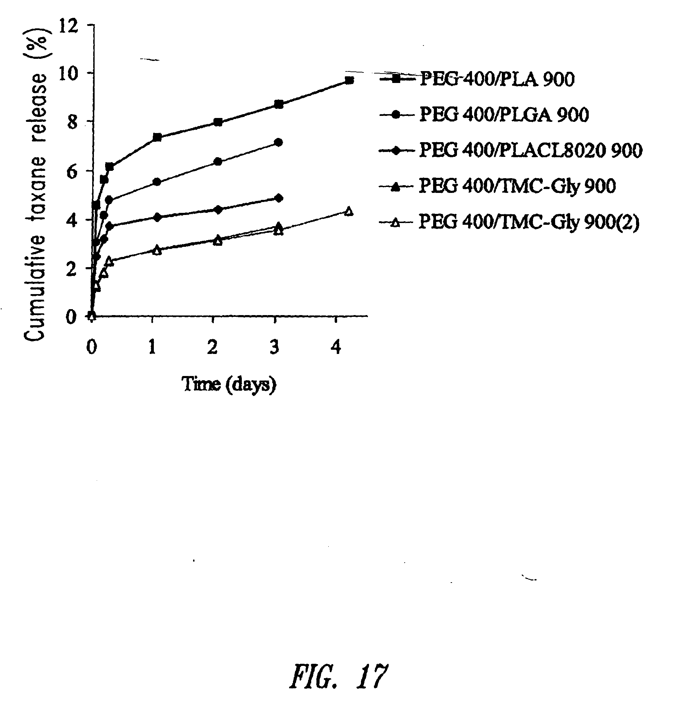

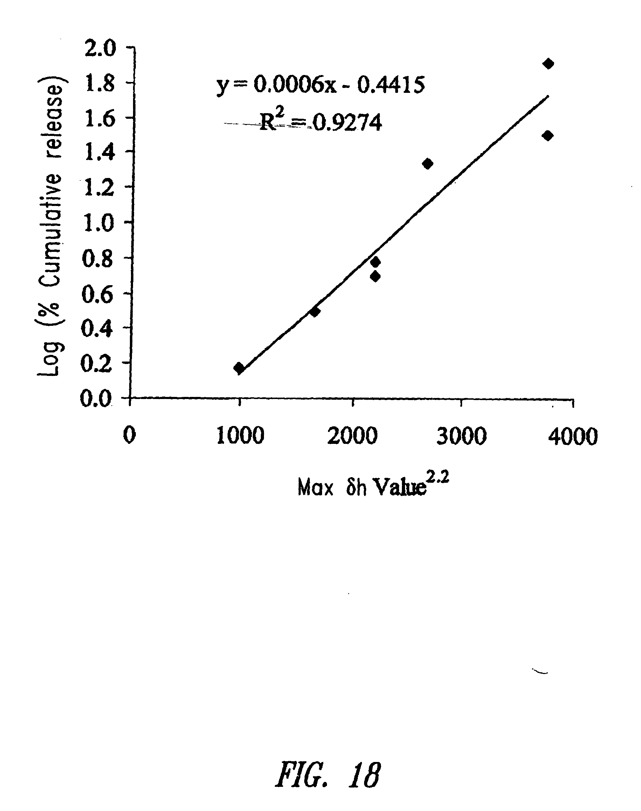

Structural analogues of PEG400/TMC-Gly(90/10)900 (e.g., triblock co-polymers composed of a PEG 400 block and two hydrophobic blocks having a combined molecular weight of 900 g/mol) were analyzed with respect to paclitaxel release characteristics. These data are summarized and compared with release from PEG400/TMC-Gly(90/10)900 in FIG. 17. The analogues were selected for release studies based on their varying solubility characteristics, expressed in maximum δh values determined in earlier solubility screens. Extent of drug release over three days varied with the chemical structure of the hydrophobic blocks in each analog and an empirical relationship (FIG. 18) relating the extent of release to solubility characteristics was established, also incorporating the data from FIG. 18. The linear regression equation (R² = 0.92) relates paclitaxel release to the polymer's maximum δh value (FIG. 18), thus in vitro release characteristics may be predicted for all analogues regardless of PEG block molecular weight, hydrophobic block monomer composition and hydrophobic block molecular weight. The relatively simple and rapid solubility screening test can thus be used to rank the performance of all of the polymers in this study and other analogues of this type.

The solubility characteristics of triblock copolymers having a hydrophilic central PEG block can be expressed as the maximum observed δh value at which the polymer was soluble. This parameter was correlated with other polymer characteristics including the percent of water soluble components in the polymer and with paclitaxel release rates from the polymer. An empirical relationship was found to relate polymer solubility characteristics to the extent of paclitaxel release observed over several days.

This release method is also suitable for the characterization of other formulations having a solid or semisolid component, for example those from Examples 6, 7, 8, 9, 10.

### EXAMPLE 22

### PHASE BEHAVIOR OF PEG400-TMC/GLY(90/10)900 / PEG 300 / WATER MIXTURES

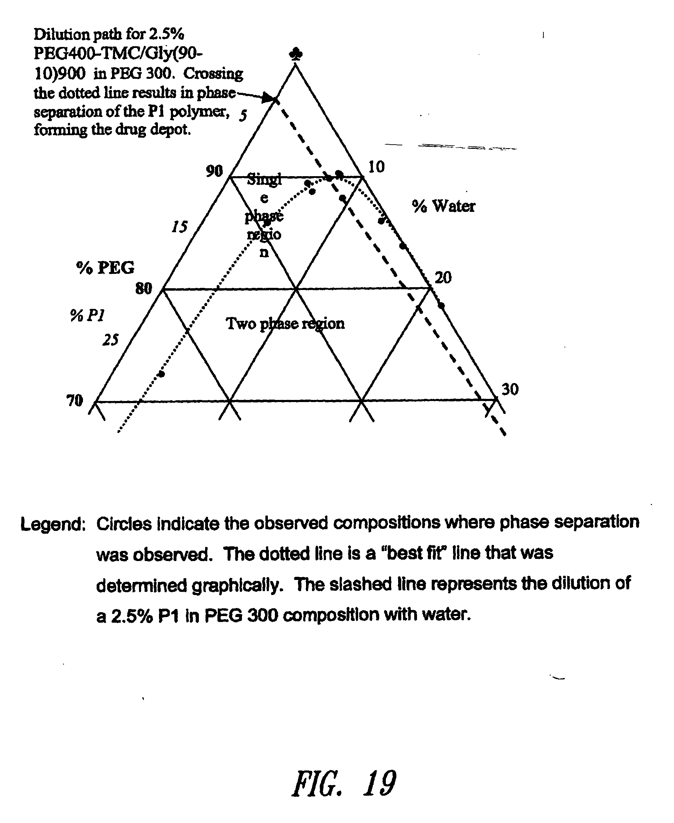

The phase separation of the PEG400-TMC/Gly(90/10)900 triblock copolymer from PEG 300 in the presence of water was evaluated to predict its behavior upon dilution in a largely aqueous physiological environment. The data, represented by a ternary phase diagram (FIG. 19), demonstrate that the mixture containing PEG 300 and the more hydrophobic PEG400-TMC/Gly(90/10)900 polymer phase separates upon addition of water. The amount of water added to effect phase separation represented less than 10% of the total mixture for most PEG400-TMC/Gly(90/10)900 /PEG 300 mixtures and decreased as the PEG400-TMC/Gly(90/10)900 content increased. Mixtures containing less than 1% did not undergo phase separation until greater than 10% water was present. The phase separation is expected to form a PEG 300-rich phase and a PEG400-TMC/Gly(90/10)900 -rich phase, the latter containing the highest proportion of water. Paclitaxel solubility in each phase was measured. Solubility in the -TMC/Gly(90/10)900 water phase was estimated by determination of the PEG400-TMC/Gly(90/10)900 /water partition coefficient for paclitaxel, which is 2000, giving an estimated solubility of 2 mg/ml (based on an aqueous solubility of paclitaxel of 1 µg/ml). Solubility in the PEG 300-rich phase was estimated from co-solvent studies of water/PEG 300 mixtures. The solubility of paclitaxel in PEG400-TMC/Gly(90/10)900 alone (not in contact with water) was estimated by visual saturation of the polymer with the drug as 250 mg/ml.

### EXAMPLE 23

### PREPARATION OF A PACLITAXEL TRIBLOCK GEL INJECTION FORMULATION

A polymer blend was prepared by dispensing 3 g of PEG400-(90/10 mol% trimethylene carbonate/glycolide)900 and 117 g of PEG300 into a beaker. The components were stirred for at least 2 hours. In a separate beaker, 15 mg of paclitaxel was dispensed and 100 ml of the blended components were added to the paclitaxel and stirred for at least 2 hours. The paclitaxel solution was then withdrawn into a large syringe. A 0.2 µm cellulose acetate syringe filter and a sterile Luer-lok union was attached to the syringe and then 3 ml syringes were filled with 1.2 ml of paclitaxel loaded triblock copolymer gel solution.

### EXAMPLE 24

### BIODISTRIBUTION OF PACLITAXEL ADMINISTERED BY INTRA-ARTICULAR INJECTION IN A COPOLYMER/PEG FORMULATION.

Animals were treated in the same way as Example 12. Administration of formulations, harvesting and tissue analysis were completed as in Example 12 except the formulations were different and the data were used to calculate median tissue levels at each time point. Two formulations were tested to evaluate a faster drug releasing formulation and a slower drug releasing formulation. For both formulations, the dose administered was the MTD, as determined at seven days according to the method of Example 13. The formulations are described by Table 5.

**TABLE 5.**

| FORMULATIONS TESTED FOR LOCAL TISSUE DISTRIBUTION OVER TIME. | | |
|---|---|---|
| Paclitaxel concentration (mg/ml) | Amount of PEG400-TMC/Gly(90/10)900 copolymer (%w/w) | Drug releasing characteristics |
| 0.15 | 2.5 | Faster releasing |
| 0.075 | 30 | Slower releasing |

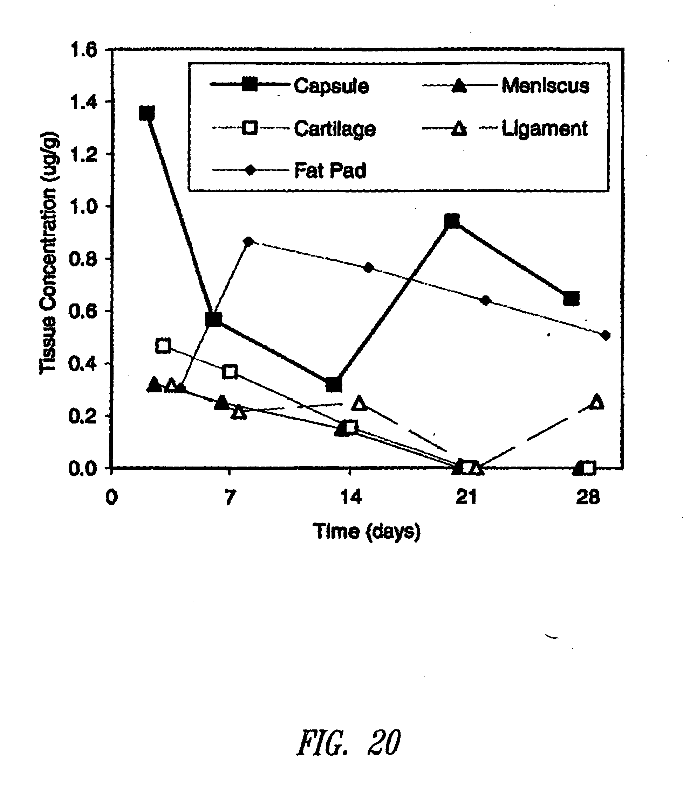

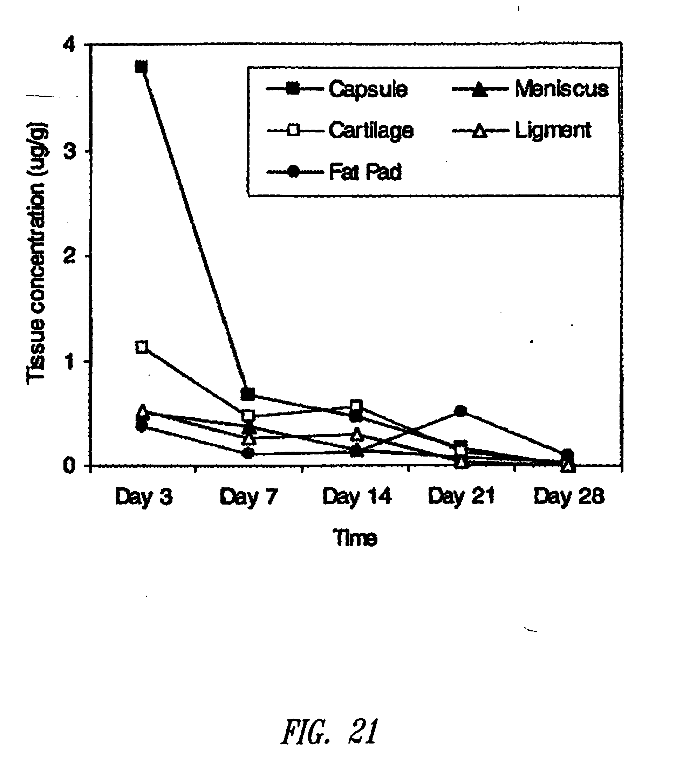

The median kinetic profiles obtained demonstrate that the slow releasing formulation results in drug retention in 3 of 5 tissues evaluated after 28 days. In comparison, the fast releasing formulation showed much lower levels after 28 days (FIGS. 20 and 21).

## Claims

1. A composition comprising:
a) a block copolymer comprising at least block A and at least block B, wherein
(i) block B is more hydrophilic than block A,
(ii) the block copolymer has a molecular weight of between about 500 g/mol and about 2000 g/mol,
(iii) the copolymer is non-thermoreversible and is a liquid at room temperature; and
b) a therapeutic agent effective in treating contracture.

2. The composition of claim 1 wherein the copolymer is a triblock copolymer.

3. The composition of claim 2 wherein the triblock copolymer comprises a carbonate monomer.

4. The composition of claim 2 wherein the triblock copolymer has an average molecular weight of between about 600 and about 1500 g/mol.

5. The composition of claim 2 wherein the triblock copolymer has a weight percent water soluble fraction of less than about 25%, about 50%, or about 75%.

6. The composition of claim 2 wherein the triblock copolymer dissolves in a solvent having a δh Hansen solubility parameter value of no less than 22, 32, or 42.

7. A composition comprising:
(a) an ABA triblock copolymer, wherein the B block comprises a polyalkylene oxide having a molecular weight of between about 200 g/mol to about 600 g/mol, and the A blocks comprise a polymer having about a 90:10 mole ratio of trimethylene carbonate (TMC) and glycolide (Gly) residues and have a total molecular weight of about 900 g/mol, and
(b) a therapeutic agent effective in treating contracture.

8. The composition of claim 7 wherein the polyalkylene oxide is PEG.

9. The composition of claim 8 wherein the PEG has a molecular weight of about 400 g/mol.

10. The composition of any one of claims 1 to 9 further comprising a diluent.

11. The composition of any one of claims 1 to 9 further comprising a liquid diluent selected from the group consisting of a PEG, PEG derivatives, polypropylene glycol, and polypropylene glycol derivatives.

12. The composition of any one of claims 1 to 9 further comprising a liquid diluent, wherein the diluent is PEG or a derivative thereof and has a molecular weight of between about 100 g/mol and about 500 g/mol.

13. The composition of claim 12 wherein the PEG or a derivative thereof has a molecular weight of about 300 g/mol.

14. The composition of claim 2 or claim 7 wherein the triblock copolymer is in a solvent at a concentration of between about 2.5% to about 33%.

15. The composition of any one of claims 1 to 14, wherein the therapeutic agent is paclitaxel, and wherein the composition comprises between about 0.01 to about 0.20 mg/ml of paclitaxel.

16. The composition of any one of claims 1 to 14 wherein the composition comprises between about 0.015 mg/ml to about 0.15 mg/ml of paclitaxel.

17. The composition of any one of claims 1 to 14 wherein the therapeutic agent is a cell cycle inhibitor selected from an anti-microtubule agent, an antimetabolite, an alkylating agent, and a vinca alkaloid.

18. The composition of any one of claims 1 to 14 wherein the therapeutic agent is docetaxel, 5-fluorouracil, halofuginone bromide, mycophenolic acid, mithramycin, puromycin, nogalamycin, 17-DMAG, nystatin, rapamycin, mitoxantrone, duanorubicin, gemcitabine, camptothecin, epothilone B, simvastatin, anisomycin, mitomycin C, epirubicin hydrochloride, topotecan, fascaplysin, podophyllotoxin, chromomycin A3, vinblastine, vincristine, or an analogue or derivative thereof.

19. The composition of any one of claims 1 to 14 wherein the therapeutic agent is a taxane, angiogenesis inhibitor, vinca alkaloid, anthracycline, doxorubicin, purine analogue, tubercidin, agent that inhibits dihydrofolate reduction, RNA synthesis inhibitor, DNA synthesis inhibitor, heat shock protein 90 antagonist, geldanamycin, sirolimus, everolimus, biolimus, tresperimus, chemokine receptor antagonist, cyclin dependent protein kinase inhibitor, epidermal growth factor kinase inhibitor, farnesyltransferaseinhibitor, hydroxymethylglutaryl coenzyme A reductase (HMGCoA reductase) inhibitor, IkappaB kinase 2 (IKK2) inhibitor, IL-1 antagonist, interleukin-1-beta-converting enzyme (ICE) antagonist, IL-1 R-associated kinase (IRAK) antagonist, pimecrolimus, ABT-578, mycophenolic acid, 1-alpha-25-dihydroxy vitamin D3, NF kappa B inhibitor, phosphodiesterase inhibitor, tumor necrosis factor alpha (TNFalpha) antagonist, TNF-alpha converting enzyme (TACE) inhibitor, fibroblast growth factor inhibitor, protein kinase inhibitor, platelet derived growth factor (PDGF) receptor kinase inhibitor, antimycotic agent, sulconizole, macrolide antibiotic, GPIIb/IIIa receptor antagonist, peroxisome proliferator-activated receptor agonist, osteoclast inhibitor, bisphosphonate, collagen antagonist, or epothilone B.

20. The composition of any one of claims 1 to 19 further comprising a second therapeutic agent selected from the group consisting of an anti-infective, an anaesthetic, an analgesic, an antibiotic, a narcotic, an anti-inflammatory agent and combinations thereof.

21. The composition of any one of claims 1-20 for treating contracture.

22. The composition according to claim 21 wherein the contracture affects a joint or affects soft tissue.

23. The composition according to claim 22, wherein the contracture affects soft tissue and wherein the soft tissue is selected from muscles, tendons, ligaments, fat, synovium, capsule, fascia, connective tissue, and combinations thereof.

24. The composition according to claim 21 wherein the contracture is a Dupuytren's contracture, a Peyronie's contracture, a Ledderhose contracture, or a Volkmann's contracture.

25. The composition according to claim 21 wherein the contracture is due to inflammation, degeneration, injury, infection, hypertrophy, a neurological condition, a metabolic condition, infection, ischemia, a genetic condition, an idiopathic condition, or a combination thereof.

26. The composition according to claim 25, wherein the injury is a fracture, a subluxation, a dislocation, a joint disruption, or due to a surgical procedure.

## Patentansprüche

1. Zusammensetzung, umfassend:
a) ein Blockcopolymer, das zumindest Block A und zumindest Block B umfasst, wobei
(i) Block B hydrophiler als Block A ist,
(ii) das Blockcopolymer ein Molekulargewicht von ungefähr 500 g/mol bis ungefähr 2000 g/mol hat,
(iii) das Copolymer nicht-thermoreversibel ist und bei Raumtemperatur eine Flüssigkeit ist; und
b) ein therapeutisches Mittel, das bei der Behandlung einer Kontraktur wirksam ist.

2. Zusammensetzung nach Anspruch 1, wobei das Copolymer ein Triblock-Copolymer ist.

3. Zusammensetzung nach Anspruch 2, wobei das Triblock-Copolymer ein Carbonatmonomer umfasst.

4. Zusammensetzung nach Anspruch 2, wobei das Triblock-Copolymer ein durchschnittliches Molekulargewicht von ungefähr 600 bis ungefähr 1500 g/mol hat.

5. Zusammensetzung nach Anspruch 2, wobei das Triblock-Copolymer einen wasserlöslichen Anteil in Gewichtsprozent von weniger als ungefähr 25%, ungefähr 50% oder ungefähr 75% hat.

6. Zusammensetzung nach Anspruch 2, wobei sich das Triblock-Copolymer in einem Lösungsmittel mit einem 8h-Hansenschen-Löslichkeitsparameterwert von mindestens 22, 32 oder 42 auflöst.

7. Zusammensetzung, umfassend:
(a) ein ABA-Triblock-Copolymer, wobei der B-Block ein Polyalkylenoxid mit einem Molekulargewicht von ungefähr 200 g/mol bis ungefähr 600 g/mol umfasst und die A-Blöcke ein Polymer mit einem Molverhältnis von ungefähr 90:10 von Trimethylencarbonat (TMC) und Glycolid (Gly)-Resten umfassen und ein Gesamtmolekulargewicht von ungefähr 900 g/mol haben, und
(b) ein therapeutisches Mittel, das zur Behandlung einer Kontraktur wirksam ist.

8. Zusammensetzung nach Anspruch 7, wobei das Polyalkylenoxid PEG ist.

9. Zusammensetzung nach Anspruch 8, wobei das PEG ein Molekulargewicht von ungefähr 400 g/mol hat.

10. Zusammensetzung nach irgendeinem der Ansprüche 1 bis 9, weiterhin umfassend ein Verdünnungsmittel.

11. Zusammensetzung nach irgendeinem der Ansprüche 1 bis 9, weiterhin umfassend ein flüssiges Verdünnungsmittel, das ausgewählt ist aus der Gruppe, bestehend aus einem PEG, PEG-Derivaten, Polypropylenglykol und Polypropylenglykolderivaten.

12. Zusammensetzung nach irgendeinem der Ansprüche 1 bis 9, weiterhin umfassend ein flüssiges Verdünnungsmittel, wobei das Verdünnungsmittel PEG oder ein Derivat davon ist und ein Molekulargewicht von ungefähr 100 g/mol bis ungefähr 500 g/mol hat.

13. Zusammensetzung nach Anspruch 12, wobei das PEG oder ein Derivat davon ein Molekulargewicht von ungefähr 300 g/mol hat.

14. Zusammensetzung nach Anspruch 2 oder Anspruch 7, wobei das Triblock-Copolymer in einem Lösungsmittel in einer Konzentration von ungefähr 2,5% bis ungefähr 33% vorliegt.

15. Zusammensetzung nach irgendeinem der Ansprüche 1 bis 14, wobei das therapeutische Mittel Paclitaxel ist und wobei die Zusammensetzung ungefähr 0,01 bis ungefähr 0,20 mg/ml Paclitaxel umfasst.

16. Zusammensetzung nach irgendeinem der Ansprüche 1 bis 14, wobei die Zusammensetzung ungefähr 0,015 mg/ml bis ungefähr 0,15 mg/ml Paclitaxel umfasst.

17. Zusammensetzung nach irgendeinem der Ansprüche 1 bis 14, wobei das therapeutische Mittel ein Zellzyklusinhibitor ist, der aus einem Antimikrotubuli-Mittel, einem Antimetabolit, einem Alkylierungsmittel und einem Vinca-Alkaloid ausgewählt ist.

18. Zusammensetzung nach irgendeinem der Ansprüche 1 bis 14, wobei das therapeutische Mittel Docetaxel, 5-Fluorouracil, Halofuginonbromid, Mycophenolsäure, Mithramycin, Puromycin, Nogalmycin, 17-DMAG, Nystatin, Rapamycin, Mitoxantron, Duanorubicin, Gemcitabin, Camptothecin, Epothilon B, Simvastatin, Anisomycin, Mitomycin C, Epirubicinhydrochlorid, Topotecan, Fascaplysin, Podophyllotoxin, Chromomycin A3, Vinblastin, Vincristin oder ein Analogon oder Derivat davon ist.

19. Zusammensetzung nach irgendeinem der Ansprüche 1 bis 14, wobei das therapeutische Mittel ein Taxan, Angiogeneseinhibitor, Vinca-Alkaloid, Anthracyclin, Doxorubicin, Purinanalogon, Tubercidin, ein Mittel, das die Dihydrofolatreduktion hemmt, RNA-Syntheseinhibitor, DNA-Syntheseinhibitor, Hitzeschockprotein-90-Antagonist, Geldanamycin, Sirolimus, Everolimus, Biolimus, Tresperimus, Chemokinrezeptorantagonist, cyclin-abhängiger Proteinkinaseinhibitor, Epidermalwuchsfaktor-Kinaseinhibitor, Farnesyltransferaseinhibitor, Hydroxymethylglutaryl-Coenzym-A-Reduktase (HMGCoA-Reduktase)-Inhibitor, IkappaB-Kinase-2 (IKK2)-Inhibitor, IL-1-Antagonist, Interleukin-1-beta-Umwandlungsenzym (ICE)-Antagonist, IL-1 R-verknüpfter Kinase (IRAK)-Antagonist, Pimecrolimus, ABT-578, Mycophenolsäure, 1-alpha-25-Dihydroxyvitamin D3, NF-Kappa-B-Inhibitor, Phosphodiesteraseinhibitor, Tumornekrosefaktor-Alpha (TNFalpha)-Antagonist, TNF-alpha-Umwandlungsenzym (TACE)-Inhibitor, Fibroblastenwachstumsfaktorinhibitor, Proteinkinaseinhibitor, Blutplättchenwachstumgsfaktor (PDGF)-Rezeptorkinaseinhibitor, ein antimycotisches Mittel, Sulconizol, Macrolid-Antibiotikum, GPIIb/IIIa-Rezeptorantagonist, peroxisomproliferator-aktivierter Rezeptoragonist, Osteoklasteninhibitor, Bisphosphonat, Collagenantagonist oder Epothilon B ist.

20. Zusammensetzung nach irgendeinem der Ansprüche 1 bis 19, weiterhin umfassend ein zweites therapeutisches Mittel, das ausgewählt ist aus der Gruppe, bestehend aus einem Antiinfektikum, einem Anästhetikum, einem Analgetikum, einem Antibiotikum, einem Narkotikum, einem entzündungshemmenden Mittel und Kombinationen davon.

21. Zusammensetzung nach irgendeinem der Ansprüche 1 bis 20 zur Behandlung einer Kontraktur.

22. Zusammensetzung nach Anspruch 21, wobei die Kontraktur ein Gelenk oder Weichteile beeinträchtigt.

23. Zusammensetzung nach Anspruch 22, wobei die Kontraktur Weichteile beeinträchtigt und wobei das Weichteil aus Muskeln, Sehnen, Bändern, Fett, Gelenkinnenhaut, Kapsel, Faszie, Bindegewebe und Kombinationen davon ausgewählt ist.

24. Zusammensetzung nach Anspruch 21, wobei die Kontraktur eine Dupuytrensche Kontraktur, eine Peyroniesche Kontraktur, eine Ledderhosensche Kontraktur oder eine Volkmannsche Kontraktur ist.

25. Zusammensetzung nach Anspruch 21, wobei die Kontraktur durch Entzündung, Degeneration, Verletzung, Infektion, Hypertrophie, eine neurologische Krankheit, eine metabolische Krankheit, Infektion, Ischämie, eine genetische Krankheit, eine idiopathische Krankheit oder eine Kombination davon bedingt ist.

26. Zusammensetzung nach Anspruch 25, wobei die Verletzung eine Fraktur, eine Subluxation, eine Dislokation, ein Gelenkbruch ist oder durch einen chirurgischen Eingriff bedingt ist.

## Revendications

1. Une composition se composant de ce qui suit :
a) un copolymère à blocs se composant d'au moins un bloc A et d'au moins un bloc B, dans lequel
(i) le bloc B est plus hydrophile que le bloc A,
(ii) le copolymère à blocs possède un poids moléculaire situé entre environ 500 g/mol et environ 2 000 g/mol,
(iii) le copolymère est non-thermoréversible et est liquide à la température ambiante ; et
b) un agent thérapeutique de traitement de la contracture.

2. La composition de la revendication 1, dans laquelle le copolymère est un copolymère tribloc.

3. La composition de la revendication 2, dans laquelle le copolymère tribloc comporte un monomère carbonate.

4. La composition de la revendication 2, dans laquelle le copolymère tribloc possède un poids moléculaire moyen situé entre environ 600 et environ 1 500 g/mol.

5. La composition de la revendication 2, dans laquelle le copolymère tribloc possède une fraction soluble dans l'eau en pourcentage de poids de moins d'environ 25 %, d'environ 50 % ou d'environ 75 %.

6. La composition de la revendication 2, dans laquelle le copolymère tribloc se dissout dans un solvant ayant une valeur de paramètre de solubilité de Hansen δh non inférieure à 22, 32 ou 42.

7. Une composition se composant de ce qui suit :
(a) un copolymère tribloc ABA, dans lequel le bloc B comporte un oxyde de polyalkylène ayant un poids moléculaire d'entre environ 200 g/mol et environ 600 g/mol, et le bloc A comporte un copolymère ayant environ un rapport moléculaire 90:10 de résidus de carbonate de triméthylène (TMC) et de glycolide (Gly) et possède un poids moléculaire total d'environ 900 g/mol, et
(b) un agent thérapeutique de traitement de la contracture.

8. La composition de la revendication 7, dans laquelle l'oxyde polyalkylène est du PEG.

9. La composition de la revendication 8, dans laquelle le PEG possède un poids moléculaire d'environ 400 g/mol.

10. La composition de n'importe laquelle des revendications 1 à 9 comprenant également un diluant.

11. La composition de n'importe laquelle des revendications 1 à 9 comprenant également un diluant liquide sélectionné dans le groupe se composant de PEG, de dérivés de PEG, de polypropylène-glycol et de dérivés de polypropylène-glycol.

12. La composition de n'importe laquelle des revendications 1 à 9 comprenant également un diluant liquide, le diluant étant du PEG ou un dérivé de PEG et possédant un poids moléculaire d'entre environ 100 g/mol et environ 500 g/mol.

13. La composition de la revendication 12, dans laquelle le PEG ou son dérivé possède un poids moléculaire d'environ 300 g/mol.

14. La composition de la revendication 2 ou 7, dans laquelle le copolymère tribloc se trouve dans un solvant à une concentration d'entre environ 2,5 % et environ 33 %.

15. La composition de n'importe laquelle des revendications 1 à 14, dans laquelle l'agent thérapeutique est du paclitaxel et dans laquelle la composition comporte entre environ 0,01 et environ 0,20 mg/ml de paclitaxel.

16. La composition de n'importe laquelle des revendications 1 à 14, dans laquelle la composition comporte entre environ 0,015 mg/ml et environ 0,15 mg/ml de paclitaxel.

17. La composition de n'importe laquelle des revendications 1 à 14, dans laquelle l'agent thérapeutique est un inhibiteur du cycle cellulaire sélectionné parmi les suivants : un agent anti-microtubule, un antimétabolite, un agent alcoylant et un alcaloïde de la pervenche.

18. La composition de n'importe laquelle des revendications 1 à 14, dans laquelle l'agent thérapeutique est une des substances suivantes : docétaxel, 5-fluoro-uracile, bromure de halofuginone, acide mycophénolique, mithramycine, puromycine, nogalamycine, 17-DMAG, nystatine, rapamycine, mitoxantrone, duanorubicine, gemcitabine, camptothecine, épothilone B, simvastatine, anisomycine, mitomycine C, hydrochlorure d'épirubicine, topotécan, fascaplysine, podophyllotoxine, chromomycine A3, vinblastine, vincristine ou un analogue ou dérivé de ces substances.

19. La composition de n'importe laquelle des revendications 1 à 14, dans laquelle l'agent thérapeutique est une des substances suivantes : taxane, inhibiteur de l'angiogenèse, alcaloïde de la pervenche, anthracycline, doxorubicine, analogue de la purine, tubercidine, inhibiteur de la réduction de dihydrofolate, inhibiteur de la synthèse d'ARN, inhibiteur de la synthèse d'ADN, antagoniste 90 de la protéine de choc thermique, geldanamycine, sirolimus, évérolimus, biolimus, tresperimus, antagoniste de récepteur de chemokine, inhibiteur de la protéine-kinase cyclinodépendante, inhibiteur de la kinase du facteur de croissance épidermique, inhibiteur de la farnésyl-transférase, inhibiteur de l'hydroxyméthylglutaryl coenzyme A réductase (HMGCoA réductase), inhibiteur de la kinase 2 (IKK2) IkappaB, antagoniste IL-1, antagoniste de l'enzyme de conversion de l'interleukine-1-beta (ICE), antagoniste de la kinase IL-1 R-associée (IRAK), pimécrolimus, ABT-578, acide mycophénolique, vitamine D3 1-alpha-25-dihydroxy, inhibiteur du NF kappa B, inhibiteur de la phosphodiestérase, inhibiteur du facteur de nécrose tumorale alpha (TNFalpha), inhibiteur de l'enzyme de conversion de TNF-alpha (TACE), inhibiteur du facteur de croissance des fibroblastes, inhibiteur de la kinase de protéine, inhibiteur de la kinase du récepteur du facteur de croissance dérivé de plaquettes (PDGF), agent antimycotique, sulconizole, antibiotique macrolide, antagoniste du récepteur GPIIb/IIIa, agoniste du récepteur activé par le proliférateur de peroxisome, inhibiteur de l'ostéoclaste, bisphosphonate, antagoniste du collagène ou épothilone B.

20. La composition de n'importe laquelle des revendications 1 à 19 comportant également un deuxième agent thérapeutique sélectionné dans le groupe se composant d'un anti-infectieux, un anesthésique, un analgésique, un antibiotique, un narcotique, un agent anti-inflammatoire et des combinaisons de ces substances.

21. La composition de n'importe laquelle des revendications 1 à 20 pour traiter la contracture.

22. La composition de la revendication 21, dans laquelle la contracture affecte une articulation ou des tissus mous.

23. La composition de la revendication 22, dans laquelle la contracture affecte les tissus mous et dans laquelle les tissus mous sont sélectionnés parmi les suivants : muscles, tendons, ligaments, graisse, synoviale, capsule, fascia, tissu conjonctif et des combinaisons de ces tissus.

24. La composition de la revendication 21, dans laquelle la contracture est une contracture de Dupuytren, une contracture de Peyronie, une contracture de Ledderhose ou une contracture de Volkmann.

25. La composition de la revendication 21, dans laquelle la contracture est due à une inflammation, une dégénérescence, une blessure, une infection, une hypertrophie, une affection neurologique, un trouble du métabolisme, une infection, une ischémie, une maladie génétique, un trouble idiopathique ou une combinaison de ces facteurs.

26. La composition de la revendication 25, dans laquelle la blessure est une fracture, une subluxation, une dislocation, une perturbation d'une articulation, ou bien est due à une intervention chirurgicale.
